# EUROPEAN PATENT SPECIFICATION

(11) **EP 3 558 235 B1**
(45) Date of publication and mention of the grant of the patent: **08.09.2021**
(21) Application number: 17821917.6
(22) Date of filing: 21.12.2017
(51) Int. Cl.: A61K 8/35, A61K 8/49, A61Q 5/06, A61K 8/46

(54) **USE OF ANTHRAQUINONE DYES AND OF FLUORESCENT DYES FOR DYEING KERATIN FIBRES, DYEING PROCESS AND COMPOSITION**
VERWENDUNG VON ANTHRACHINONFARBSTOFFEN UND FLUORESZIERENDEN FARBSTOFFEN ZUM FÄRBEN VON KERATINFASERN, FÄRBEVERFAHREN UND ZUSAMMENSETZUNG
UTILISATION DE COLORANTS ANTHRAQUINONIQUES ET DE COLORANTS FLUORESCENTS DESTINÉS À LA COLORATION DE FIBRES KÉRATINIQUES, PROCÉDÉ ET COMPOSITION DE COLORATION

(30) Priority: 22.12.2016 FR 1663123
(43) Date of publication of application: 30.10.2019
(73) Proprietor: L'Oréal, 75008 Paris (FR)
(72) Inventor: EUDELINE -LY, Annabelle, 93601 Aulnay-sous-Bois (FR); DAVID, Hervé, 93601 Aulnay-sous-Bois (FR); BLAISE, Christian, 93601 Aulnay-sous-Bois (FR); MESSAVUSSU, Abel, 93601 Aulnay-sous-Bois (FR)
(74) Representative: L'Oreal
(86) International application number: PCT/EP2017/084192
(87) International publication number: WO 2018/115337

(56) References cited:
- EP-A1- 0 226 860
- EP-A1- 0 852 943
- WO-A2-2013/174987

## Description

The present invention relates to a process for dyeing keratin fibres, in particular human keratin fibres such as the hair, using a) one or more particular anthraquinone dyes and b) one or more fluorescent direct dyes; to a composition comprising a) and b). The present invention also relates to a multi-compartment device containing the compounds defined above.

Many people have sought for a long time to modify the colour of their hair and in particular to mask their grey hair.

It is especially known practice to dye keratin fibres, in particular human keratin fibres, with dye compositions containing oxidation dye precursors, which are generally known as oxidation bases. These oxidation bases are colourless or weakly coloured compounds, which, when combined with oxidizing products, may give rise to coloured compounds via a process of oxidative condensation.

The shades obtained with these oxidation bases may be modified by combining them with couplers or colour modifiers. The variety of molecules used as oxidation bases and couplers allows a wide range of colours to be obtained.

Another well-known method consists in obtaining semi-permanent dyeing by applying to the keratin fibres direct dyes, which are coloured and colouring molecules that have affinity for said fibres.

The direct dyes conventionally used are chosen from nitrobenzene, anthraquinone, nitropyridine, azo, xanthene, acridine, azine and triarylmethane direct dyes. The chemical species may be nonionic, anionic (acidic dyes) or cationic (basic dyes). The direct dyes may also be natural dyes.

Conventional direct dyeing processes consist in applying to keratin fibres dye compositions comprising direct dyes. After application, a leave-on time is observed so as to allow the dye molecules to penetrate by diffusion into the fibres. On conclusion of the process, the fibres are rinsed.

In contrast with oxidation dyeing, these direct dyeing processes have a tendency to better protect the integrity of the fibres. The resulting colourings are generally chromatic, but, however, are only semi-temporary. The nature of the interactions that bind the direct dyes to the keratin fibres and their desorption from the surface and/or the core of the fibre are responsible for their weak dyeing power.

Although a wide range of colours is currently accessible, it generally proves necessary to combine three dyes of complementary colours - trichromatic principle - in order to obtain a natural shade (see, for example, WO 95/15144 and WO 95/01772). This tripartite combination does not, however, show good persistence with respect to repeated shampooing. It generally, or even systematically, induces an unaesthetic changing of the colour, which the consumer finds dissuasive.

Another aim of the invention is thus to be able to dye light keratin fibres efficiently in chestnut-brown, dark chestnut-brown with a glint or even black, by mixing direct dyes, and preferably only two types of direct dye.

These colourings are furthermore not sufficiently fast in the face of external agents such as light or perspiration.

Thus, there is a real need to implement processes for the direct dyeing of keratin fibres, in particular of human keratin fibres such as the hair, which do not have the drawbacks mentioned above, i.e. which make it possible especially to lead to natural colourings that have good properties, especially in terms of chromaticity, power, intensity, sheen and selectivity, and which are persistent with respect to shampooing.

The Applicant has found, surprisingly, that a process for dyeing keratin fibres using a) one or more anthraquinone dyes chosen from those of formulae **(I)** and **(II)** and b) one or more fluorescent direct dyes makes it possible to achieve the objectives mentioned above; especially to lead to natural chestnut-brown, dark chestnut-brown with a glint or even black colourings, which are not only powerful and shiny, but also shampoo-resistant.

One subject of the present invention is especially a process for dyeing keratin fibres, in particular human keratin fibres such as the hair, comprising the application to said keratin fibres of ingredients a) and b) below:
(a) one or more anthraquinone dyes chosen from the compounds of formulae **(I)** and/or **(II)** below, the optical isomers thereof, the geometrical isomers thereof, the tautomers thereof, the solvates thereof, and mixtures thereof: 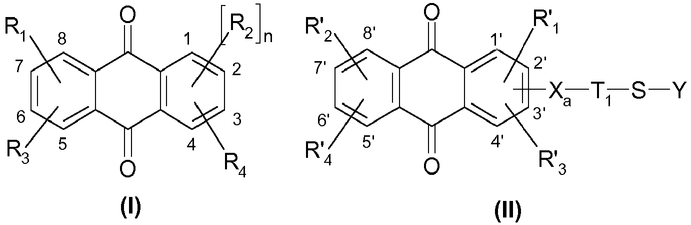 in which formula **(I)** or **(II):**
   ▪ **Xₐ**, which may be identical or different, represents a bond, a heteroatom or a group chosen from an oxygen or sulfur atom, N(Rₐ), CO, SO, SO₂, or combinations thereof such as -O-CO-, -CO-O-, -NH-CO- or -CO-NH-, with Rₐ representing a hydrogen atom or a (C₁-C₆)alkyl group optionally substituted with one or more hydroxyl groups; preferably, Xₐ represent N(Rₐ) and particularly NH;
   ▪ **Y** represents: i) a hydrogen atom; ii) an alkali metal; iii) an alkaline-earth metal; iv) an ammonium group: N⁺R^{a}R^{b}R^{g}R^{d} or a phosphonium group: P⁺R^{a}R^{b}R^{g}R^{d} with R^{a}, R^{b}, R^{g} and R^{d}, which may be identical or different, representing a (C₁-C₄)alkyl group; or v) a thiol-function protecting group; or vi) the group **(III)** below: 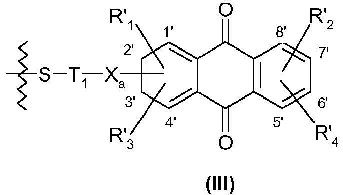
   ▪ **R₁, R₂, R₃, R₄, R'₁, R'₂, R'₃** and **R'₄**, which may be identical or different, represent an atom or a group chosen from:
      ∘ hydrogen;
      ∘ halogen such as bromine and chlorine,
      ∘ hydroxyl,
      ∘ C₁-C₄ alkoxy,
      ∘ hydroxysulfonyl (-SO₃H) or sulfonate (-SO₃⁻, M⁺), with M⁺ representing a cationic counterion, in particular an alkali metal, alkaline-earth metal or ammonium, such as Na⁺ or K⁺;
      ∘ optionally substituted C₁-C₆ alkyl,
      ∘ -NR₅R₆ in which **R₅** and **R₆**, which may be identical or different, represent an atom or radical chosen from: i) hydrogen, ii) (C₁-C₄)alkylcarbonyl such as methylcarbonyl (-COCH₃), iii) arylsulfonyl such as phenylsulfonyl (-SO₂Ph), iv) Het-ALK-C(O)- with Het representing a heterocycloalkyl group which is optionally substituted, especially with one or more (C₁-C₄)alkyl groups and ALK represents a (C₁-C₆)alkylene group optionally substituted with one or more hydroxyl or (di)(hydroxy)(C₁-C₄)(alkyl)amino groups; Het-ALK-C(O)- is such as (piperidin-1-yl)acetyl, 2-methyl-2-(piperidin-1-yl)propanoyl, 2-(morpholin-4-yl)ethyl-β-alaninoyl, v) optionally substituted aryl, in particular phenyl optionally substituted with at least one radical chosen from a) C₁-C₆ alkyl, b) hydroxyl, c) hydroxysulfonyl, d) C₁-C₄ alkoxy, e) carboxyl (-COOH), f) (C₁-C₄)alkoxycarbonyl, g) amino, h) (di)(C₁-C₄)alkylamino, one of the alkyl radicals possibly being substituted with a hydroxyl or hydroxysulfonyl radical -SO₃H, or -OSO₃H, vi) optionally substituted aryl(C₁-C₄)alkyl, in particular benzyl optionally substituted with a (di)(C₁-C₄)(alkyl)amino group, viii) optionally substituted C₁-C₂₀ alkyl, optionally interrupted with one or more heteroatoms and/or with one or more groups comprising at least one heteroatom, preferably chosen from oxygen, nitrogen and sulfur, CO, SO, SO₂ or combinations thereof. when said alkyl radical is substituted, it is substituted with one or more atoms or groups chosen from: a) halogens, preferably one or more chlorine atoms, b) hydroxyl, c) (C₁-C₆)alkylcarbonylamino, in particular acylamino, d) 5- or 6-membered heterocycloalkyl such as tetrahydro-2H-pyran-4-amine, morpholino, piperidino or piperazino, e) (di)(C₁-C₄)(alkyl)amino, f) hydroxysulfonyl(C₁-C₄)alkylamino, or hydroxysulfonyloxy(C₁-C₄)alkylamino, g) (di)(hydroxy)(C₁-C₄)(alkyl)amino, h) 5- or 6-membered heteroaryl such as imidazole, and i) formylamino (-NHCOH);
      ∘ a group of formula (a) below: -N(R₇)-X₁-W₁ (a) in which formula (a):
         **R₇** represents a hydrogen or a C₁-C₄ alkyl radical,
         **X₁** represents a divalent radical chosen from C₁-C₂₀ alkylene optionally interrupted with one or more heteroatoms or groups chosen from oxygen, nitrogen and sulfur, CO, SO, SO₂, arylene such as phenylene, or combinations thereof; preferably, X₁ represents:
            - (C₁-C₁₀)alkylene,
            - -(C₁-C₁₀)alkylcarbonyl-,
            - -carbonyl(C₁-C₁₀)alkyl-,
            - -(C₁-C₁₀)alkylaminocarbonyl(C₁-C₁₀)alkyl-,
            - -(C₁-C₁₀)alkylcarbonylamino(C₁-C₁₀)alkyl-,
            - -phenyl(C₁-C₁₀)alkyl-,
            - -(C₁-C₁₀)alkylphenyl-, or
            - phenylene,
         **W₁** represents a cationic radical chosen from: 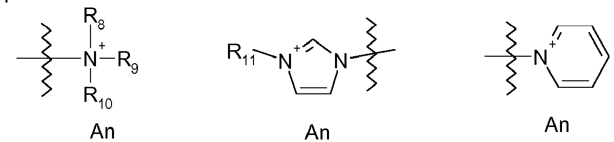 with **R₈, R₉, R₁₀** and **R₁₁,** which may be identical or different, representing a C₁-C₆ alkyl group, a benzyl radical, a C₁-C₆ alkyl sulfonate radical; the radicals **R₈** and **R₉** may form, with the nitrogen atom to which they are attached, a saturated or unsaturated, optionally substituted 5- to 7-membered heterocycle, optionally comprising another non-nitrogen heteroatom, preferably an oxygen atom;
   ▪ **n** is an integer ranging from 1 to 3; preferably, n is equal to 1 or 2, **T₁** represents a linear or branched divalent hydrocarbon-based chain comprising from 1 to 20 carbon atoms, optionally interrupted with one or more heteroatoms or groups, or combinations thereof, chosen from oxygen, sulfur, N(R_{b}), C(O), -N⁺(R₈)(R₉)- An, optionally cationic and optionally substituted heteroaryl, such as imidazolium, An with **R₈** and **R₉,** which may be identical or different, represent a C₁-C₆ alkyl radical; **R_{b}** representing a hydrogen atom or a (hydroxy)(C₁-C₄)alkyl group; preferably, said hydrocarbon-based chain is interrupted with one or more groups chosen from N(R_{b}), C(O), and a combination thereof such as -C(O)- N(R_{b})- or -N(R_{b})-C(O)-, and - N⁺(R₈)(R₉)- An, **An** is an organic or inorganic anionic counterion, which ensures the electrical neutrality of the dyes of formulae **(I)** and **(II)**;
   ▪ 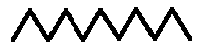 being the part of the bond that is connected to the rest of the molecule; and
(b) one or more cationic (poly)methine fluorescent dyes; it being understood that:
   - the anthraquinone dye(s) of formula (I) comprise at least one radical **R₁, R₃** or **R₄,** other than a hydrogen atom, and the anthraquinone dye(s) of formula (II) comprise at least one radical **R'₁**, **R'₂**, **R'₃** or **R'₄** other than a hydrogen atom;
   - (a) the anthraquinone dye(s) of formula (I) or (II) and (b) the fluorescent dye(s) are applied to said keratin fibres together or sequentially; and
   - when the compounds of formula (I) or (II) are cationic and comprise a sulfonate group, then M⁺ and An may be absent to ensure the electrical neutrality of said molecule. Another subject of the invention is a cosmetic composition comprising:
      (a) one or more anthraquinone dyes chosen from the compounds of formulae **(I)** and **(II)** as defined previously; and
      (b) one or more cationic (poly)methine fluorescent dyes.

The combination of anthraquinone dye(s) of formula **(I)** or **(II)** and of cationic (poly)methine fluorescent dye(s) makes it possible especially to obtain natural colourings that have good dyeing properties, especially in terms of chromaticity, power, intensity, sheen and selectivity.

Furthermore, the process and the composition according to the invention make it possible to dye light keratin materials efficiently in chestnut-brown, dark chestnut-brown with a glint or even black, by mixing direct dyes, and in particular only the ingredients a) and b) as defined previously as dyes, without the need to use additional (or complementary) dye(s) other than a) and b).

Moreover, the colourings obtained by means of the process and the composition according to the invention show good resistance to the various attacking factors to which the hair may be subjected, such as light, bad weather, washing and perspiration. They are in particular persistent with respect to shampooing, especially after at least three shampoo washes.

A subject of the present invention is also a multi-compartment device comprising a first compartment containing a composition comprising (a) one or more dyes of formula **(I)** or **(II)** as defined previously, and a second compartment containing a composition comprising (b) one or more cationic (poly)methine fluorescent dyes as defined previously.

Another subject of the invention is the use of a) cationic (poly)methine fluorescent dye(s) as defined previously, combined with b) one or more dyes of formula **(I)** or **(II)** as defined previously, for dyeing light keratin fibres, especially human keratin fibres such as the hair, in chestnut-brown, dark chestnut-brown or even black, without the need to use additional dye(s) other than a) or b).

Other subjects, characteristics, aspects and advantages of the invention will emerge even more clearly on reading the description and the examples that follow.

For the purposes of the present invention and unless otherwise indicated:
▪ the "*dyes*" according to the invention absorb light in the visible range, i.e. at a wavelength λ_{abs} particularly between 400 and 700 nm inclusive;
▪ a "*thiol-protecting group*" is a group known to those skilled in the art, for instance those described in the publications "Protective Groups in Organic Synthesis", T.W. Greene, John Wiley & Sons ed., NY, 1981, pp. 193-217; "Protecting Groups", P. Kocienski, Thieme, 3rd ed., 2005, chap. 5; and Ullmann's Encyclopedia, "Peptide Synthesis", pp. 4-5, 2005 Wiley-VCH Verlag GmbH & Co. KGaA, Weinheim 10.1002/14356007.a19 157; preferably, the protected thiol function is such that Y represents a hydrogen atom; ii) an alkali metal; iii) an alkaline-earth metal; iv) an ammonium group: N⁺R^{a}R^{b}R^{g}R^{d} or a phosphonium group: P⁺R^{a}R^{b}R^{g}R^{d} with R^{a}, R^{b}, R^{g} and R^{d}, which may be identical or different, representing a hydrogen atom or a (C₁-C₄)alkyl group;
▪ For the purposes of the present invention, the term "*direct dye*" means natural and/or synthetic dyes, which are soluble in the cosmetic medium, other than oxidation dyes; they are dyes which will diffuse superficially on the keratin fibres;
▪ For the purposes of the present invention, the term "*fluorescent dye*" means a dye which is a coloured molecule (which absorbs visible light) and which itself imparts colour, and which, in contrast with a conventional dye, transforms the absorbed energy into light of a longer wavelength emitted in the visible part of the spectrum; in particular, the "*fluorescent*" dyes of the invention are capable of absorbing light in the visible range at a wavelength λ_{abs} of between 350 and 800 nm and of re-emitting in the visible range at a longer wavelength λₑₘ than that absorbed, of between 400 and 800 nm; the difference between the absorption and emission wavelengths, also known as the Stoke's shift, is between 1 nm and 100 nm. More preferentially, the fluorescent dyes of the invention are dyes that are capable of absorbing at a wavelength λ_{abs} of between 420 nm and 550 nm and of re-emitting in the visible range at a wavelength λₑₘ of between 470 and 600 nm;
▪ the "*fluorescent dyes*" according to the present invention are to be differentiated from optical brighteners. Optical brighteners, also known as "*brighteners*" or "*fluorescent brighteners*" or "*fluorescent brightening agents*" or "*fluorescent whitening agents or FWA*" or "*whiteners*" or "*fluorescent whiteners*", are colourless compounds, which do not impart a colour and are consequently not dyes since they do not absorb in the visible light range, but only absorb in the ultraviolet range (wavelength ranging from 200 to 400 nm) and transform the absorbed energy into fluorescent light of a longer wavelength emitted in the visible part of the spectrum in the blue range. The colour impression is then generated only by the purely fluorescent light that is predominantly blue (wavelength ranging from 400 to 500 nm).
▪ the "*blue-violet*" dyes according to the invention are dyes which absorb light in the visible spectrum and which appear blue visually, i.e. which absorb light at an absorption wavelength λₘₐₓ greater than 550 nm and less than or equal to 700 nm, in particular λₘₐₓ between 560 nm and 700 nm, preferably in the blue range, i.e. λₘₐₓ between 580 and 650 nm;
▪ the term "*(hetero)aryl*" means aryl and heteroaryl groups;
▪ the "*aryl*" or "*heteroaryl*" radicals or the aryl or heteroaryl part of a radical may be substituted with at least one substituent borne by a carbon atom, chosen from:
   - a C₁-C₆ and preferably C₁-C₄ alkyl radical optionally substituted with one or more radicals chosen from hydroxyl, C₁-C₂ alkoxy, C₂-C₄ (poly)hydroxyalkoxy, acylamino, amino substituted with two C₁-C₄ alkyl radicals, which may be identical or different, optionally bearing at least one hydroxyl group, or the two radicals possibly forming, with the nitrogen atom to which they are attached, a saturated or unsaturated, optionally substituted 5- to 7-membered and preferably 5- or 6-membered heterocycle optionally comprising another nitrogen or non-nitrogen heteroatom;
   - a halogen atom;
   - a hydroxyl or thiol group;
   - a C₁-C₆ alkoxy or C₁-C₆ alkylthio radical;
   - a (poly)hydroxy(C₂-C₆)alkoxy radical;
   - an amino radical;
   - a 5- or 6-membered heterocycloalkyl radical, preferentially morpholino, piperazino, piperidino or pyrolidino, which is optionally substituted with a (C₁-C₄) alkyl radical, preferentially methyl;
   - a 5- or 6-membered heteroaryl radical, preferentially imidazolyl, optionally substituted with a (C₁-C₄)alkyl radical, preferentially methyl;
   - an amino radical substituted with one or two identical or different C₁-C₆ alkyl radicals, optionally bearing at least:
      i) a hydroxyl group,
      ii) an amino group optionally substituted with one or two optionally substituted C₁-C₃ alkyl radicals, said alkyl radicals possibly forming with the nitrogen atom to which they are attached a saturated or unsaturated, optionally substituted 5- to 7-membered heterocycle, optionally comprising at least one other nitrogen or non-nitrogen heteroatom,
      iii) a quaternary ammonium group -N⁺R'R"R''', An⁻ for which R', R" and R''', which may be identical or different, represent a C₁-C₄ alkyl group and An⁻ represents an anionic counterion,
      iv) or an optionally cationic 5- or 6-membered heteroaryl radical, preferentially imidazolium, optionally substituted with a (C₁-C₄)alkyl radical, preferentially methyl;
   - an acylamino radical (-N(R)-C(O)-R') in which the R radical is a hydrogen atom or a C₁-C₄ alkyl radical optionally bearing at least one hydroxyl group and the R' radical is a C₁-C₂ alkyl radical;
   - a carbamoyl radical ((R)₂N-C(O)-) in which the radicals R, which may be identical or different, represent a hydrogen atom or a C₁-C₄ alkyl radical optionally bearing at least one hydroxyl group;
   - an alkylsulfonylamino radical (R'-S(O)₂-N(R)-) in which the radical R represents a hydrogen atom or a C₁-C₄ alkyl radical optionally bearing at least one hydroxyl group and the radical R' represents a C₁-C₄ alkyl radical, or a phenyl radical;
   - an aminosulfonyl radical ((R)₂N-S(O)₂-) in which the radicals R, which may be identical or different, represent a hydrogen atom or a C₁-C₄ alkyl radical optionally bearing at least one hydroxyl group;
   - a carboxylic radical in acid or salified (preferably with an alkali metal or a substituted or unsubstituted ammonium) form;
   - a cyano group;
   - a nitro or nitroso group;
   - a polyhaloalkyl group, preferably trifluoromethyl;
   the cyclic or heterocyclic part of a non-aromatic radical may be substituted with at least one substituent chosen from the following groups:
   - hydroxyl;
   - C₁-C₄ alkoxy, C₂-C₄ (poly)hydroxyalkoxy;
   - C₁-C₄ alkyl;
   - alkylcarbonylamino (R-C(O)-N(R')-) in which the radical R' is a hydrogen atom or a C₁-C₄ alkyl radical optionally bearing at least one hydroxyl group, and the radical R is a C₁-C₂ alkyl radical or an amino radical optionally substituted with one or two C₁-C₄ alkyl groups, which may be identical or different, themselves optionally bearing at least one hydroxyl group, said alkyl radicals possibly forming, with the nitrogen atom to which they are attached, a saturated or unsaturated, optionally substituted 5- to 7-membered heterocycle optionally comprising at least one other nitrogen or non-nitrogen heteroatom;
   - alkylcarbonyloxy (R-C(O)-O-) in which the radical R is a C₁-C₄ alkyl radical or an amino group optionally substituted with one or two identical or different C₁-C₄ alkyl groups themselves optionally bearing at least one hydroxyl group, said alkyl radicals possibly forming with the nitrogen atom to which they are attached a saturated or unsaturated, optionally substituted 5- to 7-membered heterocycle, optionally comprising at least one other nitrogen or non-nitrogen heteroatom;
   - alkoxycarbonyl (R-X-C(O)-) in which the radical R is a C₁-C₄ alkoxy radical, X is an oxygen atom or an amino group optionally substituted with a C₁-C₄ alkyl group itself optionally bearing at least one hydroxyl group, said alkyl radical possibly forming with the nitrogen atom to which it is attached a saturated or unsaturated, optionally substituted 5- to 7-membered heterocycle, optionally comprising at least one other nitrogen or non-nitrogen heteroatom;
▪ a cyclic or heterocyclic radical, or a non-aromatic part of an aryl or heteroaryl radical, may also be substituted with one or more oxo groups;
▪ an "*aryl*" radical represents a monocyclic or fused or non-fused polycyclic carbon-based group, comprising from 6 to 22 carbon atoms, at least one ring of which is aromatic; preferentially, the aryl radical is a phenyl, biphenyl, naphthyl, indenyl, anthracenyl or tetrahydronaphthyl;
▪ a "*cationic heteroaryl radical*" is a heteroaryl group as defined above which comprises an endocyclic or exocyclic cationic group;
   - when the charge is endocyclic, it is included in the electron delocalization via the mesomeric effect; for example, it is a pyridinium, imidazolium or indolinium group: 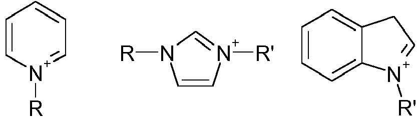 with R and R' being a heteroaryl substituent as defined previously and particularly a (hydroxy)(C₁-C₈)alkyl group, such as methyl;
   - when the charge is exocyclic, it is not included in the electron delocalization via the mesomeric effect; for example, it is an ammonium or phosphonium substituent R⁺, such as trimethylammonium, which is outside the heteroaryl, such as pyridyl, indolyl, imidazolyl or naphthalimidyl, in question: 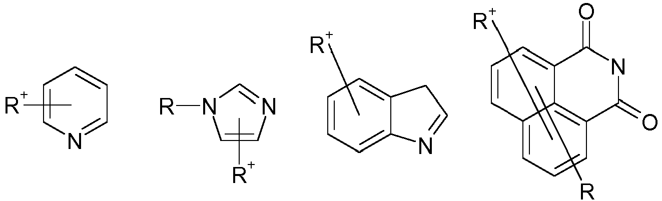
▪ with R being a heteroaryl substituent as defined below and R⁺ an ammonium RₐR_{b}R_{c}N⁺-, phosphonium RₐR_{b}R_{c}P⁺- or ammonium RₐR_{b}R_{c}N⁺-(C₁-C₆)alkylamino, RₐR_{b}R_{c}N⁺-(C₁-C₆)alkyl or RₐR_{b}R_{c}N⁺-(C₁-C₆)alkoxy group with Rₐ, R_{b} and R_{c}, which may be identical or different, being a (C₁-C₈)alkyl group such as methyl;
▪ a "*heteroaryl radical*" represents a 5- to 22-membered, monocyclic or fused or non-fused polycyclic group, comprising from 1 to 6 heteroatoms chosen from nitrogen, oxygen and sulfur, at least one ring of which is aromatic; preferentially, a heteroaryl radical is chosen from acridinyl, benzimidazolyl, benzobistriazolyl, benzopyrazolyl, benzopyridazinyl, benzoquinolyl, benzothiazolyl, benzotriazolyl, benzoxazolyl, pyridinyl, tetrazolyl, dihydrothiazolyl, imidazopyridinyl, imidazolyl, indolyl, isoquinolyl, naphthoimidazolyl, naphthoxazolyl, naphthopyrazolyl, oxadiazolyl, oxazolyl, oxazolopyridyl, phenazinyl, phenoxazolyl, pyrazinyl, pyrazolyl, pyrilyl, pyrazoyltriazyl, pyridyl, pyridinoimidazolyl, pyrrolyl, quinolyl, tetrazolyl, thiadiazolyl, thiazolyl, thiazolopyridinyl, thiazoylimidazolyl, thiopyrylyl, triazolyl, xanthylyl and the ammonium salt thereof;
▪ a *"heterocyclic radical"* is a radical which may contain one or two unsaturations, but is a monocyclic or fused or non-fused polycyclic, 5- to 22-membered non-aromatic radical comprising from 1 to 6 heteroatoms chosen from nitrogen, oxygen and sulfur;
▪ a *"heterocycloalkyl radical"* is a heterocyclic radical comprising at least one saturated ring;
▪ the term "divalent hydrocarbon-based radical" means a linear or branched, saturated or unsaturated chain, which may thus comprise one or more unsaturations, comprising from 1 to 20 carbon atoms, in particular between 2 and 14 carbon atoms, preferably between 2 and 6 carbon atoms;
▪ an *"alkyl"* radical is a linear or branched C₁-C₁₀, in particular C₁-C₈, more particularly C₁-C₆ and preferably C₁-C₄ hydrocarbon-based radical;
▪ the term *"optionally substituted"* applied to the alkyl radical implies that said alkyl radical may be substituted with one or more radicals chosen from the following radicals: i) hydroxyl, ii) C₁-C₄ alkoxy, iii) R-Z-C(X)-Y- with X, Y and Z representing an oxygen or sulfur atom or N(R'), or alternatively X and/or Z represent a bond, R and R', which may be identical or different, represent a hydrogen atom or a (C₁-C₆)alkyl group, preferably, X represents an oxygen atom, iv) amino optionally substituted with one or two identical or different C₁-C₄ alkyl radicals, said alkyl radicals possibly forming, with the nitrogen atom that bears them, a 5- to 7-membered heterocycle, optionally comprising another nitrogen or non-nitrogen heteroatom; v) or a quaternary ammonium group N⁺R'R"R''', An⁻ for which R', R" and R''', which may be identical or different, represent a C₁-C₄ alkyl group, or alternatively -N⁺R'R"R''' forms a heteroaryl such as imidazolium optionally substituted with a C₁-C₄ alkyl group and An⁻ represents the anionic counterion, vi) carboxyl C(O)H, vii) carboxylate C(O)O⁻, M⁺ with M⁺ representing a cationic counterion such as alkali metal or alkaline-earth metal, viii) sulfonic -SO₃H, ix) sulfonate -SO₃⁻, M⁺ with M⁺ as defined previously, and x) cyano;
▪ an "*alkoxy* radical" is an alkyl-oxy radical for which the alkyl radical is a linear or branched C₁-C₈ and preferentially C₁-C₆ hydrocarbon-based radical;
▪ when the alkoxy group is optionally substituted, this implies that the alkyl group is optionally substituted as defined above;
▪ the term "*organic or mineral acid salt*" is more particularly intended to mean salts chosen from a salt derived from i) hydrochloric acid HCI, ii) hydrobromic acid HBr, iii) sulfuric acid H₂SO₄, iv) alkylsulfonic acids: Alk-S(O)₂OH such as methanesulfonic acid and ethanesulfonic acid; v) arylsulfonic acids: Ar-S(O)₂OH such as benzenesulfonic acid and toluenesulfonic acid; vi) citric acid; vii) succinic acid; viii) tartaric acid; ix) lactic acid; x) alkoxysulfinic acids: Alk-O-S(O)-OH such as methoxysulfinic acid and ethoxysulfinic acid; xi) aryloxysulfinic acids such as tolueneoxysulfinic acid and phenoxysulfinic acid; xii) phosphoric acid H₃PO₄; xiii) acetic acid CH₃C(O)-OH; xiv) triflic acid CF₃SO₃H; and xv) tetrafluoroboric acid HBF₄;
▪ the term "*anionic counterion*" means an anion or an anionic group derived from an organic or mineral acid which counterbalances the cationic charge of the dye; more particularly, the anionic counterion is chosen from: i) halides such as chloride or bromide; ii) nitrates; iii) sulfonates, including C₁-C₆ alkylsulfonates: Alk-S(O)₂O⁻ such as methylsulfonate or mesylate and ethylsulfonate; iv) arylsulfonates: Ar-S(O)₂O⁻ such as benzenesulfonate and toluenesulfonate or tosylate; v) carboxylates Alk-C(O)-OH with Alk representing a (C₁-C₆)alkyl group optionally substituted with one or more hydroxyl or carboxylate groups such as citrate; vi) succinate; vii) tartrate; viii) lactate; ix) alkyl sulfates: Alk-O-S(O)O⁻ such as methyl sulfate and ethyl sulfate; x) aryl sulfates: Ar-O-S(O)O⁻ such as benzene sulfate and toluene sulfate; xi) alkoxy sulfates: Alk-O-S(O)₂O⁻ such as methoxy sulfate and ethoxy sulfate; xii) aryloxy sulfates: Ar-O-S(O)₂O⁻, xiii) phosphates O=P(OH)₂-O⁻, O=P(O⁻)₂-OH O=P(O⁻)₃, HO-[P(O)(O⁻)]_{w}-P(O)(O⁻)₂ with w being an integer; xiv) acetate; xv) triflate; and xvi) borates such as tetrafluoroborate, and xvii) disulfate (O=)₂S(O⁻)₂ or SO₄²⁻ and monosulfate HSO₄⁻;
the anionic counterion, derived from the organic or mineral acid salt, ensures the electrical neutrality of the molecule; thus, it is understood that when the anion comprises several anionic charges, then the same anion may serve for the electrical neutrality of several cationic groups in the same molecule or else may serve for the electrical neutrality of several molecules; for example, a dye which contains two cationic groups may contain either two "*singly charged*" anionic counterions or a "*doubly charged*" anionic counterion such as (O=)₂S(O⁻)₂ or O=P(O⁻)₂-OH;

In particular, the anionic counterions are chosen from halides such as chloride, bromide, fluoride or iodide; a hydroxide; a sulfate; a hydrogen sulfate; a linear or branched C₁-C₆ alkyl sulfate, such as the methylsulfate or ethylsulfate ion; carbonates and hydrogen carbonates; carboxylic acid salts such as formate, acetate, citrate, tartrate and oxalate; linear or branched C₁-C₆ alkylsulfonates, such as the methylsulfonate ion; arylsulfonates for which the aryl part, preferably phenyl, is optionally substituted with one or more C₁-C₄ alkyl radicals, for instance 4-tolylsulfonate; alkylsulfonates such as mesylate;
▪ the term "*chemical oxidizing agent*" means any oxidizing agent other than atmospheric oxygen conventionally used in the field. Thus, mention may be made of hydrogen peroxide, urea peroxide, alkali metal bromates, persalts such as perborates and persulfates, and also enzymes, among which mention may be made of peroxidases, 2-electron oxidoreductases such as uricases, and 4-electron oxygenases such as laccases. Preferably, the chemical oxidizing agent is hydrogen peroxide.
▪ Moreover, the addition salts that may be used in the context of the invention are in particular chosen from addition salts with a cosmetically acceptable base such as basifying agents as defined below, for instance alkali metal hydroxides, such as sodium hydroxide or potassium hydroxide, aqueous ammonia, amines or alkanolamines.
▪ the expression "*at least one*" is equivalent to *"one or more"*;
▪ the limits of a range of values are included in that range, in particular in the expressions "*between*" and "*ranging from* ... *to* ..."; and
▪ the expression "*inclusive*" for a range of values means that the limits of that range are included in the defined range.

### (a) Anthraquinone dyes

The process for dyeing keratin fibres according to the present invention comprises the application to said keratin fibres of (a) one or more anthraquinone dyes chosen from the compounds of formulae **(I)** and **(II)** as defined previously, the optical isomers thereof, the geometrical isomers thereof, the tautomers thereof, the solvates thereof, and mixtures thereof.

In particular, the dyes of formulae **(I)** and **(II)** are violet, blue or green.

According to a particular embodiment of the invention, a) the anthraquinone dye(s) of the invention are chosen from the dyes of formula **(I)** as defined previously.

According to a particular embodiment of the invention, a) the anthraquinone dye(s) of the invention are chosen from one or more dyes of formula **(I)** and n is equal to 0. According to another embodiment, n is equal to 1. Preferably, the substituents **R₁, R₂, R₃** and **R₄** are in positions 1, 4, 5 and 8.

According to a particular embodiment of the invention, the dyes are of formula **(I)**, with **R₁** and **R₃** representing an atom or group chosen from i) hydroxyl, ii) arylamino or aryl(C₁-C₆)alkylamino with the aryl group representing an optionally substituted aryl group, in particular a phenyl group optionally substituted with one or more groups chosen from hydroxyl, (C₁-C₄)alkyl, carboxyl, (C₁-C₄)alkoxycarbonyl, (di)(hydroxy)(C₁-C₄)(alkyl)amino, tri(C₁-C₄)alkylammonium, (C₁-C₄)alkoxy, (di)hydroxysulfonyloxy(C₁-C₄)alkylamino, hydroxysulfonyl, iii) (di)(hydroxy)(C₁-C₆)(alkyl)amino, iv) (di)(C₁-C₄)(alkyl)amino(C₁-C₆)alkylamino, v) (C₁-C₄)alkylcarbonylamino(C₁-C₆)alkylamino, vi) arylsulfonylamino with the aryl group possibly being optionally substituted, aryl preferably representing a phenyl group, vii) (di)halo(C₁-C₄)alkylamino, viii) (di)(hydroxy)(C₁-C₄)alkoxy(C₁-C₄)alkylamino, ix) (di)tri(C₁-C₄)alkylammonium(C₁-C₆)alkylamino, x) heterocycloalkyl(C₁-C₆)alkylamino, heterocycloalkyl(C₁-C₄)alkylcarbonylamino, heterocycloalkyl(C₁-C₄)alkylamino(C₁-C₄)alkylcarbonylamino or heterocycloalkyl(C₁-C₄)alkylcarbonylamino(C₁-C₆)alkylamino, said heterocycloalkyl possibly being optionally cationic and/or substituted especially with one or more (C₁-C₄)alkyl or benzyl groups, xi) halo(C₁-C₄)alkylcarbonylamino(C₁-C₆)alkylamino, xii) hydroxysulfonyl, xiii) halo such as chlorine, xiv) heteroaryl(C₁-C₆)alkylamino, said heteroaryl possibly being optionally cationic and/or substituted especially with one or more (C₁-C₄)alkyl groups, xv) heteroaryl(C₁-C₄)alkylcarbonylamino(C₁-C₆)alkylamino, said heteroaryl possibly being optionally cationic and/or substituted especially with one or more (C₁-C₄)alkyl groups, xvi) R'R"N- with R' and R", which may be identical or different, representing a hydrogen, a (halo)(C₁-C₄)alkylcarbonylamino(C₁-C₆)alkyl group, a (halo)(C₁-C₄)alkylaminocarbonyl (C₁-C₆)alkyl group, (halo)(C₁-C₄)alkylcarbonyl group, a carboxy(C₁-C₆)alkyl group, the alkyl group possibly being substituted with one or more amino or hydroxyl groups, a (poly)hydroxy(C₁-C₆)alkyl group, xvii) (C₁-C₁₆)alkylaminocarbonylamino(C₁-C₆)alkylamino, xviii) formylamino(C₁-C₆)alkylamino, xix) (hydroxy)(C₁-C₆)alkylamino(C₁-C₆)alkylamino, xix) (poly)hydroxysulfonyl(C₁-C₆)alkylamino(C₁-C₆)alkylamino, xx) sulfonato(C₁-C₆)alkyl(di(C₁-C₄)alkyl)ammonium(C₁-C₆)alkylamino, xxi) hydroxysulfonyl(C₁-C₆)alkoxy(C₁-C₆)alkylamino, xxi) heteroaryl(C₁-C₄)alkylcarbonylamino, said heteroaryl possibly being optionally cationic and/or substituted especially with one or more (C₁-C₄)alkyl groups, xxii) heterocycloalkyl(C₁-C₄)alkylcarbonylamino, said heterocycloalkyl possibly being optionally cationic and/or substituted especially with one or more (C₁-C₄)alkyl or benzyl groups, xxiii) (di)(C₁-C₄)(alkyl)amino(C₁-C₆)alkylamino, the (C₁-C₆)alkyl group possibly being substituted with one or more hydroxyl groups, xxiv) heterocycloalkylamino(C₁-C₆)alkylamino or heterocycloalkylamino, said heterocycloalkyl possibly being optionally cationic and/or substituted especially with one or more (C₁-C₄)alkyl or benzyl groups, xxv) heteroarylalkylamino(C₁-C₆)alkylamino or heteroarylalkylamino, said heteroaryl possibly being optionally cationic and/or substituted especially with one or more (C₁-C₄)alkyl groups, xxvi) tri(C₁-C₆)alkylammonium(C₁-C₆)alkylamino, xxvii) (C₁-C₄)alkoxycarbonyl(C₁-C₆)alkyl(di(C₁-C₄)alkyl)ammonium(C₁-C₆)alkylamino, xxviii) carboxylato(C₁-C₆)alkyl(di(C₁-C₄)alkyl)ammonium(C₁-C₆)alkylamino, xxix) carboxy(C₁-C₆)alkylamino(C₁-C₆)alkylamino, xxx) aryl(C₁-C₄)alkyl(di(C₁-C₄)alkyl)ammonium(C₁-C₆)alkylamino, xxxi) sulfonic SO₃H or sulfonate SO₃⁻, M⁺ with M⁺ representing a cationic counterion, xxxii) (C₁-C₆)alkyl, xxxiii) hydroxysulfonyl(C₁-C₄)amino, xxxiv) phenylsulfonylamino.

Preferably, the dyes of formula **(I)** are such that **n** is equal to 1 and the groups **R₂** and **R₄** are in positions 1 and 4.

According to a particular embodiment of the invention, the dyes are of formula **(I)** with **R₂** and **R₄** representing a hydrogen atom.

According to a particular embodiment of the invention, the dyes are of formula **(I)** with **R₂** and **R₄,** which may be identical or different, representing a hydrogen atom or a group as defined previously for R₁ and R₃ i) to xxxii), preferably chosen from:
- hydroxyl,
- (di)(C₁-C₄)(alkyl)amino,
- (C₁-C₄)alkoxy, heterocycloalkyl(C₁-C₆)alkylamino, said heterocycloalkyl possibly being optionally cationic and/or substituted especially with one or more (C₁-C₄)alkyl or benzyl groups,
- arylamino or aryl(C₁-C₆)alkylamino with the aryl group representing an optionally substituted aryl group, in particular a phenyl group optionally substituted with one or more groups chosen from (C₁-C₄)alkyl, (di)(hydroxy)(C₁-C₄)(alkyl)amino, (C₁-C₄)alkoxy and tri(C₁-C₄)alkylammonium,
- aryl(C₁-C₄)alkyl(di(C₁-C₄)alkyl)ammonium(C₁-C₆)alkylamino,
- (di)(C₁-C₄)(alkyl)amino(C₁-C₆)alkylamino, and
- hydroxy(C₁-C₄)(alkyl)amino(C₁-C₆)alkylamino.

According to another particular embodiment of the invention, the dyes are of formula **(I)**, with **R₂** and **R₃** representing a hydrogen atom, and **R₁** and **R₄** are preferably in positions 8 and 4, respectively, and **R₁** and **R₄** are as defined previously, in particular represent an atom or group as defined previously for R₁ and R₃ i) to xxxii), preferably **R₁** and **R₄** are chosen from:
- halogen such as chlorine,
- (di)(C₁-C₄)(alkyl)amino,
- (C₁-C₄)alkylcarbonylamino,
- heterocycloalkyl(C₁-C₆)alkylamino, heterocycloalkyl(C₁-C₄)alkylcarbonylamino or heterocycloalkyl(C₁-C₄)alkylamino(C₁-C₄)alkylcarbonylamino, said heterocycloalkyl may be optionally cationic and/or substituted especially with one or more (C₁-C₄)alkyl or benzyl groups,
- tri(C₁-C₄)alkylammonium(C₁-C₆)alkylamino,
- arylamino or aryl(C₁-C₆)alkylamino with the aryl group representing an optionally substituted aryl group, in particular a phenyl group optionally substituted with one or more groups chosen from (C₁-C₄)alkyl, (di)(hydroxy)(C₁-C₄)(alkyl)amino, (C₁-C₄)alkoxy and tri(C₁-C₄)alkylammonium,
- (di)(C₁-C₄)(alkyl)amino(C₁-C₆)alkylamino, and
- (C₁-C₄)alkylcarbonylamino(C₁-C₆)alkylamino.

According to another particular embodiment of the invention, the dyes are of formula **(I)**, with **R₃** and **R₄** representing a hydrogen atom, and **R₁** and **R₂** are preferably in positions 8 and 1, respectively, and **R₁** and **R₂,** are as defined previously, in particular represent an atom or group as defined previously for R₁ and R₃ i) to xxxii), preferably **R₁** and **R₂** are chosen from:
- (di)(hydroxy)(C₁-C₄)(alkyl)amino,
- (di)(hydroxy)(C₁-C₄)(alkyl)amino(C₁-C₆)alkylamino,
- tri(C₁-C₆)alkylammonium(C₁-C₆)alkylammonium,
- tri(C₁-C₆)alkylammonium(C₁-C₆)alkylamino,
- heterocycloalkyl(C₁-C₆)alkylamino, said heterocycloalkyl possibly being optionally cationic and/or substituted especially with one or more (C₁-C₄) alkyl or benzyl groups and
- arylamino or aryl(C₁-C₆)alkylamino with the aryl group representing an optionally substituted aryl group, in particular a phenyl group optionally substituted with one or more groups chosen from (C₁-C₄)alkyl, (di)(hydroxy)(C₁-C₄)(alkyl)amino, (C₁-C₄)alkoxy, tri(C₁-C₄)alkylammonium.

According to another particular embodiment of the invention, the dyes are of formula **(I)**, with **R₁** and **R₃** representing a hydrogen atom, and n is equal to 2, **R₂** and **R₄** are preferably in positions 1, 2 and 4, respectively, and **R₂** and **R₄,** are as defined previously, in particular represent an atom or group as defined previously for R₁ and R₃ i) to xxxii), preferably **R₂** and **R₄** are chosen from:
- hydroxyl,
- (C₁-C₆)alkyl,
- (di)(hydroxy)(C₁-C₄)(alkyl)amino, said (C₁-C₄)alkyl possibly being optionally substituted with a hydroxyl,
- SO₃H or SO₃⁻M⁺,
- (di)(hydroxy)(C₁-C₄)(alkyl)amino(C₁-C₆)alkylamino,
- tri(C₁-C₄)alkylammonium(C₁-C₆)alkylamino,
- halo(C₁-C₄)alkylcarbonylamino(C₁-C₄)alkylamino,
- heterocycloalkyl(C₁-C₆)alkylamino, said heterocycloalkyl possibly being optionally cationic and/or substituted especially with one or more (C₁-C₄)alkyl or benzyl groups, and
- arylamino or aryl(C₁-C₆)alkylamino with the aryl group representing an optionally substituted aryl group, in particular a phenyl group optionally substituted with one or more groups chosen from (C₁-C₄)alkyl, (di)(hydroxy)(C₁-C₄)(alkyl)amino, (C₁-C₄)alkoxy, tri(C₁-C₄)alkylammonium.

According to another particular embodiment of the invention, the dyes are of formula **(I)**, n is equal to 0, **R₁, R₃** and **R₄** are preferably in positions 8, 5 and 4, respectively, and are as defined previously, in particular represent an atom or group as defined previously for R₁ and R₃ i) to xxxii), **R₁** and **R₃** preferably representing a hydrogen atom.

According to one embodiment, n is equal to 0, **R₃** and **R₄** represent a hydrogen atom, and **R₁** is as defined previously and is in position 7 or 8.

According to another particular embodiment of the invention, a) the anthraquinone dye(s) are chosen from the symmetrical dyes bearing a disulfide group of formula **(II')**: 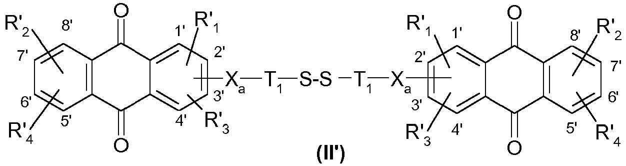 with **R'₁, R'₂, R'₃** and **R'₄, T₁** and **Xₐ** as defined previously, **Xₐ,** preferably represents a N(Rₐ) and particularly NH. More particularly, **R'₂** and **R'₄** represent a hydrogen atom and **R'₁** and **R'₃** are in particular as defined previously for **R₁** and **R₃** i) to xxxii); preferably, **R'₃** and **R'₄,** which may be identical or different, represent a group chosen from (C₁-C₆)alkyl and (di)(hydroxy)(C₁-C₄)(alkyl)amino.

Preferably, **T₁** represents a saturated linear divalent hydrocarbon-based chain comprising from 1 to 20 carbon atoms, preferably between 2 and 10 carbon atoms, optionally interrupted with one or more groups chosen from N(R_{b}), C(O), -N⁺(R₈)(R₉)- An, cationic heteroaryl such as imidazolium, An or combinations thereof, with **R₈** and **R₉**, which may be identical or different, represent a C₁-C₆ alkyl radical; **R_{b}** representing a hydrogen atom or a (C₁-C₄)alkyl group, preferably said hydrocarbon-based chain is interrupted with one or more groups chosen from -N⁺(R₈)(R₉)- An, N(R_{b}), C(O), and combinations thereof such as -C(O)-N(R_{b})- or -N(R_{b})-C(O)-, N⁺(R₈)(R₉)- An.

Preferably, **T₁** represents a divalent group **-(CH₂)ₙ-Tₐ-(CH₂)ₘ-T_{b}-(CH₂)ₚ-** with Tₐ, and T_{b}, which may be identical or different, represent a bond or a group chosen from N(R_{b}), C(O), -N⁺(R₈)(R₉)- An, cationic heteroaryl such as imidazolium, An or combinations thereof, with **R₈** and **R₉**, which may be identical or different, represent a C₁-C₆ alkyl radical; **R_{b}** representing a hydrogen atom or a (C₁-C₄)alkyl group, preferably -N⁺(R₈)(R₉)- An, -C(O)-N(R_{b})- or - N(R_{b})-C(O)-, n, m and p, which may be identical or different, represent an integer between 1 and 10 inclusive, with the sum n+m+p inclusively between 1 and 20, preferably between 2 and 10.

Preferentially, the groups **R'₁** and **R'₃** are in positions 2' and 4', and **R'₂** and **R'₄** are in positions 5' and 8'.

More particularly, a) the anthraquinone dye(s) of the invention are chosen from the symmetrical dyes bearing a disulfide group of formula **(II"):** 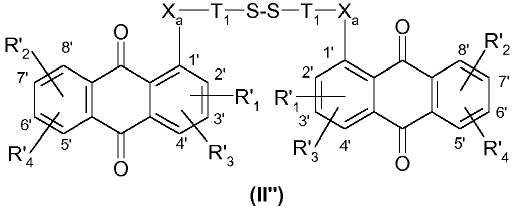 in which formula **(II") R'₁, R'₂, R'₃** and **R'₄, T₁** and **Xₐ** are as defined previously for **(II').**

According to a particular embodiment of the invention, a) the anthraquinone dye(s) of the invention are chosen from the dyes of formula **(II)** as defined previously in which Y represents a hydrogen atom or a thiol-function protecting group.

The anthraquinone dyes of the invention which contain a function SY of formula **(II)** may be in the covalent form -S-Y or the ionic form -S⁻ Y⁺ depending on the nature of Y and the pH of the medium.

One particular embodiment concerns the anthraquinone thiol dyes of formula **(II)** bearing a function SY in which Y represents a hydrogen atom or an alkali metal. Advantageously, Y represents a hydrogen atom.

In accordance with another particular embodiment of the invention, in the abovementioned formula (II), Y is a protecting group known to those skilled in the art, for instance those described in the publications "Protective Groups in Organic Synthesis", T. W. Greene, published by John Wiley & Sons, NY, 1981, pages 193-217; "Protecting Groups", P. Kocienski, Thieme, 3rd edition, 2005, chapter 5.

In particular, when Y represents a thiol-function protecting group, Y is chosen from the following radicals:
▪ (C₁-C₄)alkylcarbonyl,
▪ (C₁-C₄)alkylthiocarbonyl,
▪ (C₁-C₄)alkoxycarbonyl,
▪ (C₁-C₄)alkoxythiocarbonyl,
▪ (C₁-C₄)alkylthiothiocarbonyl,
▪ (di)(C₁-C₄)(alkyl)aminocarbonyl,
▪ (di)(C₁-C₄)(alkyl)aminothiocarbonyl,
▪ arylcarbonyl such as phenylcarbonyl,
▪ aryloxycarbonyl,
▪ aryl(C₁-C₄)alkoxycarbonyl,
▪ (di)(C₁-C₄)(alkyl)aminocarbonyl such as dimethylaminocarbonyl;
▪ (C₁-C₄)(alkyl)arylaminocarbonyl
▪ carboxyl;
▪ SO₃⁻; M⁺ with M⁺ representing an alkali metal such as sodium or potassium or alternatively M⁺ and M' of formula (II) are absent;
▪ optionally substituted aryl such as phenyl, dibenzosuberyl or 1,3,5-cycloheptatrienyl,
▪ optionally substituted heteroaryl;
▪ optionally cationic, optionally substituted heterocycloalkyl, the heterocycloalkyl group in particular represents a saturated or partially saturated 5-, 6- or 7-membered monocyclic group comprising from 1 to 4 heteroatoms chosen from oxygen, sulfur and nitrogen, such as di/tetrahydrofuranyl, di/tetrahydrothiophenyl, di/tetrahydropyrrolyl, di/tetrahydropyranyl, di/tetra/hexahydrothiopyranyl, dihydropyridyl, piperazinyl, piperidinyl, tetramethylpiperidyl, morpholinyl, di/tetra/hexahydroazepinyl, di/tetrahydropyrimidinyl, these groups being optionally substituted with one or more groups such as (C₁-C₄)alkyl, oxo or thioxo; or the heterocycle represents the following group: 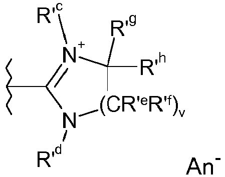 in which R'^{c}, R'^{d}, R'^{e}, R'^{f}, R'^{g} and R'^{h}, which may be identical or different, represent a hydrogen atom or a (C₁-C₄)alkyl group, or alternatively two groups R'^{g} with R'^{h}, and/or R'^{e} with R'^{f} form an oxo or thioxo group, or alternatively R'^{g} with R'^{e} together form a cycloalkyl; and v represents an integer between 1 and 3 inclusive; preferentially, R'^{c} to R'^{h} represent a hydrogen atom; and An⁻ represents a counterion;
▪ isothiouronium -C(NR'^{c}R'^{d})=N⁺R'^{e}R'^{f}; An- with R'c, R'^{d}, R'^{e} and R'^{f}, which may be identical or different, represent a hydrogen atom or a (C₁-C₄)alkyl group; preferentially, R'^{c} to R'^{f} represent a hydrogen atom; and An⁻ represents a counterion;
▪ isothiourea -C(NR'^{c}R'^{d})=NR'^{e}; with R'^{c}, R'^{d} and R'^{e} as defined previously;
▪ optionally substituted (di)aryl(C₁-C₄)alkyl such as 9-anthracenylmethyl, phenylmethyl or diphenylmethyl optionally substituted with one or more groups in particular chosen from (C₁-C₄) alkyl, (C₁-C₄) alkoxy such as methoxy, hydroxyl, alkylcarbonyl or (di)(C₁-C₄)(alkyl)amino such as dimethylamino;
▪ optionally substituted (di)heteroaryl(C₁-C₄)alkyl, the heteroaryl group in particular being a cationic or non-cationic, 5- or 6-membered monocyclic radical comprising from 1 to 4 heteroatoms chosen from nitrogen, oxygen and sulfur, such as pyrrolyl, furanyl, thiophenyl, pyridyl, pyridyl N-oxide groups such as 4-pyridyl or 2-pyridyl N-oxide, pyrylium, pyridinium or triazinyl, optionally substituted with one or more groups such as alkyl, particularly methyl; advantageously, the (di)heteroaryl(C₁-C₄)alkyl is (di)heteroarylmethyl or (di)heteroarylethyl;
▪ CR¹R²R³ with R¹, R² and R³, which may be identical or different, representing a halogen atom or a group chosen from:
   - (C₁-C₄)alkyl;
   - (C₁-C₄)alkoxy;
   - optionally substituted aryl such as phenyl optionally substituted with one or more groups, for instance (C₁-C₄)alkyl, (C₁-C₄)alkoxy or hydroxyl;
   - optionally substituted heteroaryl such as thiophenyl, furanyl, pyrrolyl, pyranyl or pyridyl, optionally substituted with a (C₁-C₄)alkyl group;
   - P(Z¹)R'¹R'²R'³ with R'¹ and R'₂, which may be identical or different, represent a hydroxyl, (C₁-C₄)alkoxy or alkyl group, represents a hydroxyl or (C₁-C₄)alkoxy group, and Z¹ represents an oxygen or sulfur atom;
▪ sterically hindered ring such as the adamantyl group; and optionally substituted alkoxy(C₁-C₄)alkyl such as methoxymethyl (MOM), ethoxyethyl (EOM) and isobutoxymethyl.

According to a particular embodiment, the thiol-protected anthraquinone dyes of formula (II) comprise a group Y chosen from i) aromatic cationic 5- or 6-membered monocyclic heteroaryl comprising from 1 to 4 heteroatoms chosen from oxygen, sulfur and nitrogen, such as oxazolium, isoxazolium, thiazolium, isothiazolium, 1,2,4-triazolium, 1,2,3-triazolium, 1,2,4-oxazolium, 1,2,4-thiadiazolium, pyrylium, pyridinium, pyrimidinium, pyrazinyl, pyrazinium, pyridazinium, triazinium, tetrazinium, oxazepinium, thiepinyl, thiepinium, imidazolium; ii) cationic 8- to 11-membered bicyclic heteroaryl such as indolinium, benzimidazolium, benzoxazolium, benzothiazolium, these monocyclic or bicyclic heteroaryl groups optionally being substituted with one or more groups such as alkyl, for instance methyl, or polyhalo(C₁-C₄)alkyl such as trifluoromethyl; iii) or the following heterocyclic: 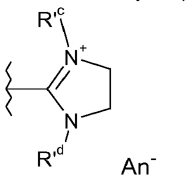 in which R'^{c} and R'^{d}, which may be identical or different, represent a hydrogen atom or a group (C₁-C₄)alkyl; preferentially, R'^{c} to R'^{d} represent a group (C₁-C₄)alkyl such as methyl; and An⁻ represents a counterion.

In particular, Y represents an alkali metal or a group chosen from oxazolium, isoxazolium, thiazolium, isothiazolium, 1,2,4-triazolium, 1,2,3-triazolium, 1,2,4-oxazolium, 1,2,4-thiadiazolium, pyrylium, pyridinium, pyrimidinium, pyrazinium, pyridazinium, triazinium and imidazolium, benzimidazolium, benzoxazolium, benzothiazolium, these groups being optionally substituted with one or more (C₁-C₄)alkyl groups, especially methyl.

In particular, Y represents an alkali metal or a protecting group such as:
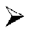 (C₁-C₄)alkylcarbonyl, for instance methylcarbonyl or ethylcarbonyl;
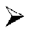 arylcarbonyl such as phenylcarbonyl;
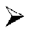 (C₁-C₄)alkoxycarbonyl;
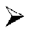 aryloxycarbonyl,
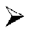 aryl(C₁-C₄)alkoxycarbonyl,
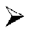 (di)(C₁-C₄)(alkyl)aminocarbonyl such as dimethylaminocarbonyl;
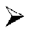 (C₁-C₄)(alkyl)arylaminocarbonyl;
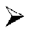 optionally substituted aryl such as phenyl;
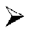 5- or 6-membered monocyclic heteroaryl such as imidazolyl or pyridyl;
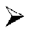 cationic 5- or 6-membered monocyclic heteroaryl such as pyrylium, pyridinium, pyrimidinium, pyrazinium, pyridazinium, triazinium or imidazolium; these groups being optionally substituted with one or more identical or different (C₁-C₄)alkyl groups such as methyl;
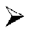 cationic 8- to 11-membered bicyclic heteroaryl such as benzimidazolium or benzoxazolium; these groups being optionally substituted with one or more identical or different (C₁-C₄)alkyl groups such as methyl;
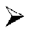 cationic heterocycle having the following formula: 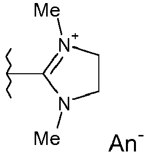
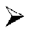 isothiouronium -C(NH₂)=N⁺H₂; An⁻;
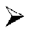 isothiourea -C(NH₂)=NH;
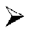 SO₃⁻; M⁺ with M⁺ representing an alkali metal such as sodium or potassium or alternatively M⁺ and M' of formula (II) are absent.

Preferably, the anthraquinone dye(s) are chosen from the following compounds, the optical isomers thereof, the geometrical isomers thereof, the tautomers thereof, the solvates thereof, and mixtures thereof: 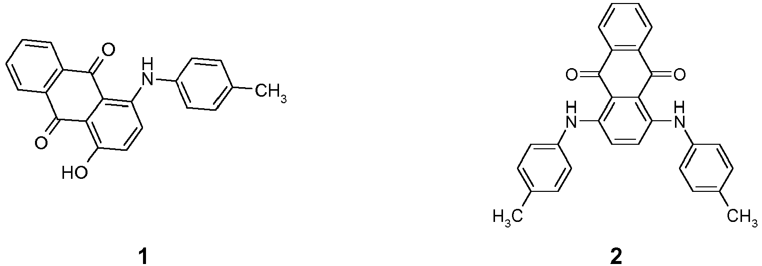 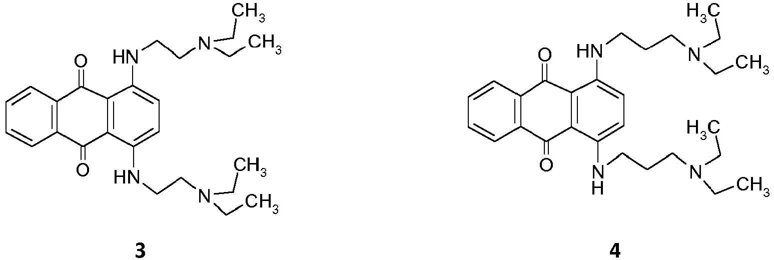 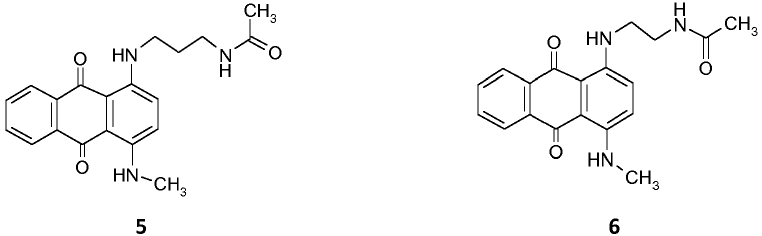 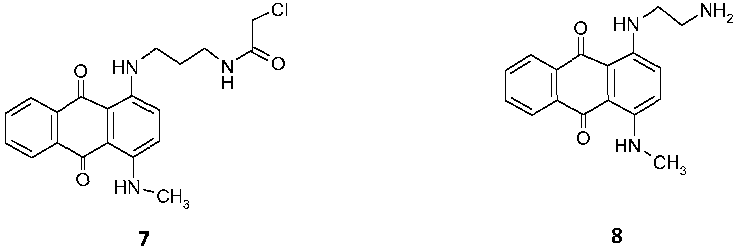 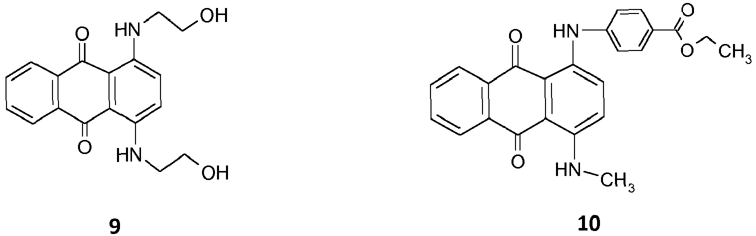 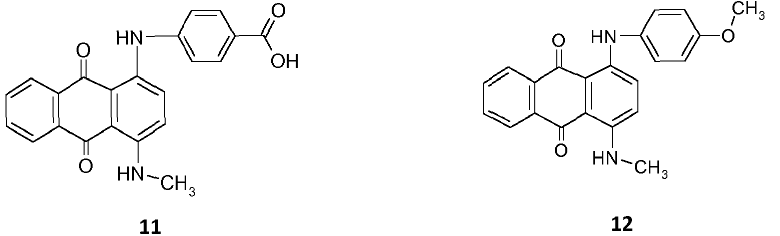 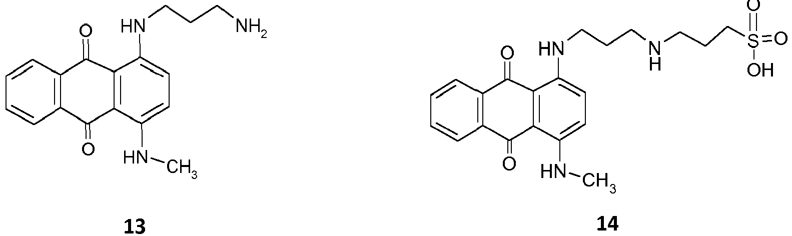 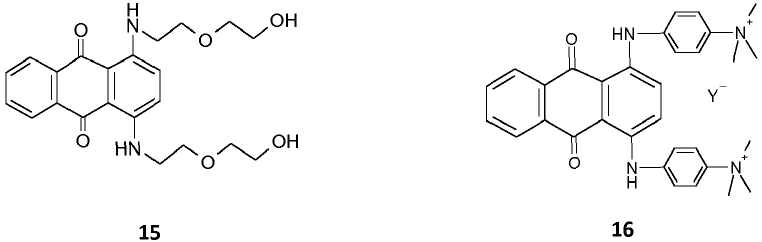 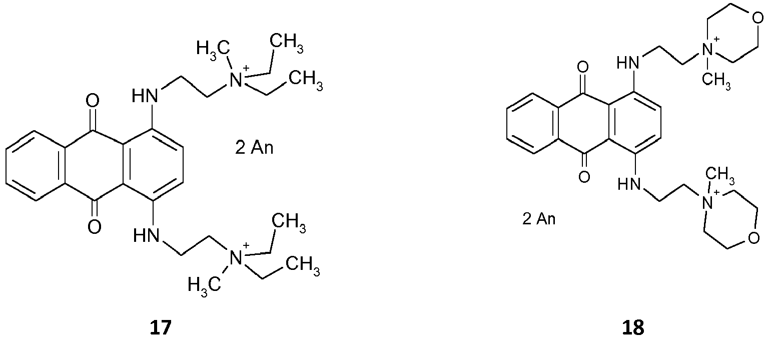 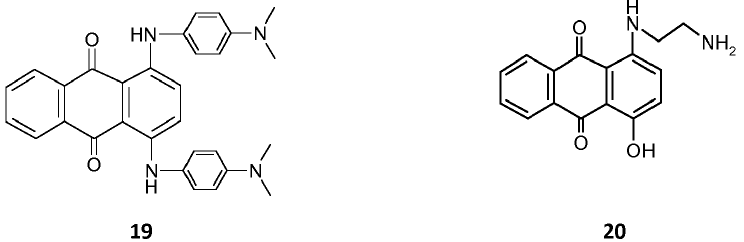 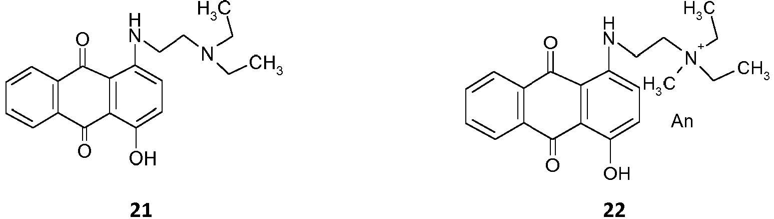 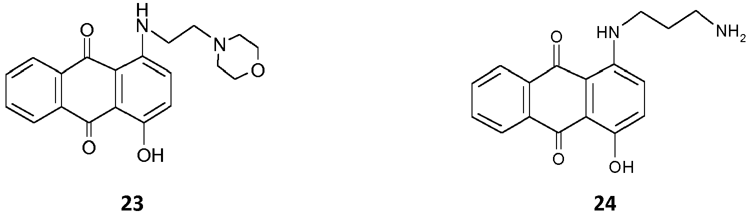 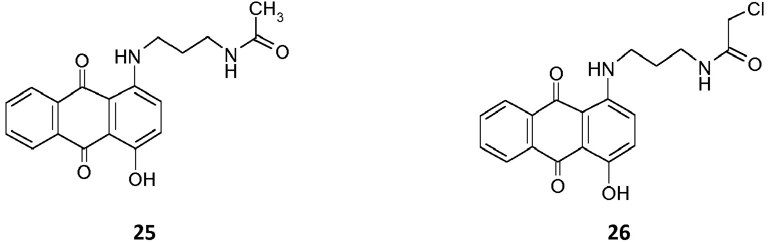 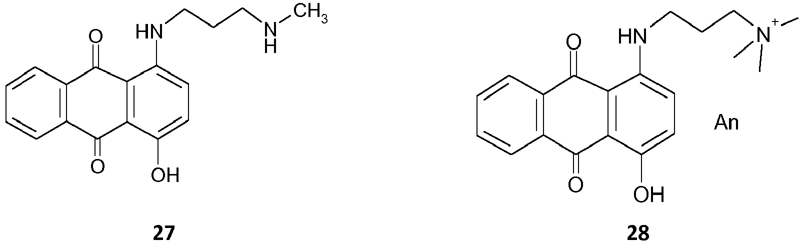 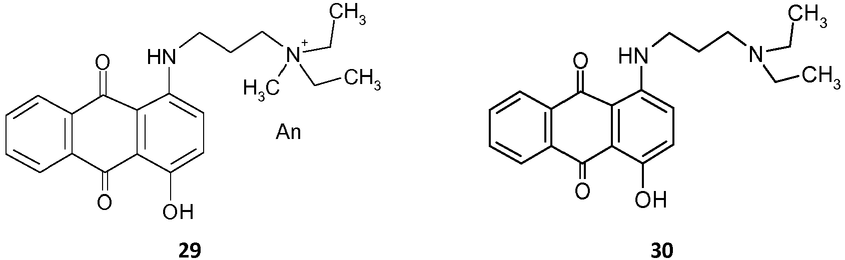 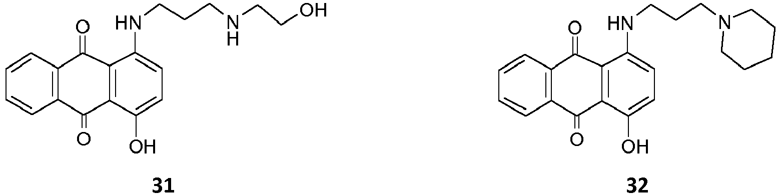 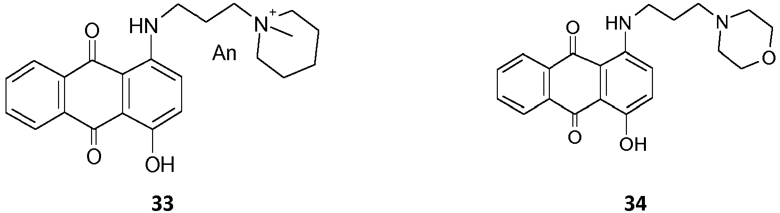 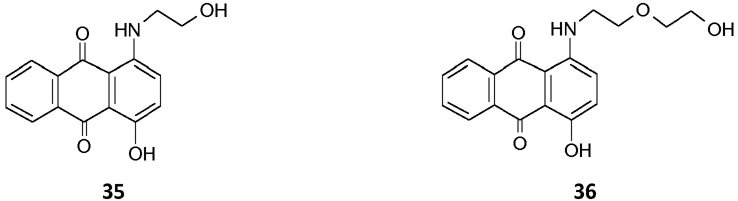 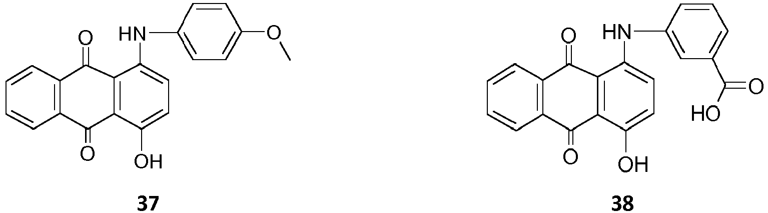 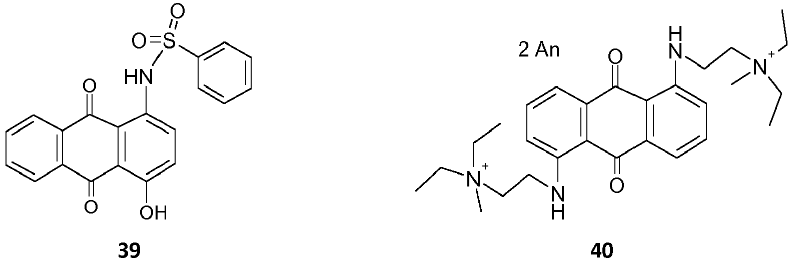 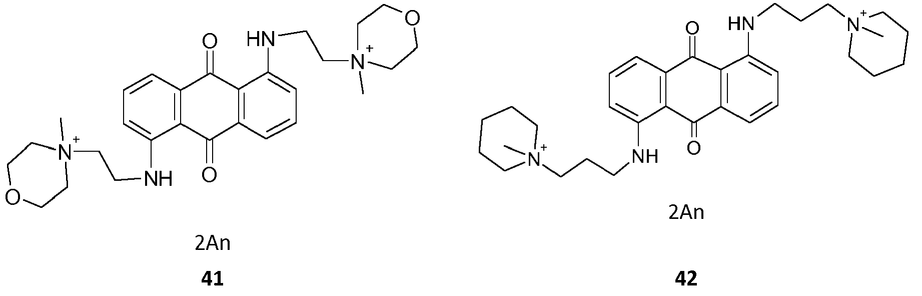 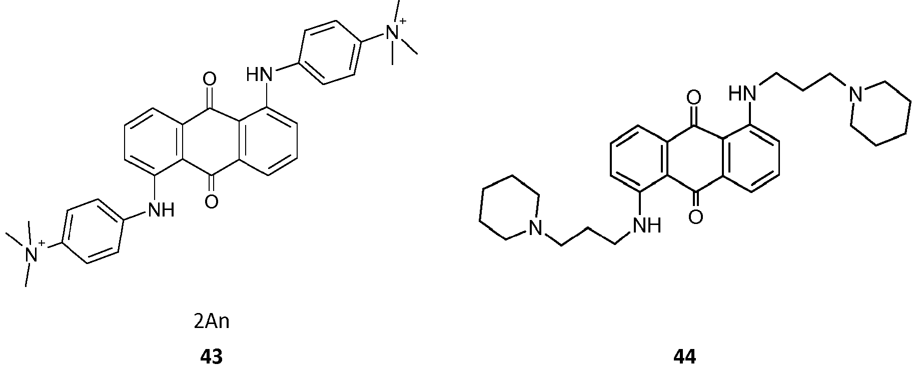 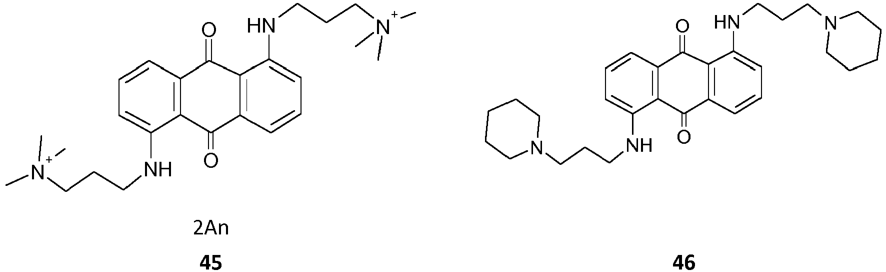 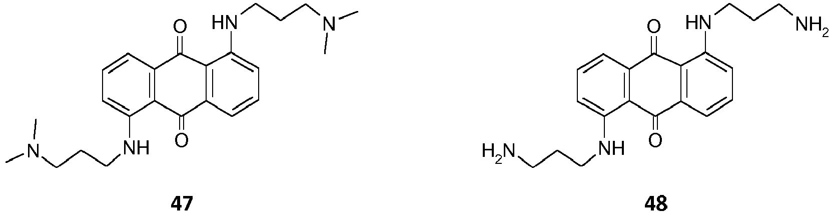 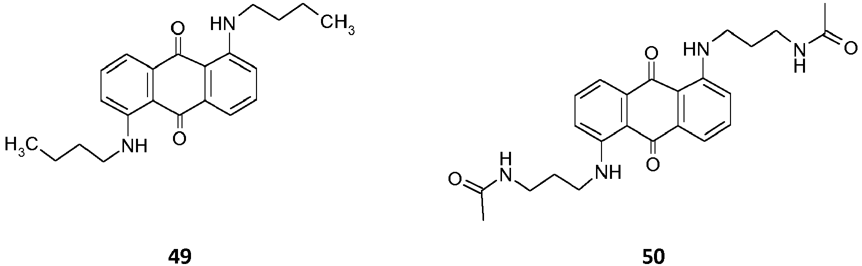 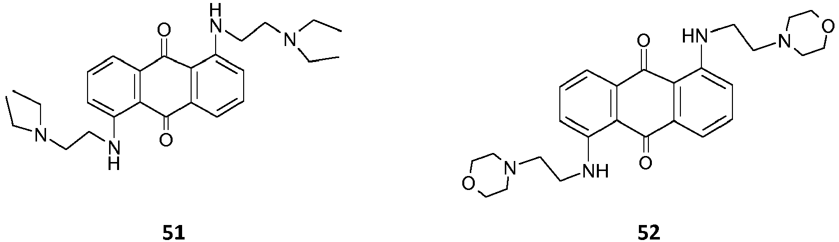 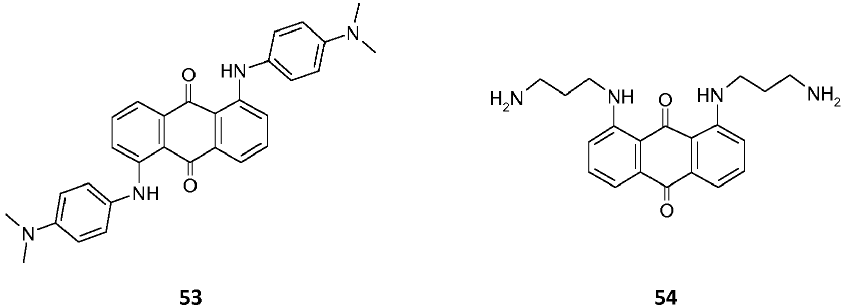 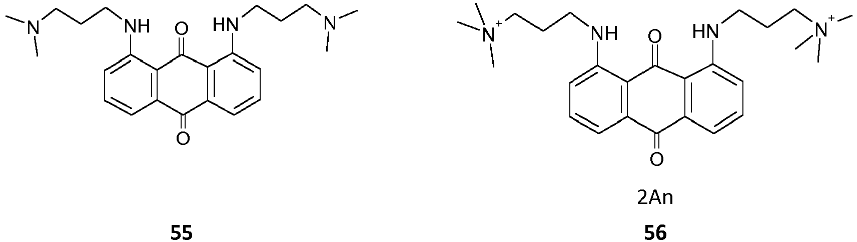 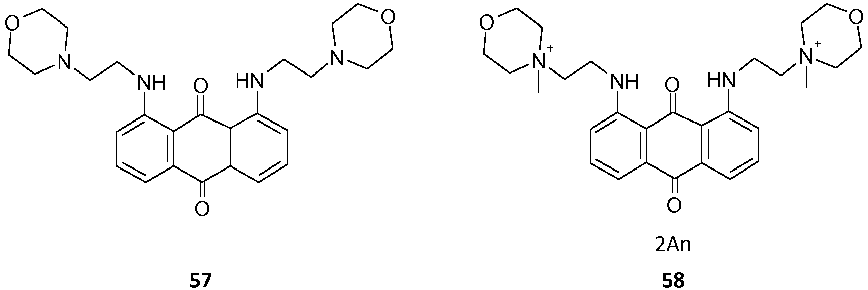 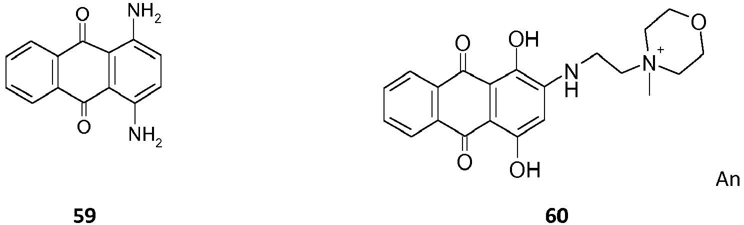 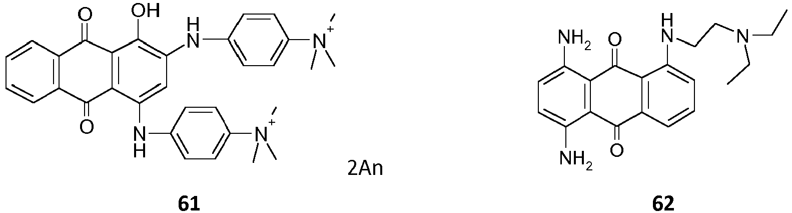 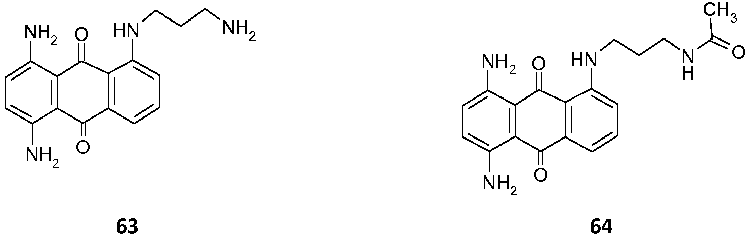 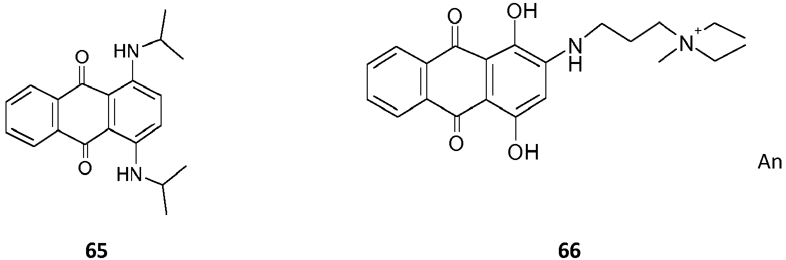 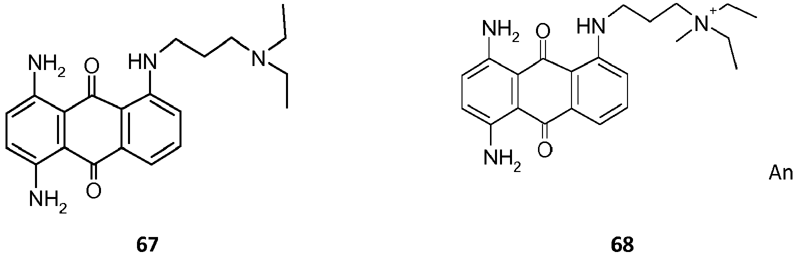 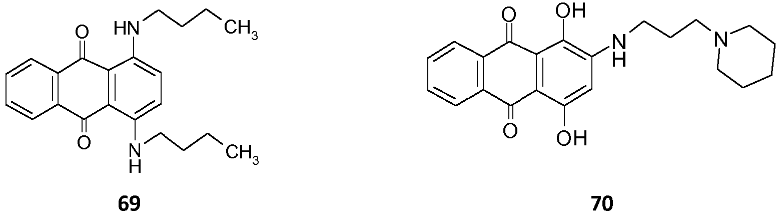 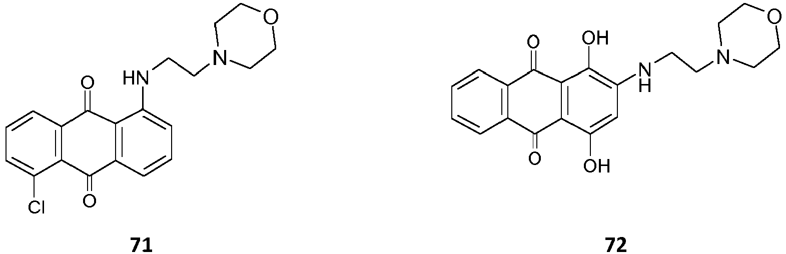 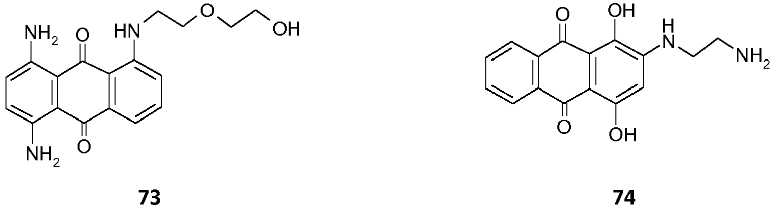 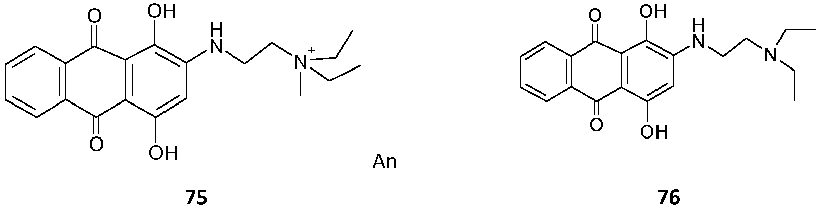 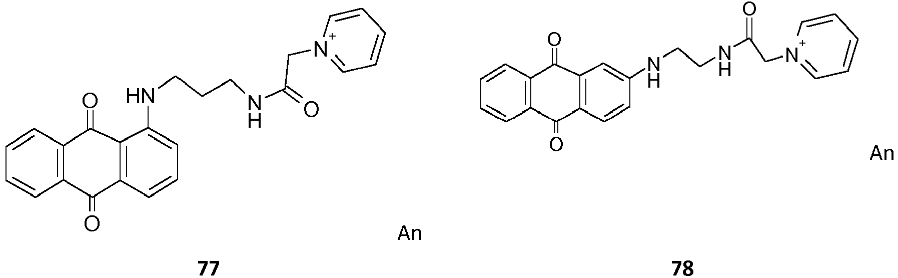 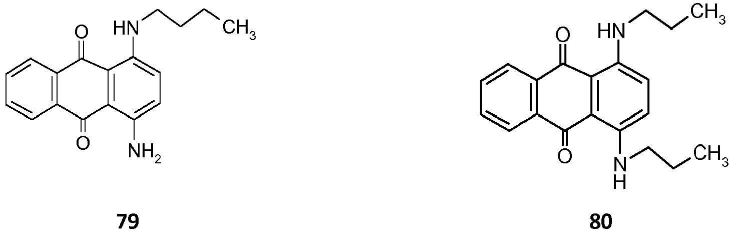 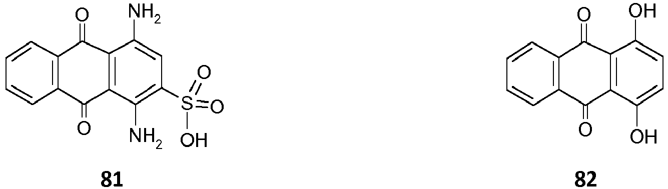 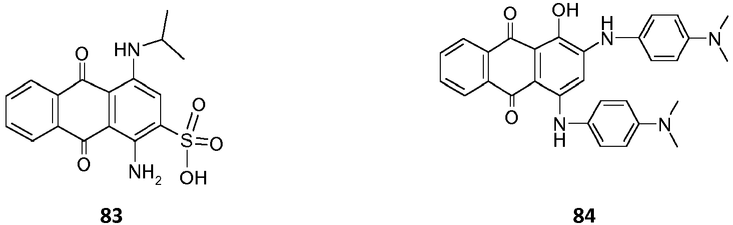 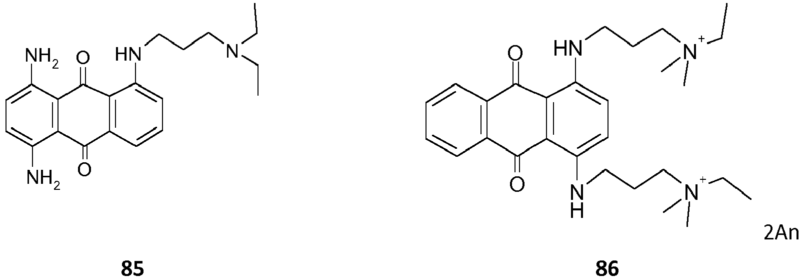 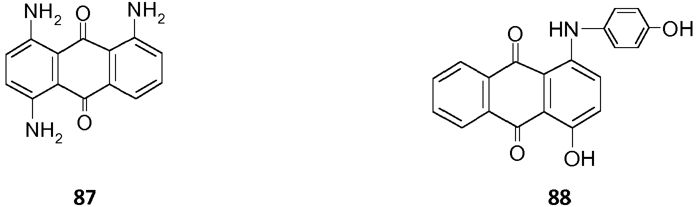 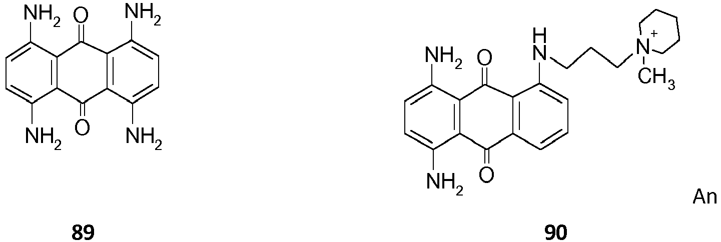 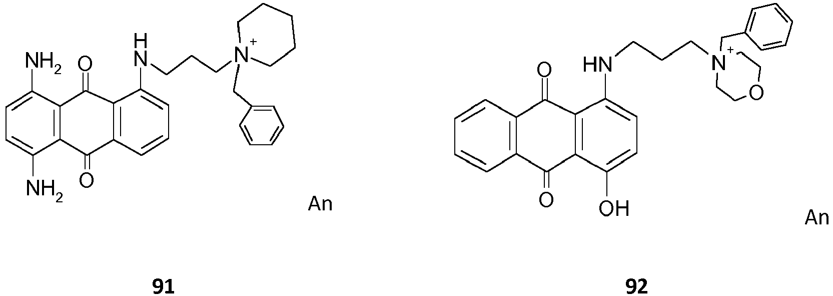 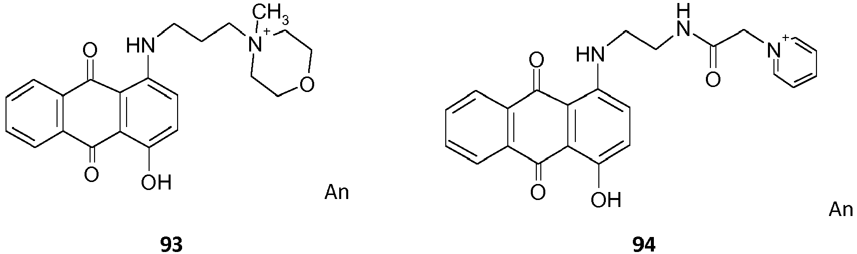 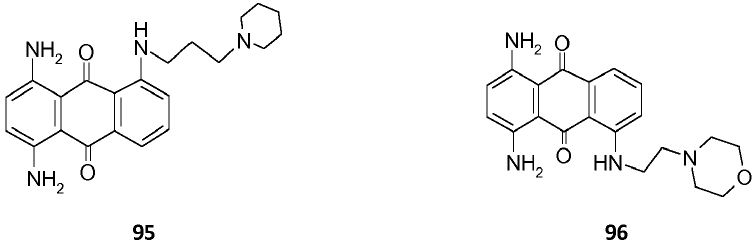 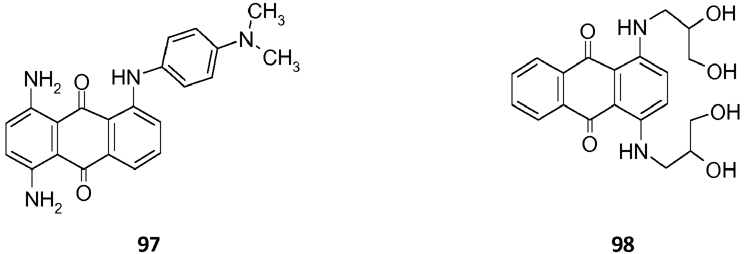 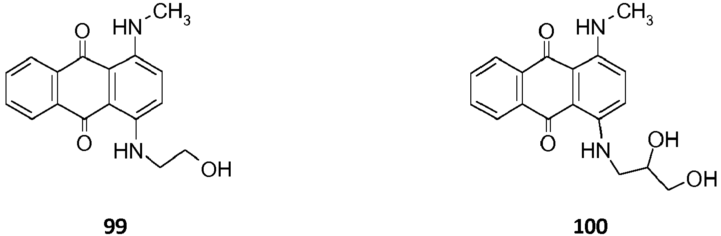 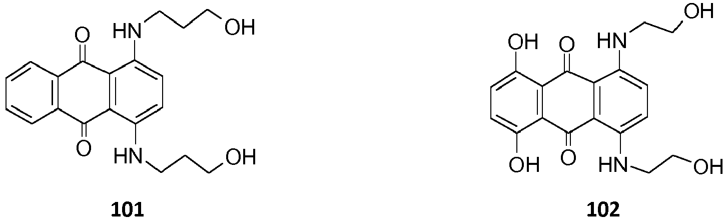 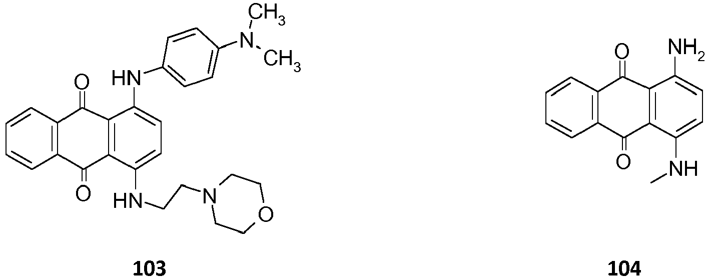 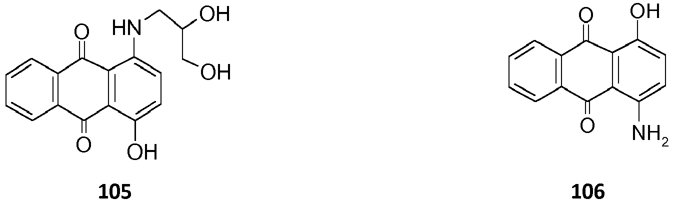 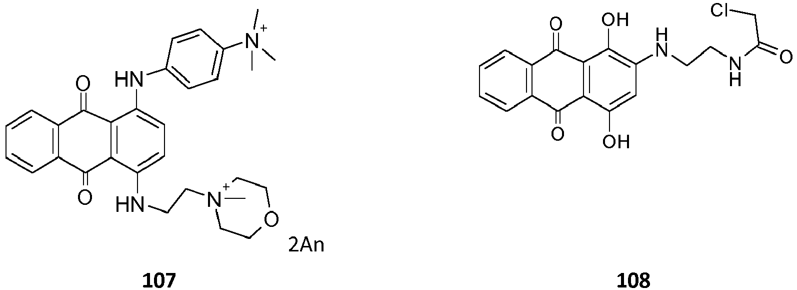 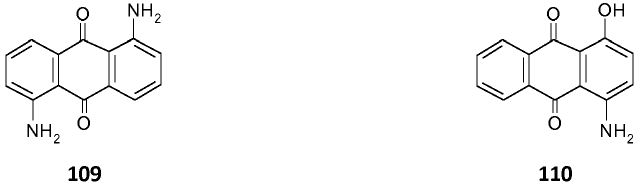 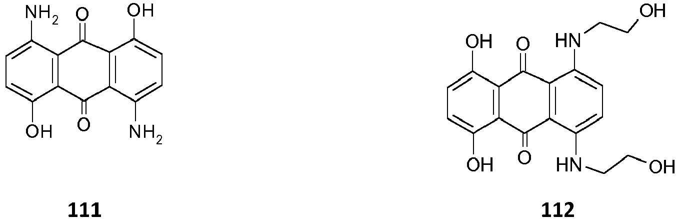 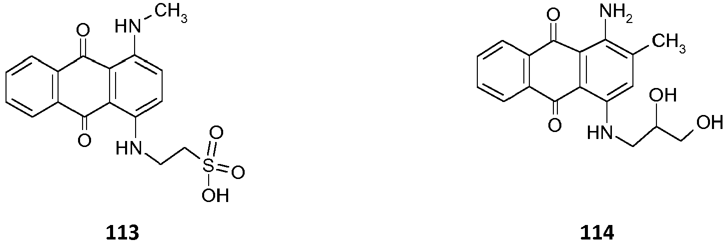 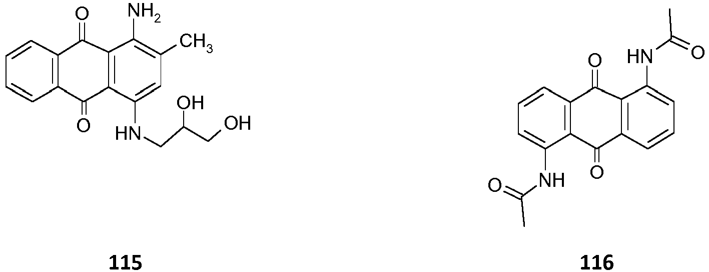 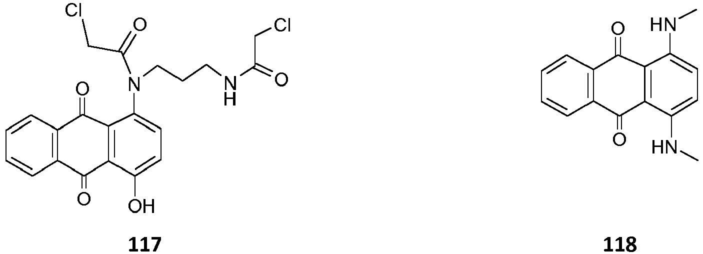 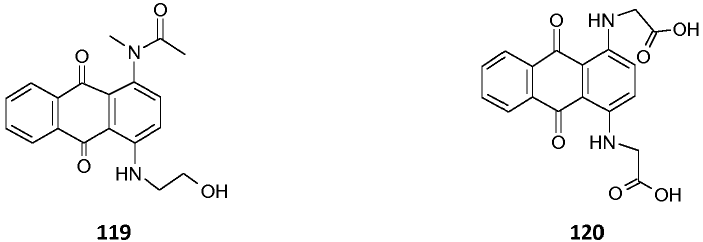 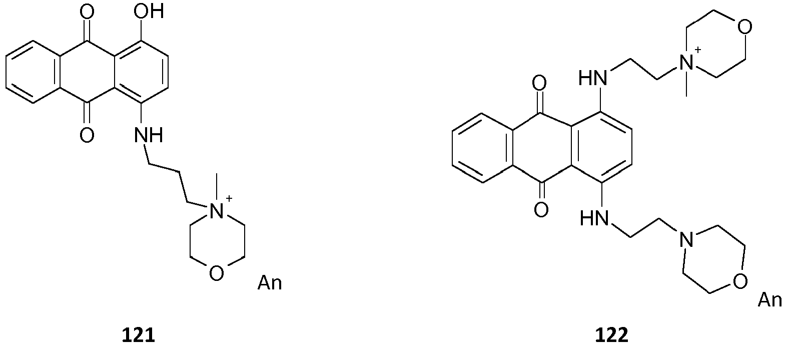 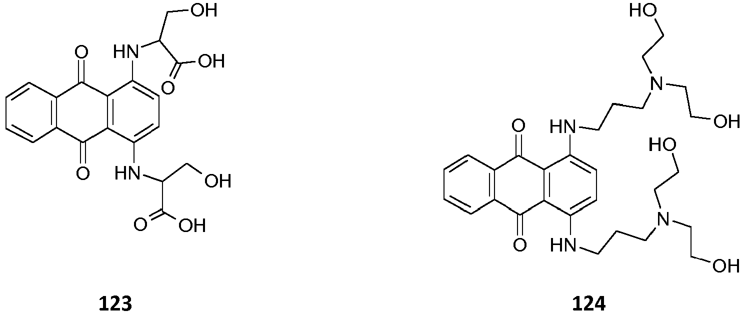 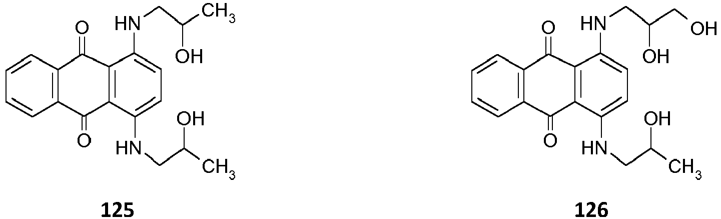 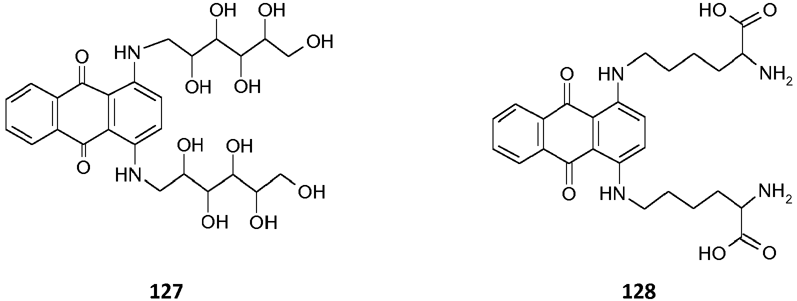 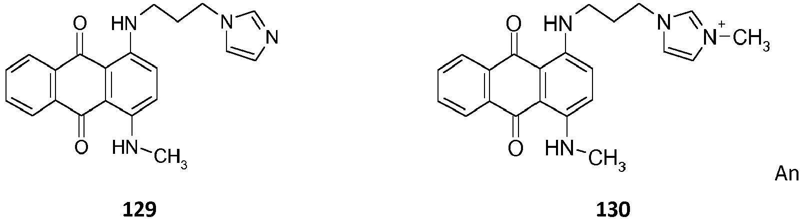 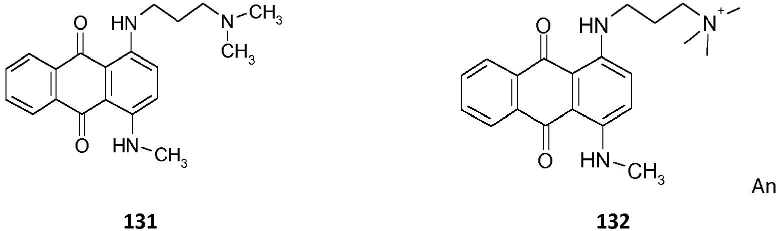 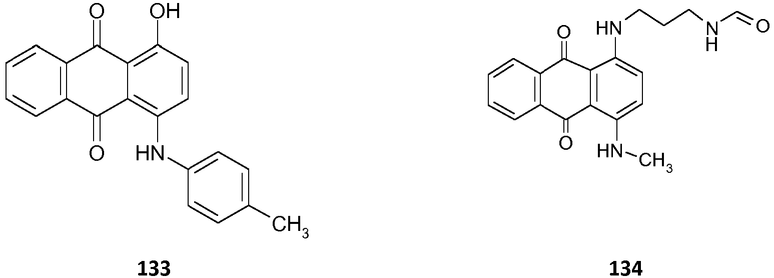 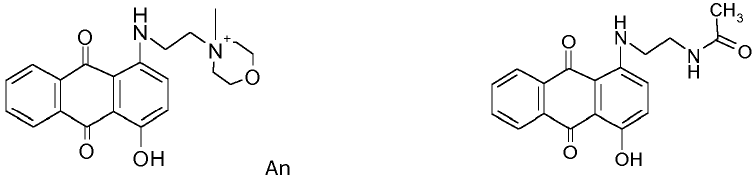 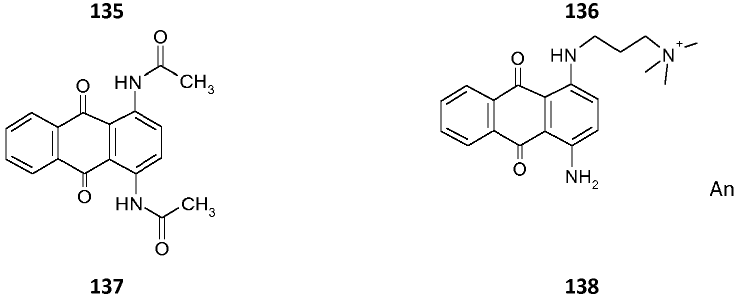 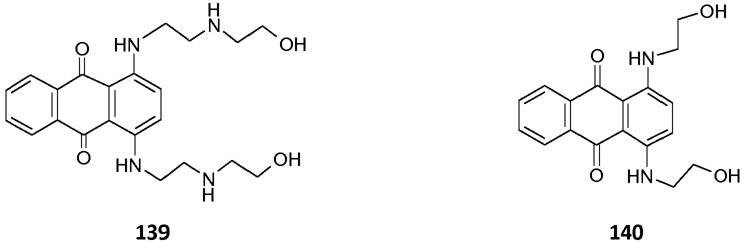 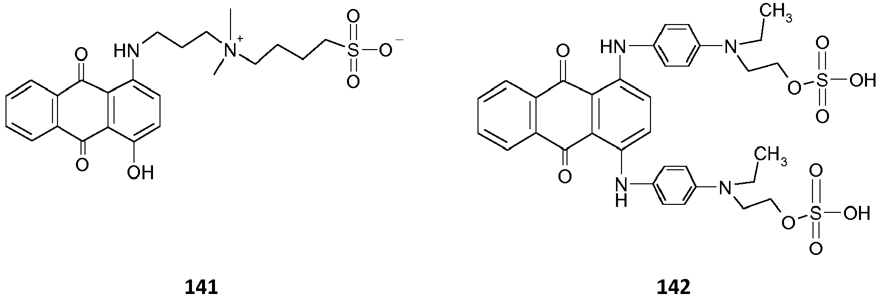 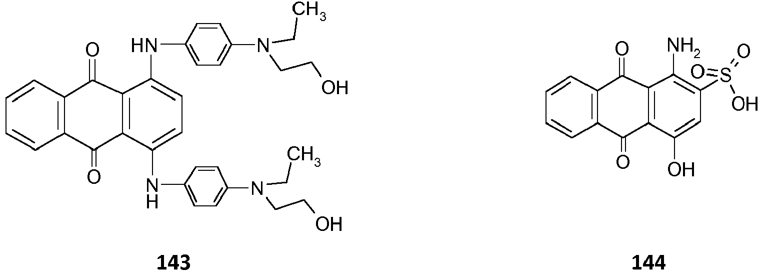 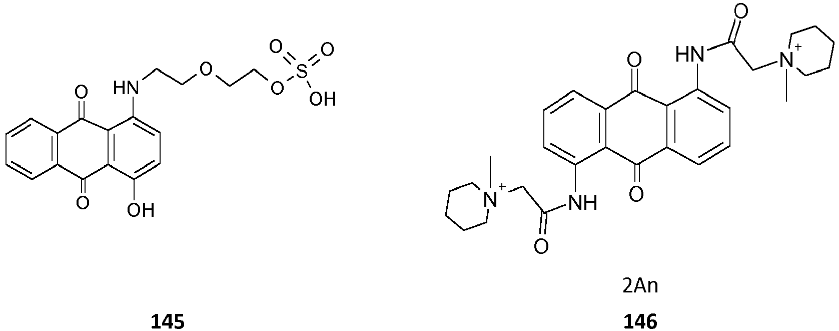 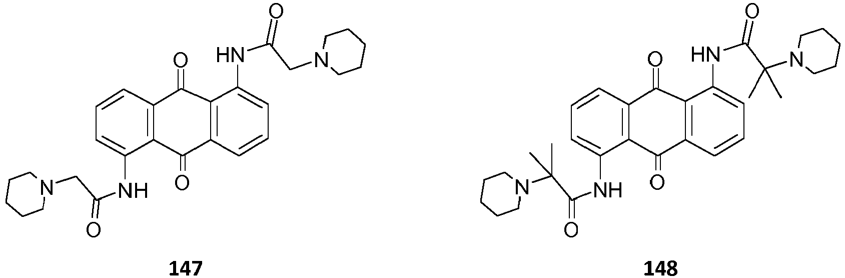 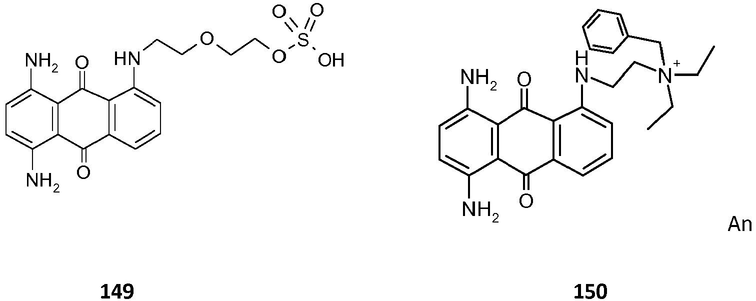 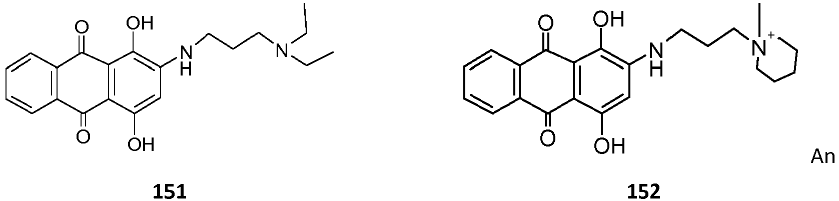 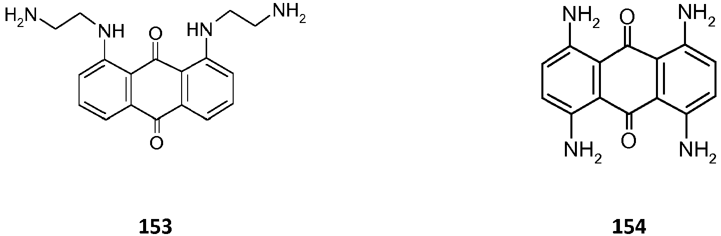 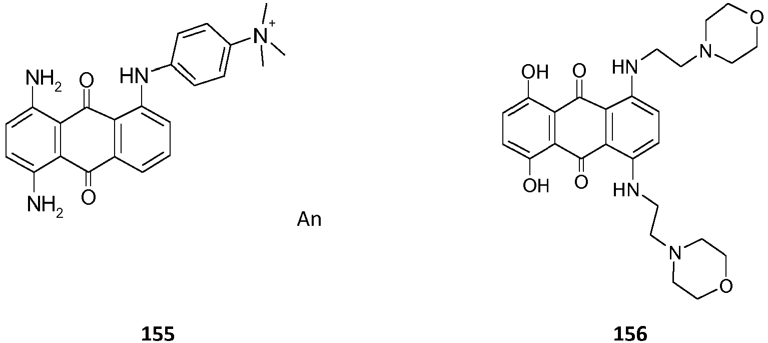 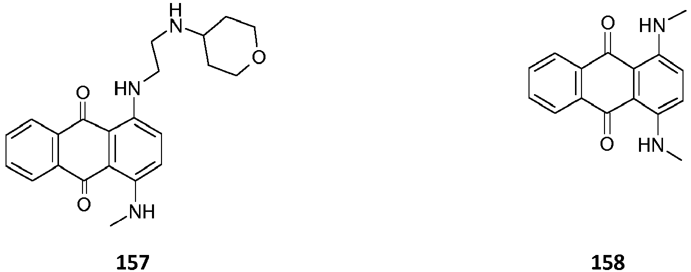 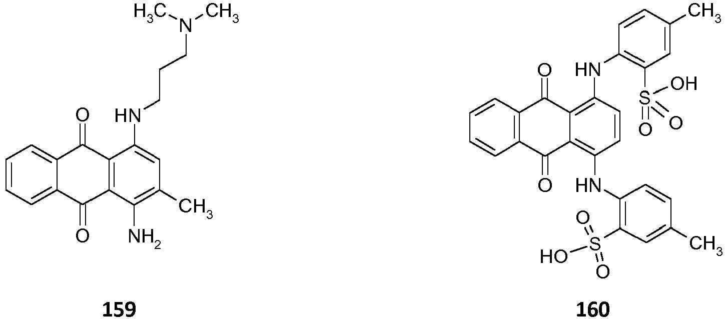 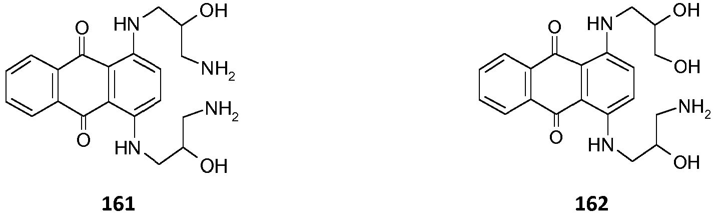 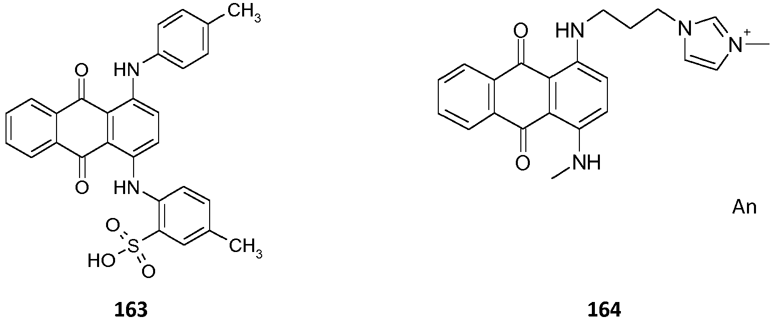 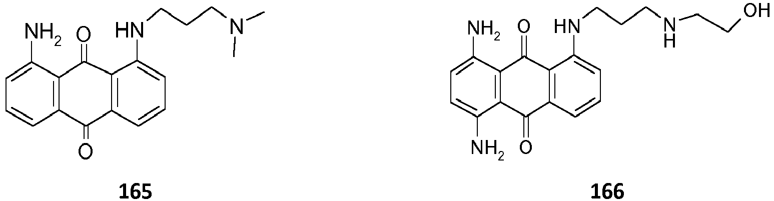 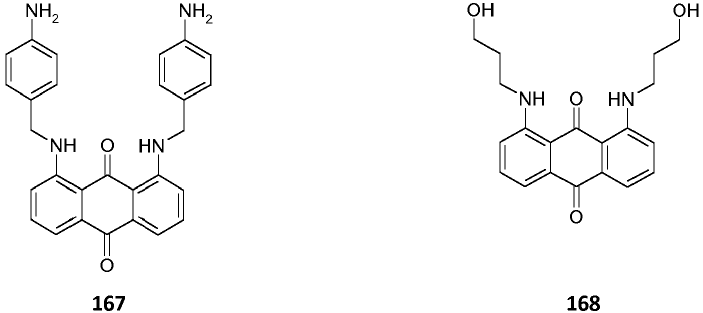 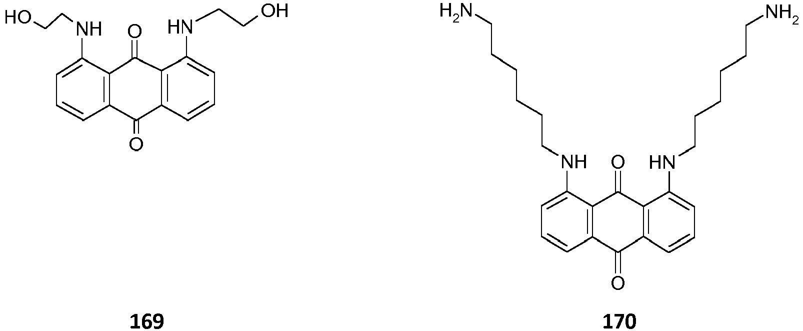 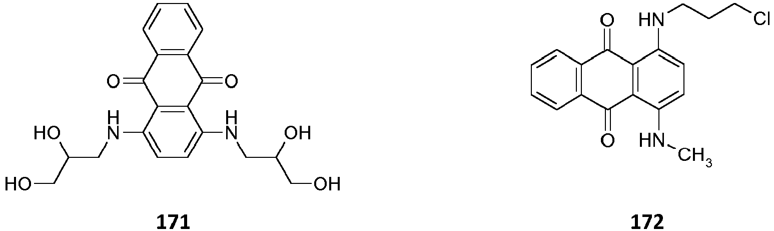 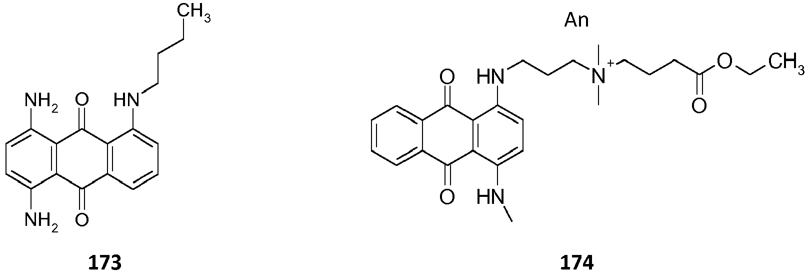 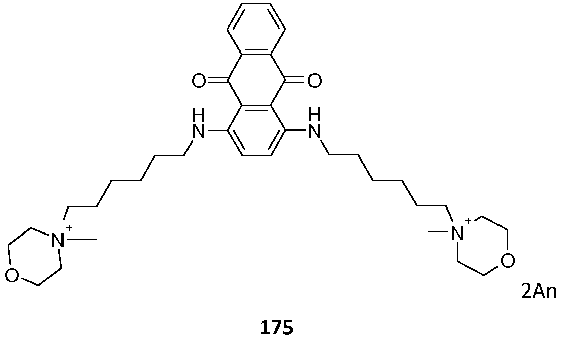 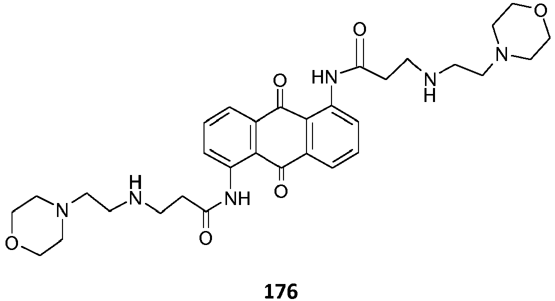 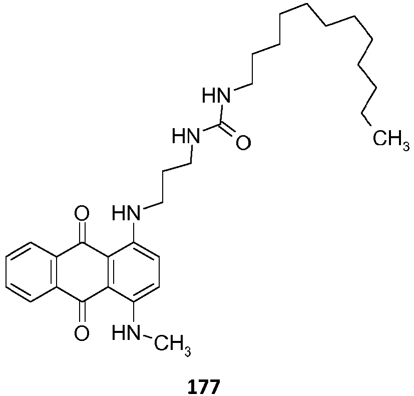 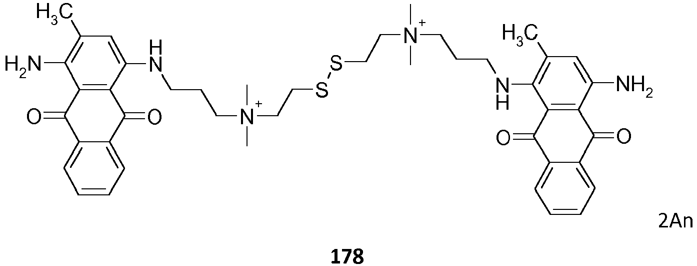 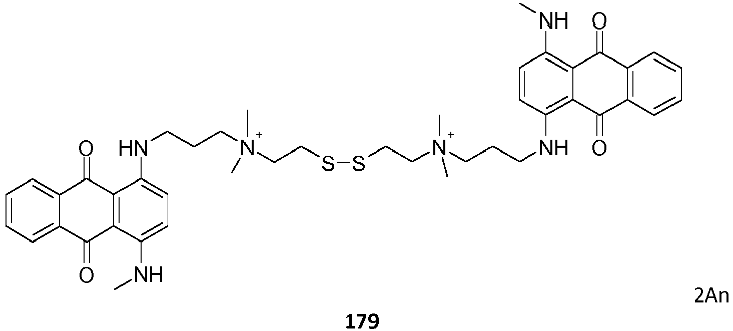 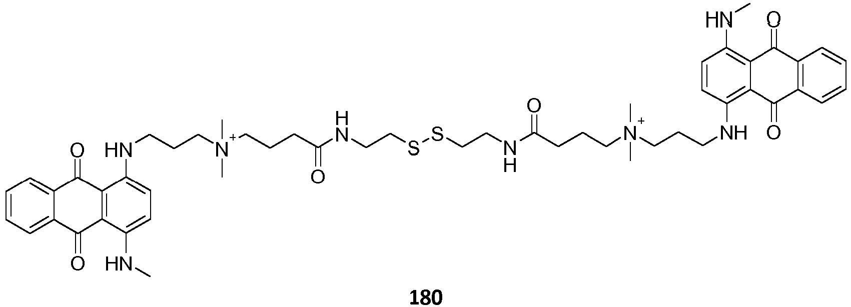 with **An**, which may be identical or different, representing an anionic counterion as defined previously, preferably chosen from mesylate, tosylate and halide such as Cl⁻ and I⁻.

The dyes of formulae (I) and (II) of the invention are known commercial products, or are accessible via standard synthetic routes known to those skilled in the art. Examples that may be mentioned include: Chemical Reviews, "The chemistry of anthraquinones", 6, pages 157- 174 (1926); and Colourage "Blue disperse dyes - structural diversities", 55 (11), pages 36, 38 and 42 (2008).

### (b) Fluorescent dyes

The process for dyeing keratin fibres according to the present invention and the composition of the invention also use, or comprise, (b) one or more cationic (poly)methine fluorescent dyes. Preferably, the fluorescent dye(s) of the invention are direct dyes. More particularly, the fluorescent dyes of the invention are other than fluorescent dyes comprising a disulfide bond; preferably, the fluorescent dyes of the invention are direct and do not comprise a bond with identical contiguous heteroatoms.

As fluorescent dyes that may be used in the present invention, mention may be made of the following dyes: (poly)methines (especially cyanins and styryls/hemicyanins), naphtalimides. The prior art knows other fluorescent dyes described in EP 1 133 975, WO 03/029 359, EP 860 636, WO 95/01772, WO 95/15144, EP 714 954 and those listed in the encyclopaedia The chemistry of synthetic dyes by K. Venkataraman, 1952, Academic Press, vol. 1 to 7, in Kirk Othmer's encyclopaedia Chemical Technology, in the chapter "Dyes and Dye Intermediates", 1993, Wiley and Sons, and in various chapters of Ullmann's Encyclopedia of Industrial Chemistry 7th edition, Wiley and Sons, especially in Ullmann's Encyclopedia of Industrial Chemistry in the chapter "Fluorescent Dyes", 2005, Wiley-VCH Verlag GmbH & Co. KGaA, Weinheim 10.1002/143560007.a11_279; in The Handbook - A Guide to Fluorescent Probes and Labeling Technologies, 10th Ed Molecular Probes/Invitrogen - Oregon 2005 circulated by Internet or in the preceding printed editions.

The fluorescent dye(s) of the invention are direct dyes chosen from (poly)methine dyes, in particular cyanin dyes and styryl/hemicyanin dyes, and naphthalimide dyes, and mixtures thereof; the fluorescent dyes are cationic; preferably, the fluorescent dye(s) (b) are chosen from styryl or hemicyanin cationic dyes.

Better still, the fluorescent dyes of the invention absorb light in the yellow, orange and red range, preferably in the absorption wavelength λ_{abs} between 400 nm and 500 nm inclusive.
According to one variant, the fluorescent dyes of the invention contain at least one cationic radical borne by, or included in, at least one of the fluorescent chromophores.
Preferably, the cationic radical is a quaternary ammonium; better still, the cationic charge is endocyclic.
These cationic radicals are, for example, a cationic radical:
- bearing an exocyclic (di/tri)(C₁-C₈)alkylammonium charge, or
- bearing an endocyclic charge, such as the following cationic heteroaryl groups: acridinium, benzimidazolium, benzobistriazolium, benzopyrazolium, benzopyridazinium, benzoquinolium, benzothiazolium, benzotriazolium, benzoxazolium, bipyridinium, bis-tetrazolium, dihydrothiazolium, imidazopyridinium, imidazolium, indolium, isoquinolium, naphthoimidazolium, naphthoxazolium, naphthopyrazolium, oxadiazolium, oxazolium, oxazolopyridinium, oxonium, phenazinium, phenoxazolium, pyrazinium, pyrazolium, pyrazoyltriazolium, pyridinium, pyridinoimidazolium, pyrrolium, pyrylium, quinolium, tetrazolium, thiadiazolium, thiazolium, thiazolopyridinium, thiazoylimidazolium, thiopyrylium, triazolium or xanthylium.

According to a preferred variant of the invention, the fluorescent dyes of the invention bear at least one cationic chromophore and comprise at least one quaternary ammonium radical such as polymethine chromophores chosen from formulae **(III)** and **(IV)** below:

**W⁺-[C(R^{c})=C(R^{d})]_{m'}-Ar'-(*)Q⁻** **(III)**

**Ar-[C(R^{d})=C(R^{c})]_{m'}-W'⁺-(*)Q⁻** **(IV)**,

in which formulae **(III)** and **(IV)**:
- **W⁺** represents a cationic heteroaryl group, in particular comprising a quaternary ammonium optionally substituted with one or more (C₁-C₈)alkyl groups optionally substituted in particular with one or more hydroxyl groups;
- **W'⁺** represents a divalent heteroaryl radical as defined for **W⁺**;
- **Ar** represents an aryl group such as phenyl or naphthyl, optionally substituted preferably with i) one or more halogen atoms such as chlorine or fluorine; ii) one or more (C₁-C₈)alkyl and preferably C₁-C₄ alkyl groups such as methyl; iii) one or more hydroxyl groups; iv) one or more (C₁-C₈)alkoxy groups such as methoxy; v) one or more hydroxy(C₁-C₈)alkyl groups such as hydroxyethyl, vi) one or more amino or (di)(C₁-C₈)alkylamino groups, preferably with the C₁-C₄ alkyl part optionally substituted with one or more hydroxyl groups, such as (di)hydroxyethylamino, vii) with one or more acylamino groups; viii) one or more heterocycloalkyl groups such as piperazinyl, piperidyl or 5- or 6-membered heteroaryl such as pyrrolidinyl, pyridyl and imidazolinyl;
- **Ar'** is a divalent aryl radical as defined for **Ar**;
- **m'** represents an integer between 1 and 4 inclusive, in particular, m is 1 or 2; better still 1;
- **R^{c}** and **R^{d}**, which may be identical or different, represent a hydrogen atom or optionally a substituted (C₁-C₈)alkyl and preferably C₁-C₄ alkyl group, or alternatively R^{c} is contiguous with **W** or **W'** and/or **R^{d}** is contiguous with **Ar** or **Ar'** and form, with the atoms that bear them, a (hetero)cycloalkyl; in particular, **R^{c}** is contiguous with **W⁺** or **W'⁺** and forms a (hetero)cycloalkyl such as cyclohexyl;
- **Q⁻** is an organic or mineral anionic counterion;
- (*) represents the part of the fluorescent chromophore that is bonded to the rest of the dye.

Preferably, **W⁺** or **W'⁺** is an imidazolium, pyridinium, benzimidazolium, pyrazolium, benzothiazolium or quinolinium radical optionally substituted with one or more identical or different C₁-C₄ alkyl radicals.

According to a particularly preferred embodiment of the invention, the fluorescent chromophore(s) of the dye **(III)** or **(IV)** are those defined previously with **m'** = 1, **Ar** representing a phenyl group substituted para to the styryl group -C(R^{d})=C(R^{c})- with a (di)(hydroxy)(C₁-C₆)(alkyl)amino group such as dihydroxy(C₁-C₄)alkylamino, and **W'⁺** representing an imidazolium or pyridinium group, preferentially ortho- or para-pyridinium.

According to another preferred variant of the invention, the fluorescent chromophore(s) of the dyes are cationic and comprise at least one quaternary ammonium radical such as a naphthimidyl bearing a cationic exocyclic charge of formula **(IIIa)** or (**IVa**): 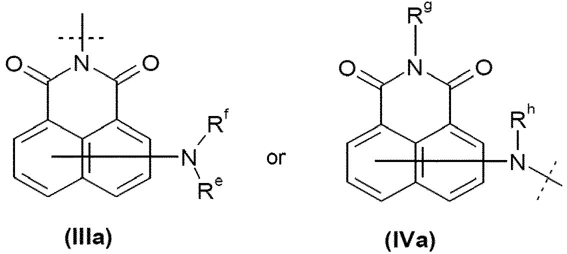 where 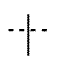 represents the bond to the dye in which formulae **(IIIa)** and **(IVa)** R^{e}, R^{f}, R^{g} and R^{h}, which may be identical or different, represent a hydrogen atom or a (C₁-C₆)alkyl group which is optionally substituted, preferably with a di(C₁-C₆)alkylamino or tri(C₁-C₆)alkylammonium group such as trimethylammonium. According to a particular embodiment, the fluorescent dye(s) (b) of the invention are chosen from those of formula **(V)**, **(VI)** or **(VII)** below: 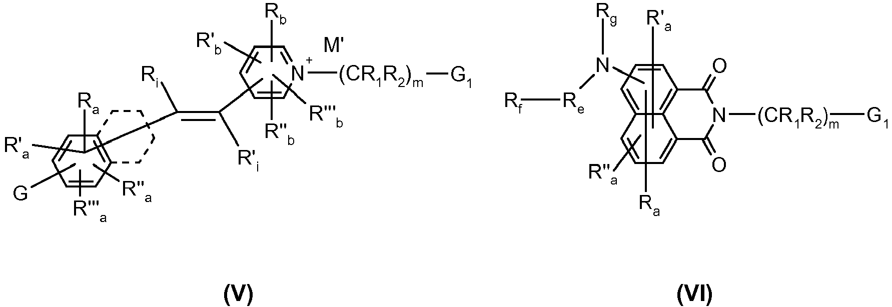 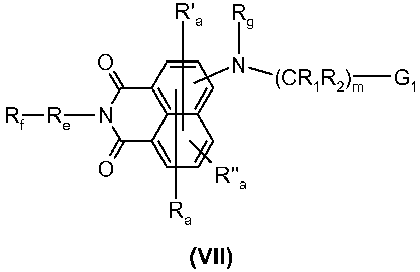 and also the organic or mineral acid or base salts thereof, the optical isomers, geometrical isomers and tautomers thereof, and the solvates thereof such as hydrates; in which formulae (V), **(VI)** and **(VII):**
- **R₁** and **R₂,** which may be identical or different, represent a hydrogen atom or a C₁-C₆ alkyl group; preferentially a hydrogen atom;
- **G₁** represents a hydrogen atom or a group chosen from NH₂ and OH preferably hydrogen;
- **Rₐ, R'ₐ, R"ₐ, R'''ₐ, R_{b}, R'_{b}, R"_{b},** and **R'''_{b},** which may be identical or different, represent a) a hydrogen atom, b) a halogen atom, a group from among: c) amino, d) (C₁-C₄)alkylamino, e) (C₁-C₄)dialkylamino, f) cyano, g) carboxyl -C(O)OH or carboxylate - C(O)O⁻, Q⁺, h) hydroxy -OH or alkoxide -O⁻Q⁺, i) (poly)halo(C₁-C₆)alkyl such as trifluoromethyl, j) acylamino, k) (C₁-C₆)alkoxy, I) (C₁-C₆)alkylthio, m) (poly)hydroxy(C₂-C₄)alkoxy, n) (C₁-C₆)alkylcarbonyloxy, o) (C₁-C₆)alkoxycarbonyl, p) (C₁-C₆)alkylcarbonylamino, q) acylamino, r) carbamoyl, s) (C₁-C₆)alkylsulfonylamino, t) aminosulfonyl, u) -SO₃H or sulfonate -SO₃⁻, Q⁺ or v) (C₁-C₆)alkyl optionally substituted with a group chosen from (C₁-C₆)alkoxy, hydroxyl, cyano, carboxyl, amino, (di)(C₁-C₄)alkylamino, or alternatively the two alkyl radicals borne by the nitrogen atom of the amino group form a 5- to 7-membered heterocycle optionally comprising another nitrogen or non-nitrogen heteroatom; particularly, **Rₐ, R'ₐ, R"ₐ, R'''ₐ, R_{b}, R'_{b} R"_{b}** and **R'''_{b}** represent a hydrogen or halogen atom or a (C₁-C₄)alkyl group, preferably a hydrogen atom;
- or alternatively two groups **Rₐ** and **R'ₐ; R_{b}** and **R'_{b},** borne by two adjacent carbon atoms, together form a benzo or indeno ring, a fused heterocycloalkyl or fused heteroaryl group; the benzo, indeno, heterocycloalkyl or heteroaryl ring being optionally substituted with a halogen atom, an amino, (C₁-C₄)alkylamino, (C₁-C₄)dialkylamino, nitro, cyano, carboxyl, hydroxyl or trifluoromethyl group, an acylamino, (C₁-C₄)alkoxy (poly)hydroxy(C₁-C₄)alkoxy, (C₁-C₄)alkylcarbonyloxy, (C₁-C₄)alkoxycarbonyl or (C₁-C₄)alkylcarbonylamino radical, an acylamino, carbamoyl or alkoxyalkylsulfonylamino radical, an aminosulfonyl radical, or a (C₁-C₆)alkyl radical optionally substituted with: a group chosen from (C₁-C₆)alkoxy, hydroxyl, cyano, carboxyl, amino, (C₁-C₄)alkylamino and (C₁-C₄)dialkylamino, or alternatively the two alkyl radicals borne by the nitrogen atom of the amino group form a 5- to 7-membered heterocycle optionally comprising another nitrogen or non-nitrogen heteroatom; preferentially, **Rₐ** and **R'ₐ** together form a benzo group;
- or alternatively, two groups Rᵢ and Rₐ; and/or a group R'ᵢ and R'ₐ together form a fused (hetero)cycloalkyl, preferentially cycloalkyl such as cyclohexyl;
- **R_{g}** represents a hydrogen atom, a (hetero)aryl(C₁-C₄)alkyl group or a (C₁-C₆)alkyl group that is optionally substituted; preferentially, R_{b} represents a hydrogen atom or a (C₁-C₃)alkyl or benzyl group;
- **Rₑ** represents a covalent bond, a linear or branched, optionally substituted (C₁-C₈)alkylene or (C₂-C₈)alkenylene hydrocarbon-based chain, preferably **Rₑ** represents an unsubstituted (C₁-C₆)alkylene;
- **R_{f}** represents a hydrogen atom, a (C₁-C₄)alkoxy group, an amino group R₃R₄N-, a quaternary ammonium group M', R₃R₄R₅N⁺- in which R₃, R₄ and R₅, which may be identical or different, represent a (C₁-C₄)alkyl group or R₃R₄N- represents an optionally substituted heteroaryl group, preferentially an optionally substituted imidazolyl group, or alternatively M', R₃R₄R₅N⁺- represents an optionally substituted cationic heteroaryl group, preferentially an imidazolinium group optionally substituted with a (C₁-C₃)alkyl group;
- **G** represents a group i) -NR_{c}R_{d}, ii) -OR with R representing a) a hydrogen atom, b) an optionally substituted, preferentially unsubstituted (C₁-C₆)alkyl group, c) an optionally substituted (hetero)aryl group, d) an optionally substituted (hetero)aryl(C₁-C₆)alkyl group such as benzyl, e) optionally substituted (hetero)cycloalkyl group, f) optionally substituted (hetero)cycloalkyl(C₁-C₆)alkyl group; according to a particular embodiment, G represents a group -NR_{c}R_{d}, according to another particular embodiment, G represents a (C₁-C₆)alkoxy group;
or alternatively when **G** represents -NR_{c}R_{d}, two groups R_{c} and R'ₐ and/or R_{d} and Rₐ together form a saturated heteroaryl or heterocycle, optionally substituted with one or more (C₁-C₆)alkyl groups, preferentially a 5- to 7-membered heterocycle containing one or two heteroatoms chosen from nitrogen and oxygen; more preferentially, the heterocycle is chosen from morpholinyl, piperazinyl, piperidyl and pyrrolidinyl groups;
- **R_{c}** and **R_{d},** which may be identical or different, represent a hydrogen atom or a group from among: a) optionally substituted (hetero)aryl such as phenyl, b) optionally substituted (hetero)aryl(C₁-C₄)alkyl, c) optionally substituted (hetero)cycloalkyl, d) optionally substituted (hetero)cycloalkyl(C₁-C₄)alkyl, f) (C₂-C₅)alkyl or g) (C₁-C₈)alkyl which is optionally substituted, preferably optionally substituted with a hydroxyl, carboxyl, carboxylate, sulfate or sulfonate group;
or alternatively two adjacent radicals **R_{c}** and **R_{d}** borne by the same nitrogen atom together form an optionally substituted heterocyclic or optionally substituted heteroaryl group;
- **Rᵢ** and **R'ᵢ,** which may be identical or different, represent a hydrogen atom or a (C₁-C₄)alkyl group;
- 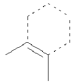 represents a (hetero)aryl group fused to the phenyl ring; or alternatively is absent from the phenyl; preferentially, when the ring is present, the ring is a benzo;
- **m** represents an integer between 1 and 18 inclusive, particularly an integer between 1 and 14 inclusive; preferentially an integer between 2 and 10 inclusive; more preferentially an integer between 3 and 8; more particularly an integer between 4 and 6;
- **M'** represents an anionic counterion, derived from a salt of an organic or mineral acid, or from an organic or mineral base that ensures the electrical neutrality of the molecule;
- **Q⁺** represents a cationic counterion, derived from a salt of an organic or mineral acid, or from an organic or mineral base that ensures the electrical neutrality of the molecule such as alkali metal, alkaline-earth metal or ammonium;
it being understood that when the molecule comprises a carboxylate, sulfonate or alkoxide group, then M' and Q⁺ may be absent to ensure the electrical neutrality of said molecule.

According to one embodiment, the fluorescent dyes of the invention are of formula (V) as defined previously.

According to a preferred embodiment, the fluorescent dyes of the invention are chosen from the styryl dyes of formula **(VIII)** below: 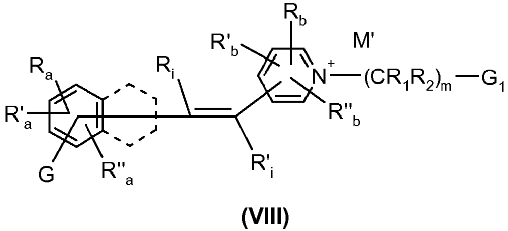 and also the organic or mineral acid or base salts thereof, the optical isomers, geometrical isomers and tautomers thereof, and the solvates thereof such as hydrates;
in which formula **(VIII) G, G₁, Rₐ, R'ₐ, R"ₐ, R_{b}, R'_{b}, R"_{b}, Rᵢ, R'ᵢ, R₁, R₂** and **m** are as defined previously for **(V)**.

In particular, the dye(s) of the invention are chosen from those of formula **(VIII)** for which:
- **R₁** and **R₂,** which may be identical or different, represent a hydrogen atom;
- **Rᵢ** and **R_{i'},** which may be identical or different, represent a hydrogen atom or a (C₁-C₄)alkyl group, preferably hydrogen;
- **Rₐ, R'ₐ** and **R"ₐ,** which may be identical or different, represent a hydrogen atom, a halogen atom such as fluorine, or an -OH, -O⁻Q⁺, (C₁-C₆)alkoxy, nitro, or cyano group, with Q⁺ as defined previously;
- **R_{b}, R'_{b}** and **R"_{b},** which may be identical or different, represent a hydrogen atom or a (C₁-C₆)alkyl group;
- or alternatively two contiguous radicals **R_{b}** and **R'_{b}** form, together with the carbon atoms that bear them, a benzo group that is condensed or fused to the pyridinium group, said benzo group possibly being substituted; preferably, said benzo group is unsubstituted;
- **G** represents a group -NR_{c}R_{d} or (C₁-C₆)alkoxy group which is optionally substituted, preferentially unsubstituted; according to a particular embodiment, G represents a group -NR_{c}R_{d}, according to another particular embodiment, G represents a (C₁-C₆)alkoxy group;
- **G₁** represents a hydrogen atom or an OH or NH₂ group, preferably hydrogen;
- **Rᵢ** and **R'ᵢ,** which may be identical or different, represent a hydrogen atom or a (C₁-C₄)alkyl group;
- 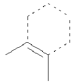 represents an aryl or heteroaryl group fused to the phenyl ring; or alternatively is absent from the phenyl ring; preferentially, when the ring is present, the ring is a benzo;
- **m** represents an integer between 1 and 18 inclusive; particularly an integer between 2 and 16 inclusive; preferentially an integer between 3 and 10; more preferentially an integer between 4 and 6;
- **R_{c}** and **R_{d},** which may be identical or different, represent a hydrogen atom, a (C₂-C₄)alkyl group or a substituted (C₁-C₈)alkyl group, preferably (C₂-C₄)alkyl substituted in particular with one or more groups chosen from i) cyano, ii) (C₁-C₃)alkoxy, iii) hydroxyl and iv) (C₁-C₃)alkylcarbonyl, preferably with one or more hydroxyl groups; and
- **M'** represents an anionic counterion, derived from a salt of an organic or mineral acid, or from an organic or mineral base that ensures the electrical neutrality of the molecule;
it being understood that when the molecule comprises an alkoxide group, then M' and Q⁺ may be absent to ensure the electrical neutrality of said molecule.

Preferably, the fluorescent dye(s) of the invention are chosen from the styryl dyes of formula **(IX)** below: 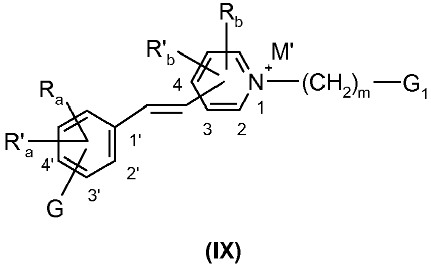 and also the organic or mineral acid salts thereof, the optical isomers, geometrical isomers and tautomers thereof, and the solvates thereof such as hydrates;
in which formula **(IX) G, G₁, Rₐ, R'ₐ, R_{b}, R'_{b}** and **m** are as defined previously.

According to a particular embodiment, the group G is in the para position relative to the - CH=CH- group, i.e. in position 4' of the phenyl group. According to another particular embodiment of the invention, the group G is in the ortho position relative to the -CH=CH-group, i.e. in position 2' of the phenyl group. According to one embodiment, the -CH=CH-group is in the para position of the pyridinium group, i.e. in position 4.

According to another advantageous variant, the -CH=CH- group is in the ortho position of the pyridinium group, i.e. in position 2.

According to a preferred embodiment of the invention, the fluorescent dyes of the invention (b) are chosen from the compounds of formulae **(X)** and **(XI)** below: 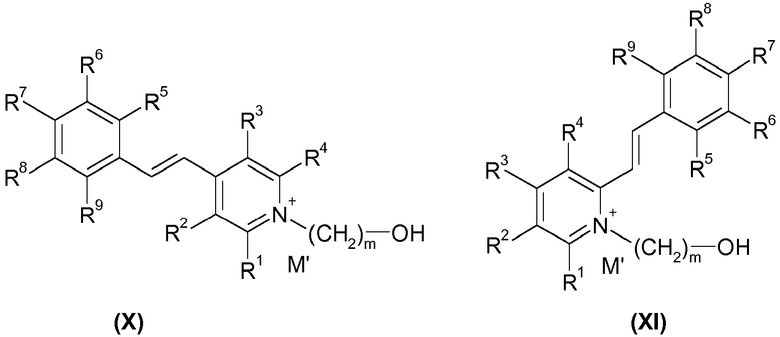 and also the organic or mineral acid salts thereof, the optical isomers, geometrical isomers and tautomers thereof, and the solvates thereof such as hydrates; in which formulae **(X)** and **(XI):**
- **R¹, R², R³** and **R⁴,** which may be identical or different, represent a hydrogen atom or a (C₁-C₆)alkyl group; preferably, **R²** and **R³** represent a hydrogen atom and **R¹** and **R⁴,** which may be identical or different, represent a hydrogen atom or a (C₁-C₄)alkyl group;
- **R⁵, R⁶, R⁷, R⁸** and **R⁹,** which may be identical or different, represent i) a hydrogen atom or ii) a halogen atom such as Cl, Br or F, iii) a group OR in which R represents a hydrogen atom or Q⁺ as described previously, or a (C₁-C₃)alkyl group, iv) aryl such as benzene, v) aryl(C₁-C₃)alkyl such as benzyl, vi) cyano, vii) nitro, viii) (C₁-C₃)alkylthio, ix) amino NR¹⁰R¹¹ with R¹⁰ and R¹¹, which may be identical or different, representing a) a hydrogen atom, b) a (C₂-C₄)alkyl group or c) a substituted (C₁-C₈)alkyl group, preferably (C₂-C₄)alkyl optionally substituted with one or more groups chosen from:
   - cyano,
   - (C₁-C₃)alkoxy,
   - hydroxyl, and
   - (C₁-C₃)alkylcarbonyl;
in particular, **R¹⁰** and **R¹¹,** which may be identical or different, represent a hydrogen atom or a (C₁-C₆)alkyl group substituted with one or more hydroxyl, cyano or (C₁-C₃)alkylcarbonyl groups such as hydroxyethyl;
- **m** represents an integer between 1 and 18 inclusive; particularly an integer between 2 and 16 inclusive; preferentially an integer between 3 and 10; more preferentially an integer between 4 and 6;
- **M'** represents an anionic counterion as defined previously;

it being understood that when the molecule comprises an alkoxide group, then M' and Q⁺ may be absent to ensure the electrical neutrality of said molecule.

According to one embodiment of the invention, the fluorescent dye(s) (b) are of formula **(X)** with:

| **R¹** | **R²** | **R³** | **R⁴** | **R⁵** | **R⁶** | **R⁷** | **R⁸** | **R⁹** | **m** |
|---|---|---|---|---|---|---|---|---|---|
| H | H | H | H | H | H | H | H | H | 1 |
| H | H | H | H | H | t-Bu | OH | t-Bu | H | 5 |
| H | H | H | H | H | t-Bu | OH | t-Bu | H | 5 |
| H | H | H | H | H | H | NH₂ | H | H | 1 |
| H | H | H | H | H | H | NH₂ | H | OCH₃ | 1 |
| H | H | H | H | H | H | OH | Br | H | 5 |
| H | H | H | H | H | OCH₃ | OH | OCH₃ | H | 5 |
| H | H | H | H | Cl | H | OH | H | Cl | 5 |
| H | H | H | H | H | H | OH | H | H | 10 |
| H | H | H | H | H | OCH₃ | OH | OCH₃ | H | 10 |
| H | H | H | H | H | t-Bu | OH | t-Bu | H | 10 |
| H | H | H | H | H | H | N(CH₂CH₃)₂ | H | H | 1 |
| H | H | H | H | H | H | N(CH₂CH₃)₂ | H | H | 1 |
| H | H | H | H | H | H | N(CH₂CH₃)₂ | H | H | 1 |
| H | H | H | H | H | t-Bu | OH | t-Bu | H | 10 |
| H | H | H | H | H | H | N(CH₂CH₃)CH₂CH₂OH | H | H | 1 |
| H | H | H | H | H | H | N(CH₂CH₃)₂ | H | H | 1 |
| H | H | H | H | H | H | N(CH₂CH₃)₂ | H | H | 1 |
| H | H | H | H | H | H | N(CH₂CH₃)₂ | H | H | 1 |
| H | H | H | H | H | H | N(CH₂CH₃)₂ | H | H | 1 |
| H | H | H | H | H | H | N(CH₂CH₃)₂ | H | H | 1 |
| H | H | H | H | H | H | N(CH₂CH₃)₂ | H | H | 1 |
| H | H | H | H | H | H | N(CH₂CH₃)₂ | H | H | 1 |
| H | H | H | H | H | H | N(CH₂CH₃)₂ | H | H | 1 |
| H | H | H | H | H | H | N(CH₂CH₃)₂ | H | H | 1 |
| H | H | H | H | H | H | n-C₆H₁₃ | H | H | 1 |
| H | H | H | H | H | H | N(CH₂CH₂OH)₂ | H | H | 2 |
| H | H | H | H | H | H | N(n-Bu)₂ | H | H | 2 |
| H | H | H | H | H | OCH₃ | OH | H | H | 10 |
| H | H | H | H | H | H | OC₂H₅OH | H | H | 1 |
| H | H | H | H | H | H | OH | H | H | 1 |
| H | H | benzo | | H | H | H | H | H | 1 |
| H | H | benzo | | H | H | N(CH₂CH₂OH)₂ | H | H | 1 |
| H | H | benzo | | H | H | N(CH₂CH₂OH)₂ | H | H | 1 |

and also the organic or mineral acid or base salts thereof, the optical isomers, geometrical isomers and tautomers thereof, and the solvates thereof such as hydrates.

According to one embodiment, the fluorescent dye(s) (b) are of formula **(XI)** with:

| **R¹** | **R²** | **R³** | **R⁴** | **R⁵** | **R⁶** | **R⁷** | **R⁸** | **R⁹** | **m** |
|---|---|---|---|---|---|---|---|---|---|
| CH₃ | H | H | H | OH | H | OCH₃ | H | H | 2 |
| CH₃ | H | H | H | H | H | OCH₃ | OCH₂-Ph | H | 2 |
| CH₃ | H | H | H | H | H | H | H | OCH₃ | 2 |
| CH₃ | H | H | H | F | H | H | H | H | 2 |
| CH₃ | H | H | H | H | H | H | OPh | H | 2 |
| CH₃ | H | H | H | H | H | N(CH₂CH₂OAC)₂ | H | H | 2 |
| CH₃ | H | H | H | OCH₃ | H | OCH₃ | OCH₃ | H | 2 |
| CH₃ | H | H | H | H | H | H | CH₃ | H | 2 |
| CH₃ | H | H | H | H | H | OH | H | H | 2 |
| CH₃ | H | H | H | H | OCH₃ | OCH₃ | OCH₃ | H | 2 |
| CH₃ | H | H | H | H | OCH₃ | OH | OH | H | 2 |
| CH₃ | H | H | H | H | H | OCH₃ | OCH₃ | OCH₃ | 2 |
| CH₃ | H | H | H | H | H | H | OCH₃ | OH | 2 |
| CH₃ | H | H | H | H | H | N(n-butyl)₂ | H | H | 2 |
| CH₃ | H | H | H | H | OCH₃ | OCH₃ | H | H | 2 |
| CH₃ | H | H | H | H | H | H | H | H | 2 |
| CH₃ | H | H | H | H | H | i-propyl | H | H | 2 |
| H | H | H | H | H | H | OH | H | H | 6 |
| H | H | H | H | H | OCH₃ | OCH₃ | OCH₃ | H | 6 |
| H | H | H | H | OH | H | OCH₃ | H | H | 2 |
| H | H | H | H | OCH₃ | H | H | OCH₃ | OCH₃ | 2 |
| H | H | H | H | H | H | H | H | Br | 2 |
| H | H | H | H | H | H | OH | H | H | 2 |
| H | H | H | H | OCH₃ | OCH₃ | OCH₃ | H | H | 6 |
| H | H | H | H | OH | OCH₃ | H | H | H | 6 |
| H | H | H | H | H | OCH₃ | OCH₃ | H | H | 6 |
| H | H | H | H | H | OCH₃ | OCH₃ | H | H | 2 |
| H | H | H | H | H | H | C(O)-OH | H | H | 6 |
| H | H | H | H | H | H | C(O)-OH | H | H | 2 |
| H | H | H | H | H | H | i-propyl | H | H | 2 |
| H | H | H | H | H | H | N(CH₃)CH₂CH₂CN | H | H | 2 |
| H | H | H | H | H | H | OCH₃ | OCH₂Ph | H | 2 |
| H | H | H | H | H | H | H | OPh | H | 2 |
| H | H | H | H | H | H | N(CH₂CH₂C(O)CH₃)₂ | H | H | 2 |
| H | H | H | H | OH | H | OCH₃ | H | H | 6 |
| H | H | H | H | H | OCH₃ | OCH₃ | OCH₃ | H | 2 |
| H | H | H | H | OCH₃ | H | OCH₃ | OCH₃ | H | 6 |
| H | H | H | H | H | H | H | CH₃ | H | 2 |
| H | H | H | H | H | H | N(CH₃)CH₂CH₂OH | H | H | 2 |

and also the organic or mineral acid or base salts thereof, the geometrical isomers and tautomers thereof, and the solvates thereof such as hydrates.

Another subject of the invention is novel dyes chosen from those of formulae **(X')** and **(XI')** below: 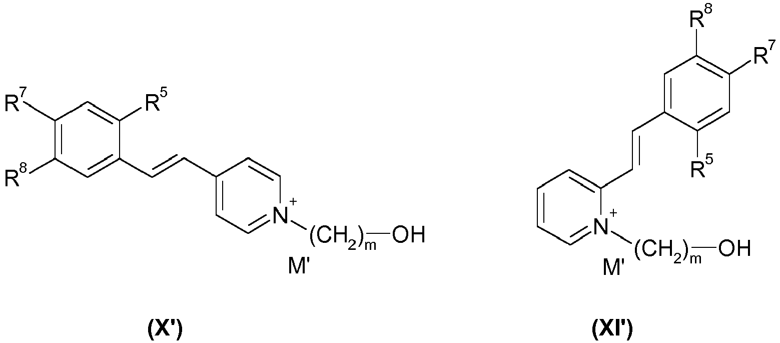 in which formulae **(X')** and **(XI') R⁵, R⁷, R⁸** and **m** are as defined previously for **(X)** and **(XI),** in particular:
- **R⁵** and **R⁸,** which may be identical or different, represent a hydrogen atom or a (C₁-C₄)alkoxy group such as methoxy;
- **R⁷** represents a (C₁-C₄)alkoxy group or NR¹⁰R¹¹ with R¹⁰ representing a) a hydrogen atom, or b) a (C₁-C₆)alkyl group substituted with one or more groups chosen from i) cyano, ii) (C₁-C₃)alkoxy, iii) hydroxyl and iv) (C₁-C₃)alkylcarbonyl and R¹¹ representing a) a (C₂-C₅)alkyl group substituted with one or more groups chosen from i) cyano, ii) (C₁-C₃)alkoxy, iii) hydroxyl and iv) (C₁-C₃)alkylcarbonyl;
in particular, NR¹⁰R¹¹ represents a (C₂-C₄)alkyl group, a (di)hydroxy(C₂-C₄)alkylamino or hydroxy(C₂-C₄)alkyl((C₁-C₄)alkyl)amino group;
- **m** represents an integer between 1 and 18 inclusive; particularly an integer between 2 and 16 inclusive; preferentially an integer between 3 and 10; more preferentially an integer between 4 and 6; and
- **M'** represents an anionic counterion, derived from a salt of an organic or mineral acid, or from an organic or mineral base that ensures the electrical neutrality of the molecule.

According to one embodiment, the fluorescent dye(s) are of formula **(X')** or **(XI')** with:

| **R⁵** | **R⁷** | **R⁸** | **m** |
|---|---|---|---|
| H | N(CH₂CH₂OH)₂ | H | 2 |
| H | N(CH₂CH₂OH)₂ | H | 3 |
| H | N(CH₂CH₂OH)₂ | H | 4 |
| H | N(CH₂CH₂OH)₂ | H | 5 |
| H | N(CH₂CH₂OH)₂ | H | 6 |
| H | N(CH₂CH₂OH)₂ | H | 8 |
| H | N(CH₂CH₂OH)₂ | H | 10 |
| H | N(CH₂CH₂OH)₂ | H | 12 |
| H | N(CH₂CH₂OH)₂ | H | 14 |
| H | N(CH₂CH₂OH)₂ | H | 16 |
| H | CH₃CH₂N(CH₂CH₂OH) | H | 2 |
| H | CH₃CH₂N(CH₂CH₂OH) | H | 4 |

and

| **R⁵** | **R⁷** | **R⁸** | **m** |
|---|---|---|---|
| OCH₃ | OCH₃ | OCH₃ | 2 |
| OCH₃ | OCH₃ | OCH₃ | 3 |
| OCH₃ | OCH₃ | OCH₃ | 3 |
| OCH₃ | OCH₃ | OCH₃ | 4 |
| OCH₃ | OCH₃ | OCH₃ | 5 |
| OCH₃ | OCH₃ | OCH₃ | 8 |
| OCH₃ | OCH₃ | OCH₃ | 10 |
| OCH₃ | OCH₃ | OCH₃ | 12 |
| OCH₃ | OCH₃ | OCH₃ | 14 |
| OCH₃ | OCH₃ | OCH₃ | 16 |

and also the organic or mineral acid or base salts thereof, the geometrical isomers and tautomers thereof, and the solvates thereof such as hydrates.

According to a preferred embodiment of the invention, the fluorescent dyes (b) of the invention are chosen from the compounds of formulae **(XII)** and **(XIII)** below: 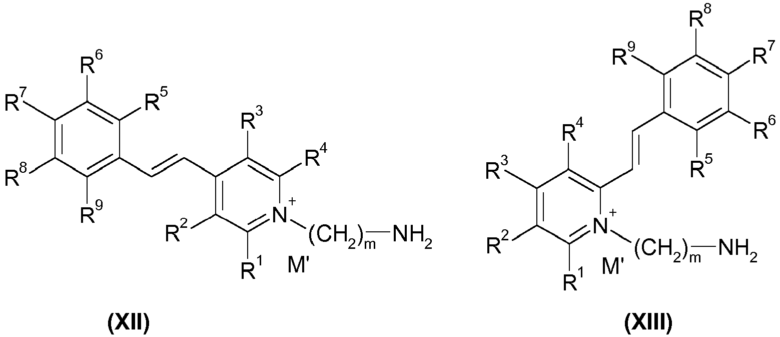 and also the organic or mineral acid salts thereof, the optical isomers, geometrical isomers and tautomers thereof, and the solvates thereof such as hydrates; in which formulae **(XII)** and **(XIII):**
- **R¹, R², R³** and **R⁴,** which may be identical or different, represent a hydrogen atom or a (C₁-C₆)alkyl group; preferably, **R²** and **R³** represent a hydrogen atom and **R¹** and **R⁴,** which may be identical or different, represent a hydrogen atom or a (C₁-C₄)alkyl group;
- **R⁵, R⁶, R⁷, R⁸** and **R⁹,** which may be identical or different, represent i) a hydrogen atom or ii) a halogen atom such as Cl, Br or F, iii) a group OR in which R represents a hydrogen atom or Q⁺ as described previously, or a (C₁-C₃)alkyl group, iv) aryl such as benzene, v) an aryl(C₁-C₃)alkyl group such as benzyl, vi) cyano, vii) nitro, viii) (C₁-C₃)alkylthio, ix) amino NR¹⁰R¹¹ with R¹⁰ and R¹¹, which may be identical or different, representing a) a hydrogen atom or b) a (C₁-C₈)alkyl group optionally substituted with one or more groups chosen from:
   - cyano,
   - (C₁-C₃)alkoxy,
   - hydroxyl, and
   - (C₁-C₃)alkylcarbonyl;
in particular, **R¹⁰** and **R¹¹**, which may be identical or different, represent a hydrogen atom or a (C₁-C₆)alkyl group substituted with one or more hydroxyl, cyano or (C₁-C₃)alkylcarbonyl groups such as methyl, ethyl, butyl, isobutyl, cyanoethyl, methylcarbonylethyl, or hydroxyethyl;
- **m** represents an integer between 1 and 18 inclusive; particularly an integer between 2 and 16 inclusive; preferentially an integer between 3 and 10; more preferentially an integer between 4 and 6;
- **M'** represents an anionic counterion derived from salts of organic or mineral acids preferably originating from **Y;**

it being understood that when the molecule comprises an alkoxide group, then M' and Q⁺ may be absent to ensure the electrical neutrality of said molecule.

According to one embodiment of the invention, the dye(s) are of formula **(XII)** with:

| **R¹** | **R²** | **R³** | **R⁴** | **R⁵** | **R⁶** | **R⁷** | **R⁸** | **R⁹** | **m** |
|---|---|---|---|---|---|---|---|---|---|
| H | H | H | H | H | H | OCH₃ | OCH₃ | OCH₃ | 2 |
| H | H | H | H | OH | OCH₃ | H | H | H | 2 |
| H | H | H | H | H | H | H | H | H | 2 |
| H | H | H | H | H | OCH₃ | OCH₃ | H | H | 2 |
| H | H | H | H | OH | H | OH | H | H | 6 |
| H | H | H | H | OCH₃ | H | OCH₃ | OCH₃ | H | 6 |
| H | H | H | H | H | H | OH | H | H | 6 |
| H | H | H | H | OCH₃ | H | H | H | F | 2 |
| H | H | H | H | H | H | C(O)-OH | H | H | 2 |
| H | H | H | H | H | H | Isopropyl | H | H | 2 |
| H | H | H | H | H | H | N(CH₂CH₂C(O)CH₃)₂ | H | H | 2 |
| H | H | H | H | OH | H | OCH₃ | H | H | 2 |
| H | H | H | H | H | H | OH | H | H | 2 |
| H | H | H | H | H | OCH₃ | OH | OH | H | 2 |
| H | H | H | H | H | CH₃ | OCH₂Ph | CH₃ | H | 2 |
| H | H | H | H | H | OCH₃ | OCH₃ | OCH₃ | H | 2 |
| H | H | H | H | H | OCH₃ | OCH₃ | OCH₃ | H | 6 |
| H | H | H | H | H | H | N(CH₃)₂ | H | H | 6 |
| H | H | H | H | H | H | OCH₃ | OCH₃ | OCH₃ | 6 |
| H | H | H | H | H | H | Phenyl | H | H | 6 |
| H | H | H | H | H | OCH₃ | OCH₃ | H | H | 6 |
| H | H | H | H | H | H | C(O)-OH | H | H | 6 |
| H | H | H | H | H | H | N(n-Butyl)₂ | H | H | 2 |
| H | H | H | H | H | H | OCH₃ | OCH₃ | H | 3 |
| H | H | H | H | H | H | OCH₃ | OCH₃ | H | 2 |
| H | H | H | H | H | H | OCH₃ | OCH₃ | H | 5 |
| H | H | H | H | H | H | OCH₃ | H | H | 3 |
| H | H | H | H | H | H | N(CH₃)₂ | H | H | 3 |
| H | H | H | H | H | H | H | H | H | 3 |

and also the organic or mineral acid or base salts thereof, the optical isomers, geometrical isomers and tautomers thereof, and the solvates thereof such as hydrates.

According to one embodiment, the dye(s) are of formula **(XIII)** with:

| **R¹** | **R²** | **R³** | **R⁴** | **R⁵** | **R⁶** | **R⁷** | **R⁸** | **R⁹** | **m** |
|---|---|---|---|---|---|---|---|---|---|
| CH₃ | H | H | H | OH | H | OCH₃ | H | H | 2 |
| CH₃ | H | H | H | H | H | OCH₃ | OCH₂-Ph | H | 2 |
| CH₃ | H | H | H | H | H | H | H | OCH₃ | 2 |
| CH₃ | H | H | H | F | H | H | H | H | 2 |
| CH₃ | H | H | H | H | H | H | OPh | H | 2 |
| CH₃ | H | H | H | H | H | N(CH₂CH₂OAc)₂ | H | H | 2 |
| CH₃ | H | H | H | OCH₃ | H | OCH₃ | OCH₃ | H | 2 |
| CH₃ | H | H | H | H | H | H | CH₃ | H | 2 |
| CH₃ | H | H | H | H | H | OH | H | H | 2 |
| CH₃ | H | H | H | H | OCH₃ | OCH₃ | OCH₃ | H | 2 |
| CH₃ | H | H | H | H | OCH₃ | OH | OH | H | 2 |
| CH₃ | H | H | H | H | H | OCH₃ | OCH₃ | OCH₃ | 2 |
| CH₃ | H | H | H | H | H | H | OCH₃ | OH | 2 |
| CH₃ | H | H | H | H | H | N(n-butyl)₂ | H | H | 2 |
| CH₃ | H | H | H | H | OCH₃ | OCH₃ | H | H | 2 |
| CH₃ | H | H | H | H | H | H | H | H | 2 |
| CH₃ | H | H | H | H | H | i-propyl | H | H | 2 |
| H | H | H | H | H | H | OH | H | H | 6 |
| H | H | H | H | H | OCH₃ | OCH₃ | OCH₃ | H | 6 |
| H | H | H | H | OH | H | OCH₃ | H | H | 2 |
| H | H | H | H | OCH₃ | H | H | OCH₃ | OCH₃ | 2 |
| H | H | H | H | H | H | H | H | Br | 2 |
| H | H | H | H | H | H | OH | H | H | 2 |
| H | H | H | H | H | H | N(CH₃)₂ | H | H | 6 |
| H | H | H | H | OCH₃ | OCH₃ | OCH₃ | H | H | 6 |
| H | H | H | H | OH | OCH₃ | H | H | H | 6 |
| H | H | H | H | H | OCH₃ | OCH₃ | H | H | 6 |
| H | H | H | H | H | OCH₃ | OCH₃ | H | H | 2 |
| H | H | H | H | H | H | C(O)-OH | H | H | 6 |
| H | H | H | H | H | H | C(O)-OH | H | H | 2 |
| H | H | H | H | H | H | i-propyl | H | H | 2 |
| H | H | H | H | H | H | N(CH₃)CH₂CH₂CN | H | H | 2 |
| H | H | H | H | H | H | OCH₃ | OCH₂Ph | H | 2 |
| H | H | H | H | H | H | H | OPh | H | 2 |
| H | H | H | H | H | H | N(CH₂CH₂C(O)CH₃)₂ | H | H | 2 |
| H | H | H | H | OH | H | OCH₃ | H | H | 6 |
| H | H | H | H | H | OCH₃ | OCH₃ | OCH₃ | H | 2 |
| H | H | H | H | OCH₃ | H | OCH₃ | OCH₃ | H | 6 |
| H | H | H | H | H | H | H | CH₃ | H | 2 |
| H | H | H | H | H | H | N(CH₃)CH₂CH₂OH | H | H | 2 |
| CH₃ | H | H | H | H | H | N(CH₃)₂ | H | H | 2 |

and also the organic or mineral acid or base salts thereof, the geometrical isomers and tautomers thereof, and the solvates thereof such as hydrates.

More particularly, the fluorescent dye (b) of the invention are chosen from those of formulae **(XII')** and **(XIII')** below: 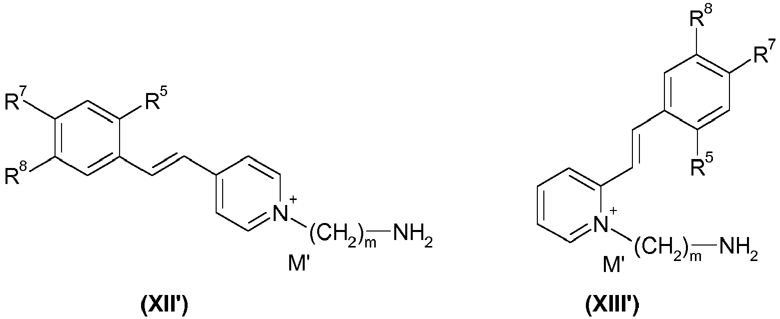 in which formulae **(XII)** and **(XIII) R⁵, R⁷, R⁸** and **m** are as defined previously for **(X)** and **(XI),** in particular:
- **R⁵** and **R⁸,** which may be identical or different, represent a hydrogen atom or a (Ci-C₄)alkoxy group such as methoxy;
- **R⁷** represents a (C₁-C₄)alkoxy group or NR¹⁰R¹¹ with R¹⁰ and R¹¹, which may be identical or different, representing a) a hydrogen atom, or b) a (C₁-C₈)alkyl group optionally substituted with one or more groups chosen from i) cyano, ii) (C₁-C₃)alkoxy, iii) hydroxyl, and iv) (C₁-C₃)alkylcarbonyl; in particular, **R¹⁰** and **R¹¹,** which may be identical or different, represent a hydrogen atom or a (C₁-C₆)alkyl group optionally substituted with one or more groups from the following: hydroxyl, cyano or (C₁-C₃)alkylcarbonyl such as methyl, ethyl, butyl, isobutyl, cyanoethyl, methylcarbonylethyl or hydroxyethyl; preferably, **R¹⁰** and **R¹¹,** which may be identical or different, represent a (C₁-C₆)alkyl group optionally substituted with one or more hydroxyl groups such as hydroxyethyl;
- **m** represents an integer between 1 and 18 inclusive; particularly an integer between 2 and 16 inclusive; preferentially an integer between 3 and 10; more preferentially an integer between 4 and 6; and
- **M'** represents an anionic counterion, derived from a salt of an organic or mineral acid, or from an organic or mineral base that ensures the electrical neutrality of the molecule.

According to one embodiment, the fluorescent dye(s) (b) of the invention are of formula **(XII')** or **(XIII')** with:

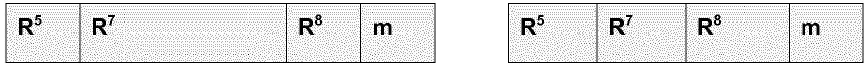

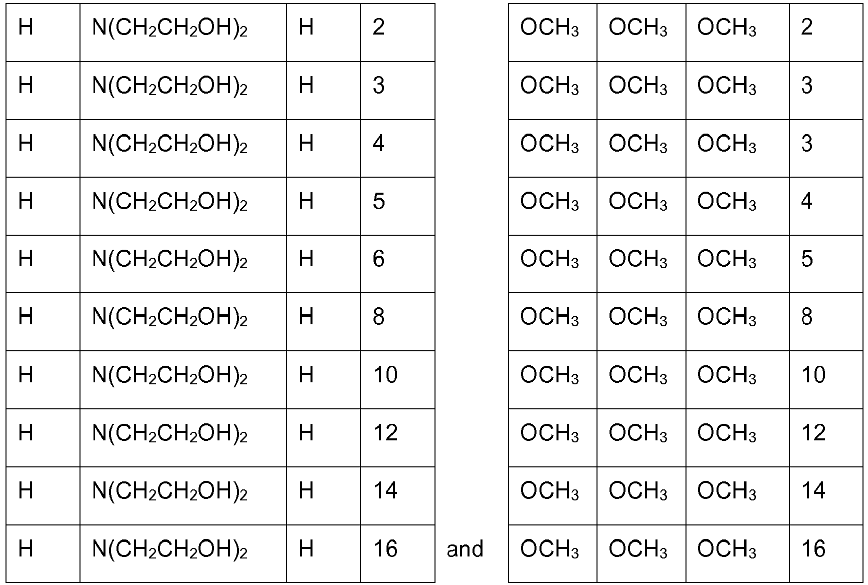

and also the organic or mineral acid or base salts thereof, the geometrical isomers and tautomers thereof, and the solvates thereof such as hydrates.

More particularly, the fluorescent dyes (b) of the invention are chosen from those of formula **(V), (VIII)** or **(IX)** as defined previously in which G represents a hydrogen atom.

More particularly, the fluorescent dyes (b) of the invention are chosen from those of formulae **(XIV)** and **(XV)** below: 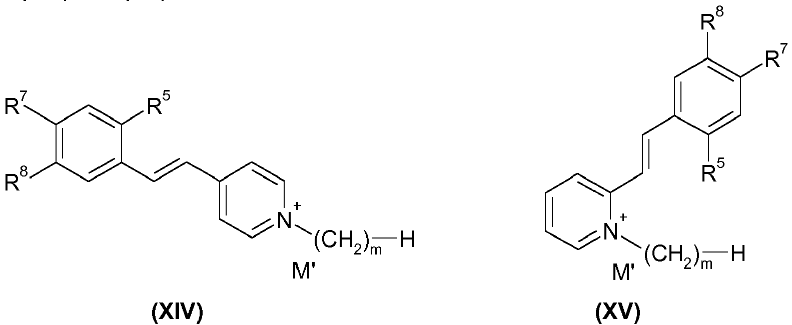 in which formulae **(XIV)** and **(XV) R⁵, R⁷, R⁸** and **m** are as defined previously for **(X)** and **(XI),** in particular:
- **R⁵** and **R⁸,** which may be identical or different, represent a hydrogen atom or a (C₁-C₄)alkoxy group such as methoxy, preferably, R⁵ and R⁸ represent a hydrogen atom;
- **R⁷** represents a (C₁-C₄)alkoxy group or NR¹⁰R¹¹ with R¹⁰ and R¹¹, which may be identical or different, representing a) a hydrogen atom, or b) a (C₁-C₈)alkyl group optionally substituted with one or more groups chosen from i) hydroxyl, ii) R-Z-C(X)-Y- with X, Y and Z representing an oxygen or sulfur atom or N(R'), or alternatively X and/or Z represent a bond, R and R', which may be identical or different, represent a hydrogen atom or a (C₁-C₆)alkyl group, preferably, X represents an oxygen atom, iii) sulfonic SO₃H, iv) sulfonate SO₃⁻, Q⁺, v) carboxylate C(O)O⁻, Q⁺ with Q⁺ representing a cationic counterion such as an alkali metal or alkaline-earth metal; in particular, R⁷ represents a group NR¹⁰R¹¹ with R¹⁰ and R¹¹, which may be identical or different, representing a) a hydrogen atom, or b) a (C₁-C₆)alkyl group optionally substituted with one or more groups chosen from i) hydroxyl, ii) carboxyl, iii) carboxylate, iv) sulfonic, and v) sulfonate, more particularly chosen from identical or different groups representing a) a hydrogen atom, or b) a (C₁-C₆)alkyl group optionally substituted with one or more groups chosen from i) hydroxyl, ii) carboxyl, and iii) carboxylate;
- **m** represents an integer between 1 and 18 inclusive; particularly an integer between 1 and 6 inclusive; preferentially an integer between 1 and 4; more preferentially an integer between 1 and 2; and
- **M'** represents an anionic counterion, derived from a salt of an organic or mineral acid, or from an organic or mineral base that ensures the electrical neutrality of the molecule;
- it being understood that when the molecule comprises a sulfonate or carboxylate group, then M' and Q⁺ may be absent to ensure the electrical neutrality of said molecule.

According to one embodiment, the fluorescent dye(s) (b) of the invention are chosen from the following compounds, the geometrical isomers thereof, the tautomers thereof, the solvates thereof, and mixtures thereof:

| | |
|---|---|
| 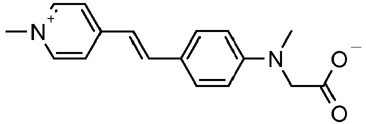 | 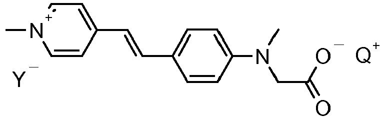 |
| **(d)** | **(d')** |
| 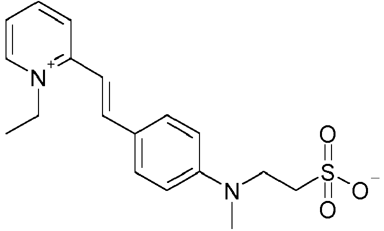 | 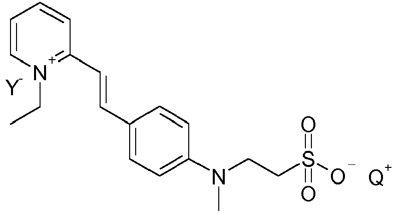 |
| **(e)** | **(e')** |
| 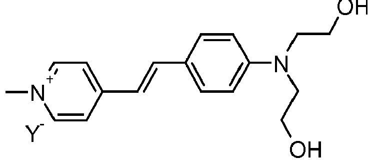 | |
| **(f)** | |
| 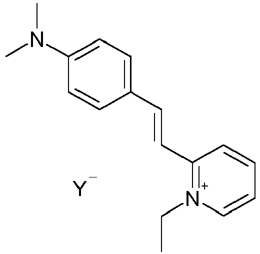 | |
| **(g)** | |
| 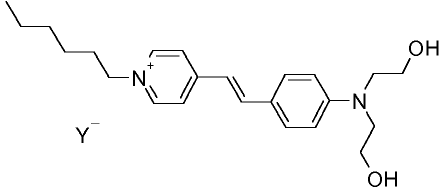 | |
| **(h)** | |

with Y- representing an anionic counterion and Q⁺ representing a cationic counterion as defined previously.

### The oxidizing agents

The process for dyeing keratin fibres according to the present invention may optionally also comprise the application to said keratin fibres of one or more oxidizing agents.

Preferably, the oxidizing agent(s) are chosen from chemical oxidizing agents.

The term "chemical oxidizing agent" means an oxidizing agent other than atmospheric oxygen.

More particularly, the chemical oxidizing agent(s) are chosen from hydrogen peroxide, hydrogen peroxide-generating systems, urea peroxide, alkali metal bromates or ferricyanides, peroxygenated salts, for instance persulfates, perborates, peracids and precursors thereof and percarbonates of alkali metals or alkaline-earth metals, and mixtures thereof.

Preferably, the chemical oxidizing agent(s) are chosen from hydrogen peroxide and hydrogen peroxide-generating systems.

According to a preferred embodiment, the hydrogen peroxide-generating system(s) are chosen from urea peroxide, polymeric complexes that can release hydrogen peroxide, chosen from polyvinylpyrrolidone/H₂O₂; oxidases; perborates; and percarbonates.

Preferably, the chemical oxidizing agent is hydrogen peroxide, and more preferentially aqueous hydrogen peroxide solution.

The chemical oxidizing agent(s) are advantageously applied in the form of an aqueous solution of which the content of chemical oxidizing agents is preferably between 0.05% and 5% by weight and more preferentially between 0.1% and 2% by weight, relative to the total weight of the aqueous solution.

According to a preferred embodiment of the invention, the dyeing process does not use any oxidizing agent. According to a preferred embodiment of the invention, the cosmetic composition comprising ingredients (a) and (b) does not comprise any oxidizing agent.

### The reducing agents

The process for dyeing keratin fibres according to the present invention may optionally also comprise the application to said keratin fibres of one or more reducing agents. The reducing agent(s) that are useful in the present invention are advantageously chosen from the compounds of formula **(XVI)** below, and also the addition salts thereof, and mixtures thereof:

**H(X)_{q}(R₁₀)ₜ** **(XVI)**

in which compound of formula **(XVI):**
- X represents P, S or SO₂,
- q represents an integer equal to 0 or 1,
- t represents an integer equal to 1 or 2, and
- R represents a linear or branched, saturated or unsaturated C₁ to C₂₀ alkyl radical, optionally interrupted with a heteroatom, and/or optionally substituted with one or more radicals chosen from hydroxyl, halo, amine, carboxyl, ((C₁-C₃₀)alkoxy)carbonyl, amido, ((C₁-C₃₀)alkyl)aminocarbonyl, (C₁-C₃₀)acyl)amino, mono- or dialkylamino, and mono- or dihydroxylamino radicals.

Preferably, the reducing agent(s) are chosen from thioglycolic acid, thiolactic acid, glyceryl monothioglycolate, cysteamine, N-acetylcysteamine, N-propionylcysteamine, cysteine, N-acetylcysteine, thiomalic acid, pantetheine, 2,3-dimercaptosuccinic acid, N-(mercaptoalkyl)-ω-hydroxyalkylamides, N-mono- or N,N-dialkylmercapto-4-butyramides, aminomercaptoalkylamides, N-(mercaptoalkyl)succinamic acid and N-(mercaptoalkyl)succinimide derivatives, alkylamino mercaptoalkylamides, the azeotropic mixture of 2-hydroxypropyl thioglyconate and of (2-hydroxy-1-methyl)ethyl thioglycolate, mercaptoalkylaminoamides, N-mercaptoalkylalkanediamides and formamidinesulfinic acid derivatives, salts thereof, and mixtures thereof.

The chemical reducing agent(s) are advantageously applied in the form of an aqueous solution of which the content of chemical reducing agents is preferably between 0.01% and 10% by weight and more preferentially between 0.1% and 5% by weight, relative to the total weight of the aqueous solution.

According to a preferred embodiment of the invention, the dyeing process does not use any reducing agent. According to a preferred embodiment of the invention, the cosmetic composition comprising ingredients (a) and (b) does not comprise any reducing agent.

### The cosmetic medium and the solvents

The anthraquinone compound(s) of formula **(I)** or **(II)** as defined previously, and (b) the fluorescent dye(s) as defined previously, and also, when they are present, the oxidizing agent(s) and/or the reducing agent(s), may be dissolved beforehand before being applied to the keratin fibres.

In other words, the ingredients used in the dyeing process of the present invention may be present in one or more compositions.

The composition(s) comprising the ingredients according to the present invention are cosmetic compositions, i.e. they are preferably aqueous. Besides water, they may comprise one or more organic solvents, or mixtures thereof.

Examples of organic solvents that may be mentioned include linear or branched C₂ to C₄ alkanols, such as ethanol and isopropanol; glycerol; polyols and polyol ethers, for instance 2-butoxyethanol, propylene glycol, hexylene glycol, dipropylene glycol, propylene glycol monomethyl ether, diethylene glycol monomethyl ether and monoethyl ether, and also aromatic alcohols or ethers, for instance benzyl alcohol or phenoxyethanol, and mixtures thereof.

### The pH:

The pH of the composition(s) used in the dyeing process of the invention and of the composition of the invention comprising ingredients (a) and (b) is particularly between 2 and 12 approximately and preferably between 3 and 11 approximately. It may be adjusted to the desired value by means of acidifying or alkaline agents usually used in the dyeing of keratin fibres, or alternatively using standard buffer systems.

The pH of the composition which comprises (a) and (b) and that of the composition(s) used in the dyeing process of the invention is preferentially between 6 and 11 inclusive, particularly between 7 and 10 and more particularly between 7.5 and 9.5, such as between 9 and 9.5.

Among the acidifying agents, mineral and organic acids as defined previously, mention may be made, by way of example, of mineral or organic acids, for instance hydrochloric acid, orthophosphoric acid or sulfuric acid, carboxylic acids, for instance acetic acid, tartaric acid, citric acid and lactic acid, and sulfonic acids.

Among the alkaline agents that may be mentioned, for example, are aqueous ammonia, alkali metal carbonates, alkanolamines such as monoethanolamines, diethanolamines and triethanolamines, and other alkaline agents as defined previously.

### Forms of the composition:

The composition(s) comprising the dye(s) of formula **(I)** as defined previously, and the fluorescent dye(s) as defined previously, may be in various presentation forms, such as in the form of liquids, lotions, creams or gels or in any other form that is suitable for dyeing keratin fibres. It may also be packaged under pressure in an aerosol container in the presence of a propellant or in a non-aerosol container and may form a foam.

### Additives

When the ingredients used in the dyeing process according to the present invention are present in one or more composition(s), said composition(s) may also optionally comprise one or more additives, different from the ingredients of the invention and among which mention may be made of fatty substances, cationic, anionic, nonionic, amphoteric or zwitterionic surfactants, cationic, anionic, nonionic or amphoteric polymers or mixtures thereof, antidandruff agents, anti-seborrhoea agents, agents for preventing hair loss and/or for promoting hair regrowth, vitamins and provitamins including panthenol, sunscreens, mineral or organic pigments, sequestrants, plasticizers, solubilizers, acidifying agents, mineral or organic thickeners, especially polymeric thickeners, opacifiers or nacreous agents, antioxidants, hydroxy acids, fragrances, preserving agents, pigments and ceramides.

Needless to say, a person skilled in the art will take care to select this or these optional additional compound(s) such that the advantageous properties intrinsically associated with the composition(s) in accordance with the invention are not, or are not substantially, adversely affected by the envisaged addition(s).

The above additives may generally be present in an amount, for each of them, of between 0 and 20% by weight relative to the total weight of the composition comprising them.

### The dyeing process

The process for dyeing keratin fibres according to the present invention comprises the application to said keratin fibres of the following ingredients:
(a) one or more anthraquinone dyes of formulae **(I)** and/or **(II)** as defined previously, and
(b) one or more fluorescent dyes as defined previously,
it being understood that the anthraquinone dye(s) of formulae (I) and/or (II) (ingredients (a)) and the fluorescent dye(s) (ingredients (b)) are applied to said keratin fibres together or sequentially.

In other words, the dyeing process according to the present invention may be performed in one or more steps.

According to a particularly preferred embodiment, the anthraquinone dye(s) of formulae (I) and/or (II) and the fluorescent dye(s) (b), as defined previously, are applied together (or jointly), i.e. simultaneously, to the keratin fibres. According to this embodiment, the dyeing process is performed in one step. One variant of the dyeing process of the invention involves applying a cosmetic composition according to the invention which comprises (a) the anthraquinone compound(s) of formulae (I) and/or (II) and (b) the fluorescent dye(s) as defined previously to the keratin fibres.

According to another particularly preferred embodiment, the anthraquinone dye(s) of formulae (I) and/or (II) as defined previously and the fluorescent dye(s) as defined previously are applied sequentially, i.e. successively. According to this other embodiment, the dyeing process is performed in at least two steps.

According to a first embodiment in at least two steps, the fluorescent dye(s) as defined previously are applied to the keratin fibres subsequently to the anthraquinone dye(s) of formulae (I) and/or (II) as defined previously. In other words, the fluorescent dye(s) as defined previously are applied after the anthraquinone dye(s) of formulae (I) and/or (II) as defined previously.

According to this first embodiment, the process for dyeing keratin fibres comprises the following steps:
- a first step of applying to said keratin fibres a cosmetic composition comprising one or more anthraquinone dye(s) of formulae (I) and/or (II) as defined previously, followed by
- a second step of applying to said keratin fibres a cosmetic composition which comprises one or more fluorescent dyes as defined previously.

According to a preferred embodiment in at least two steps, the anthraquinone dye(s) of formulae (I) and/or (II) as defined previously are applied to the keratin fibres subsequently to the fluorescent dye(s). In other words, the anthraquinone dye(s) of formulae (I) and/or (II) are applied after the fluorescent direct dye(s).

According to this preferred embodiment, the process for dyeing keratin fibres comprises the following steps:
- a first step of applying to said keratin fibres a cosmetic composition comprising one or more fluorescent dyes as defined previously, followed by
- a second step of applying to said keratin fibres a cosmetic composition comprising one or more anthraquinone dye(s) of formulae **(I)** and/or **(II)** as defined previously.

Preferably, ingredients (a) and (b) are applied to the keratin fibres in a bath ratio that may range from 0.1 to 10 and more particularly from 0.2 to 8. For the purposes of the present invention, the term "bath ratio" means the ratio between the total weight of ingredient (a) or (b) and the total weight of keratin fibres to be treated.

When the dyeing process is performed in one step, ingredients (a) and (b) are advantageously left to stand on the keratin fibres for a time ranging from 1 to 90 minutes and more preferentially for a time ranging from 5 to 60 minutes.

When the dyeing process is performed in at least two steps, each of the ingredients (a) and (b) may be advantageously left to stand on the keratin fibres for a time ranging from 1 to 60 minutes and more preferentially for a time ranging from 5 to 45 minutes.

On conclusion of the dyeing process according to the invention, in one or at least two steps, the keratin fibres are advantageously rinsed with water. They may optionally be washed with a shampoo, followed by rinsing with water, before being dried or left to dry.

When the dyeing process is performed in at least two steps, the keratin fibres are advantageously rinsed with water between each step. In other words, the dyeing process may comprise an intermediate rinsing step between the application of the first ingredient and the application of the second ingredient. During this intermediate rinsing step, the keratin fibres may optionally be washed with a shampoo, followed by rinsing with water, before being dried or left to dry.

The dyeing process according to the present invention may be performed at room temperature (25°C) or with heating.

When they are present, the reducing agent(s) may be applied separately or together with one of the ingredients (a) or (b). Preferably, when they are present, the reducing agent(s) are applied together with the ingredient (b).

When they are present, the oxidizing agent(s) may be applied separately or together with one of the ingredients (a) or (b). Preferably, when they are present, the oxidizing agent(s) are applied after application of ingredients (a) and (b).

According to a particular embodiment, the process for dyeing keratin fibres according to the present invention comprises the following successive steps:
- a first step of applying to said keratin fibres a cosmetic composition comprising (a) one or more anthraquinone dye(s) of formulae (I) and/or (II) as defined previously, followed by
- a second step of applying to said keratin fibres a cosmetic composition comprising: (b) one or more fluorescent dyes as defined previously, and (c) one or more reducing agents as defined previously.

According to a particular embodiment of the dyeing process of the invention, no step of said process involves an oxidizing agent.

According to an advantageous embodiment of the dyeing process of the invention, no step of said process involves a reducing agent.

The dyeing process according to the present invention may be applied to wet or dry, preferably dry, keratin fibres.

### The multi-compartment device

The present invention also relates to a multi-compartment device comprising a first compartment comprising one or more anthraquinone dyes of formula (I) and/or (II) as defined previously, and a second compartment comprising one or more fluorescent dyes as defined previously.

According to a particular embodiment, the "keratin fibres" are human keratin fibres and more particularly the hair.

The examples that follow serve to illustrate the invention.

### EXAMPLES

### 1. APPLICATION TESTS PERFORMED:

The tests were performed:
- at a molar concentration of 5x10⁻³ mol% of each dye (Example 1)
- at a molar concentration of 5x10⁻³ mol% of anthraquinone dye and of 1.66x10⁻³ mol% of fluorescent dye (Example 2)
- The solutions are adjusted to pH 9-9.5 with aqueous ammonia solution.

The compounds tested on a 0.5 g lock of hair containing 90% white hairs are the following: The violet-blue dyes, of the anthraquinone disulfide series or not, evaluated in the context of the invention are the following:

| Dye (a) | Structure of violet-blue dyes |
|---|---|
| Dye 1 | 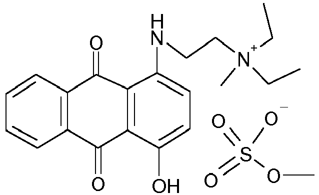 |
| Dye 2 | 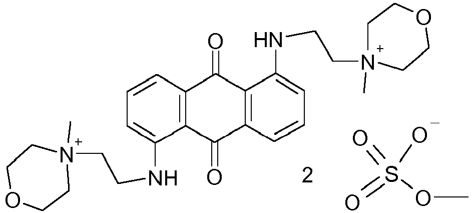 |
| Dye 3 | 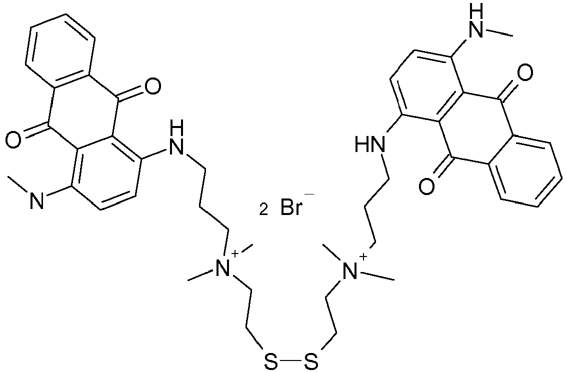 |

The fluorescent dyes of the styrylpyridinium series evaluated in the context of the invention are the following:

| Dye (b) | Structure of the fluorescent dyes |
|---|---|
| Dye a | 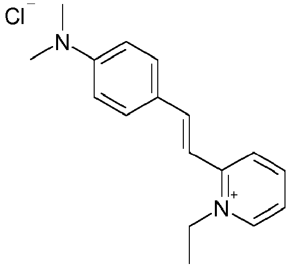 |
| Dye b | 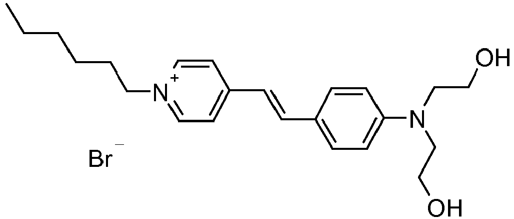 |

The combinations of dyeing compounds were prepared on a 0.5 g lock of natural hair containing 90% white hairs as a mixture (NW90) according to 3 application modes:
- *In reducing medium:*
   ▪ "blue direct dye" then "fluorescent dye" (application sense 1 - AS1)
- *In non-reducing medium:*
   ▪ "fluorescent dye" then "blue direct dye" (application sense 2 - AS2)
   ▪ "blue direct dye" then "fluorescent dye" (application sense 3 - AS3)
   ▪ "blue direct dye" and "fluorescent dye" as a mixture before application (application sense 4 - AS4).

For application sense 1 - AS1, the products were applied as follows:
Application condition for the blue direct dye/fluorescent dye:
   The amount of a) dye of formula **(I)** or **(II)** according to the invention [blue] and of b) fluorescent dyes = 5×10⁻³ mol% in water
      - 9 ml of this mixture + 1 ml of Dulcia DV2 (bath ratio = 5)
      - 20 minutes at room temperature (RT = 25°C) + rinsing + H₂O₂ (0.48 g%) 5 minutes at RT
      - rinsing + 1 shampooing + drying under a hood for 10 minutes per g of hair

For application senses 2 (AS2), 3 (AS3) and 4 (AS4), the products were applied as follows:
Application condition for the blue direct dye of formula **(I)** or **(II):** 5×10⁻³ mol% in water, 30 minutes at RT + rinsing + 1 shampooing + drying under a hood for 10 minutes per g of hair.
Application condition for the fluorescent dye: 5×10⁻³ mol% in water, 20 minutes at RT + rinsing + 1 shampooing + drying under a hood for 10 minutes per g of hair
Application condition for the mixture "blue direct dye of formula (I) or (II) + fluorescent dye": 5×10⁻³ mol% of each of the dyes in water, 45 minutes at RT + rinsing + 1 shampooing + drying under a hood for 10 minutes per g of hair

### Example 1:

### The following hair dyeing results were obtained:

| Blue dye | Fluorescent dye | Application sense | Colour obtained |
|---|---|---|---|
| Dye 1 | Dye a | AS2 | Brown with a coppery glint |
| Dye 1 | Dye a | AS3 | Brown with a coppery glint |
| Dye 1 | Dye a | AS4 | Brown with a coppery glint |
| Dye 1 | Dye b | AS1 | Brown with a coppery glint |
| Dye 2 | Dye a | AS1 | Brown with a coppery glint |
| Dye 2 | Dye b | AS1 | Brown with a coppery glint |
| Dye 2 | Dye b | AS2 | Brown with a coppery glint |
| Dye 2 | Dye b | AS3 | Brown with a coppery glint |
| Dye 2 | Dye b | AS4 | Brown with a coppery glint |
| Dye 3 | Dye a | AS1 | Dull brown |
| Dye 3 | Dye b | AS1 | Brown |

It is seen that the process of the invention makes it possible to obtain very aesthetic and powerful brown colours just by combining a blue dye and a fluorescent dye.

### Comparative data:

### COMPARATIVE EXPERIMENT

3 series of locks (90 % natural with hair - 90 NW) have been carried out with an equimolar association of a reductive or not medium vs. comparative prior art EP0852943A1.

The tests has been carried out at a molar concentration of 5 × 10-3 mol % for each dye. The compositions are adjusted to pH 9-9.5 with an ammonia solution. The compounds tested on locks of 0.5 g of natural white hair (90 NW) are the following:
The blue disulfide anthraquinone dye, and the hemicyanin dyes have been evaluated in the context of the comparison as follows:

| Dye (a) | chemical structure of Blue-violet dye |
|---|---|
| Dye 3 | 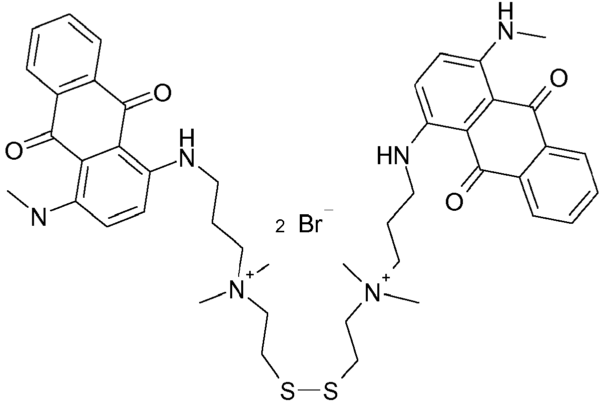 |

| Dye (b) | chemical structure of hemicyanin fluorescent dyes |
|---|---|
| Dye a EP0852943A1 Comparative | 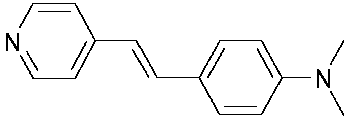 |
| Dye b Invention | 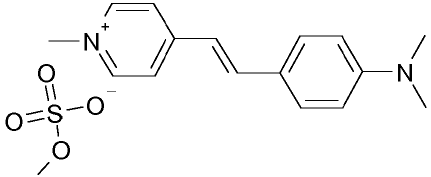 |

In reducing medium:
- *"blue disulfide direct dye" and "fluorescent dye" (application sense 1* - *SA1)*

Application condition of the blue disulfide direct dye / fluorescent dye:
The amount of a) dye 3 and b) fluorescent dyes = 5.10-3 mol % in water
   - 9 ml of this mixture + 1 ml dulcia DV2
   - 20 '/ TA + rinse + H2O2 (0.48 g %) 5' / TA
   - rinsing + 1 shampoo + drying with headphones 10 '/ g of hair
   - In a non-reducing medium:
      - *"blue disulfide direct dye" and "fluorescent dye" mixed before application (method 2* - *SA2).*

The method of applying the mixture « blue disulfide direct dye 3 + fluorescent dye » 5.10⁻³ mol % in water, for 45 ' at room temperature (27 °C) /TA + rinsing + 1 shampoo + dryeing au hair 10'/g of hair

### Results

| Blue direct dye | Fluorescent dye | application sense | Color after treatment |
|---|---|---|---|
| Dye 3 | | AS1 | Blue |
| Dye 3 | | AS2 | Blue |
| | Dye a | AS1 | No coloration |
| | Dye a | AS2 | No coloration |
| | Dye b | AS1 | Red |
| | Dye b | AS2 | Red |
| Dye 3 | Dye a | AS1 | Red |
| Dye 3 | Dye b | AS1 | Brown with shade |
| Dye 3 | Dye a | AS2 | Blue |
| Dye 3 | Dye b | AS2 | Cold brown |

After treating keratin fibers with dyes and shampoo locks have been dried and color on the locks has been measured with spectrocolorimeter Minolta CM2600d (angle 10°, illuminant D65) in the CIEL*a*b* system.

In this system, L* represents intensity of the color, a* represents the green/red color axis and b* the blue/yellow color axis .

The lowest L* measured, the most intensive is the color.

| Blue Dye | Fluorescent dye | Application sense | L* | a* | b* |
|---|---|---|---|---|---|
| Dye 3 | | AS1 | 32.03 | -5.72 | -19 |
| Dye 3 | | AS2 | 35.41 | -7.06 | -14.56 |
| | Dye a | AS1 | 59.86 | 0.54 | 15.83 |
| | Dye a | AS2 | 61.59 | 0.65 | 10.98 |
| | Dye b | AS1 | 28.23 | 38.77 | 25.94 |
| | Dye b | AS2 | 33.27 | 43.76 | 31.17 |
| Dye 3 | Dye a | AS1 | 32.25 | -5.03 | -18.65 |
| Dye 3 | Dye b | AS1 | 19.29 | 10.66 | 8.56 |
| Dye 3 | Dye a | AS2 | 35.82 | -5.89 | -17.44 |
| Dye 3 | Dye b | AS2 | 24.3 | 18.77 | 16.51 |

These results of the table show that the method according to the invention, using only two direct fluorescent dyes b), anthraquinone dye of formula (I) and / or (II) of the invention associated with a cationic fluorescent dye, makes it possible to obtain natural brown colors. This is not the case of the combination of an anthraquinone dye of formula (I) and / or (II) of EP0852943A1 combined with a non-cationic fluorescent dye a) for which the color obtained is blue (non natural color).

## Claims

1. Process for dyeing keratin fibres, in particular human keratin fibres such as the hair, comprising the application to said keratin fibres of ingredients (a) and (b) below:
(a) one or more anthraquinone dyes chosen from the compounds of formulae **(I)** and/or **(II)** below, the optical isomers thereof, the geometrical isomers thereof, the tautomers thereof, the solvates thereof, and mixtures thereof: 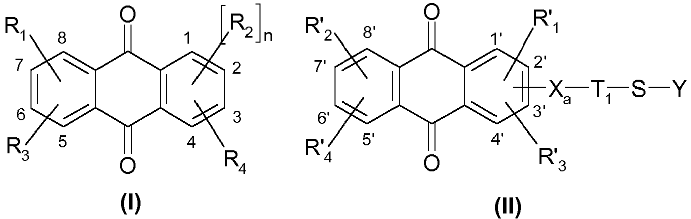 in which formula **(I)** or **(II):**
▪ **Xₐ,** which may be identical or different, represents a bond, a heteroatom or a group chosen from an oxygen or sulfur atom, N(Rₐ), CO, SO, SO₂, or combinations thereof such as -O-CO-, -CO-O-, -NH-CO- or -CO-NH-, with Rₐ representing a hydrogen atom or a (C₁-C₆)alkyl group optionally substituted with one or more hydroxyl groups; preferably, Xₐ represent N(Rₐ) and particularly NH;
▪ **Y** represents: i) a hydrogen atom; ii) an alkali metal; iii) an alkaline-earth metal; iv) an ammonium group: N⁺R^{a}R^{b}R^{g}R^{d} or a phosphonium group: P⁺R^{a}R^{b}R^{g}R^{d} with R^{a}, R^{b}, R^{g} and R^{d}, which may be identical or different, representing a hydrogen atom or a (C₁-C₄)alkyl group; or v) a thiol-function protecting group; or vi) the group **(III)** below: 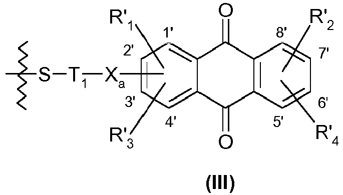
▪ **R₁, R₂, R₃, R₄, R'₁, R'₂, R'₃** and **R'₄,** which may be identical or different, represent an atom or a group chosen from:
∘ hydrogen;
∘ halogen such as bromine and chlorine,
∘ hydroxyl,
∘ C₁-C₄ alkoxy,
∘ hydroxysulfonyl (-SO₃H) or sulfonate (-SO₃⁻, M⁺), with M⁺ representing a cationic counterion, in particular an alkali metal, alkaline-earth metal or ammonium, such as Na⁺ or K⁺;
∘ optionally substituted C₁-C₆ alkyl,
∘ -NR₅R₆ in which **R₅** and **R₆,** which may be identical or different, represent an atom or radical chosen from: i) hydrogen, ii) (C₁-C₄)alkylcarbonyl such as methylcarbonyl (-COCH₃), iii) arylsulfonyl such as phenylsulfonyl (-SO₂Ph), iv) Het-ALK-C(O)- with Het representing a heterocycloalkyl group which is optionally substituted, especially with one or more (C₁-C₄)alkyl groups and ALK represents a (C₁-C₆)alkylene group optionally substituted with one or more hydroxyl or (di)(hydroxy)(C₁-C₄)(alkyl)amino groups; Het-ALK-C(O)- is such as (piperidin-1-yl)acetyl, 2-methyl-2-(piperidin-1-yl)propanoyl,2-(morpholin-4-yl)ethyl-β-alaninoyl, v) optionally substituted aryl, in particular phenyl optionally substituted with at least one radical chosen from a) C₁-C₆ alkyl, b) hydroxyl, c) hydroxysulfonyl, d) C₁-C₄ alkoxy, e) carboxyl (-COOH), f) (C₁-C₄)alkoxycarbonyl, g) amino, h) (di)(C₁-C₄)alkylamino, one of the alkyl radicals possibly being substituted with a hydroxyl or hydroxysulfonyl radical -SO₃H, or -OSO₃H, vi) optionally substituted aryl(Ci-C₄)alkyl, in particular benzyl optionally substituted with a (di)(C₁-C₄)(alkyl)amino group, viii) optionally substituted C₁-C₂₀ alkyl, optionally interrupted with one or more heteroatoms and/or with one or more groups comprising at least one heteroatom, preferably chosen from oxygen, nitrogen and sulfur, CO, SO, SO₂ or combinations thereof. when said alkyl radical is substituted, it is substituted with one or more atoms or groups chosen from: a) halogens, preferably one or more chlorine atoms, b) hydroxyl, c) (C₁-C₆)alkylcarbonylamino, in particular acylamino, d) 5- or 6-membered heterocycloalkyl such as tetrahydro-2H-pyran-4-amine, morpholino, piperidino or piperazino, e) (di)(C₁-C₄)(alkyl)amino, f) hydroxysulfonyl(C₁-C₄)alkylamino, or hydroxysulfonyloxy(C₁-C₄)alkylamino, g) (di)(hydroxy)(C₁-C₄)(alkyl)amino, h) 5- or 6-membered heteroaryl such as imidazole, and i) formylamino (-NHCOH);
∘ a group of formula (a) below: -N(R₇)-X₁-W₁ (a) in which formula (a):
**R₇** represents a hydrogen or a C₁-C₄ alkyl radical,
**X₁** represents a divalent radical chosen from C₁-C₂₀ alkylene optionally interrupted with one or more heteroatoms or groups chosen from oxygen, nitrogen and sulfur, CO, SO, SO₂, arylene such as phenylene, or combinations thereof; preferably, X₁ represents:
• (C₁-C₁₀)alkylene,
• -(C₁-C₁₀)alkylcarbonyl-,
• -carbonyl(C₁-C₁₀)alkyl-,
• -(C₁-C₁₀)alkylaminocarbonyl(C₁-C₁₀)alkyl-,
• -(C₁-C₁₀)alkylcarbonylamino(C₁-C₁₀)alkyl-,
• -phenyl(C₁-C₁₀)alkyl-,
• -(C₁-C₁₀)alkylphenyl-, or
• phenylene,
**W₁** represents a cationic radical chosen from: 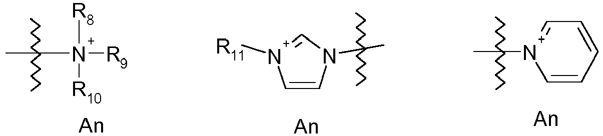 with **R₈, R₉, R₁₀** and **R₁₁,** which may be identical or different, representing a C₁-C₆ alkyl group, a benzyl radical, a C₁-C₆ alkyl sulfonate radical; the radicals **R₈** and **R₉** may form, with the nitrogen atom to which they are attached, a saturated or unsaturated, optionally substituted 5- to 7-membered heterocycle, optionally comprising another non-nitrogen heteroatom, preferably an oxygen atom;
▪ n is an integer ranging from 1 to 3; preferably, n is equal to 1 or 2, **T₁** represents a linear or branched divalent hydrocarbon-based chain comprising from 1 to 20 carbon atoms, optionally interrupted with one or more heteroatoms or groups, or combinations thereof, chosen from oxygen, sulfur, N(R_{b}), C(O), -N⁺(R₈)(R₉)- An, optionally cationic and optionally substituted heteroaryl, such as imidazolium, An with **R₈** and **R₉,** which may be identical or different, represent a C₁-C₆ alkyl radical; **R_{b}** representing a hydrogen atom or a (hydroxy)(C₁-C₄)alkyl group; preferably, said hydrocarbon-based chain is interrupted with one or more groups chosen from N(R_{b}), C(O), and a combination thereof such as -C(O)- N(R_{b})- or -N(R_{b})-C(O)-, and-N⁺(R₈)(R₉)- An, **An** is an organic or inorganic anionic counterion, which ensures the electrical neutrality of the dyes of formulae **(I)** and **(II);**
▪ 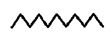 being the part of the bond that is connected to the rest of the molecule; and
(b) one or more cationic (poly)methine fluorescent dyes; it being understood that:
- the anthraquinone dye(s) of formula **(I)** comprise at least one radical **R₁, R₃** or **R₄,** other than a hydrogen atom, and the anthraquinone dye(s) of formula (II) comprise at least one radical **R'₁, R'₂, R'₃** or **R'₄** other than a hydrogen atom;
- (a) the anthraquinone dye(s) of formula **(I)** or **(II)** and (b) the fluorescent dye(s) are applied to said keratin fibres together or sequentially; and
- when the compounds of formula (I) or (II) are cationic and comprise a sulfonate group, then M⁺ and An may be absent to ensure the electrical neutrality of said molecule.

2. Process according to the preceding claim, in which the anthraquinone dye(s) of formulae **(I)** and/or **(II)** are dyes which absorb light in the blue-violet range, preferably the blue range.

3. Process according to either of the preceding claims, in which the anthraquinone dye(s) of formulae **(I)** and/or **(II)** are dyes of formula **(I)** and n is equal to 0 or 1; preferably, the substituents **R₁, R₂, R₃** and **R₄** are in positions 1, 4, 5 and 8.

4. Process according to any one of the preceding claims, in which the anthraquinone dye(s) of formula **(I)** and/or **(II)** are dyes are of formula **(I),** with **R₁** and **R₃** representing an atom or group chosen from i) hydroxyl, ii) arylamino or aryl(C₁-C₆)alkylamino with the aryl group representing an optionally substituted aryl group, in particular a phenyl group optionally substituted with one or more groups chosen from (C₁-C₄)alkyl, carboxyl, (C₁-C₄)alkoxycarbonyl, (di)(hydroxy)(C₁-C₄)(alkyl)amino, tri(C₁-C₄)alkylammonium, (C₁-C₄)alkoxy, (di)hydroxysulfonyloxy(C₁-C₄)alkylamino, hydroxysulfonyl, iii) (di)(hydroxy)(C₁-C₆)(alkyl)amino, iv) (di)(C₁-C₄)(alkyl)amino(C₁-C₆)alkylamino, v) (C₁-C₄)alkylcarbonylamino(C₁-C₆)alkylamino, vi) arylsulfonylamino with the aryl group possibly being optionally substituted, aryl preferably representing a phenyl group, vii) (di)halo(C₁-C₄)alkylamino, viii) (di)(hydroxy)(C₁-C₄)alkoxy(C₁-C₄)alkylamino, ix) (di)tri(C₁-C₄)alkylammonium(C₁-C₆)alkylamino, x) heterocycloalkyl(C₁-C₆)alkylamino, heterocycloalkyl(C₁-C₄)alkylcarbonylamino, heterocycloalkyl(C₁-C₄)alkylamino(C₁-C₄)alkylcarbonylamino or heterocycloalkyl(C₁-C₄)alkylcarbonylamino(C₁-C₆)alkylamino, said heterocycloalkyl possibly being optionally cationic and/or substituted especially with one or more (C₁-C₄)alkyl or benzyl groups, xi) halo(C₁-C₄)alkylcarbonylamino(C₁-C₆)alkylamino, xii) hydroxysulfonyl, xiii) halo such as chlorine, xiv) heteroaryl(C₁-C₆)alkylamino, said heteroaryl possibly being optionally cationic and/or substituted especially with one or more (C₁-C₄)alkyl groups, xv) heteroaryl(C₁-C₄)alkylcarbonylamino(C₁-C₆)alkylamino, said heteroaryl possibly being optionally cationic and/or substituted especially with one or more (C₁-C₄)alkyl groups, xvi) R'R"N- with R' and R", which may be identical or different, representing a (halo)(C₁-C₄)alkylcarbonylamino(C₁-C₆)alkyl group, a (halo)(C₁-C₄)alkylaminocarbonyl (C₁-C₆)alkyl group, a (halo)(C₁-C₄)alkylcarbonyl group, a carboxy(C₁-C₆)alkyl group, the alkyl group possibly being substituted with one or more amino or hydroxyl groups, a (poly)hydroxy(C₁-C₆)alkyl group, xvii) (C₁-C₁₆)alkylaminocarbonylamino(C₁-C₆)alkylamino, xviii) formylamino(C₁-C₆)alkylamino, xix) (hydroxy)(C₁-C₆)alkylamino(C₁-C₆)alkylamino, xix) hydroxysulfonyl(C₁-C₆)alkylamino(C₁-C₆)alkylamino, xx) sulfonato(C₁-C₆)alkyl(di(C₁-C₄)alkyl)ammonium(C₁-C₆)alkylamino, xxi) hydroxysulfonyl(C₁-C₆)alkoxy(C₁-C₆)alkylamino, xxi) heteroaryl(C₁-C₄)alkylcarbonylamino, said heteroaryl possibly being optionally cationic and/or substituted especially with one or more (C₁-C₄)alkyl groups, xxii) heterocycloalkyl(C₁-C₄)alkylcarbonylamino, said heterocycloalkyl possibly being optionally cationic and/or substituted especially with one or more (C₁-C₄)alkyl or benzyl groups, xxiii) (di)(C₁-C₄)(alkyl)amino(C₁-C₆)alkylamino, the (C₁-C₆)alkyl group possibly being substituted with one or more hydroxyl groups, xxiv) heterocycloalkylamino(C₁-C₆)alkylamino or heterocycloalkylamino, said heterocycloalkyl possibly being optionally cationic and/or substituted especially with one or more (C₁-C₄)alkyl or benzyl groups, xxv) heteroarylalkylamino(C₁-C₆)alkylamino or heteroarylalkylamino, said heteroaryl possibly being optionally cationic and/or substituted especially with one or more (C₁-C₄)alkyl groups, xxvi) tri(C₁-C₆)alkylammonium(C₁-C₆)alkylamino, xxvii) (C₁-C₄)alkoxycarbonyl(C₁-C₆)alkyl(di(C₁-C₄)alkyl)ammonium(C₁-C₆)alkylamino, xxviii) carboxylato(C₁-C₆)alkyl(di(C₁-C₄)alkyl)ammonium(C₁-C₆)alkylamino, xxix) carboxy(C₁-C₆)alkylamino(C₁-C₆)alkylamino, xxx) aryl(C₁-C₄)alkyl(di(C₁-C₄)alkyl)ammonium(C₁-C₆)alkylamino, xxxi) sulfonic SO₃H or sulfonate SO₃⁻, M⁺ with M⁺ representing a cationic counterion, xxxii) (C₁-C₆)alkyl, xxxiii) hydroxysulfonyl(C₁-C₄)amino, xxxiv) phenylsulfonylamino.

5. Process according to any one of the preceding claims, in which the anthraquinone dye(s) of formulae **(I)** and/or **(II)** are dyes of formula **(I)** and **R₂** and **R₄,** which may be identical or different, represent a hydrogen atom or a group as defined previously for R₁ and R₃ i) to xxxii) in the preceding claim:
- hydroxyl,
- (di)(C₁-C₄)(alkyl)amino,
- (C₁-C₄)alkoxy, heterocycloalkyl(C₁-C₆)alkylamino, said heterocycloalkyl possibly being optionally cationic and/or substituted especially with one or more (C₁-C₄)alkyl or benzyl groups,
- arylamino or aryl(C₁-C₆)alkylamino with the aryl group representing an optionally substituted aryl group, in particular a phenyl group optionally substituted with one or more groups chosen from (C₁-C₄)alkyl, (di)(hydroxy)(C₁-C₄)(alkyl)amino, (C₁-C₄)alkoxy and tri(C₁-C₄)alkylammonium,
- aryl(C₁-C₄)alkyl(di(C₁-C₄)alkyl)ammonium(C₁-C₆)alkylamino,
- (di)(C₁-C₄)(alkyl)amino(C₁-C₆)alkylamino, and
- hydroxy(C₁-C₄)(alkyl)amino(C₁-C₆)alkylamino.

6. Process according to any one of the preceding claims, in which the anthraquinone dye(s) of formulae **(I)** and/or **(II)** are dyes of formula **(I)** and **R₂** and **R₄** represent a hydrogen atom.

7. Process according to any one of Claims 1 to 3, in which the anthraquinone dye(s) of formulae **(I)** and/or **(II)** are dyes of formula **(I)** with **R₂** and **R₃** representing a hydrogen atom, and **R₁** and **R₄** are preferably in positions 8 and 4, respectively, and **R₁** and **R₄** are as defined in Claim 1, and in particular represent an atom or group as defined previously for R₁ and R₃ i) to xxxii) in Claim 4, preferably **R₁** and **R₄** are chosen from:
- halogen such as chlorine,
- (di)(C₁-C₄)(alkyl)amino,
- (C₁-C₄)alkylcarbonylamino,
- heterocycloalkyl(C₁-C₆)alkylamino, heterocycloalkyl(C₁-C₄)alkylcarbonylamino or heterocycloalkyl(C₁-C₄)alkylamino(C₁-C₄)alkylcarbonylamino, said heterocycloalkyl may be optionally cationic and/or substituted especially with one or more (C₁-C₄)alkyl or benzyl groups,
- tri(C₁-C₄)alkylammonium(C₁-C₆)alkylamino,
- arylamino or aryl(C₁-C₆)alkylamino with the aryl group representing an optionally substituted aryl group, in particular a phenyl group optionally substituted with one or more groups chosen from (C₁-C₄)alkyl, (di)(hydroxy)(C₁-C₄)(alkyl)amino, (C₁-C₄)alkoxy and tri(C₁-C₄)alkylammonium,
- (di)(C₁-C₄)(alkyl)amino(C₁-C₆)alkylamino, and
- (C₁-C₄)alkylcarbonylamino(C₁-C₆)alkylamino.

8. Process according to any one of Claims 1 to 3, in which the anthraquinone dye(s) of formulae **(I)** and/or **(II)** are dyes of formula **(I)** with **R₃** and **R₄** representing a hydrogen atom, and **R₁** and **R₂** are preferably in positions 8 and 1, respectively, and **R₁** and **R₂** are as defined in Claim 1, and in particular represent an atom or group as defined previously for R₁ and R₃ i) to xxxii) in Claim 4, preferably **R₁** and **R₂** are chosen from:
- (di)(hydroxy)(C₁-C₄)(alkyl)amino,
- (di)(hydroxy)(C₁-C₄)(alkyl)amino(C₁-C₆)alkylamino,
- tri(C₁-C₆)alkylammonium(C₁-C₆)alkylammonium,
- tri(C₁-C₆)alkylammonium(C₁-C₆)alkylamino,
- heterocycloalkyl(C₁-C₆)alkylamino, said heterocycloalkyl possibly being optionally cationic and/or substituted especially with one or more (C₁-C₄) alkyl or benzyl groups and
- arylamino or aryl(C₁-C₆)alkylamino with the aryl group representing an optionally substituted aryl group, in particular a phenyl group optionally substituted with one or more groups chosen from (C₁-C₄)alkyl, (di)(hydroxy)(C₁-C₄)(alkyl)amino, (C₁-C₄)alkoxy, tri(C₁-C₄)alkylammonium.

9. Process according to any one of Claims 1 to 3, in which the anthraquinone dye(s) of formulae **(I)** and/or **(II)** are dyes of formula **(I)** with **R₁** and **R₃** representing a hydrogen atom and n is equal to 2, and **R₂** and **R₄** are preferably in positions 1, 2 and 4, respectively, and **R₂** and **R₄** are as defined in Claim 1, and in particular represent an atom or group as defined previously for R₁ and R₃ i) to xxxii) in Claim 4, preferably **R₂** and **R₄** are chosen from:
- (C₁-C₆)alkyl,
- (di)(hydroxy)(C₁-C₄)(alkyl)amino,
- SO₃H or SO₃⁻M⁺,
- (di)(hydroxy)(C₁-C₄)(alkyl)amino(C₁-C₆)alkylamino,
- tri(C₁-C₄)alkylammonium(C₁-C₆)alkylamino, and
- heterocycloalkyl(C₁-C₆)alkylamino, said heterocycloalkyl possibly being optionally cationic and/or substituted especially with one or more (C₁-C₄)alkyl or benzyl groups, and
- arylamino or aryl(C₁-C₆)alkylamino with the aryl group representing an optionally substituted aryl group, in particular a phenyl group optionally substituted with one or more groups chosen from (C₁-C₄)alkyl, (di)(hydroxy)(C₁-C₄)(alkyl)amino, (C₁-C₄)alkoxy, tri(C₁-C₄)alkylammonium.

10. Process according to any one of Claims 1 to 3, in which the anthraquinone dye(s) of formulae **(I)** and/or **(II)** are dyes of formula **(II'):** 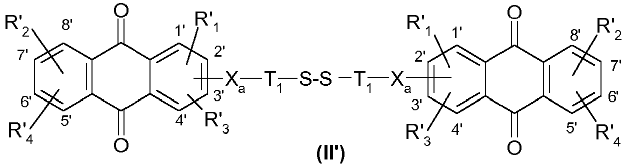 with **R'₁, R'₂, R'₃** and **R'₄, T₁** and **Xₐ** as defined in Claim 1, **Xₐ** preferably represents an N(Rₐ) and particularly NH, more particularly **R'₂** and **R'₄** represent a hydrogen atom and **R'₁** and **R'₃** are in particular as defined in Claim 4 for **R₁** and **R₃** i) to xxxii), preferably, **R'₃** and **R'₄,** which may be identical or different, represent a group chosen from (C₁-C₆)alkyl and (di)(hydroxy)(C₁-C₄)(alkyl)amino; preferably, **T₁** represents a saturated linear divalent hydrocarbon-based chain comprising from 1 to 20 carbon atoms, preferably between 2 and 10 carbon atoms, optionally interrupted with one or more groups chosen from N(R_{b}), C(O),-N⁺(R₈)(R₉)- An, cationic heteroaryl such as imidazolium, An or combinations thereof, with **R₈** and **R₉,** which may be identical or different, representing a C₁-C₆ alkyl radical; **R_{b}** representing a hydrogen atom or a (C₁-C₄)alkyl group; preferably, said hydrocarbon-based chain is interrupted with one or more groups chosen from -N⁺(R₈)(R₉)- An, N(R_{b}), C(O), and a combination thereof such as -C(O)- N(R_{b})- or -N(R_{b})-C(O)-, N⁺(R₈)(R₉)- An; more preferentially, **T₁** represents a divalent group **-(CH₂)ₙ-Tₐ-(CH₂)ₘ-T_{b}-(CH₂)ₚ-** with Tₐ and T_{b}, which may be identical or different, representing a bond, or a group chosen from N(R_{b}), C(O), -N⁺(R₈)(R₉)- An, cationic heteroaryl such as imidazolium, An or combinations thereof, with **R₈** and **R₉,** which may be identical or different, representing a C₁-C₆ alkyl radical; **R_{b}** representing a hydrogen atom or a (C₁-C₄)alkyl group; preferably, -N⁺(R₈)(R₉)- An, -C(O)-N(R_{b})- or -N(R_{b})-C(O)-, n, m and p, which may be identical or different, represent an integer between 1 and 10 inclusive, with the sum n+m+p between 1 and 20 inclusive, preferably between 2 and 10; preferentially, the groups **R'₁** and **R'₃** are in positions 2' and 4', and **R'₂** and **R'₄** are in positions 5' and 8'.

11. Process according to the preceding claim, in which the anthraquinone dye(s) of formulae **(I)** and/or **(II)** are dyes of formula **(II"):** 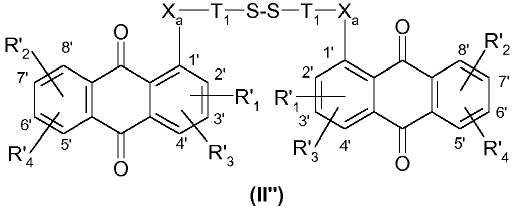 in which formula **(II") R'₁, R'₂, R'₃** and **R'₄, T₁** and **Xₐ** are as defined in the preceding claim for **(II').**

12. Process according to any one of the preceding claims, in which (a) the anthraquinone dye(s) of formulae **(I)** and/or **(II)** are chosen from the following compounds, the optical isomers thereof, the geometrical isomers thereof, the tautomers thereof, the solvates thereof, and mixtures thereof: 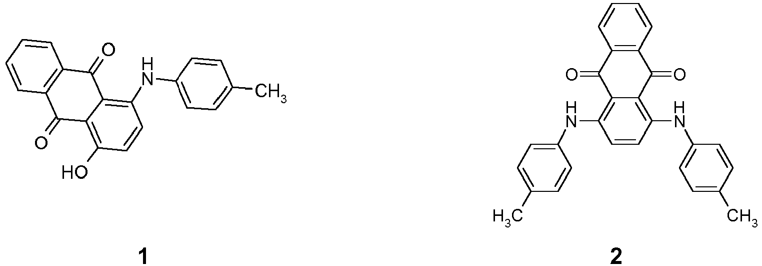 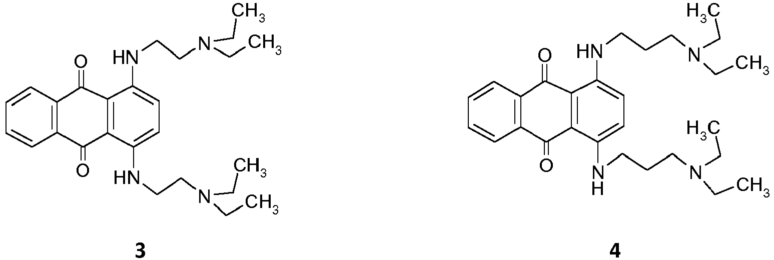 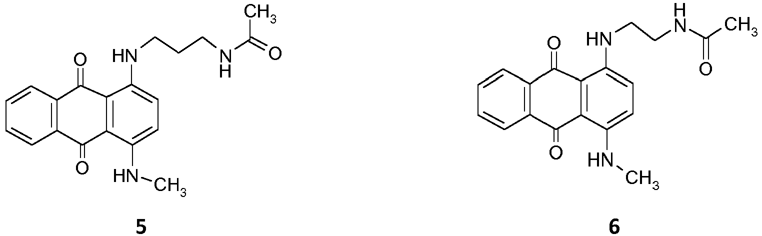 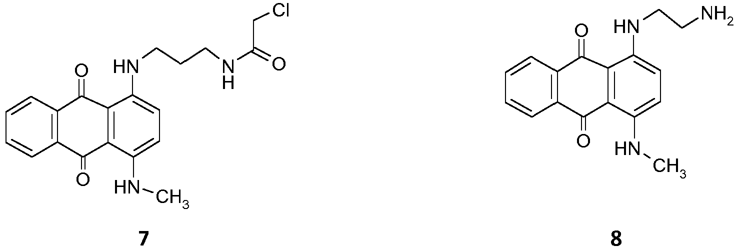 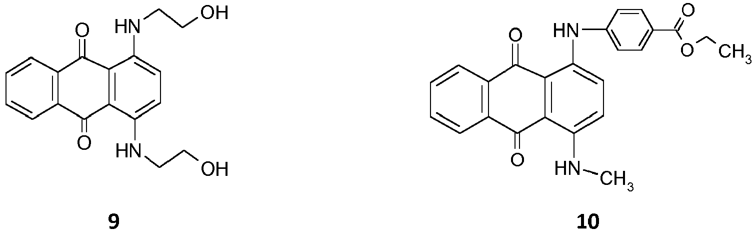 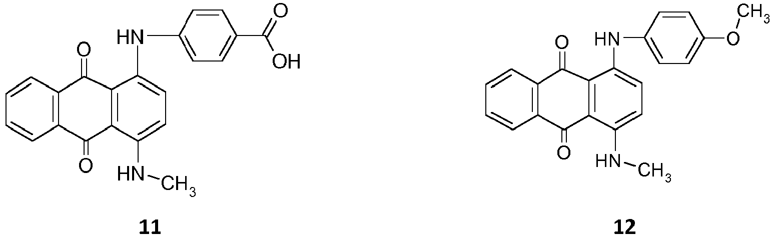 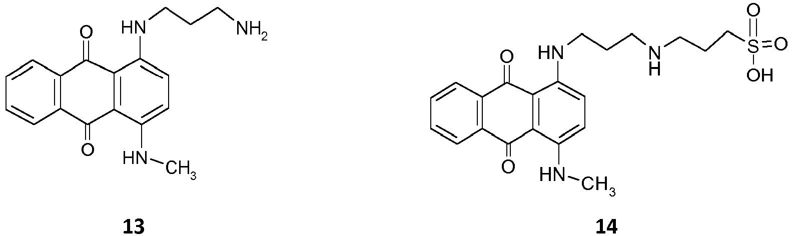 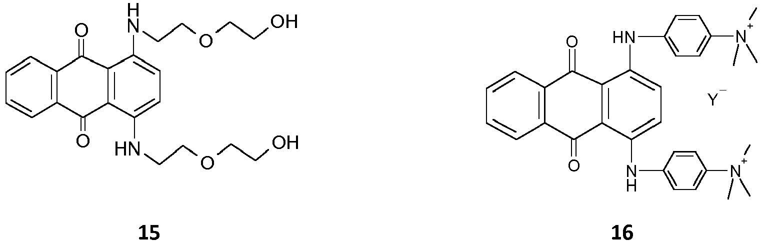 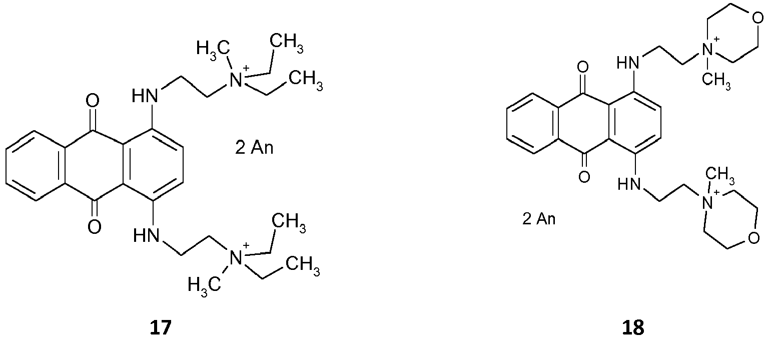 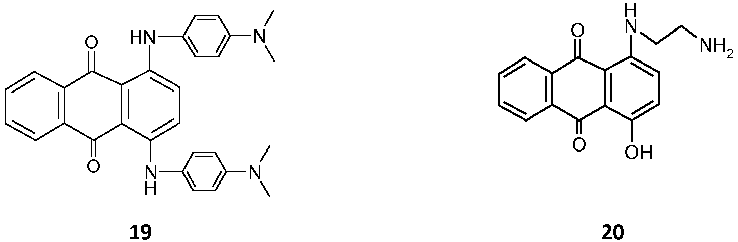 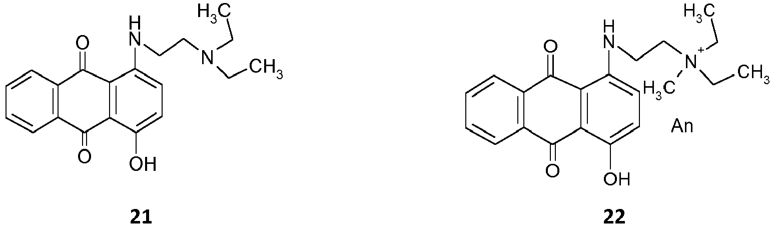 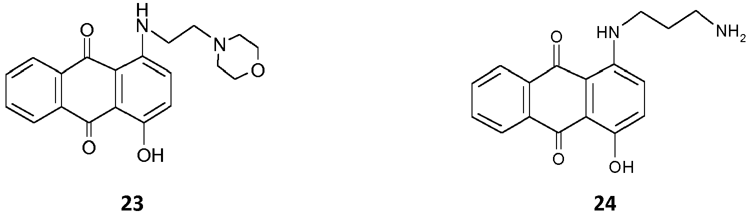 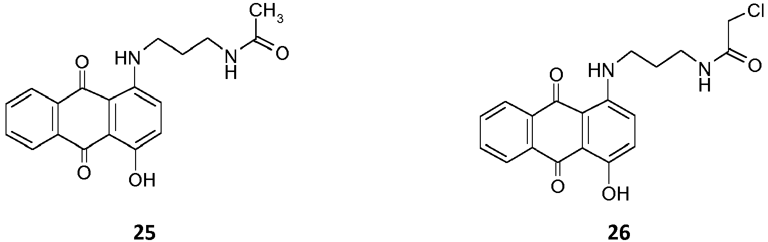 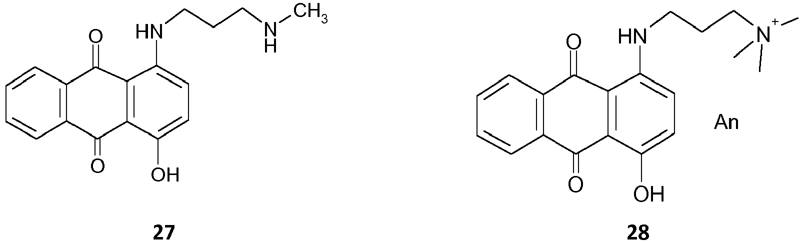 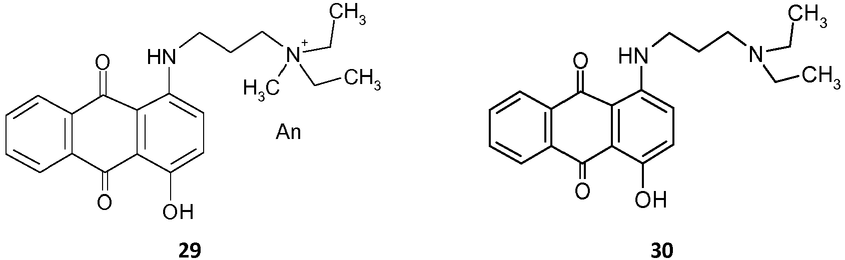 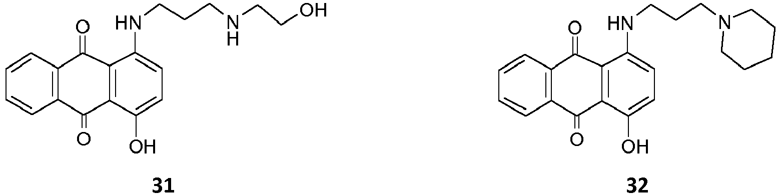 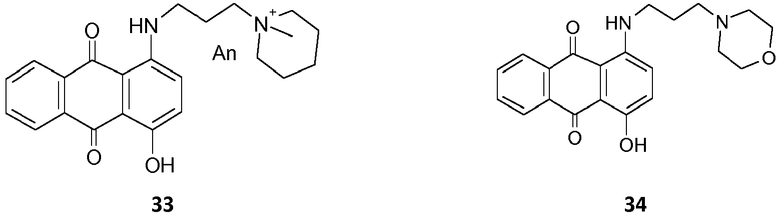 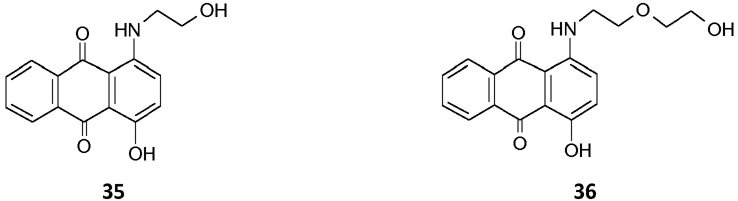 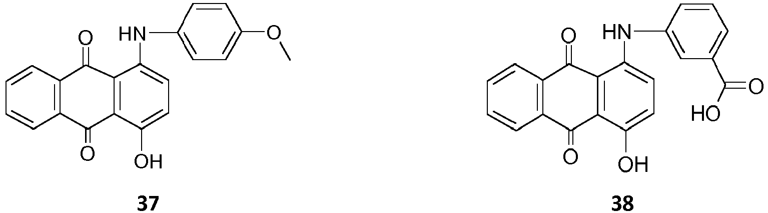 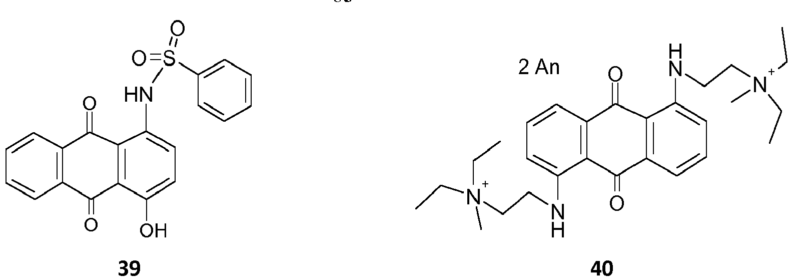 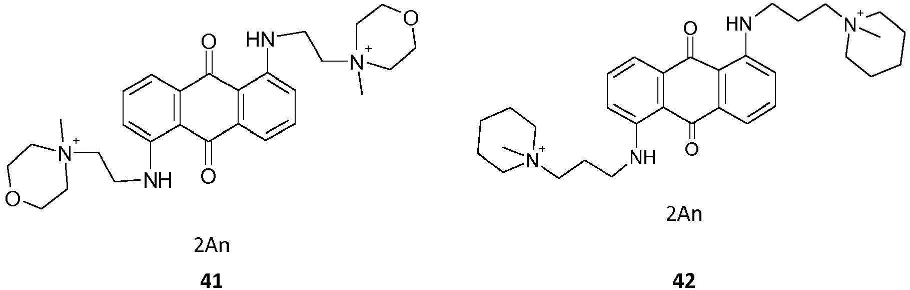 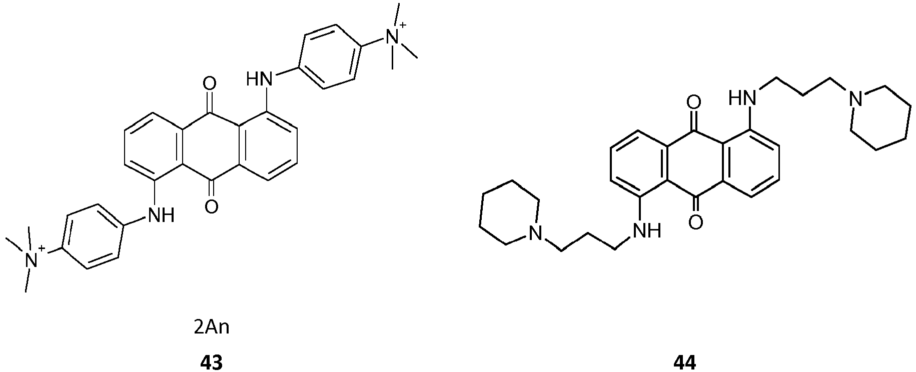 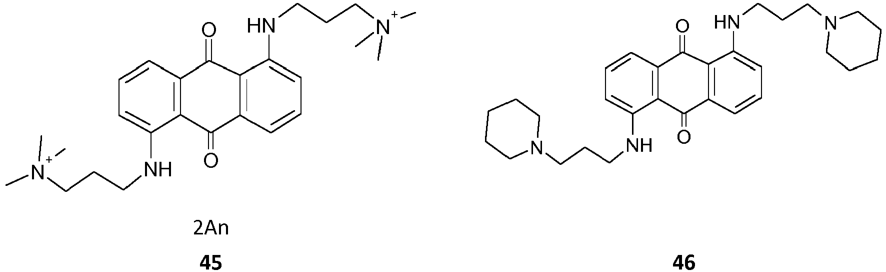 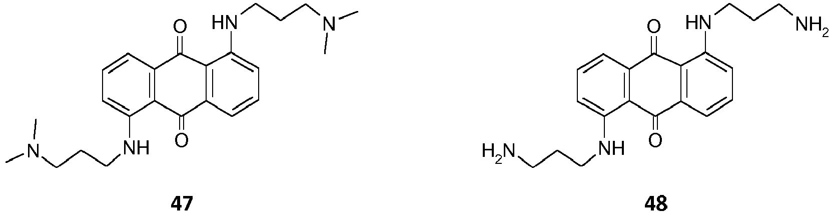 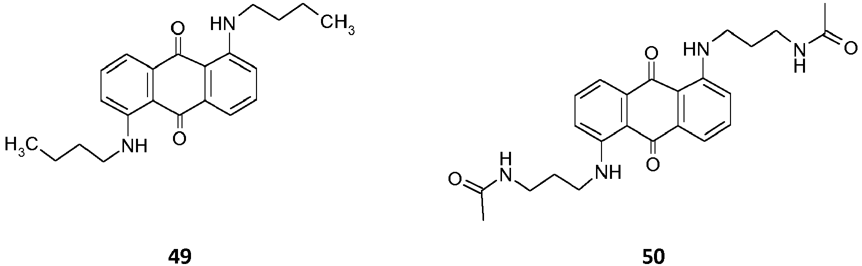 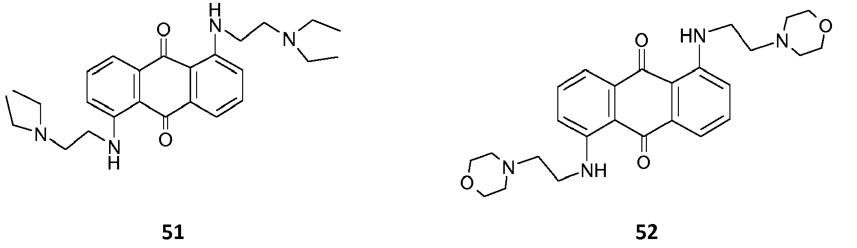 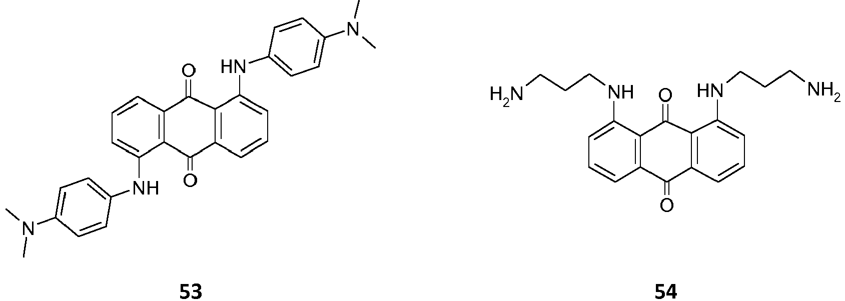 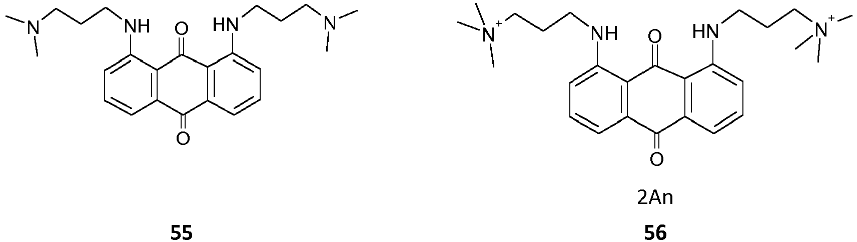 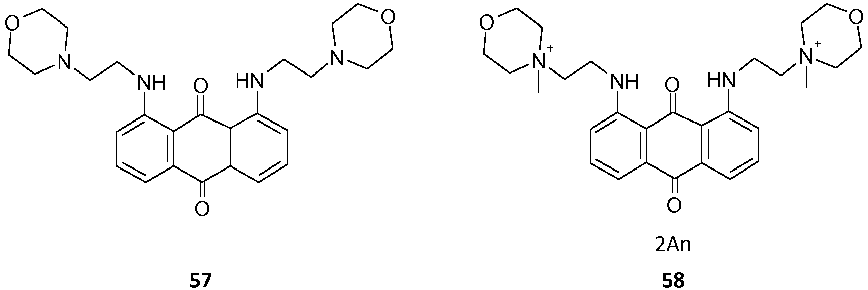 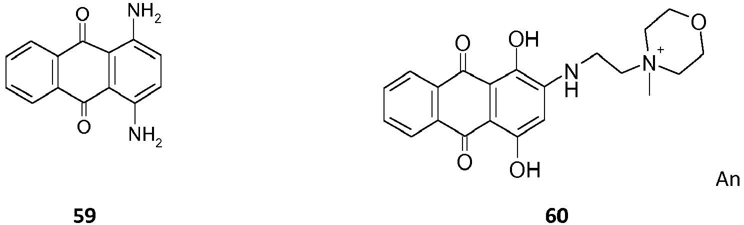 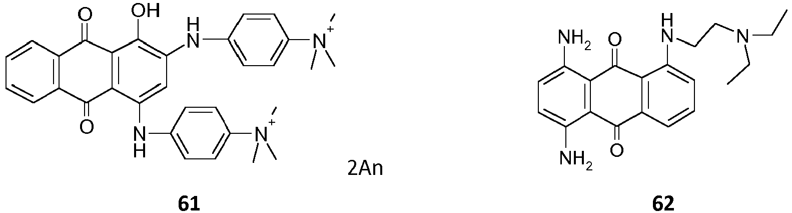 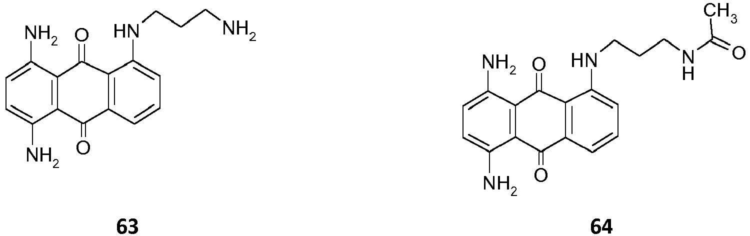 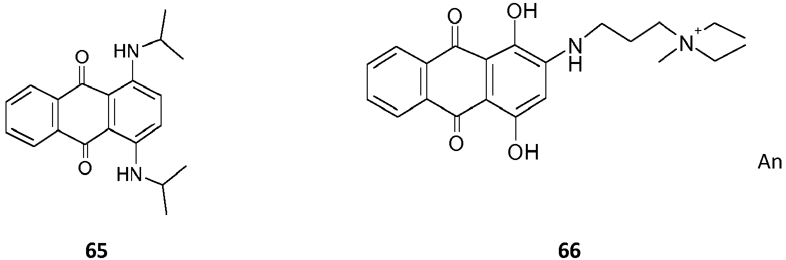 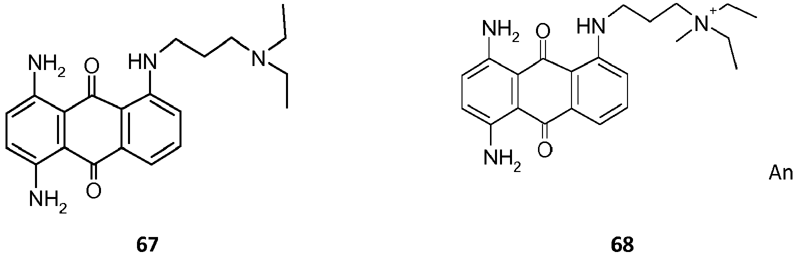 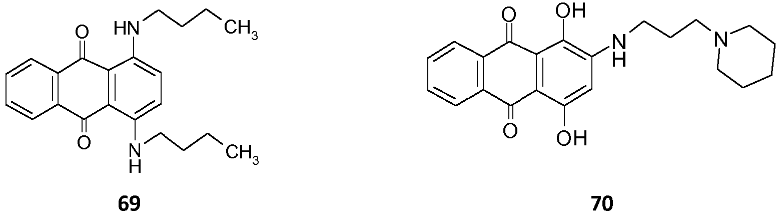 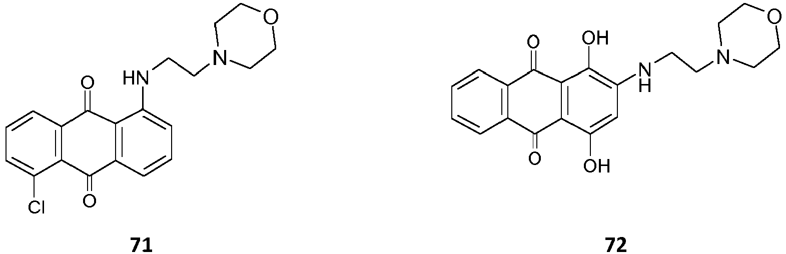 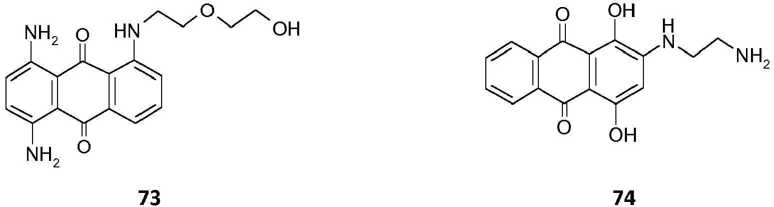 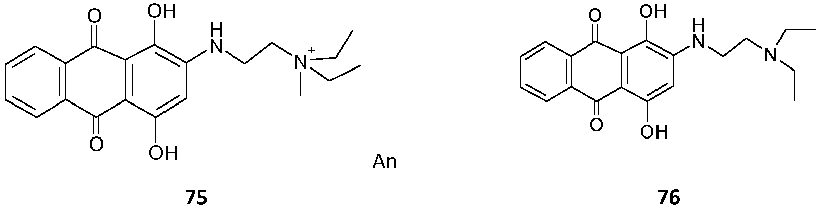 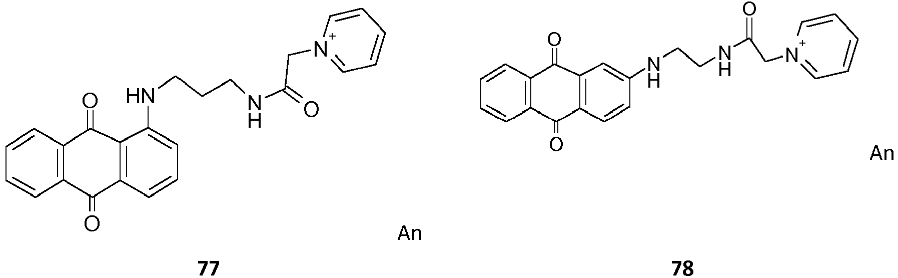 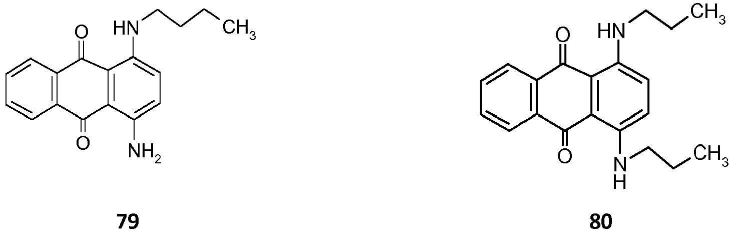 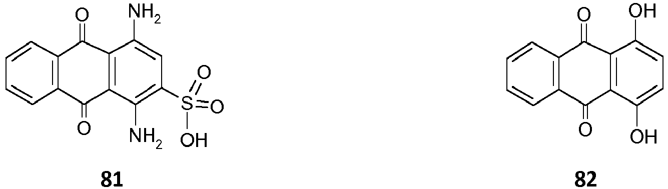 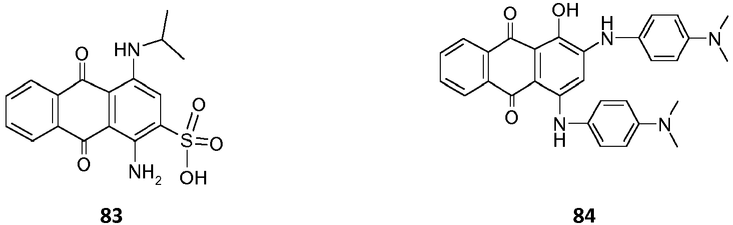 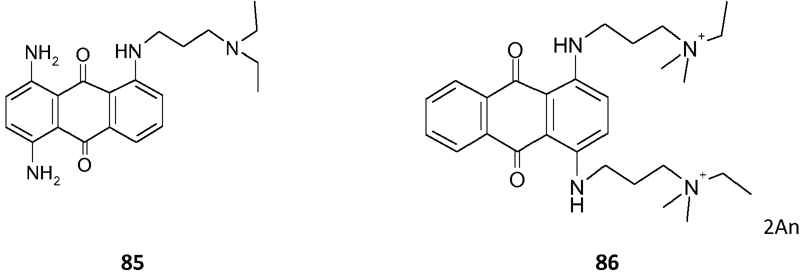 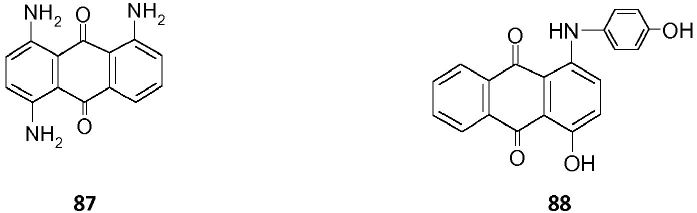 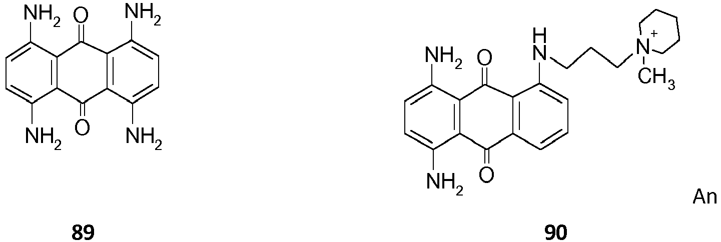 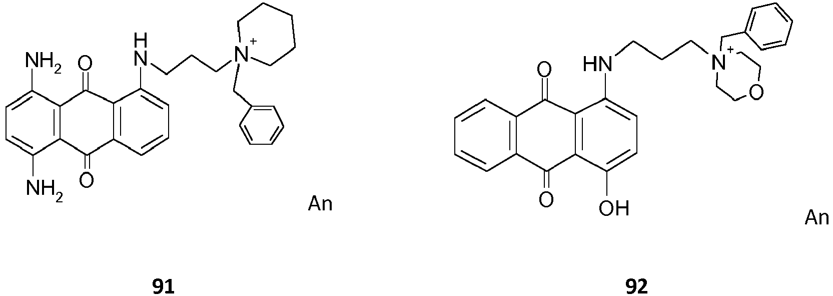 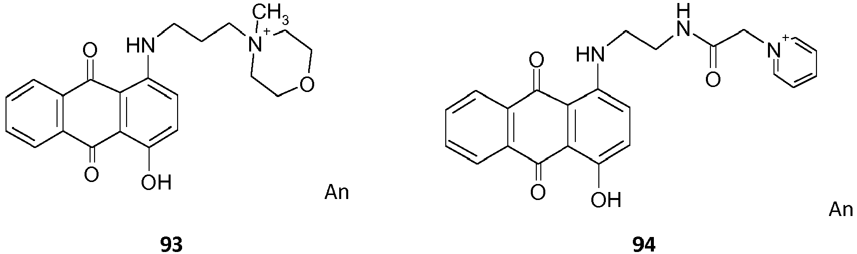 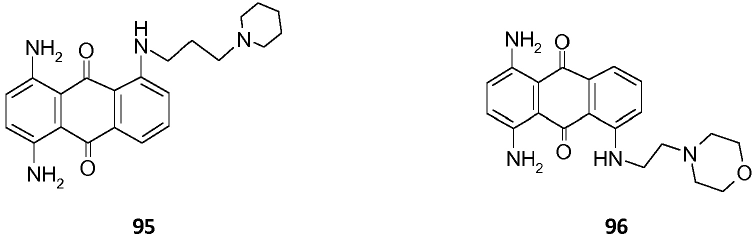 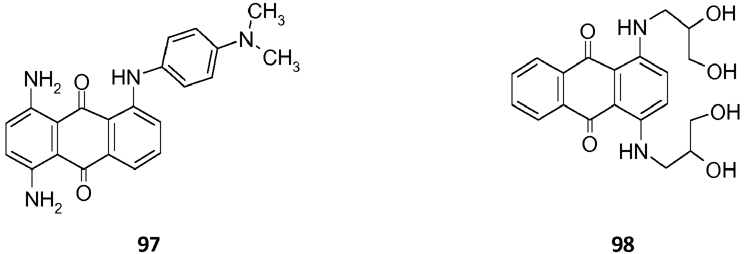 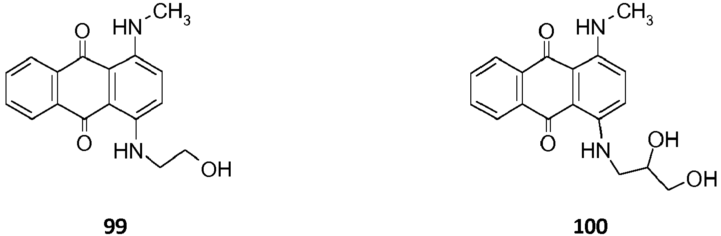 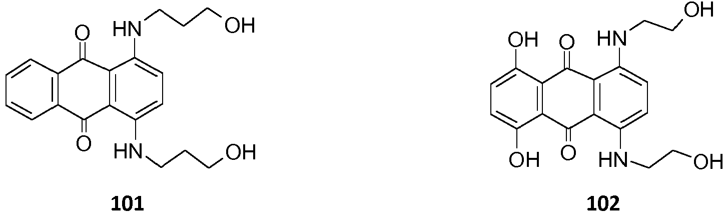 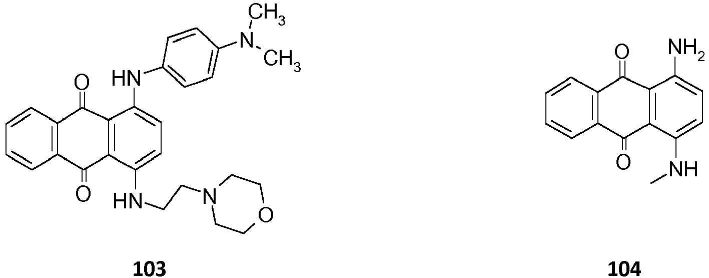 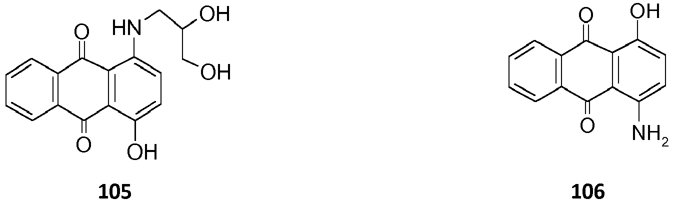 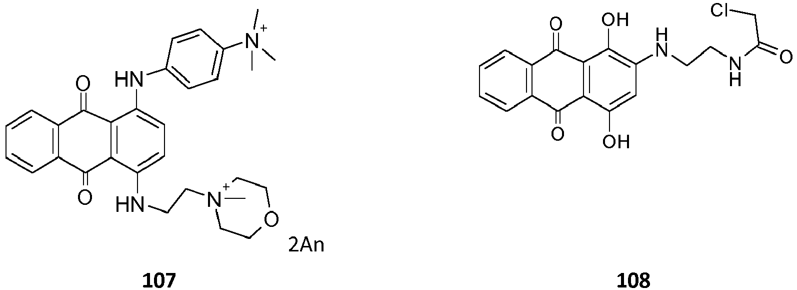 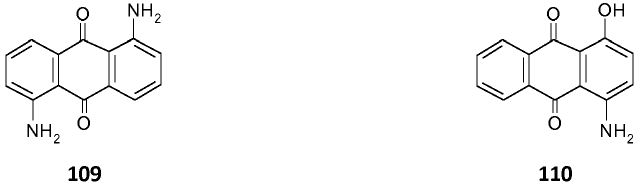 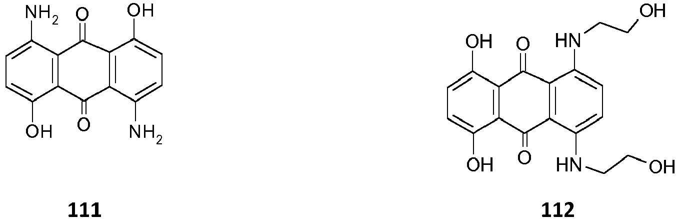 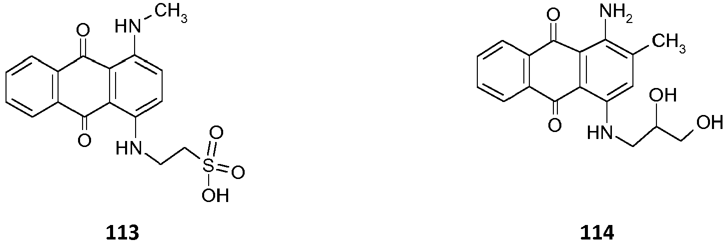 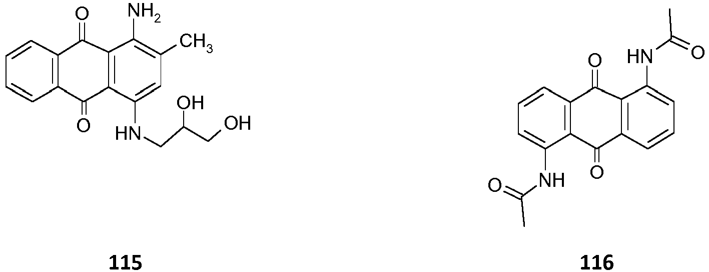 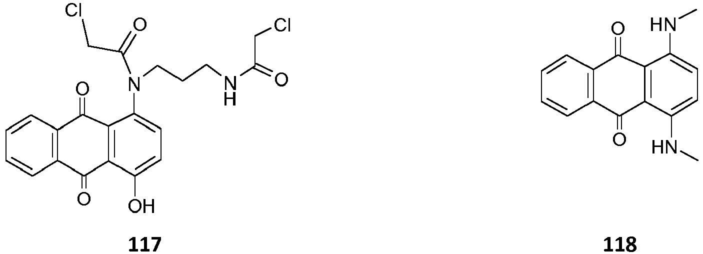 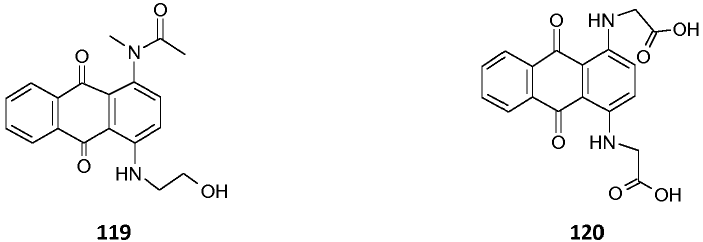 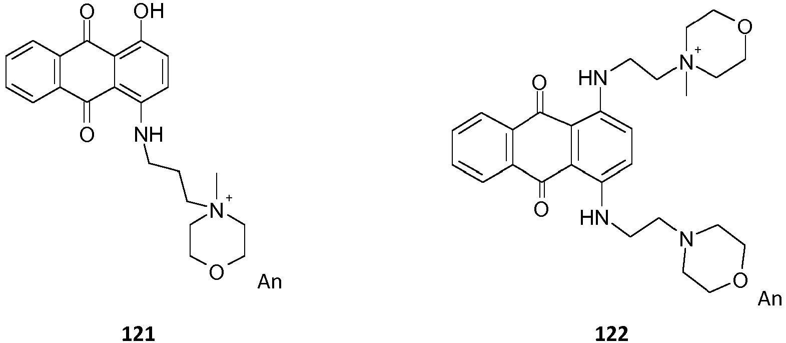 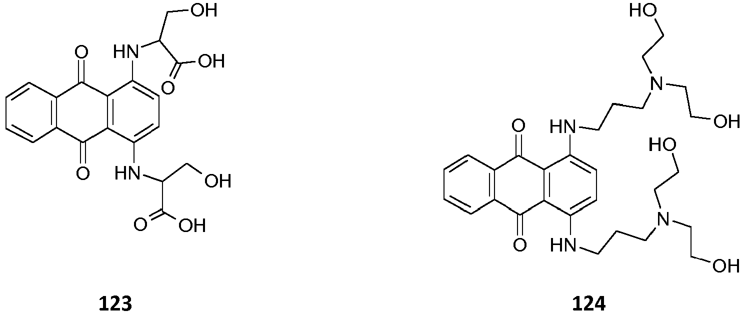 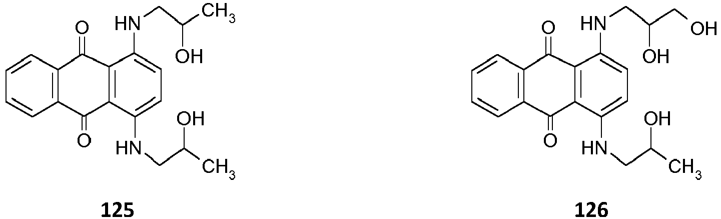 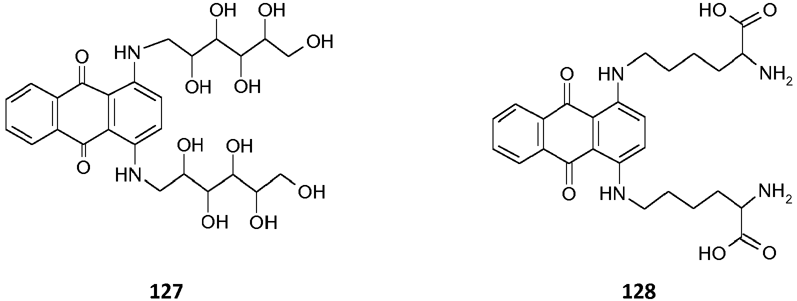 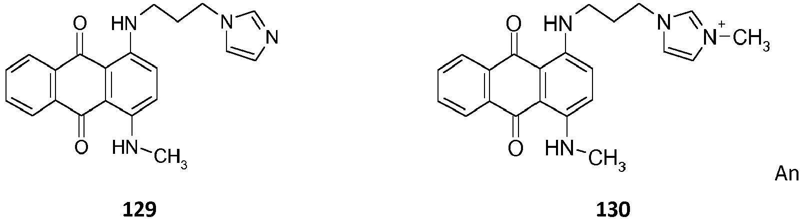 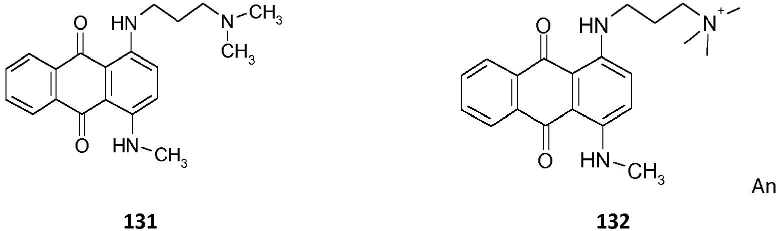 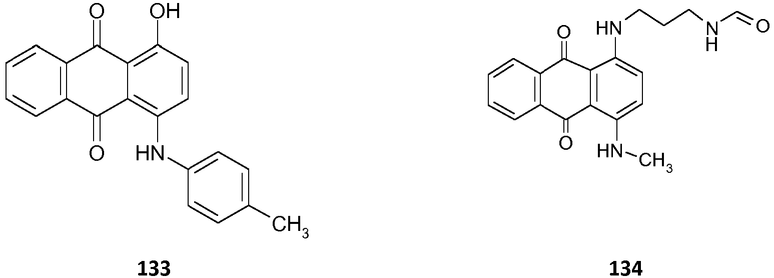 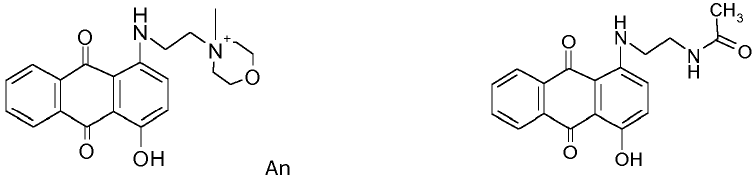 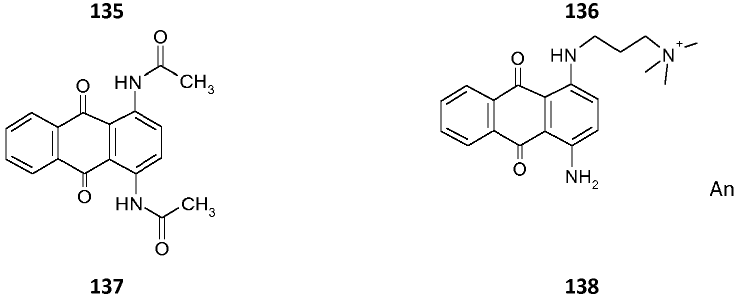 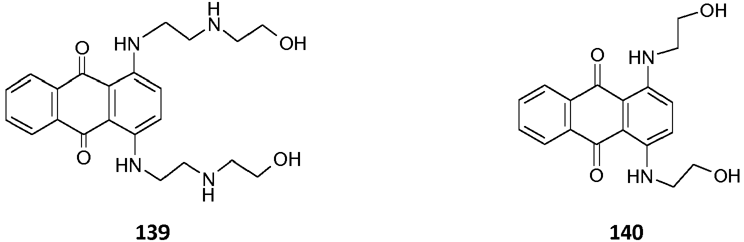 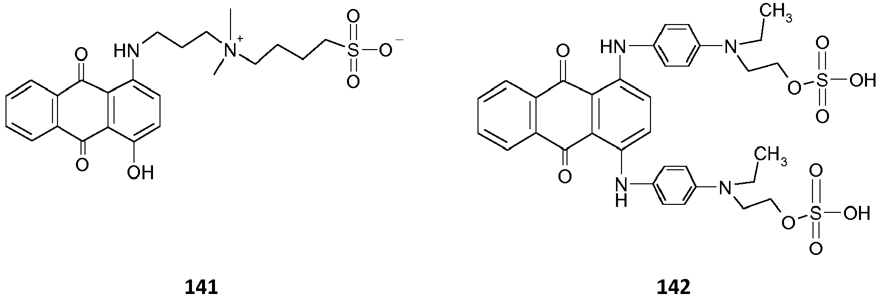 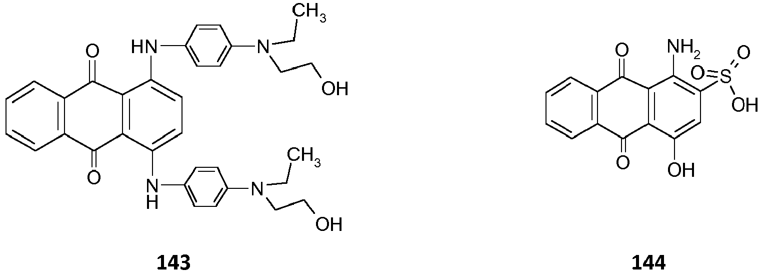 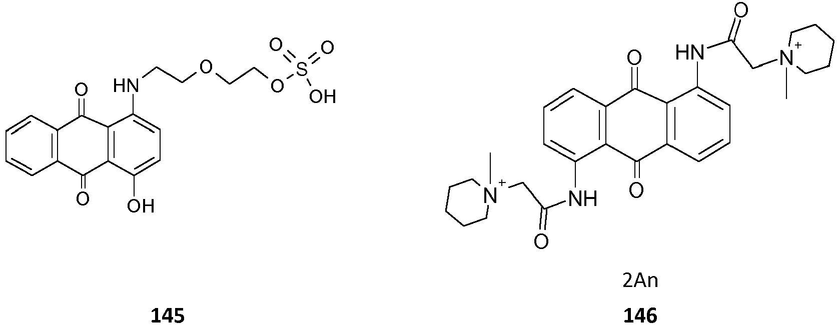 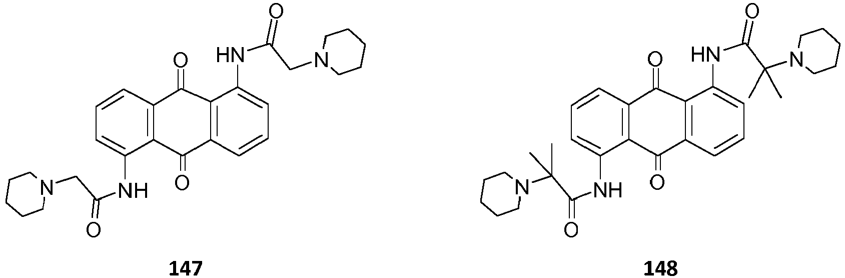 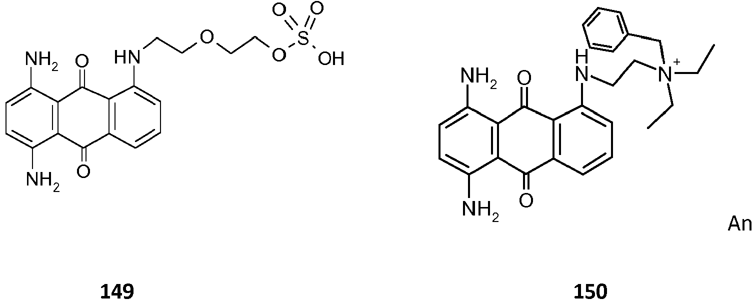 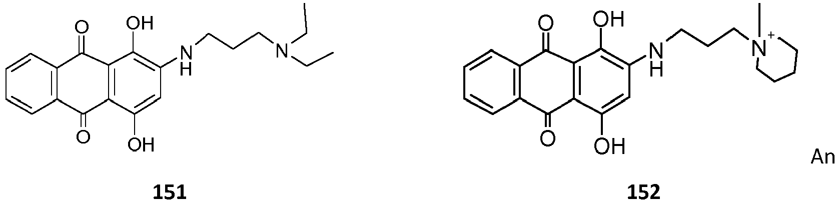 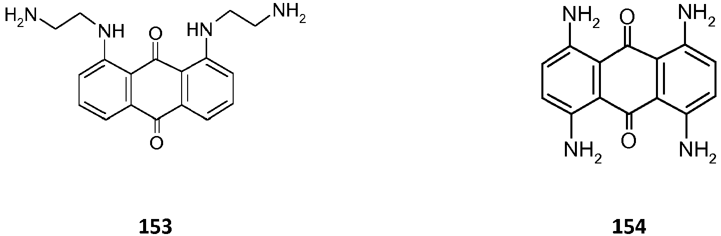 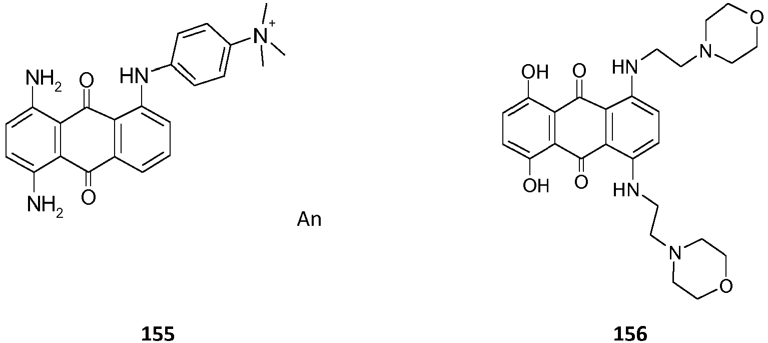 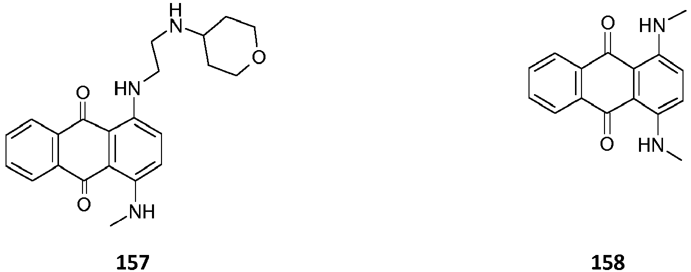 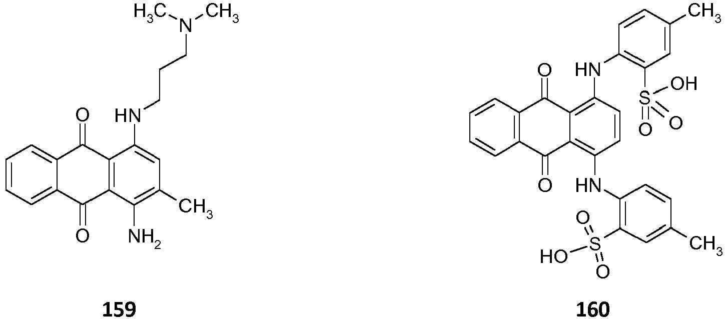 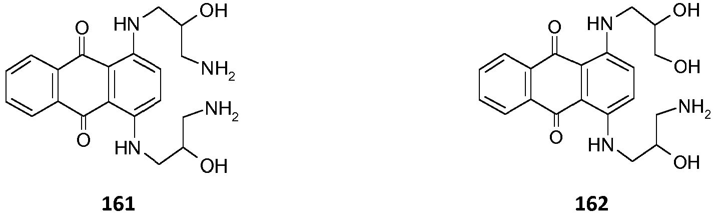 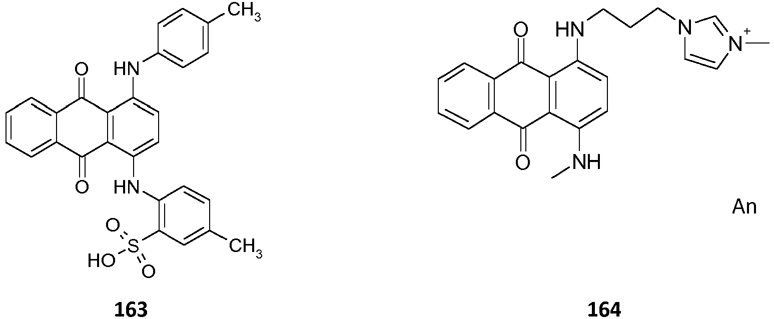 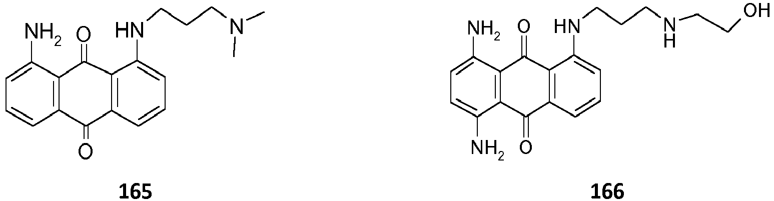 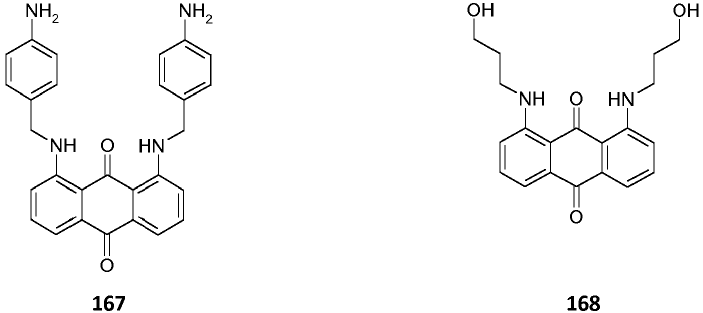 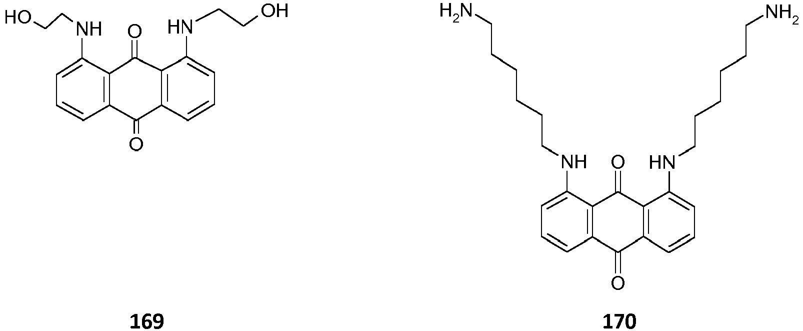 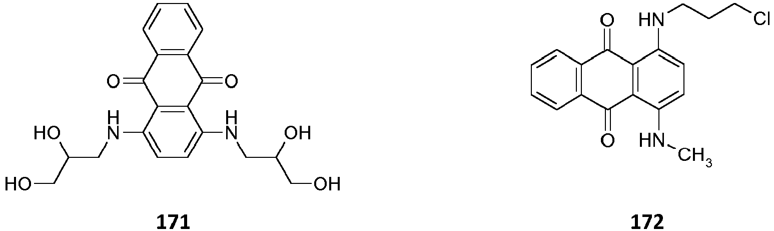 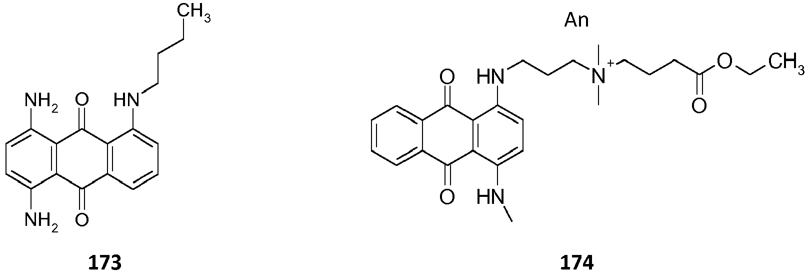 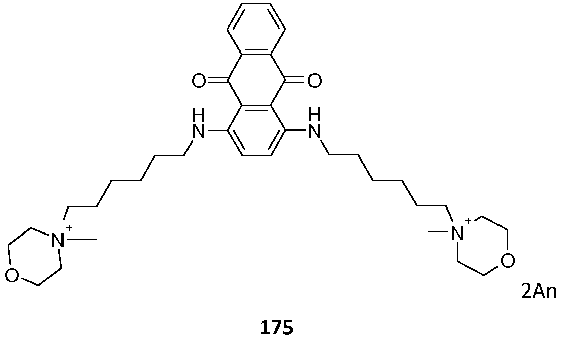 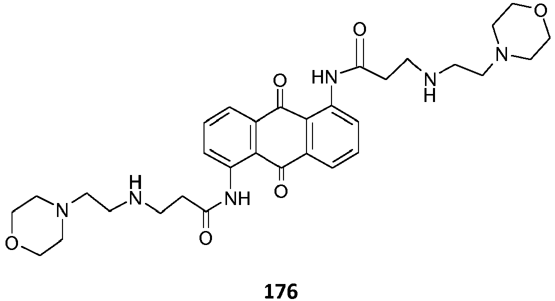 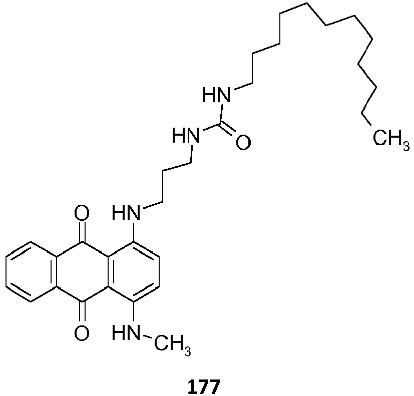 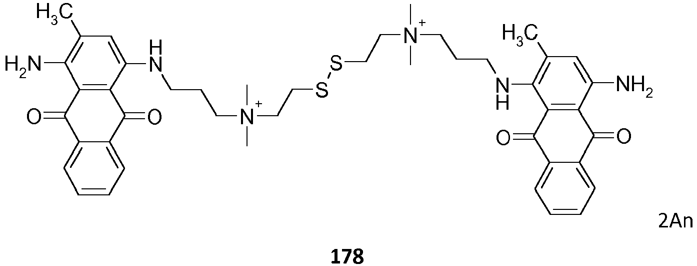 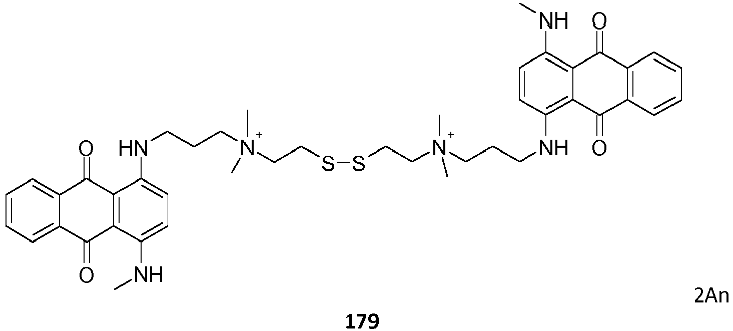 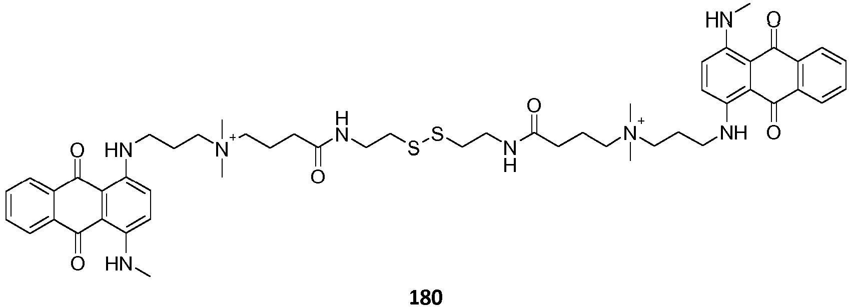 with **An,** which may be identical or different, representing an anionic counterion as defined previously, preferably chosen from mesylate, tosylate and halide such as Cl⁻ and I⁻.

13. Process according to any one of the preceding claims, in which the cationic (poly)methine fluorescent dye(s) are direct dyes chosen from cyanin dyes and styryl/hemicyanin dyes, and naphthalimide dyes, and mixtures thereof; more particularly, the cationic fluorescent dyes are direct and are chosen from styryl or hemicyanin cationic dyes.

14. Process according to any one of the preceding claims, in which the cationic (poly)methine fluorescent dye(s) (b) are dyes which absorb light in the yellow, orange and red range, preferably in the absorption wavelength λ_{abs} between 400 nm and 500 nm inclusive.

15. Process according to any one of the preceding claims, in which the cationic (poly)methine fluorescent dye(s) (b) are dyes which bear at least one cationic chromophore chosen from formulae **(III), (IV), (IIIa)** and **(IVa)** below:
**W⁺-[C(R^{c})=C(R^{d})]_{m'}-Ar'-(*)Q⁻** **(III)**
**Ar-[C(R^{d})=C(R^{c})]_{m'}-W'⁺-(*)Q⁻** **(IV),**
in which formulae **(III)** and **(IV):**
• **W⁺** represents a cationic heteroaryl group, in particular comprising a quaternary ammonium optionally substituted with one or more (C₁-C₈)alkyl groups optionally substituted in particular with one or more hydroxyl groups;
• **W'⁺** represents a divalent heteroaryl radical as defined for **W⁺;**
• **Ar** represents an aryl group such as phenyl or naphthyl, optionally substituted preferably with i) one or more halogen atoms such as chlorine or fluorine; ii) one or more (C₁-C₈)alkyl and preferably C₁-C₄ alkyl groups such as methyl; iii) one or more hydroxyl groups; iv) one or more (C₁-C₈)alkoxy groups such as methoxy; v) one or more hydroxy(C₁-C₈)alkyl groups such as hydroxyethyl, vi) one or more amino or (di)(C₁-C₈)alkylamino groups, preferably with the C₁-C₄ alkyl part optionally substituted with one or more hydroxyl groups, such as (di)hydroxyethylamino, vii) with one or more acylamino groups; viii) one or more heterocycloalkyl groups such as piperazinyl, piperidyl or 5- or 6-membered heteroaryl such as pyrrolidinyl, pyridyl and imidazolinyl;
• **Ar'** is a divalent aryl radical as defined for **Ar;**
• **m'** represents an integer between 1 and 4 inclusive, in particular, m is 1 or 2; better still 1;
• **R^{c}** and **R^{d},** which may be identical or different, represent a hydrogen atom or optionally a substituted (C₁-C₈)alkyl and preferably C₁-C₄ alkyl group, or alternatively **R^{c}** is contiguous with **W** or **W'** and/or **R^{d}** is contiguous with **Ar** or **Ar'** and form, with the atoms that bear them, a (hetero)cycloalkyl; in particular, **R^{c}** is contiguous with **W⁺** or **W'⁺** and forms a (hetero)cycloalkyl such as cyclohexyl;
• **Q⁻** is an organic or mineral anionic counterion;
• **(*)** represents the part of the fluorescent chromophore that is bonded to the rest of the dye;
preferably, **W⁺** or **W'⁺** is an imidazolium, pyridinium, benzimidazolium, pyrazolium, benzothiazolium or quinolinium radical optionally substituted with one or more identical or different C₁-C₄ alkyl radicals; particularly preferably, the fluorescent chromophore(s) are chosen from those with **m'** = 1, **Ar** representing a phenyl group substituted para to the styryl group -C(R^{d})=C(R^{c})- with a (di)(hydroxy)(C₁-C₆)(alkyl)amino group such as dihydroxy(C₁-C₄)alkylamino, and **W'⁺** representing an imidazolium or pyridinium group, preferably ortho- or para-pyridinium; 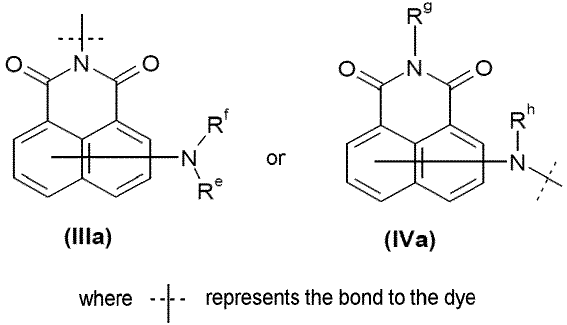 in which formulae **(IIIa)** and **(IVa)** R^{e}, R^{f}, R^{g} and R^{h}, which may be identical or different, represent a hydrogen atom or a (C₁-C₆)alkyl group which is optionally substituted, preferably with a di(C₁-C₆)alkylamino or tri(C₁-C₆)alkylammonium group such as trimethylammonium.

16. Process according to any one of the preceding claims, in which the cationic (poly)methine fluorescent dye(s) (b) are chosen from the dyes of formulae (V), **(VI)** and **(VII):** 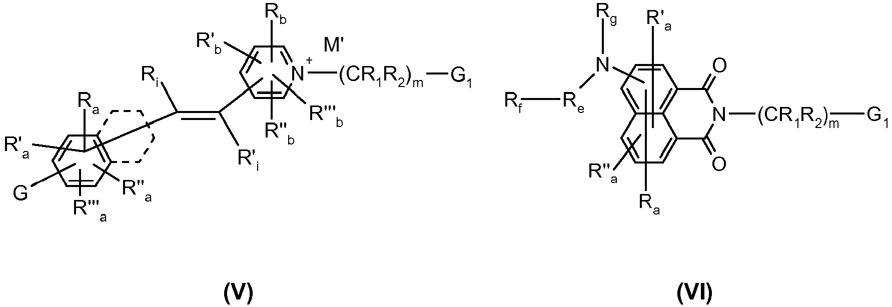 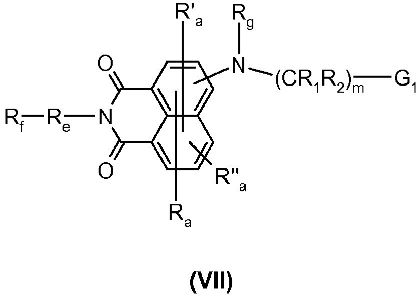 and also the organic or mineral acid or base salts thereof, the optical isomers, geometrical isomers and tautomers thereof, and the solvates thereof such as hydrates;
in which formulae **(V), (VI)** and **(VII):**
• **R₁** and **R₂,** which may be identical or different, represent a hydrogen atom or a C₁-C₆ alkyl group; preferentially a hydrogen atom;
• **G₁** represents a hydrogen atom or a group chosen from NH₂ and OH; preferably hydrogen;
• **Rₐ, R'ₐ, R"ₐ, R'''ₐ, R_{b}, R'_{b}, R"_{b},** and **R'''_{b},** which may be identical or different, represent a) a hydrogen atom, b) a halogen atom, a group from among: c) amino, d) (C₁-C₄)alkylamino, e) (C₁-C₄)dialkylamino, f) cyano, g) carboxyl -C(O)OH or carboxylate-C(O)O⁻, Q⁺, h) hydroxy -OH or alkoxide -O⁻Q⁺, i) (poly)halo(C₁-C₆)alkyl such as trifluoromethyl, j) acylamino, k) (C₁-C₆)alkoxy, I) (C₁-C₆)alkylthio, m) (poly)hydroxy(C₂-C₄)alkoxy, n) (C₁-C₆)alkylcarbonyloxy, o) (C₁-C₆)alkoxycarbonyl, p) (C₁-C₆)alkylcarbonylamino, q) acylamino, r) carbamoyl, s) (C₁-C₆)alkylsulfonylamino, t) aminosulfonyl, u) -SO₃H or sulfonate -SO₃⁻, Q⁺ or v) (C₁-C₆)alkyl optionally substituted with a group chosen from (C₁-C₆)alkoxy, hydroxyl, cyano, carboxyl, amino, (di)(C₁-C₄)alkylamino, or alternatively the two alkyl radicals borne by the nitrogen atom of the amino group form a 5- to 7-membered heterocycle optionally comprising another nitrogen or non-nitrogen heteroatom; particularly, **Rₐ, R'ₐ, R"ₐ, R'''ₐ, R_{b}, R'_{b}**, **R"_{b}** and **R'''_{b}** represent a hydrogen or halogen atom or a (C₁-C₄)alkyl group, preferably a hydrogen atom;
• or alternatively two groups **Rₐ** and **R'ₐ; R_{b}** and **R'_{b},** borne by two adjacent carbon atoms, together form a benzo or indeno ring, a fused heterocycloalkyl or fused heteroaryl group; the benzo, indeno, heterocycloalkyl or heteroaryl ring being optionally substituted with a halogen atom, an amino, (C₁-C₄)alkylamino, (C₁-C₄)dialkylamino, nitro, cyano, carboxyl, hydroxyl or trifluoromethyl group, an acylamino, (C₁-C₄)alkoxy (poly)hydroxy(C₁-C₄)alkoxy, (C₁-C₄)alkylcarbonyloxy, (C₁-C₄)alkoxycarbonyl or (C₁-C₄)alkylcarbonylamino radical, an acylamino, carbamoyl or alkoxyalkylsulfonylamino radical, an aminosulfonyl radical, or a (C₁-C₆)alkyl radical optionally substituted with: a group chosen from (C₁-C₆)alkoxy, hydroxyl, cyano, carboxyl, amino, (C₁-C₄)alkylamino and (C₁-C₄)dialkylamino, or alternatively the two alkyl radicals borne by the nitrogen atom of the amino group form a 5- to 7-membered heterocycle optionally comprising another nitrogen or non-nitrogen heteroatom; preferentially, **Rₐ** and **R'ₐ** together form a benzo group;
• or alternatively, two groups Rᵢ and Rₐ; and/or a group R'ᵢ and R'ₐ together form a fused (hetero)cycloalkyl, preferentially cycloalkyl such as cyclohexyl;
• **R_{g}** represents a hydrogen atom, a (hetero)aryl(C₁-C₄)alkyl group or a (C₁-C₆)alkyl group that is optionally substituted; preferentially, R_{b} represents a hydrogen atom or a (C₁-C₃)alkyl or benzyl group;
• **Rₑ** represents a covalent bond, a linear or branched, optionally substituted (C₁-C₈)alkylene or (C₂-C₈)alkenylene hydrocarbon-based chain, preferably **Rₑ** represents an unsubstituted (C₁-C₆)alkylene;
• **R_{f}** represents a hydrogen atom, a (C₁-C₄)alkoxy group, an amino group R₃R₄N-, a quaternary ammonium group M', R₃R₄R₅N⁺- in which R₃, R₄ and R₅, which may be identical or different, represent a hydrogen atom or a (C₁-C₄)alkyl group or R₃R₄N-represents an optionally substituted heteroaryl group, preferentially an optionally substituted imidazolyl group, or alternatively M', R₃R₄R₅N⁺- represents an optionally substituted cationic heteroaryl group, preferentially an imidazolinium group optionally substituted with a (C₁-C₃)alkyl group;
• **G** represents a group i) -NR_{c}R_{d}, ii) -OR with R representing a) a hydrogen atom, b) an optionally substituted, preferentially unsubstituted (C₁-C₆)alkyl group, c) an optionally substituted (hetero)aryl group, d) an optionally substituted (hetero)aryl(C₁-C₆)alkyl group such as benzyl, e) optionally substituted (hetero)cycloalkyl group, f) optionally substituted (hetero)cycloalkyl(C₁-C₆)alkyl group; according to a particular embodiment, G represents a group -NR_{c}R_{d}, according to another particular embodiment, G represents a (C₁-C₆)alkoxy group;
or alternatively when **G** represents -NR_{c}R_{d}, two groups R_{c} and R'ₐ and/or R_{d} and Rₐ together form a saturated heteroaryl or heterocycle, optionally substituted with one or more (C₁-C₆)alkyl groups, preferentially a 5- to 7-membered heterocycle containing one or two heteroatoms chosen from nitrogen and oxygen; more preferentially, the heterocycle is chosen from morpholinyl, piperazinyl, piperidyl and pyrrolidinyl groups;
• **R_{c}** and **R_{d},** which may be identical or different, represent a hydrogen atom or a group from among: a) optionally substituted (hetero)aryl such as phenyl, b) optionally substituted (hetero)aryl(C₁-C₄)alkyl, c) optionally substituted (hetero)cycloalkyl, d) optionally substituted (hetero)cycloalkyl(C₁-C₄)alkyl, f) (C₂-C₅)alkyl or g) (C₁-C₈)alkyl which is optionally substituted, preferably optionally substituted with a hydroxyl, carboxyl, carboxylate, sulfate or sulfonate group;
or alternatively two adjacent radicals **R_{c}** and **R_{d}** borne by the same nitrogen atom together form an optionally substituted heterocyclic or optionally substituted heteroaryl group;
• **Rᵢ** and **R'ᵢ,** which may be identical or different, represent a hydrogen atom or a (C₁-C₄)alkyl group;
• 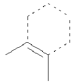 represents a (hetero)aryl group fused to the phenyl ring; or alternatively is absent from the phenyl; preferentially, when the ring is present, the ring is a benzo;
• **m** represents an integer between 1 and 18 inclusive, particularly an integer between 1 and 14 inclusive; preferentially an integer between 2 and 10 inclusive; more preferentially an integer between 3 and 8; more particularly an integer between 4 and 6;
• **M'** represents an anionic counterion, derived from a salt of an organic or mineral acid, or from an organic or mineral base that ensures the electrical neutrality of the molecule;
• **Q⁺** represents a cationic counterion, derived from a salt of an organic or mineral acid, or from an organic or mineral base that ensures the electrical neutrality of the molecule such as alkali metal, alkaline-earth metal or ammonium;
it being understood that when the molecule comprises a carboxylate, sulfonate or alkoxide group, then M' and Q⁺ may be absent to ensure the electrical neutrality of said molecule.

17. Process according to any one of the preceding claims, in which the cationic (poly)methine fluorescent dye(s) (b) are chosen from the styryl dyes of formula **(VIII)** below: 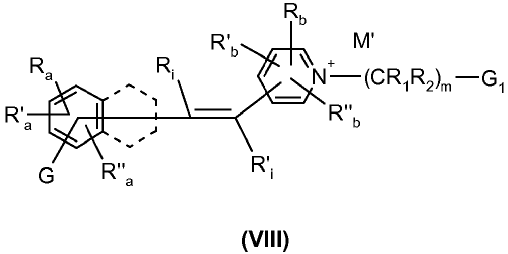 and also the organic or mineral acid or base salts thereof, the optical isomers, geometrical isomers and tautomers thereof, and the solvates thereof such as hydrates;
in which formula **(VIII) G, G₁, Rₐ, R'ₐ, R"ₐ, R_{b}, R'_{b}, R"_{b}, Rᵢ, R'ᵢ, R₁, R₂** and **m** are as defined in the preceding claim;
particularly:
• **R₁** and **R₂**, which may be identical or different, represent a hydrogen atom;
• **Rᵢ** and **R**_{**i**'}, which may be identical or different, represent a hydrogen atom or a (C₁-C₄)alkyl group, preferably hydrogen;
• **Rₐ, R'ₐ** and **R"ₐ**, which may be identical or different, represent a hydrogen atom, a halogen atom such as fluorine, or an -OH, -O⁻Q⁺, (C₁-C₆)alkoxy, nitro, or cyano group, with Q⁺ as defined in the preceding claim;
• **R_{b}, R'_{b}** and **R"_{b}**, which may be identical or different, represent a hydrogen atom or a (C₁-C₆)alkyl group;
• or alternatively two contiguous radicals **R_{b}** and **R'_{b}** form, together with the carbon atoms that bear them, a benzo group that is condensed or fused to the pyridinium group, said benzo group possibly being substituted; preferably, said benzo group is unsubstituted;
• **G** represents a group -NR_{c}R_{d} or (C₁-C₆)alkoxy group which is optionally substituted, preferentially unsubstituted; according to a particular embodiment, G represents a group -NR_{c}R_{d}, according to another particular embodiment, G represents a (C₁-C₆)alkoxy group;
• **G₁** represents a hydrogen atom or an OH or NH₂ group, preferably hydrogen;
• **Rᵢ** and **R'ᵢ**, which may be identical or different, represent a hydrogen atom or a (C₁-C₄)alkyl group;
• 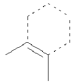 represents an aryl or heteroaryl group fused to the phenyl ring; or alternatively is absent from the phenyl ring; preferentially, when the ring is present, the ring is a benzo;
• **m** represents an integer between 1 and 18 inclusive; particularly an integer between 2 and 16 inclusive; preferentially an integer between 3 and 10; more preferentially an integer between 4 and 6;
• **R_{c}** and **R_{d},** which may be identical or different, represent a hydrogen atom, a (C₂-C₄)alkyl group or a substituted (C₁-C₈)alkyl group, preferably (C₂-C₄)alkyl substituted in particular with one or more groups chosen from i) cyano, ii) (C₁-C₃)alkoxy, iii) hydroxyl and iv) (C₁-C₃)alkylcarbonyl, preferably with one or more hydroxyl groups; and
• **M'** represents an anionic counterion, derived from a salt of an organic or mineral acid, or from an organic or mineral base that ensures the electrical neutrality of the molecule;
it being understood that when the molecule comprises an alkoxide group, then M' and Q⁺ may be absent to ensure the electrical neutrality of said molecule.

18. Process according to any one of the preceding claims, in which the cationic (poly)methine fluorescent dye(s) (b) are chosen from the dyes of the compounds of formulae **(X), (XI), (XII)** and **(XIII)** below: 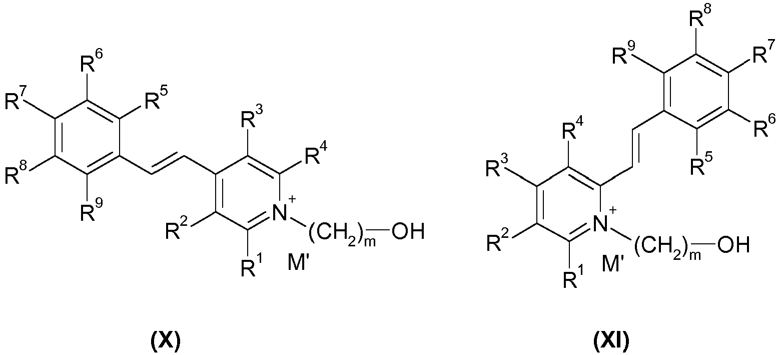 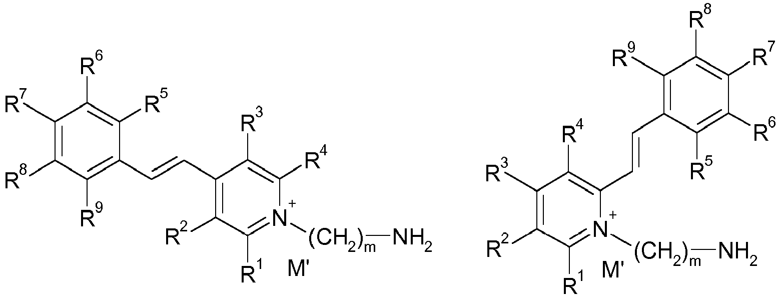 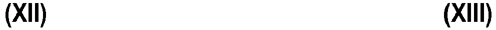 and also the organic or mineral acid salts thereof, the optical isomers, geometrical isomers and tautomers thereof, and the solvates thereof such as hydrates;
in which formula **(X),(XI), (XII)** or **(XIII):**
• **R¹, R², R³** and **R⁴,** which may be identical or different, represent a hydrogen atom or a (C₁-C₆)alkyl group; preferably, **R²** and **R³** represent a hydrogen atom and **R¹** and **R⁴,** which may be identical or different, represent a hydrogen atom or a (C₁-C₄)alkyl group;
• **R⁵, R⁶, R⁷, R⁸** and **R⁹,** which may be identical or different, represent i) a hydrogen atom or ii) a halogen atom such as Cl, Br or F, iii) a group OR in which R represents a hydrogen atom or Q⁺ as described previously, or a (C₁-C₃)alkyl group, iv) aryl such as benzene, v) an aryl(C₁-C₃)alkyl group such as benzyl, vi) cyano, vii) nitro, viii) (C₁-C₃)alkylthio, ix) amino NR¹⁰R¹¹ with R¹⁰ and R¹¹, which may be identical or different, representing a) a hydrogen atom, b) a (C₂-C₄)alkyl group or c) a substituted (C₁-C₈)alkyl group, preferably (C₂-C₄)alkyl optionally substituted with one or more groups chosen from:
- cyano,
- (C₁-C₃)alkoxy,
- hydroxyl, and
- (C₁-C₃)alkylcarbonyl;
in particular, **R¹⁰** and **R¹¹,** which may be identical or different, represent a hydrogen atom or a (C₁-C₆)alkyl group substituted with one or more hydroxyl, cyano or (C₁-C₃)alkylcarbonyl groups such as hydroxyethyl;
• **m** represents an integer between 1 and 18 inclusive; particularly an integer between 2 and 16 inclusive; preferentially an integer between 3 and 10; more preferentially an integer between 4 and 6;
• **M'** represents an anionic counterion as defined in the preceding claim;
it being understood that when the molecule comprises an alkoxide group, then M' and Q⁺ may be absent to ensure the electrical neutrality of said molecule.

19. Process according to any one of Claims 1 to 18, in which the cationic (poly)methine fluorescent dye(s) (b) of the invention are chosen from those of formulae **(XIV)** and **(XV)** below: 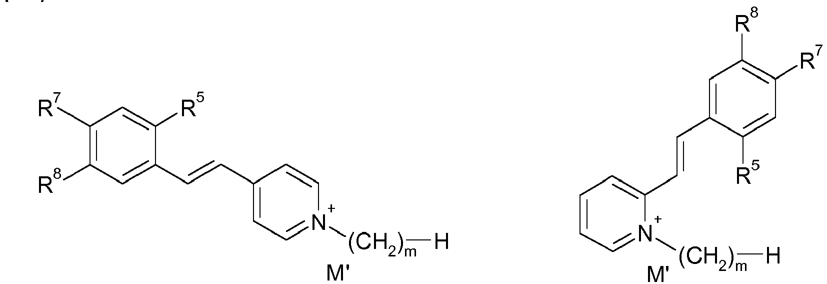 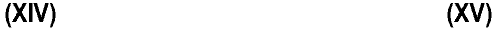 in which formulae **(XIV)** and **(XV) R⁵, R⁷, R⁸** and **m** are as defined previously for **(X)** and **(XI)**, in particular:
• **R⁵** and **R⁸,** which may be identical or different, represent a hydrogen atom or a (C₁-C₄)alkoxy group such as methoxy, preferably, R⁵ and R⁸ represent a hydrogen atom;
• **R⁷** represents a (C₁-C₄)alkoxy group or NR¹⁰R¹¹ with R¹⁰ and R¹¹, which may be identical or different, representing a) a hydrogen atom, or b) a (C₁-C₈)alkyl group optionally substituted with one or more groups chosen from i) hydroxyl, ii) R-Z-C(X)-Y- with X, Y and Z representing an oxygen or sulfur atom or N(R'), or alternatively X and/or Z represent a bond, R and R', which may be identical or different, represent a hydrogen atom or a (C₁-C₆)alkyl group, preferably, X represents an oxygen atom, iii) sulfonic SO₃H, iv) sulfonate SO₃⁻, Q⁺, v) carboxylate C(O)O⁻, Q⁺ with Q⁺ representing a cationic counterion such as an alkali metal or alkaline-earth metal; in particular, R⁷ represents a group NR¹⁰R¹¹ with R¹⁰ and R¹¹, which may be identical or different, representing a) a hydrogen atom, or b) a (C₁-C₆)alkyl group optionally substituted with one or more groups chosen from i) hydroxyl, ii) carboxyl, iii) carboxylate, iv) sulfonic, and v) sulfonate, more particularly chosen from identical or different groups representing a) a hydrogen atom, or b) a (C₁-C₆)alkyl group optionally substituted with one or more groups chosen from i) hydroxyl, ii) carboxyl, and iii) carboxylate;
• **m** represents an integer between 1 and 18 inclusive; particularly an integer between 1 and 6 inclusive; preferentially an integer between 1 and 4; more preferentially an integer between 1 and 2; and
• **M'** represents an anionic counterion, derived from a salt of an organic or mineral acid, or from an organic or mineral base that ensures the electrical neutrality of the molecule;
• it being understood that when the molecule comprises an alkoxide group, then M' and Q⁺ may be absent to ensure the electrical neutrality of said molecule.

20. Process according to any one of the preceding claims, in which:
• the cationic (poly)methine fluorescent dye(s) (b) are of formula **(X)** with:
| **R¹** | **R²** | **R³** | **R⁴** | **R⁵** | **R⁶** | **R⁷** | **R⁸** | **R⁹** | **m** |
|---|---|---|---|---|---|---|---|---|---|
| H | H | H | H | H | H | H | H | H | 1 |
| H | H | H | H | H | t-Bu | OH | t-Bu | H | 5 |
| H | H | H | H | H | t-Bu | OH | t-Bu | H | 5 |
| H | H | H | H | H | H | NH₂ | H | H | 1 |
| H | H | H | H | H | H | NH₂ | H | OCH₃ | 1 |
| H | H | H | H | H | H | OH | Br | H | 5 |
| H | H | H | H | H | OCH₃ | OH | OCH₃ | H | 5 |
| H | H | H | H | Cl | H | OH | H | Cl | 5 |
| H | H | H | H | H | H | OH | H | H | 10 |
| H | H | H | H | H | OCH₃ | OH | OCH₃ | H | 10 |
| H | H | H | H | H | t-Bu | OH | t-Bu | H | 10 |
| H | H | H | H | H | H | N(CH₂CH₃)₂ | H | H | 1 |
| H | H | H | H | H | H | N(CH₂CH₃)₂ | H | H | 1 |
| H | H | H | H | H | H | N(CH₂CH₃)₂ | H | H | 1 |
| H | H | H | H | H | t-Bu | OH | t-Bu | H | 10 |
| H | H | H | H | H | H | N(CH₂CH₃)CH₂CH₂OH | H | H | 1 |
| H | H | H | H | H | H | N(CH₂CH₃)₂ | H | H | 1 |
| H | H | H | H | H | H | N(CH₂CH₃)₂ | H | H | 1 |
| H | H | H | H | H | H | N(CH₂CH₃)₂ | H | H | 1 |
| H | H | H | H | H | H | N(CH₂CH₃)₂ | H | H | 1 |
| H | H | H | H | H | H | N(CH₂CH₃)₂ | H | H | 1 |
| H | H | H | H | H | H | N(CH₂CH₃)₂ | H | H | 1 |
| H | H | H | H | H | H | N(CH₂CH₃)₂ | H | H | 1 |
| H | H | H | H | H | H | N(CH₂CH₃)₂ | H | H | 1 |
| H | H | H | H | H | H | N(CH₂CH₃)₂ | H | H | 1 |
| H | H | H | H | H | H | n-C₆H₁₃ | H | H | 1 |
| H | H | H | H | H | H | N(CH₂CH₂OHh | H | H | 2 |
| H | H | H | H | H | H | N(n-Bu)₂ | H | H | 2 |
| H | H | H | H | H | OCH₃ | OH | H | H | 10 |
| H | H | H | H | H | H | OC₂H₅OH | H | H | 1 |
| H | H | H | H | H | H | OH | H | H | 1 |
| H | H | benzo | | H | H | H | H | H | 1 |
| H | H | benzo | | H | H | N(CH₂CH₂OHh | H | H | 1 |
| H | H | benzo | | H | H | N(CH₂CH₂OHh | H | H | 1 |
and also the organic or mineral acid or base salts thereof, the optical isomers, geometrical isomers and tautomers thereof, and the solvates thereof such as hydrates;
The cationic (poly)methine fluorescent dye(s) (b) of formula **(XI)** with:
| **R¹** | **R²** | **R³** | **R⁴** | **R⁵** | **R⁶** | **R⁷** | **R⁸** | **R⁹** | **m** |
|---|---|---|---|---|---|---|---|---|---|
| CH₃ | H | H | H | OH | H | OCH₃ | H | H | 2 |
| CH₃ | H | H | H | H | H | OCH₃ | OCH₂-Ph | H | 2 |
| CH₃ | H | H | H | H | H | H | H | OC H₃ | 2 |
| CH₃ | H | H | H | F | H | H | H | H | 2 |
| CH₃ | H | H | H | H | H | H | OPh | H | 2 |
| CH₃ | H | H | H | H | H | N(CH₂CH₂OAc)₂ | H | H | 2 |
| CH₃ | H | H | H | OCH₃ | H | OCH₃ | OCH₃ | H | 2 |
| CH₃ | H | H | H | H | H | H | CH₃ | H | 2 |
| CH₃ | H | H | H | H | H | OH | H | H | 2 |
| CH₃ | H | H | H | H | OCH₃ | OCH₃ | OCH₃ | H | 2 |
| CH₃ | H | H | H | H | OCH₃ | OH | OH | H | 2 |
| CH₃ | H | H | H | H | H | OCH₃ | OCH₃ | OC H₃ | 2 |
| CH₃ | H | H | H | H | H | H | OCH₃ | OH | 2 |
| CH₃ | H | H | H | H | H | N(n-butyl)₂ | H | H | 2 |
| CH₃ | H | H | H | H | OCH₃ | OCH₃ | H | H | 2 |
| CH₃ | H | H | H | H | H | H | H | H | 2 |
| CH₃ | H | H | H | H | H | i-propyl | H | H | 2 |
| H | H | H | H | H | H | OH | H | H | 6 |
| H | H | H | H | H | OCH₃ | OCH₃ | OCH₃ | H | 6 |
| H | H | H | H | OH | H | OCH₃ | H | H | 2 |
| H | H | H | H | OCH₃ | H | H | OCH₃ | OC H₃ | 2 |
| H | H | H | H | H | H | H | H | Br | 2 |
| H | H | H | H | H | H | OH | H | H | 2 |
| H | H | H | H | OCH₃ | OCH₃ | OCH₃ | H | H | 6 |
| H | H | H | H | OH | OCH₃ | H | H | H | 6 |
| H | H | H | H | H | OCH₃ | OCH₃ | H | H | 6 |
| H | H | H | H | H | OCH₃ | OCH₃ | H | H | 2 |
| H | H | H | H | H | H | C(O)-OH | H | H | 6 |
| H | H | H | H | H | H | C(O)-OH | H | H | 2 |
| H | H | H | H | H | H | i-propyl | H | H | 2 |
| H | H | H | H | H | H | N(CH₃)CH₂CH₂CN | H | H | 2 |
| H | H | H | H | H | H | OCH₃ | OCH₂Ph | H | 2 |
| H | H | H | H | H | H | H | OPh | H | 2 |
| H | H | H | H | H | H | N(CH₂CH₂C(O)CH₃)₂ | H | H | 2 |
| H | H | H | H | OH | H | OCH₃ | H | H | 6 |
| H | H | H | H | H | OCH₃ | OCH₃ | OCH₃ | H | 2 |
| H | H | H | H | OCH₃ | H | OCH₃ | OCH₃ | H | 6 |
| H | H | H | H | H | H | H | CH₃ | H | 2 |
| H | H | H | H | H | H | N(CH₃)CH₂CH₂OH | H | H | 2 |
and also the organic or mineral acid or base salts thereof, the geometrical isomers and tautomers thereof, and the solvates thereof such as hydrates;
• the cationic (poly)methine fluorescent dye(s) (b) of formulae **(X')** and **(XI'):** 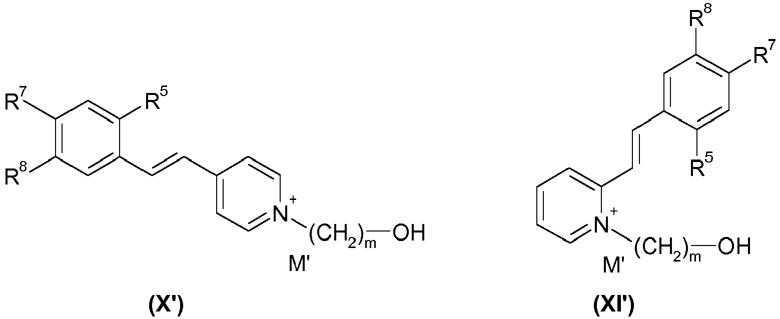 with M' as defined in the preceding claim; and
| **R⁵** | **R**⁷ | **R⁸** | **m** | | **R⁵** | **R**⁷ | **R⁸** | **m** |
|---|---|---|---|---|---|---|---|---|
| H | N(CH₂CH₂OH)₂ | H | 2 | | OCH₃ | OCH₃ | OCH₃ | 2 |
| H | N(CH₂CH₂OH)₂ | H | 3 | | OCH₃ | OCH₃ | OCH₃ | 3 |
| H | N(CH₂CH₂OH)₂ | H | 4 | | OCH₃ | OCH₃ | OCH₃ | 3 |
| H | N(CH₂CH₂OH)₂ | H | 5 | | OCH₃ | OCH₃ | OCH₃ | 4 |
| H | N(CH₂CH₂OH)₂ | H | 6 | | OCH₃ | OCH₃ | OCH₃ | 5 |
| H | N(CH₂CH₂OH)₂ | H | 8 | | OCH₃ | OCH₃ | OCH₃ | 8 |
| H | N(CH₂CH₂OH)₂ | H | 10 | | OCH₃ | OCH₃ | OCH₃ | 10 |
| H | N(CH₂CH₂OH)₂ | H | 12 | | OCH₃ | OCH₃ | OCH₃ | 12 |
| H | N(CH₂CH₂OH)₂ | H | 14 | | OCH₃ | OCH₃ | OCH₃ | 14 |
| H | N(CH₂CH₂OH) ₂ | H | 16 | | OCH₃ | OCH₃ | OCH₃ | 16 |
| H | CH₃CH₂N(CH₂CH₂OH) | H | 2 | | | | | |
| H | CH₃CH₂N(CH₂CH₂OH) | H | 4 | and | | | | |
and also the organic or mineral acid or base salts thereof, the geometrical isomers and tautomers thereof, and the solvates thereof such as hydrates;
the cationic (poly)methine fluorescent dye(s) of formula **(XII)** with:
| **R¹** | **R²** | **R³** | **R⁴** | **R⁵** | **R⁶** | **R⁷** | **R⁸** | **R⁹** | **m** |
|---|---|---|---|---|---|---|---|---|---|
| H | H | H | H | H | H | OCH₃ | OCH₃ | OCH₃ | 2 |
| H | H | H | H | OH | OCH₃ | H | H | H | 2 |
| H | H | H | H | H | H | H | H | H | 2 |
| H | H | H | H | H | OCH₃ | OCH₃ | H | H | 2 |
| H | H | H | H | OH | H | OH | H | H | 6 |
| H | H | H | H | OCH₃ | H | OCH₃ | OCH₃ | H | 6 |
| H | H | H | H | H | H | OH | H | H | 6 |
| H | H | H | H | OCH₃ | H | H | H | F | 2 |
| H | H | H | H | H | H | C(O)-OH | H | H | 2 |
| H | H | H | H | H | H | Isopropyl | H | H | 2 |
| H | H | H | H | H | H | N(CH₂CH₂C(O)CH₃)₂ | H | H | 2 |
| H | H | H | H | OH | H | OCH₃ | H | H | 2 |
| H | H | H | H | H | H | OH | H | H | 2 |
| H | H | H | H | H | OCH₃ | OH | OH | H | 2 |
| H | H | H | H | H | CH₃ | OCH₂Ph | CH₃ | H | 2 |
| H | H | H | H | H | OCH₃ | OCH₃ | OCH₃ | H | 2 |
| H | H | H | H | H | OCH₃ | OCH₃ | OCH₃ | H | 6 |
| H | H | H | H | H | H | N(CH₃)₂ | H | H | 6 |
| H | H | H | H | H | H | OCH₃ | OCH₃ | OCH₃ | 6 |
| H | H | H | H | H | H | Phenyl | H | H | 6 |
| H | H | H | H | H | OCH₃ | OCH₃ | H | H | 6 |
| H | H | H | H | H | H | C(O)-OH | H | H | 6 |
| H | H | H | H | H | H | N(n-Butyl)₂ | H | H | 2 |
| H | H | H | H | H | H | OCH₃ | OCH₃ | H | 3 |
| H | H | H | H | H | H | OCH₃ | OCH₃ | H | 2 |
| H | H | H | H | H | H | OCH₃ | OCH₃ | H | 5 |
| H | H | H | H | H | H | OCH₃ | H | H | 3 |
| H | H | H | H | H | H | N(CH₃)₂ | H | H | 3 |
| H | H | H | H | H | H | H | H | H | 3 |
and also the organic or mineral acid or base salts thereof, the optical isomers, geometrical isomers and tautomers thereof, and the solvates thereof such as hydrates;
• the cationic (poly)methine fluorescent dye(s) of formula **(XIII)** with:
| **R¹** | **R²** | **R³** | **R⁴** | **R⁵** | **R⁶** | **R⁷** | **R⁸** | **R⁹** | **m** |
|---|---|---|---|---|---|---|---|---|---|
| CH₃ | H | H | H | OH | H | OCH₃ | H | H | 2 |
| CH₃ | H | H | H | H | H | OCH₃ | OCH₂-Ph | H | 2 |
| CH₃ | H | H | H | H | H | H | H | OCH₃ | 2 |
| CH₃ | H | H | H | F | H | H | H | H | 2 |
| CH₃ | H | H | H | H | H | H | OPh | H | 2 |
| CH₃ | H | H | H | H | H | N(CH₂CH₂OAC)₂ | H | H | 2 |
| CH₃ | H | H | H | OCH₃ | H | OCH₃ | OCH₃ | H | 2 |
| CH₃ | H | H | H | H | H | H | CH₃ | H | 2 |
| CH₃ | H | H | H | H | H | OH | H | H | 2 |
| CH₃ | H | H | H | H | OCH₃ | OCH₃ | OCH₃ | H | 2 |
| CH₃ | H | H | H | H | OCH₃ | OH | OH | H | 2 |
| CH₃ | H | H | H | H | H | OCH₃ | OCH₃ | OCH₃ | 2 |
| CH₃ | H | H | H | H | H | H | OCH₃ | OH | 2 |
| CH₃ | H | H | H | H | H | N(n-butyl)₂ | H | H | 2 |
| CH₃ | H | H | H | H | OCH₃ | OCH₃ | H | H | 2 |
| CH₃ | H | H | H | H | H | H | H | H | 2 |
| CH₃ | H | H | H | H | H | i-propyl | H | H | 2 |
| H | H | H | H | H | H | OH | H | H | 6 |
| H | H | H | H | H | OCH₃ | OCH₃ | OCH₃ | H | 6 |
| H | H | H | H | OH | H | OCH₃ | H | H | 2 |
| H | H | H | H | OCH₃ | H | H | OCH₃ | OCH₃ | 2 |
| H | H | H | H | H | H | H | H | Br | 2 |
| H | H | H | H | H | H | OH | H | H | 2 |
| H | H | H | H | H | H | N(CH₃)₂ | H | H | 6 |
| H | H | H | H | OCH₃ | OCH₃ | OCH₃ | H | H | 6 |
| H | H | H | H | OH | OCH₃ | H | H | H | 6 |
| H | H | H | H | H | OCH₃ | OCH₃ | H | H | 6 |
| H | H | H | H | H | OCH₃ | OCH₃ | H | H | 2 |
| H | H | H | H | H | H | C(O)-OH | H | H | 6 |
| H | H | H | H | H | H | C(O)-OH | H | H | 2 |
| H | H | H | H | H | H | i-propyl | H | H | 2 |
| H | H | H | H | H | H | N(CH₃)CH₂CH₂CN | H | H | 2 |
| H | H | H | H | H | H | OCH₃ | OCH₂Ph | H | 2 |
| H | H | H | H | H | H | H | OPh | H | 2 |
| H | H | H | H | H | H | N(CH₂CH₂C(O)CH₃)₂ | H | H | 2 |
| H | H | H | H | OH | H | OCH₃ | H | H | 6 |
| H | H | H | H | H | OCH₃ | OCH₃ | OCH₃ | H | 2 |
| H | H | H | H | OCH₃ | H | OCH₃ | OCH₃ | H | 6 |
| H | H | H | H | H | H | H | CH₃ | H | 2 |
| H | H | H | H | H | H | N(CH₃)CH₂CH₂OH | H | H | 2 |
| CH₃ | H | H | H | H | H | N(CH₃)₂ | H | H | 2 |
and also the organic or mineral acid or base salts thereof, the geometrical isomers and tautomers thereof, and the solvates thereof such as hydrates;
• the cationic (poly)methine fluorescent dye(s) (b) of formulae **(XII')** and **(XIII')** below: 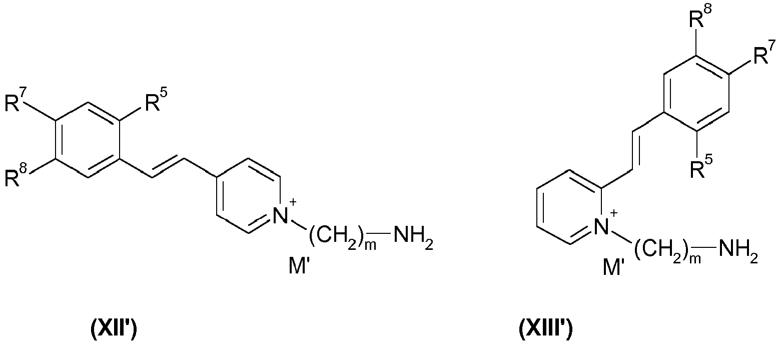 with:
| **R⁵** | **R**⁷ | **R⁸** | **m:** | | **R⁵** | **R**⁷ | **R⁸** | **m** |
|---|---|---|---|---|---|---|---|---|
| H | N(CH₂CH₂OH)₂ | H | 2 | | OCH₃ | OCH₃ | OCH₃ | 2 |
| H | N(CH₂CH₂OH)₂ | H | 3 | | OCH₃ | OCH₃ | OCH₃ | 3 |
| H | N(CH₂CH₂OH)₂ | H | 4 | | OCH₃ | OCH₃ | OCH₃ | 3 |
| H | N(CH₂CH₂OH)₂ | H | 5 | | OCH₃ | OCH₃ | OCH₃ | 4 |
| H | N(CH₂CH₂OH)₂ | H | 6 | | OCH₃ | OCH₃ | OCH₃ | 5 |
| H | N(CH₂CH₂OH)₂ | H | 8 | | OCH₃ | OCH₃ | OCH₃ | 8 |
| H | N(CH₂CH₂OH)₂ | H | 10 | | OCH₃ | OCH₃ | OCH₃ | 10 |
| H | N(CH₂CH₂OH)₂ | H | 12 | | OCH₃ | OCH₃ | OCH₃ | 12 |
| H | N(CH₂CH₂OH)₂ | H | 14 | | OCH₃ | OCH₃ | OCH₃ | 14 |
| H | N(CH₂CH₂OH)₂ | H | 16 | and | OCH₃ | OCH₃ | OCH₃ | 16 |
and also the organic or mineral acid or base salts thereof, the geometrical isomers and tautomers thereof, and the solvates thereof such as hydrates;
• the cationic (poly)methine fluorescent dye(s) (b) chosen from the following compounds:
| | |
|---|---|
| 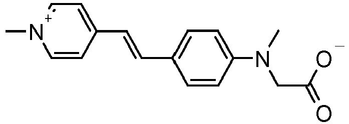 | 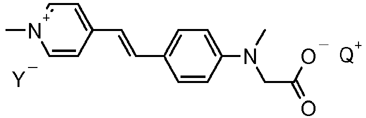 |
| **(d)** | **(d')** |
| 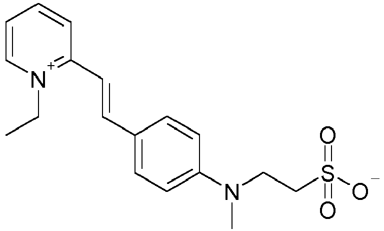 | 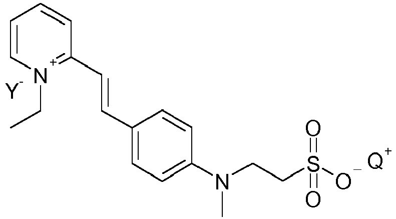 |
| **(e)** | **(e')** |
| 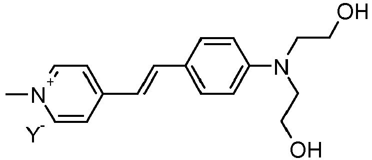 | |
| **(f)** | |
| 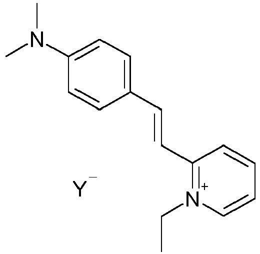 | |
| **(g)** | |
| 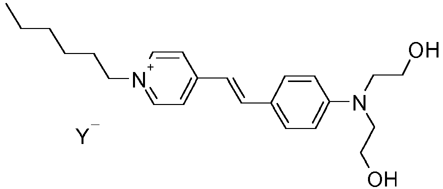 | |
| **(h)** | |
and also the organic or mineral acid or base salts thereof, the geometrical isomers and tautomers thereof, and the solvates thereof such as hydrates;
with Y and Q⁺as defined in the preceding claim.

21. Process according to any one of the preceding claims, **characterized in that** it also comprises the application to said keratin fibres of a cosmetic composition comprising c) one or more reducing agents, preferably chosen from i) the reducing agents of formula (XVI) below, and also the addition salts thereof, and mixtures thereof:
H(X)_{q}(R₁₀)ₜ (XVI)
in which formula (XVI):
- X represents P, S or SO₂,
- q represents an integer equal to 0 or 1,
- t represents an integer equal to 1 or 2, and
- R represents a linear or branched, saturated or unsaturated C₁ to C₂₀ alkyl radical, optionally interrupted with a heteroatom, and/or optionally substituted with one or more radicals chosen from hydroxyl, halo, amine, carboxyl, ((C₁-C₃₀)alkoxy)carbonyl, amido, ((C₁-C₃₀)alkyl)aminocarbonyl, ((C₁-C₃₀)acyl)amino, mono- or dialkylamino, and mono- or dihydroxylamino radicals;
ii) thioglycolic acid, iii) thiolactic acid, iv) glyceryl monothioglycolate, v) cysteamine, vi) N-acetylcysteamine, vii) N-propionylcysteamine, viii) cysteine, ix) N-acetylcysteine, x) thiomalic acid, xi) pantetheine, xii) 2,3-dimercaptosuccinic acid, xiii) N-(mercaptoalkyl)-ω-hydroxyalkylamides, xiv) N-mono- or N,N-dialkylmercapto-4-butyramides, xv) aminomercaptoalkylamides, xvi) N-(mercaptoalkyl)succinamic acid derivatives, xvii) N-(mercaptoalkyl)succinimide derivatives, xviii) alkylamino mercaptoalkylamides, xix) the azeotropic mixture of 2-hydroxypropyl thioglyconate and of (2-hydroxy-1-methyl)ethyl thioglycolate, xx) mercaptoalkylaminoamides, xxi) N-mercaptoalkylalkanediamides, xxii) formamidinesulfinic acid derivatives, addition salts thereof, and mixtures thereof.

22. Process according to any one of Claims 1 to 20, not using any reducing agent.

23. Process according to any one of the preceding claims, in which the anthraquinone compound(s) (a), as defined in any one of Claims 1 to 11, and the cationic (poly)methine fluorescent direct dye(s) (b), as defined in any one of Claims 1 and 12 to 20, are applied together to the keratin fibres; preferably, the process comprises a step of applying to the keratin fibres a cosmetic composition which comprises a) one or more anthraquinone dyes of formulae **(I)** and/or **(II),** as defined in any one of Claims 1 to 11, and (b) one or more cationic (poly)methine fluorescent dye(s), as defined in any one of Claims 1 and 12 to 20.

24. Process according to any one of Claims 1 to 22, which comprises at least two successive steps:
- a step of applying to the keratin fibres a cosmetic composition comprising (b) one or more cationic (poly)methine fluorescent dyes, as defined in any one of Claims 1 and 12 to 20, followed by,
- a step of applying to said keratin fibres a cosmetic composition comprising (a) one or more anthraquinone dyes of formula (I) and/or (II), as defined in any one of Claims 1 to 11.

25. Process according to any one of Claims 1 to 22, which comprises at least two successive steps:
- a step of applying to said fibres a cosmetic composition comprising (a) one or more anthraquinone dyes of formula (I) and/or (II), as defined in any one of Claims 1 to 11, followed by
- a step of applying to the keratin fibres a cosmetic composition comprising (b) one or more cationic (poly)methine fluorescent dyes, as defined in any one of Claims 1 and 12 to 20.

26. Process according to any one of the preceding claims, in which the pH of the cosmetic composition(s) is between 6 and 11 inclusive, particularly between 7 and 10, more particularly between 7.5 and 9.5, such as between 9 and 9.5.

27. Cosmetic composition comprising (a) one or more anthraquinone dyes of formula **(I)** and/or **(II),** as defined in any one of Claims 1 to 11, and (b) one or more cationic (poly)methine fluorescent dyes, as defined in any one of Claims 1 and 13 to 20, optionally at a pH as defined in the preceding claim.

## Patentansprüche

1. Verfahren zum Färben von Keratinfasern, insbesondere menschlichen Keratinfasern wie dem Haar, bei dem man auf die Keratinfasern die nachstehenden Bestandteile (a) und (b) aufbringt:
(a) einen oder mehrere Anthrachinon-Farbstoffe, die aus den Verbindungen der nachstehenden Formeln **(I)** und/oder **(II),** den optischen Isomeren davon, den geometrischen Isomeren davon, den Tautomeren davon, den Solvaten davon und Mischungen davon ausgewählt sind: 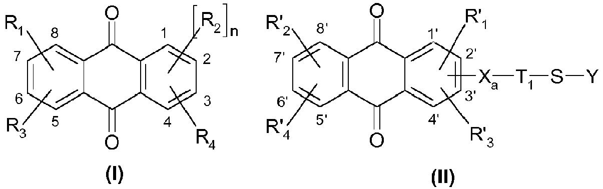 wobei in Formel **(I)** oder **(II):**
▪ **Xₐ,** das gleich oder verschieden sein kann, für eine Bindung, ein Heteroatom oder eine aus einem Sauerstoff- oder Schwefelatom, N(Rₐ), CO, SO, SO₂ oder Kombinationen davon wie -O-CO-, -CO-O-, - NH-CO- or -CO-NH- steht, wobei Rₐ für ein Wasserstoffatom oder eine (C₁-C₆)Alkylgruppe, die gegebenenfalls durch eine oder mehrere Hydroxylgruppen substituiert ist, steht; vorzugsweise Xₐ für N(Rₐ) und insbesondere NH steht;
▪ Y für i) ein Wasserstoffatom; ii) ein Alkalimetall; iii) ein Erdalkalimetall; iv) eine Ammoniumgruppe: N⁺R^{a}R^{b}R^{g}R^{d} oder eine Phosphoniumgruppe: P⁺R^{a}R^{b}R^{g}R^{d} steht, wobei R^{a}, R^{b}, R^{g} und R^{d}, die gleich oder verschieden sein können, für ein Wasserstoffatom oder eine (Ci-C₄)Alkylgruppe stehen; oder v) eine Schutzgruppe für die Thiolfunktion oder vi) die nachstehende Gruppe **(III)** steht: 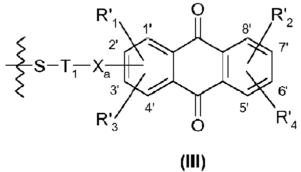
▪**R₁, R₂, R₃, R₄, R'₁, R'₂, R'₃** und **R'₄,** die gleich oder verschieden sein können, für ein Atom oder eine Gruppe, das bzw. die aus
∘ Wasserstoff,
∘ Halogen wie Brom und Chlor,
∘ Hydroxyl,
∘ C₁-C₄-Alkoxy,
∘ Hydroxysulfonyl (-SO₃H) oder Sulfonat (-SO₃⁻, M⁺), wobei M⁺ für ein kationisches Gegenion, insbesondere ein Alkalimetall, Erdalkalimetall oder Ammonium, wie Na⁺ oder K⁺, steht;
o gegebenenfalls substituiertem C₁-C₆-Alkyl,
o -NR₅R₆, worin **R₅** und **R₆,** die gleich oder verschieden sein können, für ein Atom oder einen Rest stehen, das bzw. der aus i) Hydrogen, ii) (C₁-C₄) Alkylcarbonyl wie Methylcarbonyl (-COCH₃), iii) Arylsulfonyl wie Phenylsulfonyl (-SO₂Ph), iv) Het-ALK-C(O)-, wobei Het für eine Heterocycloalkylgruppe, die gegebenenfalls substituiert ist, insbesondere durch eine oder mehrere (C₁-C₄)Alkylgruppen, steht und ALK für eine (C₁-C₆)-Alkylengruppe, die gegebenenfalls durch eine oder mehrere Hydroxyl- oder (Di)(hydroxy)(C₁-C₄)(alkyl)aminogruppen substituiert ist, steht; Het-ALK-C(O)- wie (Piperidin-1-yl)acetyl, 2-Methyl-2-(piperidin-1-yl)propanoyl, 2-(Morpholin-4-yl)ethyl-β-alaninoyl, v) gegebenenfalls substituiertem Aryl, insbesondere Phenyl, das gegebenenfalls durch mindestens einen Rest, der aus a) C₁-C₆-Alkyl, b) Hydroxyl, c) Hydroxysulfonyl, d) Ci-C₄-Alkoxy, e) Carboxyl (-COOH), f) (Ci-C₄)Alkoxycarbonyl, g) Amino, h) (Di) (C₁-C₄)alkylamino, wobei einer der Alkylreste gegebenenfalls durch einen Hydroxyl- oder Hydroxysulfonylrest -SO₃H oder -OSO₃H substituiert ist, ausgewählt ist, substituiert sein kann, vi) gegebenenfalls substituiertem Aryl (C₁-C₄) alkyl, insbesondere Benzyl, das gegebenenfalls durch eine (Di)(Ci-C₄)(alkyl)aminogruppe substituiert ist, viii) gegebenenfalls substituiertem C₁-C₂₀-Alkyl, das gegebenenfalls durch ein oder mehrere Heteroatome und/oder durch eine oder mehrere Gruppen, die mindestens ein Heteroatom umfassen, die vorzugsweise aus Sauerstoff, Stickstoff und Schwefel, CO, SO, SO₂ oder Kombinationen davon ausgewählt sind, unterbrochen ist, wobei der Alkylrest dann, wenn er substituiert ist, durch ein oder mehrere Atome oder eine oder mehrere Gruppen, die aus a) Halogenen, vorzugsweise einem oder mehreren Chloratomen, b) Hydroxyl, c) (C₁-C₆)-Alkylcarbonylamino, insbesondere Acylamino, d) 5- oder 6-gliedrigem Heterocycloalkyl wie Tetrahydro-2H-pyran-4-amin, Morpholino, Piperidino oder Piperazino, e) (Di) (C₁-C₄)-(alkyl)amino, f) Hydroxysulfonyl(C₁-C₄)alkylamino oder Hydroxysulfonyloxy(C₁-C₄)alkylamino, g) (Di) (hydroxy) (C₁-C₄) (alkyl) amino, h) 5- oder 6-gliedrigem Heteroaryl wie Imidazol und i) Formylamino (-NHCOH) ausgewählt sind, substituiert ist ausgewählt ist;
∘ einer Gruppe der nachstehenden Formel (a): -N(R₇)-X₁-W₁ (a),
wobei in Formel (a):
**R₇** für einen Wasserstoff oder einen C₁-C₄-Alkylrest steht,
**X₁** für einen zweiwertigen Rest steht, der aus C₁-C₂₀-Alkylen, das gegebenenfalls durch ein oder mehrere Heteroatome und/oder durch eine oder mehrere Gruppen, die aus Sauerstoff, Stickstoff und Schwefel, CO, SO, SO₂, Arylen wie Phenylen oder Kombinationen davon ausgewählt sind, unterbrochen ist;
vorzugsweise X₁ für
• (C₁-C₁₀)Alkylen,
• -(C₁-C₁₀)Alkylcarbonyl-,
• -Carbonyl (C₁-C₁₀) alkyl-,
• -(C₁-C₁₀)Alkylaminocarbonyl (C₁-C₁₀) alkyl-,
• -(C₁-C₁₀)Alkylcarbonylamino (C₁-C₁₀) alkyl-,
• -Phenyl (C₁-C₁₀) alkyl,
• - (C₁-C₁₀)Alkylphenyl- oder
• Phenylen
steht;
**W₁** für einen kationischen Rest, der aus 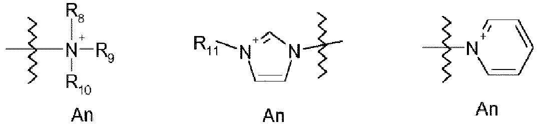 ausgewählt ist, steht, wobei **R₈, R₉, R₁₀** und **R₁₁,** die gleich oder verschieden sein können, für eine C₁-C₆-Alkylgruppe, einen Benzylrest oder einen C₁-C₆-Alkylsulfonatrest stehen; die Reste
**R₈** und **R₉** mit dem Stickstoffatom, an das sie gebunden sind, einen gesättigten oder ungesättigten, gegebenenfalls substituierten 5- bis 7-gliedrigen Heterocyclus, der gegebenenfalls ein weiteres Nicht-Stickstoff-Heteroatom, vorzugsweise ein Sauerstoffatom, umfasst, bilden können;
ausgewählt ist, stehen;
▪ n für eine ganze Zahl im Bereich von 1 bis 3 steht; vorzugsweise n gleich 1 oder 2 ist, **T₁** für eine lineare oder verzweigte zweiwertige Kette auf Kohlenwasserstoffbasis mit 1 bis 20 Kohlenstoffatomen, die gegebenenfalls durch ein oder mehrere Heteroatome oder eine oder mehrere Gruppen oder Kombinationen davon, die aus Sauerstoff, Schwefel, N(R_{b}), C(O), -N⁺ (R₈) (R₉) - An und gegebenenfalls kationischem und gegebenenfalls substituiertem Heteroaryl, wie Imidazolium, An ausgewählt sind, unterbrochen ist, wobei **R₈** und **R₉**, die gleich oder verschieden sein können für einen C₁-C₆-Alkylrest stehen; wobei **R_{b}** für ein Wasserstoffatom oder eine (Hydroxy) (C₁-C₄) alkylgruppe steht; vorzugsweise die Kette auf Kohlenwasserstoffbasis durch eine oder mehrere Gruppen, die aus N(R_{b}), C(O) und einer Kombination davon wie -C(O)- N(R_{b})- oder - N(R_{b})-C(O)- und -N⁺(R₈) (R₉)- An ausgewählt sind, unterbrochen ist, wobei **An** für ein organisches oder anorganisches Gegenion steht, das die elektrische Neutralität der Farbstoffe der Formeln **(I)** und **(II)** gewährleistet;
▪ wobei 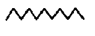 Teil der Bindung ist, die mit dem Rest des Moleküls verbunden ist; und
(b) einen oder mehrere kationische (Poly)methin-Fluoreszenzfarbstoffe;
wobei es sich versteht, dass:
- der bzw. die Anthrachinon-Farbstoffe der Formel **(I)** mindestens einen Rest **R₁, R₃** oder **R₄,** der von einem Wasserstoffatom verschieden ist, umfassen und der bzw. die Anthrachinon-Farbstoffe der Formel **(II)** mindestens einen Rest **R'₁, R'₂, R'₃** oder **R'₄,** der von einem Wasserstoffatom verschieden ist, umfassen;
- (a) der bzw. die Anthrachinon-Farbstoffe der Formel **(I)** oder **(II)** und (b) der bzw. die Fluoreszenzfarbstoffe zusammen oder nacheinander auf die Keratinfasern aufgebracht werden und
- dann, wenn die Verbindungen der Formel (I) oder (II) kationisch sind und eine Sulfonatgruppe umfassen, M⁺ und An fehlen können, um die elektrische Neutralität des Moleküls zu gewährleisten.

2. Verfahren nach dem vorhergehenden Anspruch, wobei es sich bei dem bzw. den Anthrachinon-Farbstoffen der Formeln **(I)** und/oder **(II)** um Farbstoffe handelt, die Licht im blau-violetten Bereich, vorzugsweise im blauen Bereich, absorbieren.

3. Verfahren nach einem der vorhergehenden Ansprüche, wobei es sich bei dem bzw. den Anthrachinon-Farbstoffen der Formeln **(I)** und/oder **(II)** um Farbstoffe der Formel **(I)** handelt und n gleich 0 oder 1 ist; vorzugsweise die Substituenten **R₁, R₂, R₃** und **R₄** in den Positionen 1, 4, 5 und 8 stehen.

4. Verfahren nach einem der vorhergehenden Ansprüche, wobei es sich bei dem bzw. den Anthrachinon-Farbstoffen der Formeln **(I)** und/oder **(II)** um Farbstoffe der Formel **(I)** handelt, wobei **R₁** und **R₃** für ein Atom oder eine Gruppe stehen, das bzw. die aus i) Hydroxyl, ii) Arylamino oder Aryl(C₁-C₆)alkylamino, wobei die Arylgruppe für eine gegebenenfalls substituierte Arylgruppe, insbesondere eine Phenylgruppe, die gegebenenfalls durch eine oder mehrere Gruppen, die aus (C₁-C₄)-Alkyl, Carboxyl, (C₁-C₄)Alkoxycarbonyl, (Di)-(hydroxy) (C₁-C₄) (alkyl) amino, Tri (C₁-C₄)alkylammonium, (C₁-C₄)Alkoxy, (Di)hydroxysulfonyloxy(C₁-C₄)alkylamino und Hydroxysulfonyl ausgewählt sind, substituiert ist, steht, iii) (Di)(hydroxy)(C₁-C₆) (alkyl) amino, iv) (Di) (C₁-C₄) (alkyl) amino (C₁-C₆)-alkylamino, v) (C₁-C₄) Alkylcarbonylamino (C₁-C₆) - alkylamino, vi) Arylsulfonylamino, wobei die Arylgruppe gegebenenfalls substituiert sein kann, wobei Aryl vorzugsweise für eine Phenylgruppe steht, vii) (Di)halogen (C₁-C₄) alkylamino, viii) (Di)-(hydroxy) (C₁-C₄) alkoxy (C₁-C₄) alkylamino, ix) (Di)-tri (C₁-C₄)alkylammonium (C₁-C₆)alkylamino, x) Heterocycloalkyl(C₁-C₆)alkylamino, Heterocycloalkyl(C₁-C₄)alkylcarbonylamino, Heterocycloalkyl(C₁-C₄)-alkylamino(C₁-C₄)alkylcarbonylamino oder Heterocycloalkyl (C₁-C₄)alkylcarbonylamino (C₁-C₆)alkylamino, wobei das Heterocycloalkyl gegebenenfalls kationisch und/oder substituiert, insbesondere durch eine oder mehrere (C₁-C₄)Alkyl- oder Benzylgruppen, sein kann, xi) Halogen(C₁-C₄)alkylcarbonylamino(C₁-C₆)alkylamino, xii) Hydroxysulfonyl, xiii) Halogen wie Chlor, xiv) Heteroaryl (C₁-C₆)alkylamino, wobei das Heteroaryl gegebenenfalls kationisch und/oder substituiert, insbesondere durch eine oder mehrere (C₁-C₄) Alkylgruppen, sein kann, xv) Heteroaryl (C₁-C₄)alkylcarbonylamino (C₁-C₆)alkylamino, wobei das Heteroaryl gegebenenfalls kationisch und/oder substituiert, insbesondere durch eine oder mehrere (C₁-C₄)Alkylgruppen, sein kann, xvi) R'R''N-, wobei R' und R'', die gleich oder verschieden sein können, für eine (Halogen) (C₁-C₄)alkylcarbonylamino (C₁-C₆)alkylgruppe, eine (Halogen) (C₁-C₄)alkylaminocarbonyl (C₁-C₆)alkylgruppe, eine (Halogen)(C₁-C₄)alkylcarbonylgruppe, eine Carboxy (C₁-C₆) alkylgruppe, wobei die Alkylgruppe gegebenenfalls durch eine oder mehrere Amino- oder Hydroxylgruppen substituiert sein kann, oder eine (Poly)hydroxy(C₁-C₆)alkylgruppe stehen, xvii) (C₁-C₁₆) Alkylaminocarbonylamino (C₁-C₆)alkylamino, xviii) Formylamino (C₁-C₆)alkylamino, xix) (Hydroxy) (C₁-C₆)alkylamino (C₁-C₆)alkylamino, xix) Hydroxysulfonyl (C₁-C₆)alkylamino (C₁-C₆)alkylamino, xx) Sulfonato (C₁-C₆) alkyl (di (C₁-C₄) alkyl) ammonium (C₁-C₆) alkylamino, xxi) Hydroxysulfonyl (C₁-C₆) alkoxy (C₁-C₆) alkylamino, xxi) Heteroaryl(C₁-C₄)alkylcarbonylamino, wobei das Heteroaryl gegebenenfalls kationisch und/oder substituiert, insbesondere durch eine oder mehrere (C₁-C₄) Alkylgruppen, sein kann, xxii) Heterocycloalkyl(C₁-C₄)alkylcarbonylamino, wobei das Heterocycloalkyl gegebenenfalls kationisch und/oder substituiert, insbesondere durch eine oder mehrere (C₁-C₄)Alkyl- oder Benzylgruppen, sein kann, xxiii) (Di) (C₁-C₄) (alkyl) amino (C₁-C₆) alkylamino, wobei die (C₁-C₆)Alkylgruppe gegebenenfalls durch eine oder mehrere Hydroxylgruppen substituiert sein kann, xxiv) Heterocycloalkylamino(C₁-C₆)alkylamino oder Heterocycloalkylamino, wobei das Heterocycloalkyl gegebenenfalls kationisch und/oder substituiert, insbesondere durch eine oder mehrere (C₁-C₄)Alkyloder Benzylgruppen, sein kann, xxv) Heteroarylalkylamino(C₁-C₆)alkylamino oder Heteroarylalkylamino, wobei das Heteroaryl gegebenenfalls kationisch und/oder substituiert, insbesondere durch eine oder mehrere (C₁-C₄)Alkylgruppen, sein kann, xxvi) Tri (C₁-C₆) alkylammonium(C₁-C₆) alkylamino, xxvii) (C₁-C₄)Alkoxycarbonyl (C₁-C₆) alkyl-(di (C₁-C₄) alkyl) ammonium (C₁-C₆) alkylamino, xxviii) Carboxylato (C₁-C₆) alkyl (di (C₁-C₄) alkyl) ammonium (C₁-C₆)alkylamino, xxix) Carboxy (C₁-C₆) alkylamino (C₁-C₆) alkylamino, xxx) Aryl (C₁-C₄) alkyl (di (C₁-C₄) - alkyl)ammonium(C₁-C₆) alkylamino, xxxi) Sulfonsäure SO₃H oder Sulfonat SO₃⁻, M⁺, wobei M⁺ für ein kationisches Gegenion steht, xxxii) (C₁-C₆)Alkyl, xxxiii) Hydroxysulfonyl(C₁-C₄)amino und xxxiv) Phenylsulfonylamino ausgewählt ist.

5. Verfahren nach einem der vorhergehenden Ansprüche, wobei es sich bei dem bzw. den Anthrachinon-Farbstoffen der Formeln **(I)** und/oder **(II)** um Farbstoffe der Formel **(I)** handelt, und wobei **R₂** und **R₄,** die gleich oder verschieden sein können, für ein Wasserstoffatom oder eine Gruppe gemäß der oben für R₁ und R₃ i) bis xxxii) im vorhergehenden Anspruch angegebenen Definition stehen:
- Hydroxyl,
- (Di) (C₁-C₄) (alkyl) amino,
- (C₁-C₄)Alkoxy, Heterocycloalkyl(C₁-C₆)alkylamino, wobei das Heterocycloalkyl gegebenenfalls kationisch und/oder substituiert, insbesondere durch eine oder mehrere (C₁-C₄) Alkyl- oder Benzylgruppen, sein kann,
- Arylamino oder Aryl (C₁-C₆) alkylamino, wobei die Arylgruppe für eine gegebenenfalls substituierte Arylgruppe, insbesondere eine Phenylgruppe, die gegebenenfalls durch eine oder mehrere Gruppen, die aus (C₁-C₄)Alkyl, (Di) (hydroxy) (C₁-C₄) (alkyl) amino, (C₁-C₄)Alkoxy und Tri (C₁-C₄)-alkylammonium ausgewählt sind, substituiert ist, steht,
- Aryl (C₁-C₄) alkyl (di (C₁-C₄) alkyl) ammonium (C₁-C₆) - alkylamino,
- (Di) (C₁-C₄) (alkyl) amino (C₁-C₆) alkylamino und
- Hydroxy (C₁-C₄) (alkyl) amino (C₁-C₆) alkylamino.

6. Verfahren nach einem der vorhergehenden Ansprüche, wobei es sich bei dem bzw. den Anthrachinon-Farbstoffen der Formeln **(I)** und/oder **(II)** um Farbstoffe der Formel **(I)** handelt und **R₂** und **R₄** für ein Wasserstoffatom stehen.

7. Verfahren nach einem der Ansprüche 1 bis 3, wobei es sich bei dem bzw. den Anthrachinon-Farbstoffen der Formeln **(I)** und/oder **(II)** um Farbstoffe der Formel **(I)** handelt, wobei **R₂** und **R₃** für ein Wasserstoffatom stehen und **R₁** und **R₄** vorzugsweise in den Positionen 8 bzw. 4 stehen und **R₁** und **R₄** wie in Anspruch 1 definiert sind und insbesondere für ein Atom oder eine Gruppe gemäß der oben für R₁ und R₃ i) bis xxxii) in Anspruch 4 angegebenen Definition stehen, vorzugsweise **R₁** und **R₄** aus
- Halogen wie Chlor,
- (Di) (C₁-C₄) (alkyl)amino,
- (C₁-C₄) Alkylcarbonylamino,
- Heterocycloalkyl(C₁-C₆)alkylamino, Heterocycloalkyl(C₁-C₄)alkylcarbonylamino oder Heterocycloalkyl (C₁-C₄) alkylamino (C₁-C₄) alkylcarbonylamino, wobei das Heterocycloalkyl gegebenenfalls kationisch und/oder substituiert, insbesondere durch eine oder mehrere (C₁-C₄)Alkyl- oder Benzylgruppen, sein kann,
- Tri (C₁-C₄) alkylammonium (C₁-C₆) alkylamino,
- Arylamino oder Aryl (C₁-C₆) alkylamino, wobei die Arylgruppe für eine gegebenenfalls substituierte Arylgruppe, insbesondere eine Phenylgruppe, die gegebenenfalls durch eine oder mehrere Gruppen, die aus (C₁-C₄)Alkyl, (Di) (hydroxy) (C₁-C₄) (alkyl) amino, (C₁-C₄)Alkoxy und Tri(C₁-C₄) alkylammonium ausgewählt sind, substituiert ist, steht,
- (Di) (C₁-C₄) (alkyl) amino (C₁-C₆) alkylamino und
- (C₁-C₄)Alkylcarbonylamino (C₁-C₆) alkylamino ausgewählt sind.

8. Verfahren nach einem der Ansprüche 1 bis 3, wobei es sich bei dem bzw. den Anthrachinon-Farbstoffen der Formeln **(I)** und/oder **(II)** um Farbstoffe der Formel **(I)** handelt, wobei **R₃** und **R₄** für ein Wasserstoffatom stehen und **R₁** und **R₂** vorzugsweise in den Positionen 8 bzw. 1 stehen und **R₁** und **R₂** wie in Anspruch 1 definiert sind und insbesondere für ein Atom oder eine Gruppe gemäß der oben für R₁ und R₃ i) bis xxxii) in Anspruch 4 angegebenen Definition stehen, vorzugsweise **R₁** und **R₂** aus
- (Di) (hydroxy) (C₁-C₄) (alkyl)amino,
- (Di) (hydroxy) (C₁-C₄) (alkyl) amino (C₁-C₆) alkylamino,
- Tri (C₁-C₆) alkylammonium (C₁-C₆) alkylammonium,
- Tri (C₁-C₆) alkylammonium (C₁-C₆) alkylamino,
- Heterocycloalkyl(C₁-C₆)alkylamino, wobei das Heterocycloalkyl gegebenenfalls kationisch und/oder substituiert, insbesondere durch eine oder mehrere (C₁-C₄)Alkyl- oder Benzylgruppen, sein kann, und
- Arylamino oder Aryl(C₁-C₆)alkylamino, wobei die Arylgruppe für eine gegebenenfalls substituierte Arylgruppe, insbesondere eine Phenylgruppe, die gegebenenfalls durch eine oder mehrere Gruppen, die aus (C₁-C₄)Alkyl, (Di) (hydroxy) (C₁-C₄) (alkyl) amino, (C₁-C₄)Alkoxy und Tri(C₁-C₄)alkylammonium ausgewählt sind, substituiert ist, steht,
ausgewählt sind.

9. Verfahren nach einem der Ansprüche 1 bis 3, wobei es sich bei dem bzw. den Anthrachinon-Farbstoffen der Formeln **(I)** und/oder **(II)** um Farbstoffe der Formel **(I)** handelt, wobei **R₁** und **R₃** für ein Wasserstoffatom stehen und n gleich 2 ist und **R₂** und **R₄** vorzugsweise in den Positionen 1, 2 bzw. 4 stehen und **R₂** und **R₄** wie in Anspruch 1 definiert sind und insbesondere für ein Atom oder eine Gruppe gemäß der oben für R₁ und R₃ i) bis xxxii) in Anspruch 4 angegebenen Definition stehen, vorzugsweise R₂ und **R₄** aus
- (C₁-C₆) Alkyl,
- (Di) (hydroxy) (C₁-C₄) (alkyl) amino,
- SO₃H oder SO₃⁻M⁺,
- (Di) (hydroxy) (C₁-C₄) (alkyl) amino (C₁-C₆) alkylamino,
- Tri (C₁-C₄) alkylammonium (C₁-C₆) alkylamino und
- Heterocycloalkyl(C₁-C₆)alkylamino, wobei das Heterocycloalkyl gegebenenfalls kationisch und/oder substituiert, insbesondere durch eine oder mehrere (C₁-C₄)Alkyl- oder Benzylgruppen, sein kann, und
- Arylamino oder Aryl(C₁-C₆)alkylamino, wobei die Arylgruppe für eine gegebenenfalls substituierte Arylgruppe, insbesondere eine Phenylgruppe, die gegebenenfalls durch eine oder mehrere Gruppen, die aus (C₁-C₄)Alkyl, (Di) (hydroxy) (C₁-C₄) (alkyl) amino, (C₁-C₄)Alkoxy und Tri(C₁-C₄) alkylammonium ausgewählt sind, substituiert ist, steht,
ausgewählt sind.

10. Verfahren nach einem der Ansprüche 1 bis 3, wobei es sich bei dem bzw. den Anthrachinon-Farbstoffen der Formeln **(I)** und/oder **(II)** um Farbstoffe der Formel **(II')** handelt: 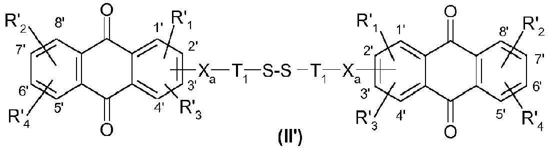 wobei **R'₁, R'₂, R'₃** und **R'₄, T₁** und **Xₐ** wie in Anspruch 1 definiert sind, **Xₐ** vorzugsweise für ein N(Rₐ) und insbesondere NH steht, spezieller **R'₂** und **R'₄** für ein Wasserstoffatom stehen und **R'₁** und **R'₃** insbesondere wie in Anspruch 4 für **R₁** und **R₃** i) bis xxxii) definiert sind, vorzugsweise, **R'₃** und **R'₄,** die gleich oder verschieden sein können, für eine Gruppe, die aus (C₁-C₆)Alkyl und (Di) (hydroxy) (C₁-C₄) (alkyl)amino ausgewählt ist, stehen; vorzugsweise **T₁** für eine gesättigte lineare zweiwertige Kette auf Kohlenwasserstoffbasis mit 1 bis 20 Kohlenstoffatomen, vorzugsweise zwischen 2 und 10 Kohlenstoffatomen, die gegebenenfalls durch eine oder mehrere Gruppen, die aus N(R_{b}), C(O), -N⁺ (R₈) (R₉)- An, kationischem Heteroaryl wie Imidazolium, An oder Kombinationen davon ausgewählt sind, unterbrochen ist, wobei **R₈** und **R₉**, die gleich oder verschieden sein können, für einen C₁-C₆-Alkylrest stehen; wobei **R_{b}** für ein Wasserstoffatom oder eine (C₁-C₄)Alkylgruppe steht; vorzugsweise die Kette auf Kohlenwasserstoffbasis durch eine oder mehrere Gruppen, die aus -N⁺ (R₈) (R₉)- An, N(R_{b}), C(O) und einer Kombination davon wie -C(O)- N(R_{b})- oder -N(R_{b})-C(O)- und N⁺(R₈) (R₉)- An ausgewählt sind, unterbrochen ist; weiter bevorzugt **T₁** für eine zweiwertige Gruppe **-(CH2)ₙ-Tₐ-(CH2)ₘ-Tb-(CH2)_{P}⁻** steht, wobei Tₐ und T_{b}, die gleich oder verschieden sein können, für eine Bindung oder eine Gruppe, die aus N(R_{b}) C(O), -N⁺(R₈) (R₉)- An, kationischem Heteroaryl wie Imidazolium, An oder Kombinationen davon ausgewählt ist, stehen, wobei **R₈** und **R₉,** die gleich oder verschieden sein können, für einen C₁-C₆-Alkylrest stehen; wobei **R_{b}** für ein Wasserstoffatom oder eine (C₁-C₄)Alkylgruppe steht; vorzugsweise -N⁺(R₈) (R₉)- An, -C(O)-N(R_{b})- oder - N(R_{b})-C(O)-, n, m und p, die gleich oder verschieden sein können, für eine ganze Zahl zwischen 1 und 10 inklusive stehen, wobei die Summe n+m+p zwischen 1 und 20 inklusive, vorzugsweise zwischen 2 und 10, liegt; bevorzugt die Gruppen **R'₁** und **R'₃** in den Positionen 2' und 4' stehen und **R'₂** und **R'₄** in den Positionen 5' und 8' stehen.

11. Verfahren nach dem vorhergehenden Anspruch, wobei es sich bei dem bzw. den Anthrachinon-Farbstoffen der Formeln **(I)** und/oder **(II)** um Farbstoffe der Formel **(II'')** handelt: 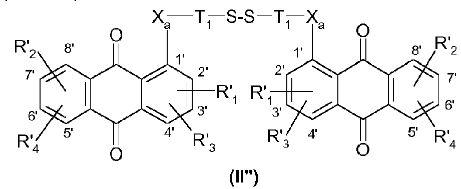 wobei in Formel **(II'') R'₁, R'₂, R'₃** und **R'₄, T₁** und **Xₐ** wie im vorhergehenden Anspruch für **(II')** definiert sind.

12. Verfahren nach einem der vorhergehenden Ansprüche, wobei (a) der bzw. die Anthrachinon-Farbstoffe der Formeln **(I)** und/oder **(II)** aus den folgenden Verbindungen, den optischen Isomeren davon, den geometrischen Isomeren davon, den Tautomeren davon, den Solvaten davon und Mischungen davon ausgewählt sind: 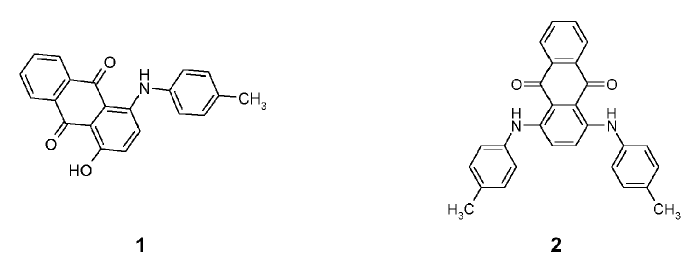 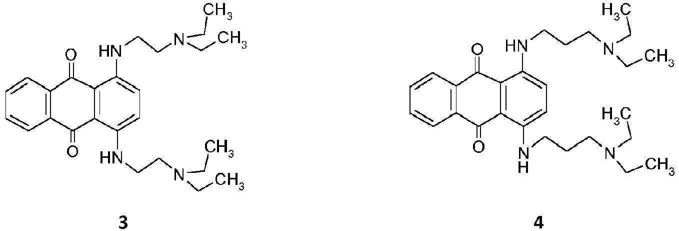 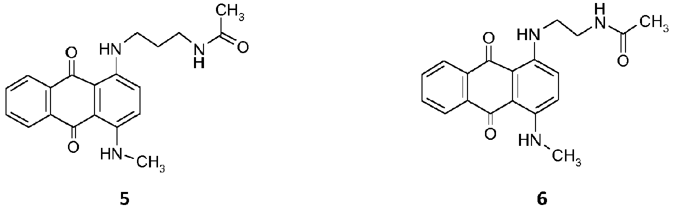 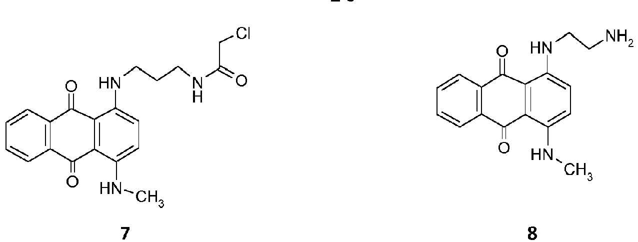 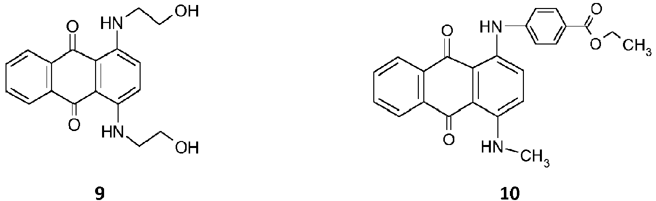 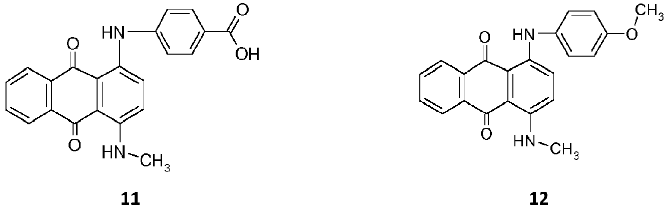 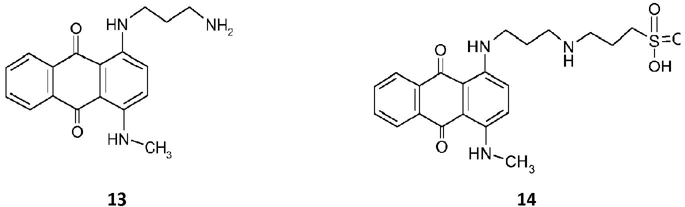 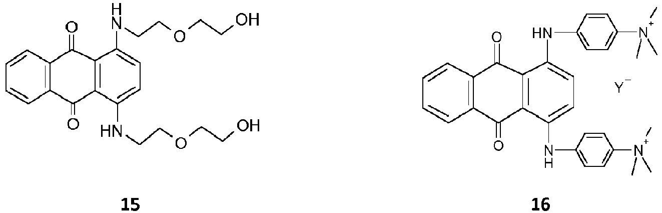 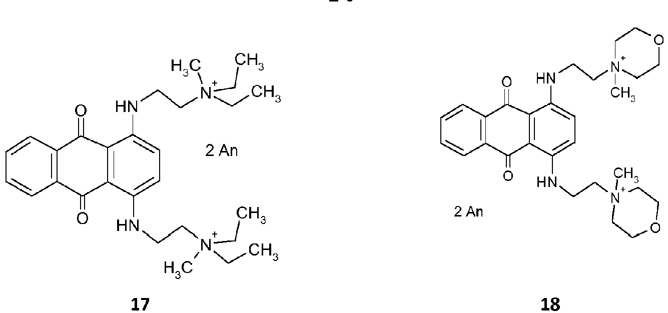 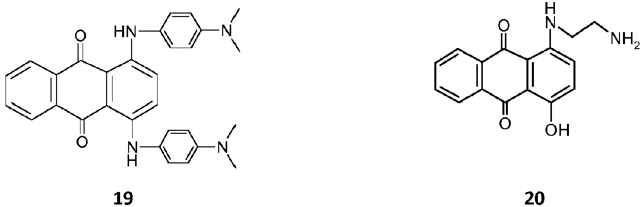 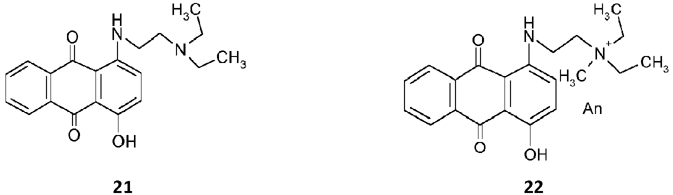 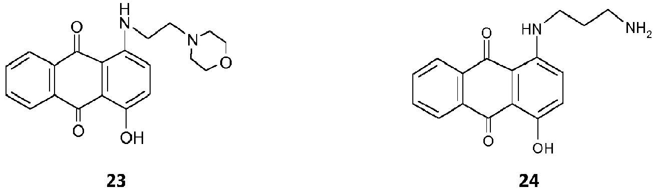 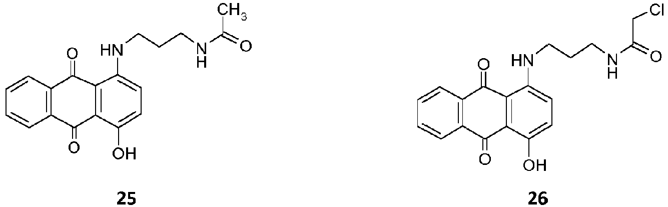 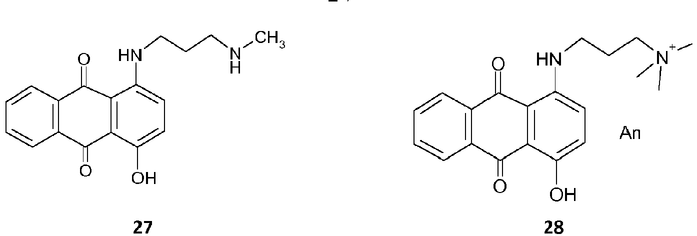 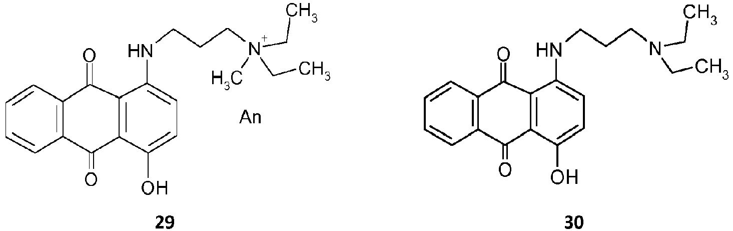 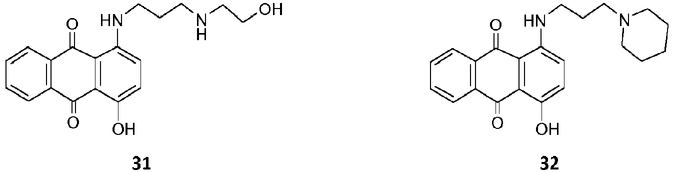 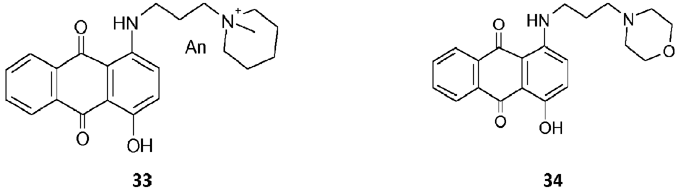 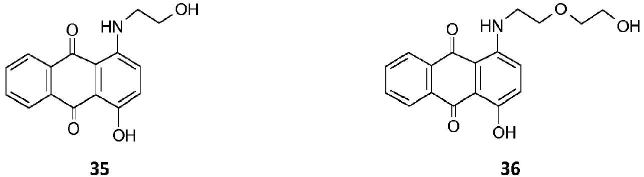 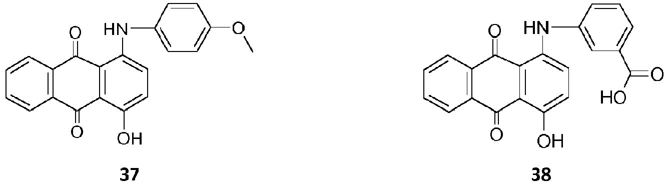 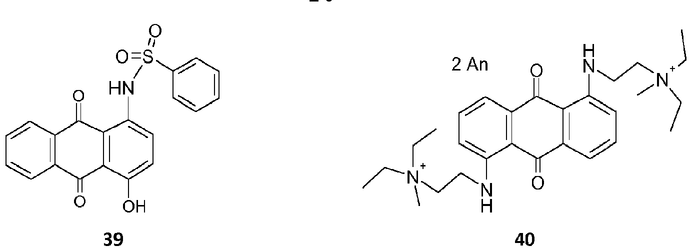 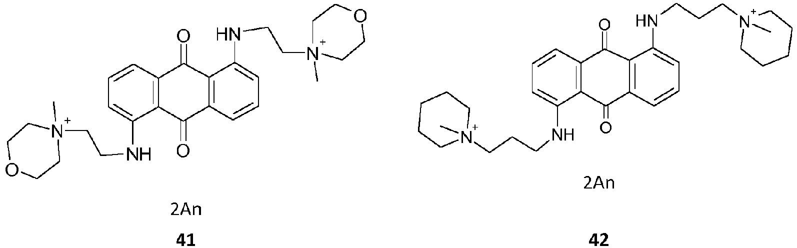 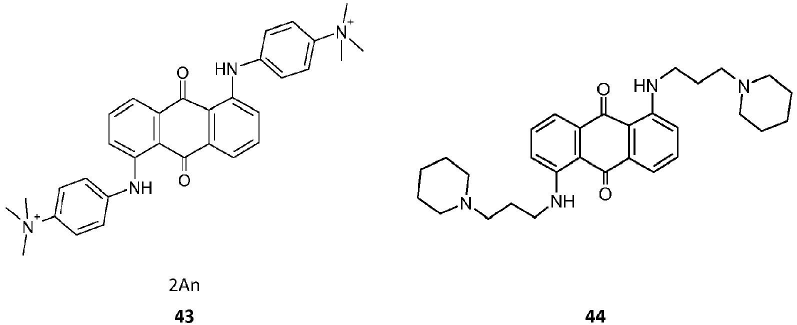 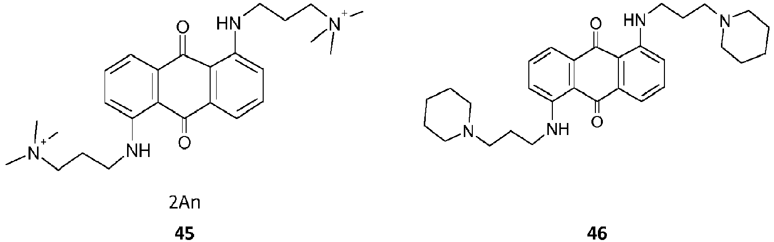 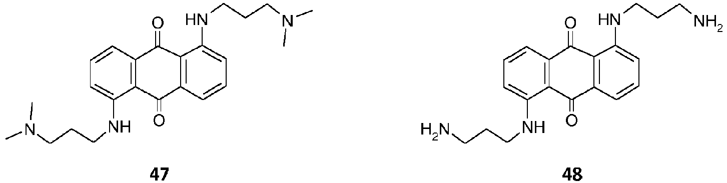 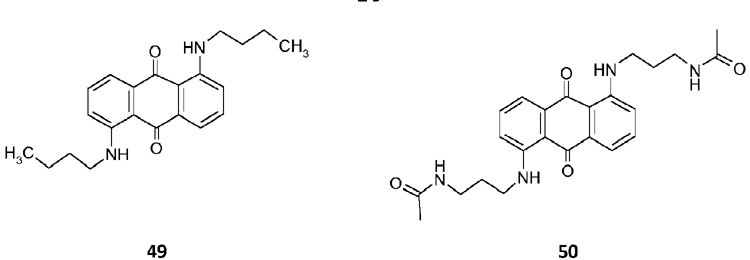 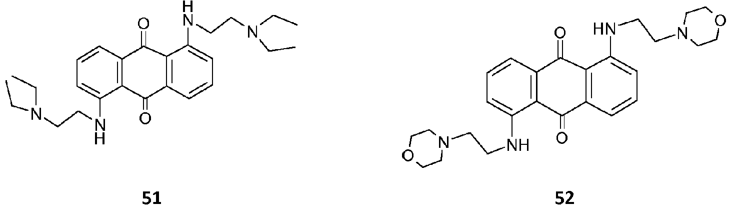 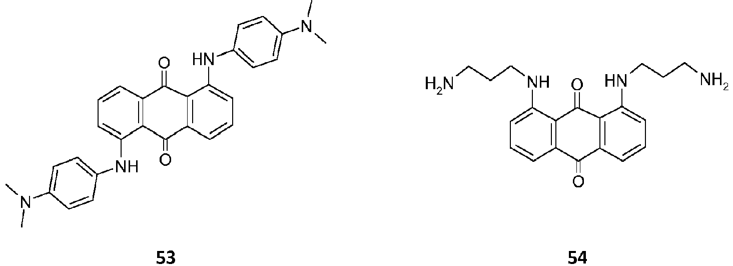 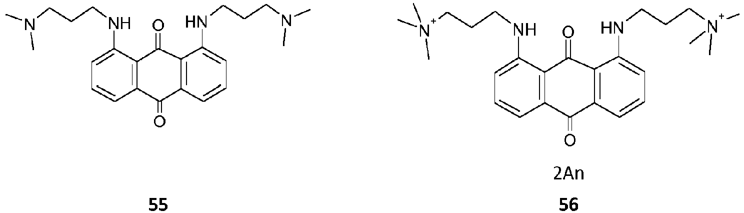 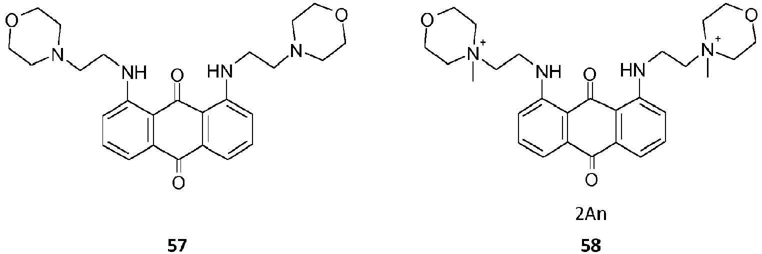 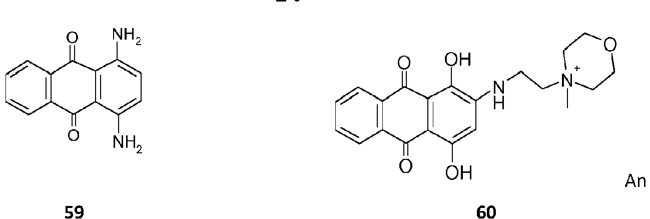 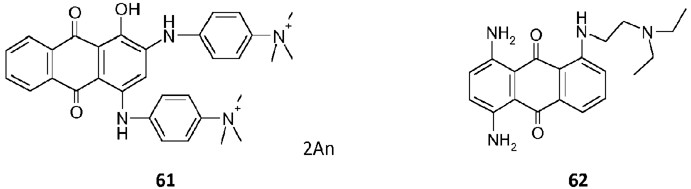 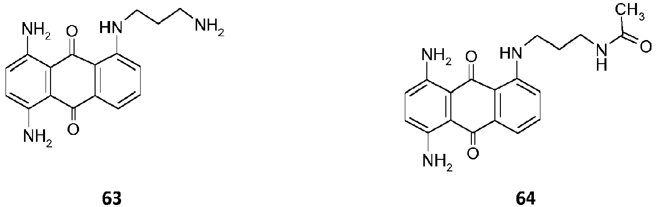 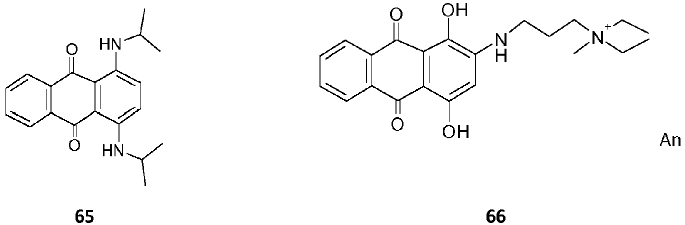 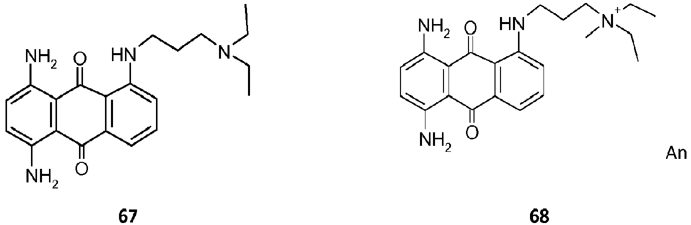 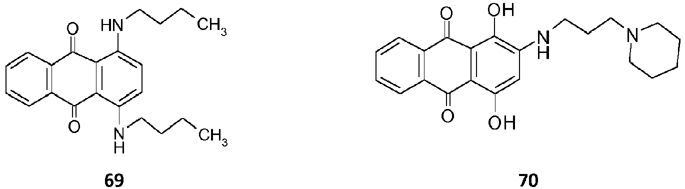 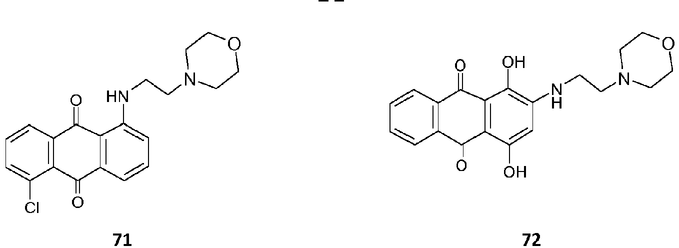 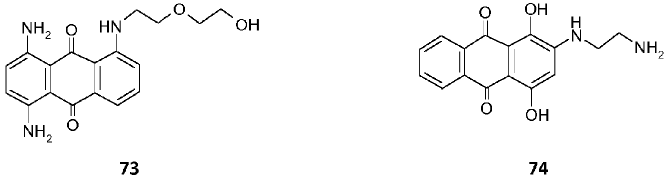 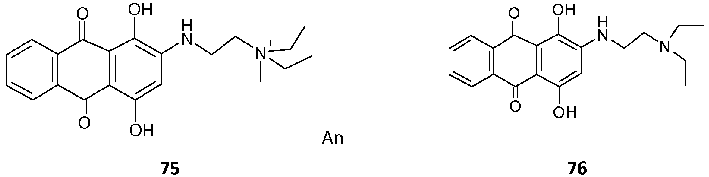 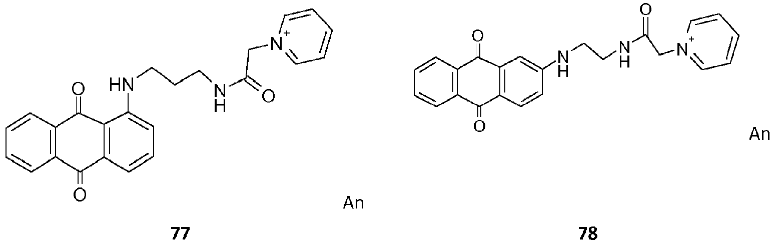 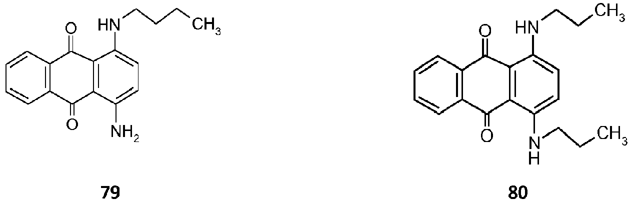 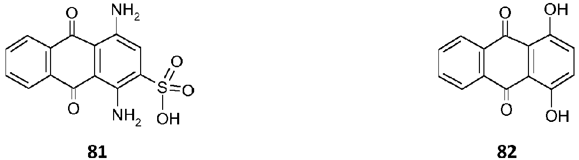 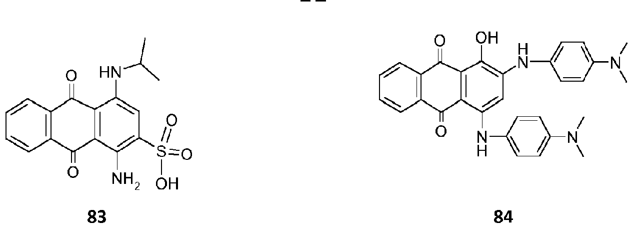 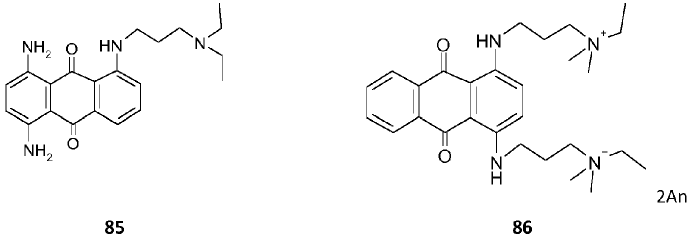 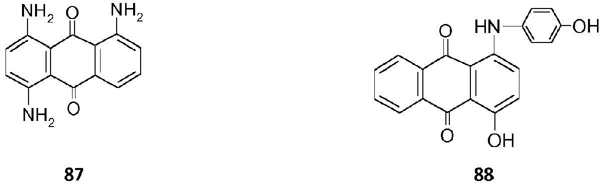 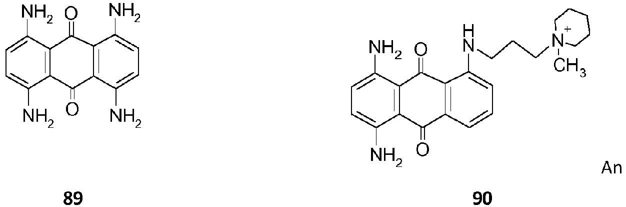 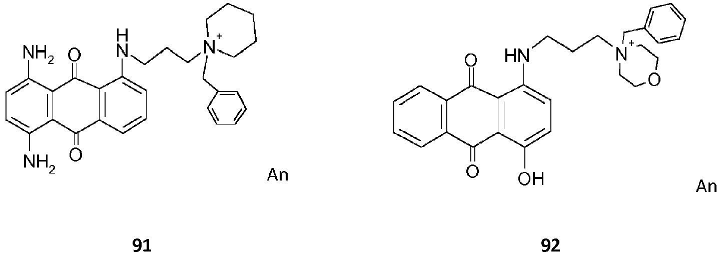 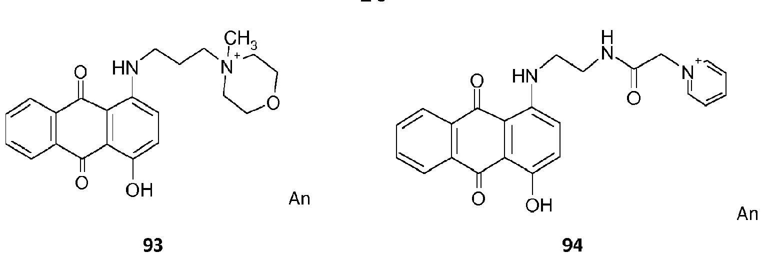 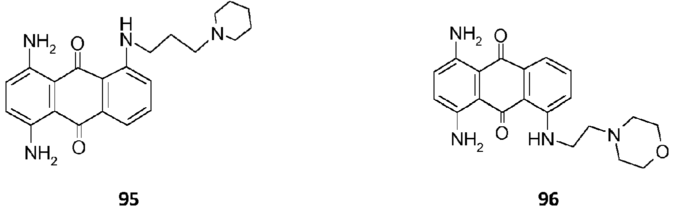 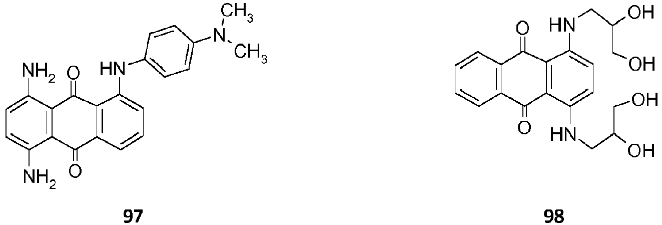 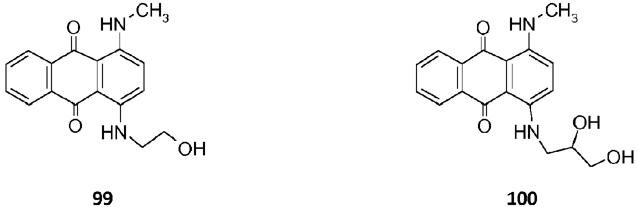 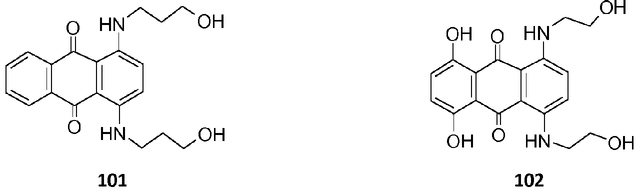 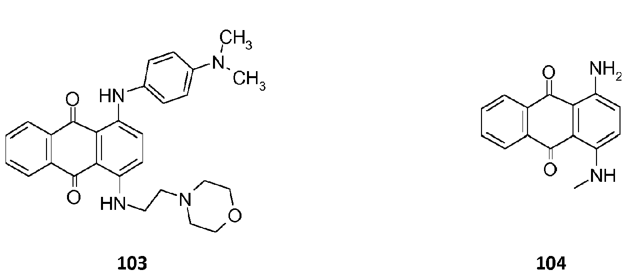 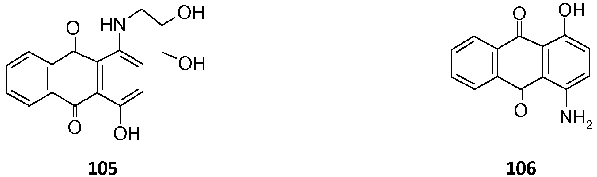 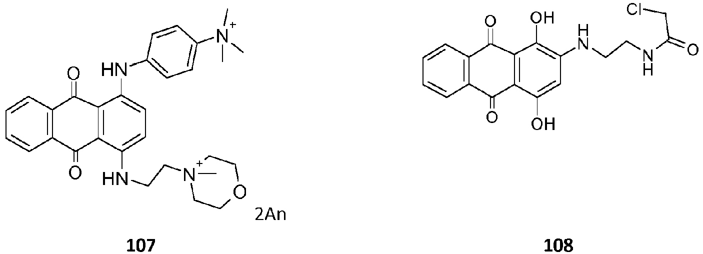 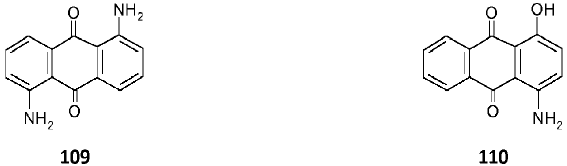 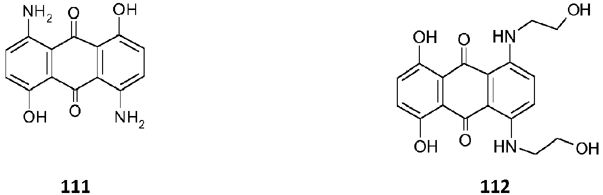 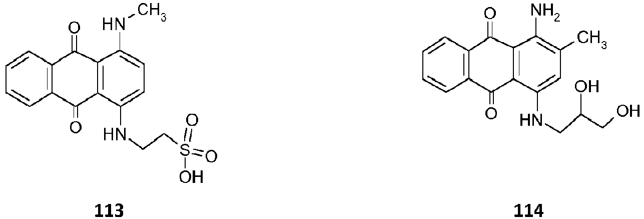 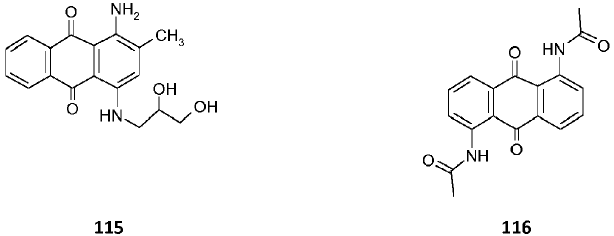 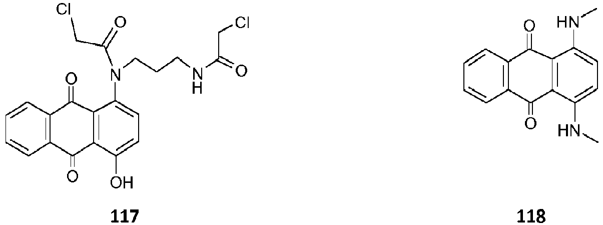 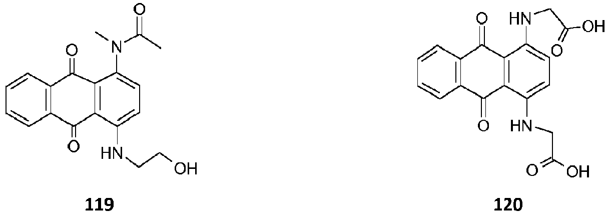 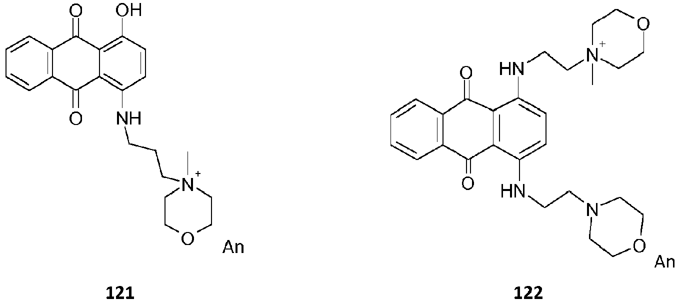 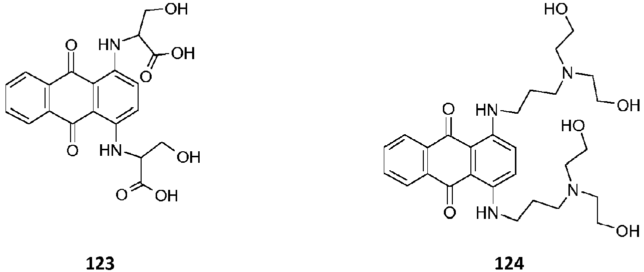 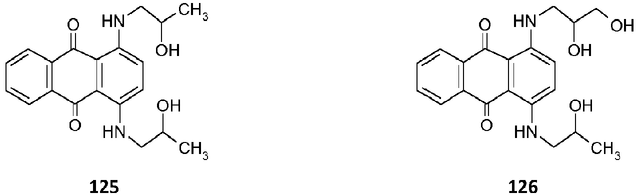 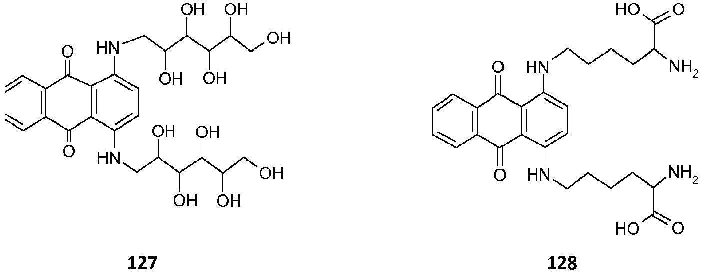 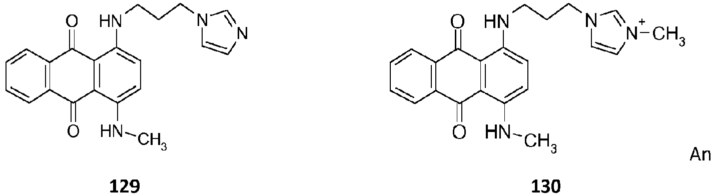 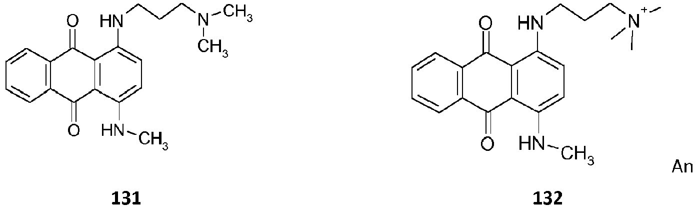 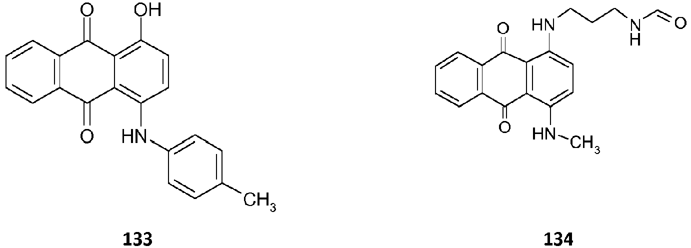 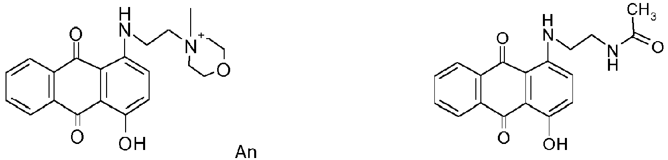 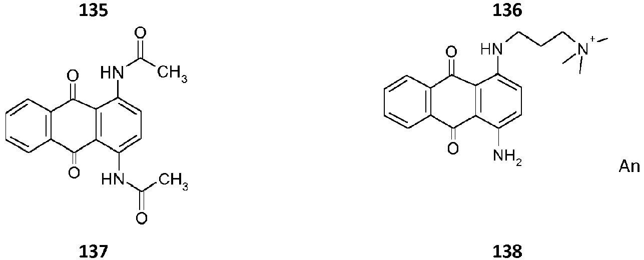 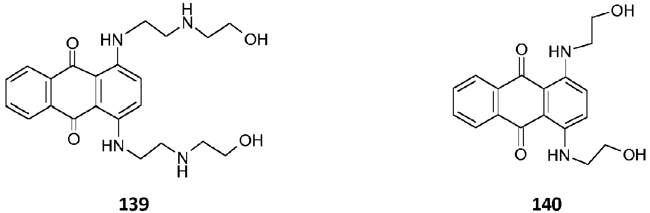 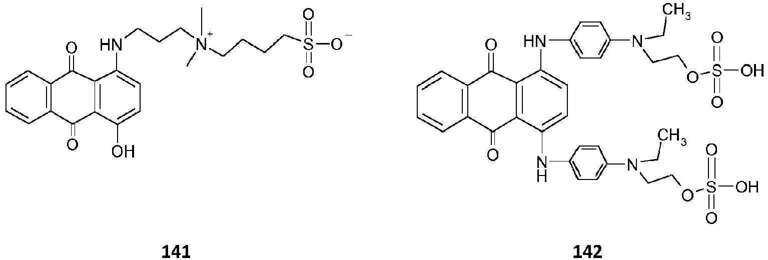 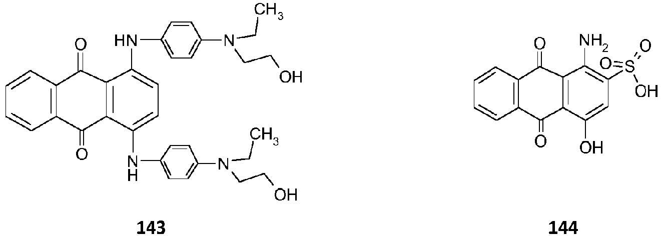 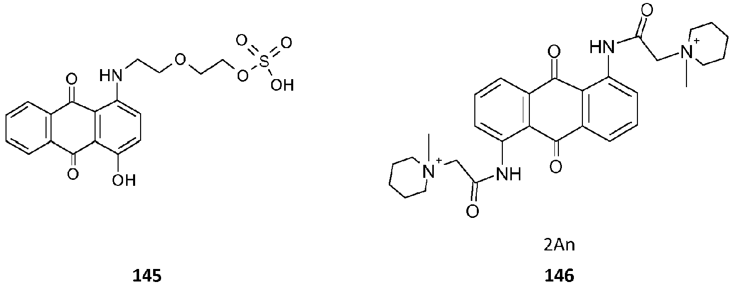 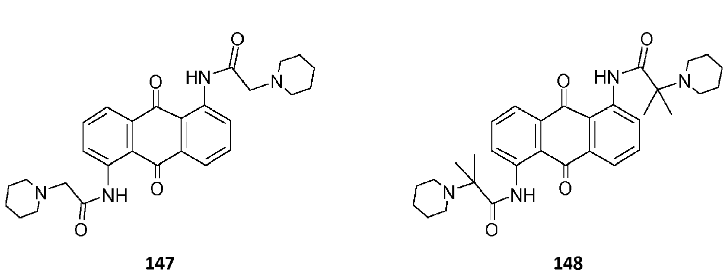 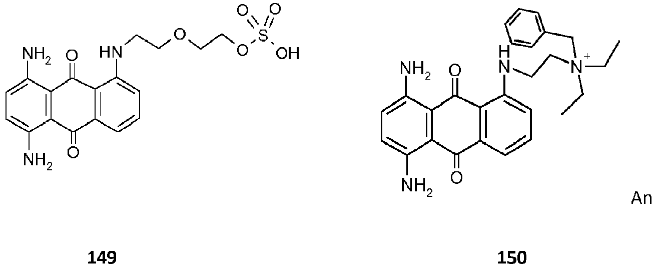 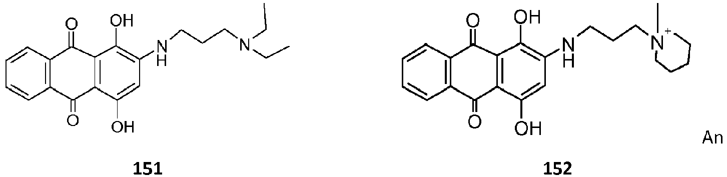 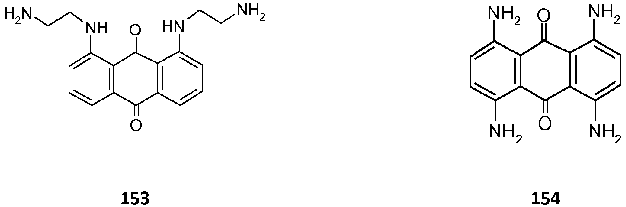 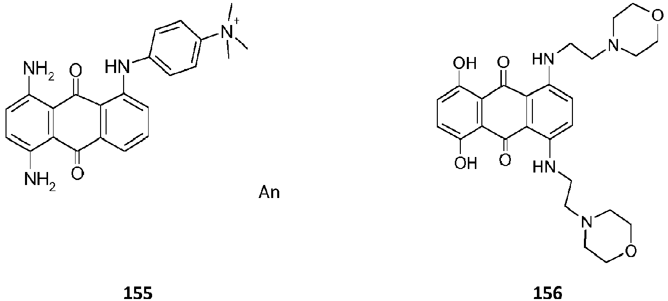 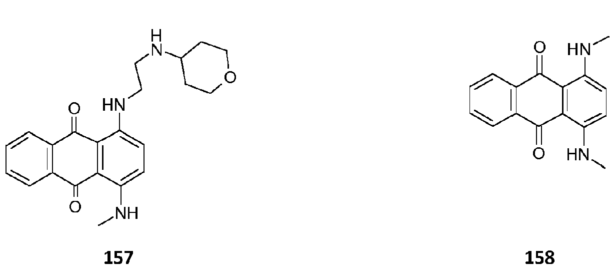 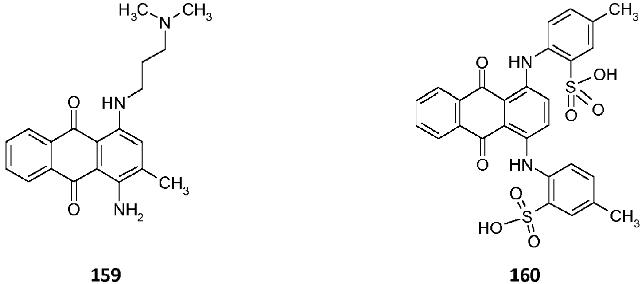 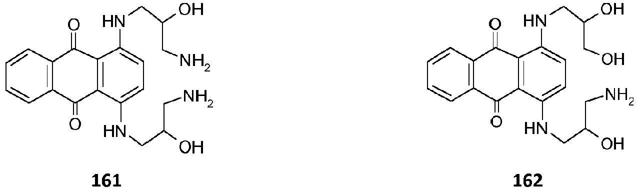 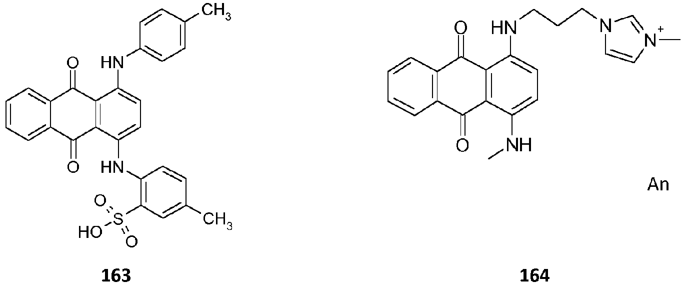 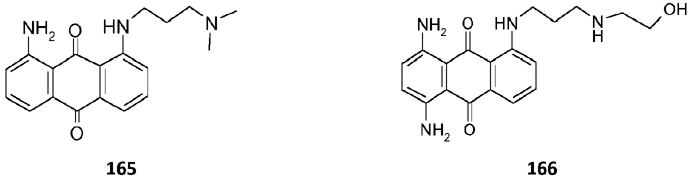 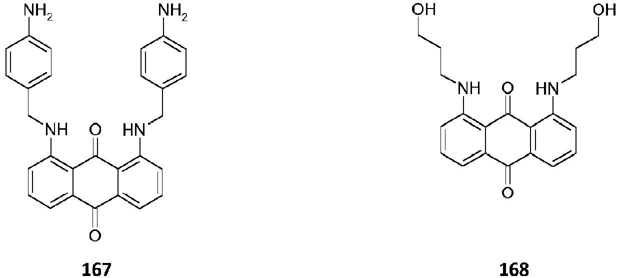 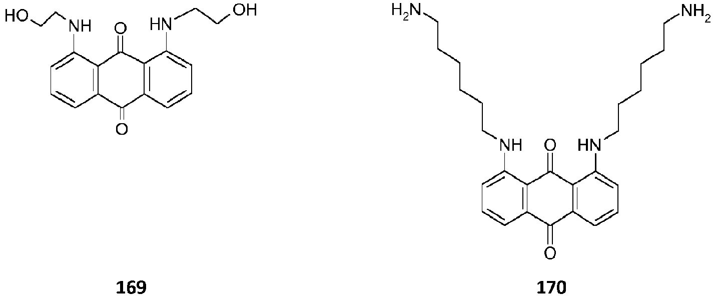 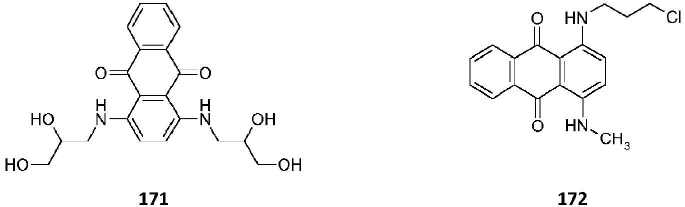 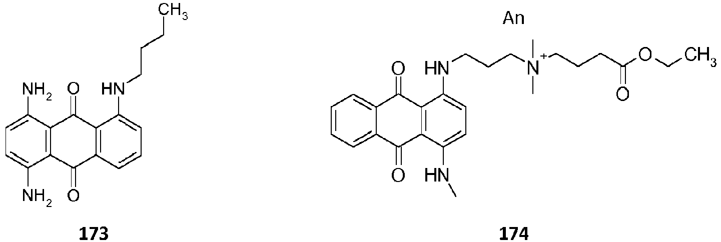 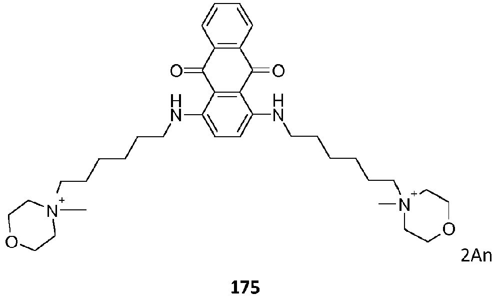 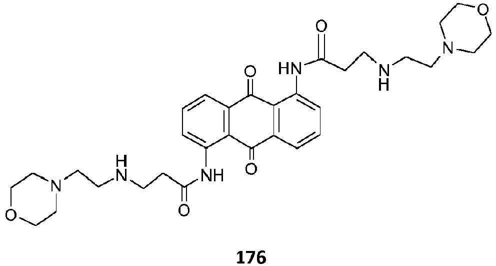 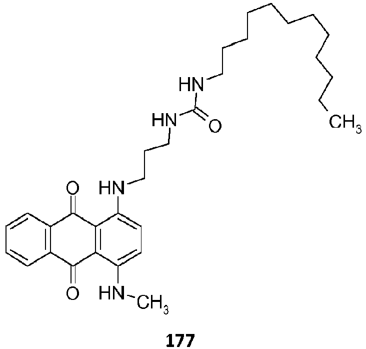 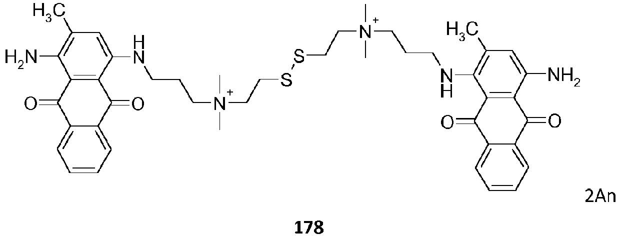 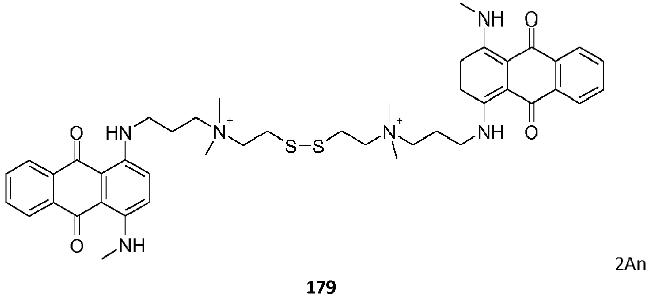 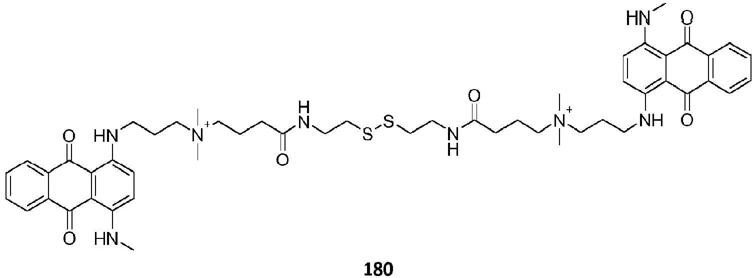 wobei An, das gleich oder verschieden sein kann, für ein anionisches Gegenion gemäß obiger Definition steht, vorzugsweise ausgewählt aus Mesylat, Tosylat und Halogenid wie Cl⁻ und I⁻.

13. Verfahren nach einem der vorhergehenden Ansprüche, wobei es sich bei dem bzw. den kationischen (Poly)-methin-Fluoreszenzfarbstoffen um Direktfarbstoffe handelt, die aus Cyanin-Farbstoffen und Styryl/Hemicyanin-Farbstoffen und Naphthalimid-Farbstoffen und Mischungen davon ausgewählt sind; spezieller die kationischen Fluoreszenzfarbstoffe Direktfarbstoffe sind und aus kationischen Styryloder Hemicyanin-Farbstoffen ausgewählt sind.

14. Verfahren nach einem der vorhergehenden Ansprüche, wobei es sich bei dem bzw. den kationischen (Poly)-methin-Fluoreszenzfarbstoffen (b) um Farbstoffe handelt, die Licht im gelben, orangefarbenen und roten Bereich absorbieren, vorzugsweise bei der Absorptionswellenlänge λ_{abs} zwischen 400 nm und 500 nm inklusive.

15. Verfahren nach einem der vorhergehenden Ansprüche, wobei es sich bei dem bzw. den kationischen (Poly)-methin-Fluoreszenzfarbstoffen (b) um Farbstoffe handelt, die mindestens einen kationischen Chromophor tragen, der aus den nachstehenden Formeln **(III), (IV), (IIIa)** und **(IVa)** ausgewählt ist:
**W⁺-[C(R^{c})=C(R^{d})]_{m'}-Ar'-(*) Q⁻** **(III)**
**Ar-[C(R^{d})=C(R^{c})]_{m'}-W'⁺-(*) Q⁻** **(IV),**
wobei in den Formeln **(III)** und **(IV):**
• **W⁺** für eine kationische Heteroarylgruppe, die insbesondere ein quaternäres Ammonium umfasst, das gegebenenfalls durch eine oder mehrere (C₁-Cs)Alkylgruppen, die gegebenenfalls durch eine oder mehrere Hydroxylgruppen substituiert sind, substituiert ist;
• **W'⁺** für einen zweiwertigen Heteroarylrest wie für **W⁺** definiert steht;
• **Ar** für eine Arylgruppe wie Phenyl oder Naphthyl, die gegebenenfalls durch i) ein oder mehrere Halogenatome wie Chlor oder Fluor; ii) ein oder mehrere (C₁-C₈)Alkyl- und vorzugsweise C₁-C₄-Alkylgruppen wie Methyl; iii) eine oder mehrere Hydroxylgruppen; iv) eine oder mehrere (Ci-C₈)Alkoxygruppen wie Methoxy; v) eine oder mehrere Hydroxy(C₁-C₈)alkylgruppen wie Hydroxyethyl, vi) eine oder mehrere Amino- oder (Di) (C₁-Cs)alkylaminogruppen, wobei vorzugsweise der Ci-C₄-Alkylteil gegebenenfalls durch eine oder mehrere Hydroxylgruppen substituiert ist, wie (Di)hydroxyethylamino, vii) eine oder mehrere Acylaminogruppen; viii) eine oder mehrere Heterocycloalkylgruppen wie Piperazinyl, Piperidinyl oder 5- oder 6-gliedriges Heteroaryl wie Pyrrolidinyl, Pyridyl und Imidazolinyl substituiert ist, steht;
• **Ar'** ein zweiwertiger Rest wie für **Ar** definiert ist;
• m' für eine ganze Zahl zwischen 1 und 4 inklusive steht, insbesondere m gleich 1 oder 2, noch besser 1, ist;
• **R^{c}** und **R^{d},** die gleich oder verschieden sein können, für ein Wasserstoffatom oder gegebenenfalls eine substituierte (C₁-C₈)Alkylund vorzugsweise C₁-C₄-Alkylgruppe stehen oder alternativ dazu **R^{c}** an **W** oder **W'** angrenzt und/oder **R^{d}** an **Ar** oder **Ar'** angrenzt und mit den Atomen die sie tragen, ein (Hetero)cycloalkyl bilden; insbesondere **R^{c}** an **W⁺** oder **W'⁺** angrenzt und ein (Hetero)cycloalkyl wie Cyclohexyl bildet;
• **Q⁻** ein organisches oder mineralisches anionisches Gegenion ist;
• **(*)** für den Teil des fluoreszierenden Chromophors steht, der an den Rest des Farbstoffs gebunden ist;
vorzugsweise wobei **W⁺** oder **W'⁺** ein Imidazolium-, Pyridinium-, Benzimidazolium-, Pyrazolium-, Benzothiazolium- oder Chinoliniumrest, der gegebenenfalls durch einen oder mehrere gleiche oder verschiedene C₁-C₄-Alkyl Reste substituiert ist; besonders bevorzugt der oder die fluoreszierenden Chromophoren aus denjenigen mit **m'** = 1 ausgewählt sind, wobei **Ar** für eine Phenylgruppe, die in para-Position zur Styrylgruppe -C(R^{d})=C(R^{c})- durch eine (Di) (hydroxy) (C₁-C₆) (alkyl)aminogruppe wie Dihydroxy(C₁-C₄)alkylamino substituiert ist, steht und wobei **W'⁺** für eine Imidazolium- oder Pyridiniumgruppe, vorzugsweise ortho- oder para-Pyridinium, steht; 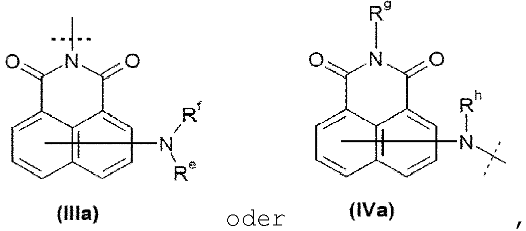 wobei 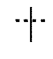 für eine Bindung an den Farbstoff steht, wobei in den Formeln **(IIIa)** und **(IVa)** R^{e}, R^{f}, R^{g} und R^{h}, die gleich oder verschieden sein können, für ein Wasserstoffatom oder eine (C₁-C₆)Alkylgruppe, die gegebenenfalls substituiert ist, vorzugsweise durch eine **D**i (C₁-C₆) alkylamino- oder Tri(C₁-C₆)alkylammoniumgruppe wie Trimethylammonium, stehen.

16. Verfahren nach einem der vorhergehenden Ansprüche, wobei der bzw. die kationischen (Poly)methin-Fluoreszenzfarbstoffe (b) aus den Farbstoffen der Formeln **(V), (VI)** und **(VII):** 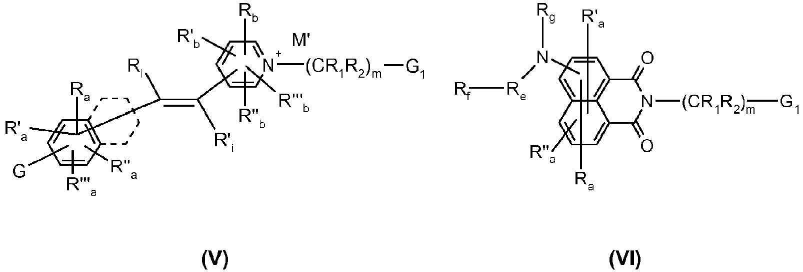 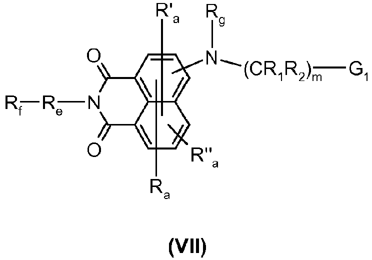 sowie den organischen oder mineralischen Säure- oder Basensalzen davon, den optischen Isomeren, geometrischen Isomeren und Tautomeren davon und den Solvaten davon wie Hydraten ausgewählt ausgewählt sind;
wobei in den Formeln **(V), (VI)** und **(VII):**
• **R₁** und **R₂,** die gleich oder verschieden sein können, für ein Wasserstoffatom oder eine C₁-C₆-Alkylgruppe, bevorzugt ein Wasserstoffatom, stehen;
• **G₁** für ein Wasserstoffatom oder eine aus NH₂ und OH ausgewählte Gruppe, vorzugsweise Wasserstoff, steht;
• **Rₐ, R'_{a,} R''ₐ, R'''ₐ, R_{b}, R'_{b}, R''_{b}** und **R'''_{b},** die gleich oder verschieden sein können, für a) ein Wasserstoffatom b) ein Halogenatom, eine Gruppe von c) Amino, d) (C₁-C₄) Alkylamino, e) (Ci-C₄)Dialkylamino, f) Cyano, g) Carboxyl -C(O)OH oder Carboxylat -C(O)O⁻, Q⁺, h) Hydroxy -OH oder Alkoxid -O⁻Q⁺, i) (Poly) halogen (C₁-C₆) alkyl wie Trifluormethyl, j) Acylamino, k) (C₁-C₆)Alkoxy, 1) (C₁-C₆)Alkylthio, m) (Poly) hydroxy (C₂-C₄) - alkoxy, n) (C₁-C₆)Alkylcarbonyloxy, o) (C₁-C₆)-Alkoxycarbonyl, p) (C₁-C₆)Alkylcarbonylamino, q) Acylamino, r) Carbamoyl, s) (C₁-C₆)Alkylsulfonylamino, t) Aminosulfonyl, u) -SO₃H oder Sulfonat -SO₃⁻, Q⁺ oder v) (C₁-C₆)Alkyl, das gegebenenfalls durch eine Gruppe, die aus (C₁-C₆)Alkoxy, Hydroxyl, Cyano, Carboxyl, Amino und (Di) (C₁-C₄)alkylamino ausgewählt ist, substituiert ist, stehen oder alternativ dazu die beiden Alkylreste, die von dem Stickstoffatom der Aminogruppe getragen werden, einen 5- bis 7-gliedrigen Heterocyclus, der gegebenenfalls einen weiteren Stickstoff oder ein weiteres nicht Stickstoff-Heteroatom umfasst, bilden; insbesondere **Rₐ, R'ₐ, R''ₐ, R'''ₐ, Rb, R'_{b}, R''_{b}** und **R"'_{b}** für ein Wasserstoff- oder Halogenatom oder eine (C₁-C₄)Alkylgruppe, vorzugsweise ein Halogenatom, stehen;
• oder alternativ dazu zwei Gruppen **Rₐ** und **R'ₐ; R_{b}** und **R'_{b},** die von zwei benachbarten Kohlenstoffatomen getragen werden, zusammen einen Benzo- oder Indenoring oder eine anellierte Heterocycloalkyl- oder anellierte Heteroarylgruppe bilden; wobei der Benzo-, Indeno-, Heterocycloalkyl- oder Heteroarylring gegebenenfalls durch ein Halogenatom, eine Amino-, (C₁-C₄)Alkylamino-, (C₁-C₄)Dialkylamino-, Nitro-, Cyano-, Carboxyl-, Hydroxyl- oder Trifluormethylgruppe, einen Acylamino-, (Ci-C₄)Alkoxy-, (Poly)hydroxy(C₁-C₄)alkoxy-, (C₁-C₄)-Alkylcarbonyloxy-, (C₁-C₄)Alkoxycarbonyl oder (C₁-C₄)Alkylcarbonylaminorest, einen Acylamino-, Carbamoyl- oder Alkoxyalkylsulfonylaminorest, einen Aminosulfonylrest oder einen (Ci-C₆)Alkylrest, der gegebenenfalls durch eine Gruppe, die aus (C₁-C₆)Alkoxy, Hydroxyl, Cyano, Carboxyl, Amino, (C₁-C₄)Alkylamino und (Ci-C₄) Dialkylamino ausgewählt ist, substituiert ist, steht oder alternativ dazu die beiden Alkylreste, die von dem Stickstoffatom der Aminogruppe getragen werden, einen 5- bis 7-gliedrigen Heterocyclus, der gegebenenfalls einen weiteren Stickstoff oder ein weiteres nicht Stickstoff-Heteroatom umfasst, bilden; bevorzugt **Rₐ** und **R'ₐ** zusammen eine Benzogruppe bilden;
• oder alternativ dazu zwei Gruppen Rᵢ und Rₐ und/oder eine Gruppe R'ᵢ und R'ₐ zusammen ein anelliertes (Hetero)cycloalkyl, bevorzugt Cycloalkyl wie Cyclohexyl, bilden;
• **R_{g}** für ein Wasserstoffatom, eine (Hetero)aryl(C₁-C₄)alkylgruppe oder eine (C₁-C₆)Alkylgruppe, die gegebenenfalls substituiert ist, steht; bevorzugt R_{b} für ein Wasserstoffatom oder eine (C₁-C₃)Alkyl- oder Benzylgruppe steht;
• **Rₑ** für eine kovalente Bindung oder eine lineare oder verzweigte, gegebenenfalls substituierte (C₁-C₈) Alkylen- oder (C₂-C₈) Alkenylenkette auf Kohlenwasserstoffbasis steht, vorzugsweise **Rₑ** für ein unsubstituiertes (C₁-C₆)Alkylen steht;
• **R_{f}** für ein Wasserstoffatom, eine (Ci-C₄)Alkoxygruppe, eine Aminogruppe R₃R₄N-, eine quaternäre Ammoniumgruppe M', R₃R₄R₅N⁺-, wobei R₃, R₄ und R₅, die gleich oder verschieden sein können, für ein Wasserstoffatom oder eine (C₁-C₄)Alkylgruppe stehen, steht oder R₃R₄N- für eine gegebenenfalls substituierte Heteroarylgruppe, bevorzugt eine gegebenenfalls substituierte Imidazolylgruppe, steht oder alternativ dazu M', R₃R₄R₅N⁺- für eine gegebenenfalls substituierte kationische Heteroarylgruppe, bevorzugt eine Imidazoliniumgruppe, die gegebenenfalls durch eine (C₁-C₃)Alkylgruppe substituiert ist, steht;
• **G** für eine Gruppe i) -NR_{c}R_{d}, ii) -OR steht, wobei R für a) ein Wasserstoffatom, b) eine gegebenenfalls substituierte, vorzugsweise unsubstituierte (C₁-C₆)Alkylgruppe, c) eine gegebenenfalls substituierte (Hetero)arylgruppe, d) eine gegebenenfalls substituierte (Hetero) aryl (C₁-C₆) alkylgruppe wie Benzyl, e) eine gegebenenfalls substituierte (Hetero)cycloalkylgruppe, f) eine gegebenenfalls substituierte (Hetero) cycloalkyl(C₁-C₆)alkylgruppe steht; gemäß einer besonderen Ausführungsform G für eine Gruppe -NR_{c}R_{d} steht, gemäß einer anderen besonderen Ausführungsform G für eine (C₁-C₆)Alkoxygruppe steht;
oder alternativ dazu dann, wenn G für -NR_{c}R_{d} steht, zwei Gruppen R_{c} und R'ₐ und/oder R_{d} und Rₐ zusammen ein gesättigtes Heteroaryl oder einen gesättigten Heterocyclus, das bzw. der gegebenenfalls durch eine oder mehrere (Ci-C₆)Alkylgruppen substituiert ist, vorzugsweise einen 5- bis 7-gliedrigen Heterocyclus mit einem oder zwei Heteroatomen, die aus Stickstoff und Sauerstoff ausgewählt sind, bilden; weiter bevorzugt der Heterocyclus aus Morpholinyl-, Piperazinyl-, Piperidyl- und Pyrrolidinylgruppen ausgewählt ist;
• **R_{c}** und **R_{d},** die gleich oder verschieden sein können, für ein Wasserstoffatom oder eine Gruppe von a) gegebenenfalls substituiertem (Hetero)aryl wie Phenyl, b) gegebenenfalls substituiertem (Hetero) aryl (C₁-C₄) alkyl, c) gegebenenfalls substituiertem (Hetero)cycloalkyl, d) gegebenenfalls substituiertem (Hetero) cycloalkyl (C₁-C₄) alkyl, f) (C₂-C₅)Alkyl oder g) (C₁-C₈)Alkyl, das gegebenenfalls substituiert ist, vorzugsweise gegebenenfalls durch eine Hydroxyl-, Carboxyl-, Carboxylat-, Sulfat- oder Sulfonatgruppe substituiert ist, stehen;
oder alternativ dazu zwei benachbarte Reste **R_{c}** und **R_{d},** die von demselben Stickstoffatom getragen werden, zusammen eine gegebenenfalls substituierte heterocyclische Gruppe oder gegebenenfalls substituierte Heteroarylgruppe bilden;
• **Rᵢ** und **R'ᵢ,** die gleich oder verschieden sein können, für ein Wasserstoffatom oder eine (C₁-C₄)-Alkylgruppe stehen;
• 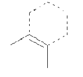 für eine an den Phenylring anellierte (Hetero)arylgruppe steht oder alternativ dazu an dem Phenyl fehlt; bevorzugt der Ring dann, wenn er vorhanden ist, ein Benzo ist;
• m für eine ganze Zahl zwischen 1 und 18 inklusive, insbesondere eine ganze Zahl zwischen 1 und 14 inklusive, bevorzugt eine ganze Zahl zwischen 2 und 10 inklusive, weiter bevorzugt eine ganze Zahl zwischen 3 und 8, spezieller eine ganze Zahl zwischen 4 und 6, steht;
• **M'** für ein anionisches Gegenion steht, das sich von einem Salz einer organischen Säure oder Mineralsäure oder von einer organischen Base oder Mineralbase ableitet und die elektrische Neutralität des Moleküls gewährleistet;
• **Q⁺** für ein anionisches Gegenion steht, das sich von einem Salz einer organischen Säure oder Mineralsäure oder von einer organischen Base oder Mineralbase ableitet und die elektrische Neutralität des Moleküls gewährleistet, wie Alkalimetall, Erdalkalimetall oder Ammonium; wobei es sich versteht, dass dann, wenn das Molekül eine Carboxylat-, Sulfonat- oder Alkoxidgruppe umfasst, M' und **Q⁺** fehlen können, um die elektrische Neutralität des Moleküls zu gewährleisten.

17. Verfahren nach einem der vorhergehenden Ansprüche, wobei der bzw. die kationischen (Poly)methin-Fluoreszenzfarbstoffe (b) aus den Styrylfarbstoffen der nachstehenden Formel (VIII): 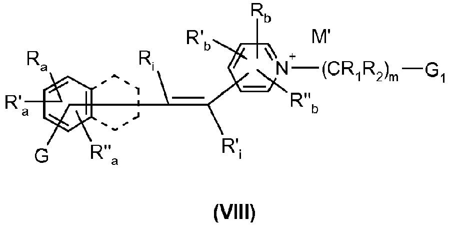 sowie den organischen oder mineralischen Säure- oder Basensalzen davon, den optischen Isomeren, geometrischen Isomeren und Tautomeren davon und den Solvaten davon wie Hydraten ausgewählt ausgewählt sind;
wobei in Formel **(VIII) G, G₁, R_{a,} R'_{a,} R''_{a,} Rb, R'_{b}, R''_{b}, Rᵢ, R'ᵢ, R₁, R₂** und **m** wie im vorhergehenden Anspruch definiert sind;
insbesondere:
• **R₁** und **R₂,** die gleich oder verschieden sein können, für ein Wasserstoffatom stehen;
• **Rᵢ** und **R_{i'},** die gleich oder verschieden sein können, für ein Wasserstoffatom oder eine (C₁-C₄)Alkylgruppe, vorzugsweise Wasserstoff, stehen;
• **R_{a,} R'ₐ** und **R''ₐ,** die gleich oder verschieden sein können, für ein Wasserstoffatom, ein Halogenatom wie Fluor oder eine -OH-, -O⁻Q⁺, (C₁-C₆)Alkoxy-, Nitro- oder Cyanogruppe stehen, wobei Q⁺ wie im vorhergehenden Anspruch definiert ist;
• **R_{b}**, **R'_{b}** und **R''_{b},** die gleich oder verschieden sein können, für ein Wasserstoffatom oder eine (C₁-C₆)Alkylgruppe stehen;
• oder alternativ dazu zwei angrenzende Reste **R_{b}** und **R'_{b}** zusammen mit den Kohlenstoffatomen, die sie tragen, eine Benzogruppe bilden, die mit der Pyridiniumgruppe kondensiert oder anelliert ist, wobei die Benzogruppe gegebenenfalls substituiert ist; vorzugsweise die Benzogruppe unsubstituiert ist;
• **G** für eine Gruppe -NR_{c}R_{d} oder eine (C₁-C₆)Alkoxygruppe, die gegebenenfalls substituiert, vorzugsweise unsubstituiert, ist; gemäß einer besonderen Ausführungsform G für eine Gruppe - NR_{c}R_{d} steht, gemäß einer anderen besonderen Ausführungsform G für eine (C₁-C₆)Alkoxygruppe steht;
• **G₁** für ein Wasserstoffatom oder eine OH- oder NH₂-Gruppe, vorzugsweise Wasserstoff, steht;
• **Rᵢ** und **R_{i'},** die gleich oder verschieden sein können, für ein Wasserstoffatom oder eine (C₁-C₄)Alkylgruppe stehen;
• 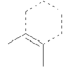 für eine an den Phenylring anellierte Aryl-oder Heteroarylgruppe steht oder alternativ dazu an dem Phenyl fehlt; bevorzugt der Ring dann, wenn er vorhanden ist, ein Benzo ist;
• **m** für eine ganze Zahl zwischen 1 und 18 inklusive, insbesondere eine ganze Zahl zwischen 2 und 16 inklusive, bevorzugt eine ganze Zahl zwischen 3 und 10, weiter bevorzugt eine ganze Zahl zwischen 4 und 6, steht;
• **R_{c}** und **R_{d},** die gleich oder verschieden sein können, für ein Wasserstoffatom, eine (C₂-C₄)Alkylgruppe oder eine substituierte (Ci-Cs)Alkylgruppe, vorzugsweise (C₂-C₄)Alkyl, das insbesondere durch eine oder mehrere Gruppen, die aus i) Cyano, ii) (C₁-C₃)Alkoxy, iii) Hydroxyl und iv) (C₁-C₃)Alkylcarbonyl ausgewählt sind, vorzugsweise durch ein oder mehrere Hydroxylgruppen, substituiert ist, stehen; und
• **M'** für ein anionisches Gegenion steht, das sich von einem Salz einer organischen Säure oder Mineralsäure oder von einer organischen Base oder Mineralbase ableitet und die elektrische Neutralität des Moleküls gewährleistet;
wobei es sich versteht, dass dann, wenn das Molekül eine Alkoxidgruppe umfasst, M' und Q⁺ fehlen können, um die elektrische Neutralität des Moleküls zu gewährleisten.

18. Verfahren nach einem der vorhergehenden Ansprüche, wobei der bzw. die kationischen (Poly)methin-Fluoreszenzfarbstoffe (b) aus den Farbstoffen der nachstehenden Formeln (X), (XI), (XII) und (XIII): 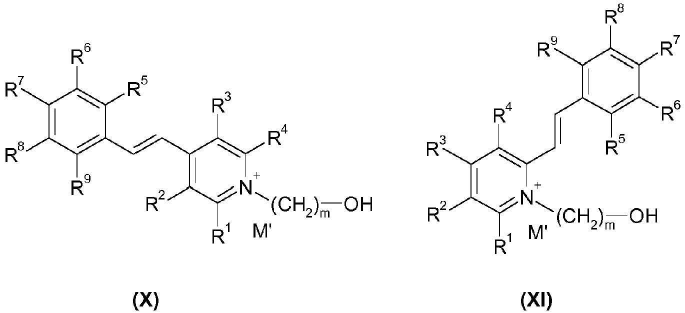 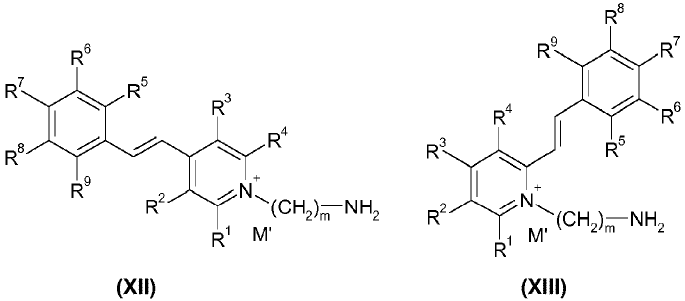 sowie den organischen oder mineralischen Säure- oder Basensalzen davon, den optischen Isomeren, geometrischen Isomeren und Tautomeren davon und den Solvaten davon wie Hydraten ausgewählt ausgewählt sind;
wobei in den Formeln **(X), (XI), (XII)** und **(XIII):**
• **R¹**, **R², R³** und **R⁴**, die gleich oder verschieden sein können, für ein Wasserstoffatom oder eine (C₁-C₆)Alkylgruppe stehen; vorzugsweise **R²** und **R³** für ein Wasserstoffatom stehen und **R¹** und **R⁴**, die gleich oder verschieden sein können, für ein Wasserstoffatom oder eine (C₁-C₄)Alyklgruppe stehen;
• **R⁵**, **R⁶**, **R⁷**, **R⁸** und **R⁹,** die gleich oder verschieden sein können, für i) ein Wasserstoffatom oder ii) ein Halogenatom wie Cl, Br oder F, iii) eine Gruppe OR, in der R für ein Wasserstoffatom oder Q⁺, das wie zuvor beschrieben ist oder eine (C₁-C₃)Alkylgruppe steht, iv) Aryl wie Benzol, v) Aryl (C₁-C₃) alkyl wie Benzyl, vi) Cyano, vii) Nitro, viii) (C₁-C₃)Alkylthio, ix) Amino NR¹⁰R¹¹ stehen, wobei R¹⁰ und R¹¹, die gleich oder verschieden sein können, für a) ein Wasserstoffatom, b) eine (C₂-C₄)Alkylgruppe oder c) eine substituierte (C₁-C₈)Alkylgruppe, vorzugsweise eine (C₂-C₄)Alkylgruppe, die gegebenenfalls durch eine oder mehrere Gruppen, die aus
- Cyano,
- (C₁-C₃)Alkoxy,
- Hydroxyl und
- (C₁-C₃)Alkylcarbonyl
ausgewählt sind, substituiert ist, stehen; insbesondere **R¹⁰** und **R¹¹,** die gleich oder verschieden sein können, für ein Wasserstoffatom oder eine (C₁-C₆)Alkylgruppe, die durch eine oder mehrere Hydroxyl-, Cyano- oder (C₁-C₃)Alkylcarbonylgruppen substituiert ist, wie Hydroxyethyl, stehen;
• **m** für eine ganze Zahl zwischen 1 und 18 inklusive, insbesondere eine ganze Zahl zwischen 2 und 16 inklusive, bevorzugt eine ganze Zahl zwischen 3 und 10, weiter bevorzugt eine ganze Zahl zwischen 4 und 6, steht;
• **M'** für ein anionisches Gegenion steht, das wie im vorhergehenden Anspruch definiert ist; wobei es sich versteht, dass dann, wenn das Molekül eine Alkoxidgruppe umfasst, M' und Q⁺ fehlen können, um die elektrische Neutralität des Moleküls zu gewährleisten.

19. Verfahren nach einem der Ansprüche 1 bis 18, wobei der bzw. die kationischen (Poly)methin-Fluoreszenzfarbstoffe (b) der Erfindung aus denjenigen der nachstehenden Formeln **(XIV)** und **(XV)** ausgewählt sind: 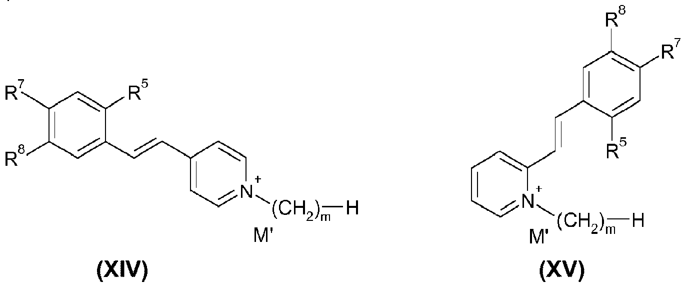 wobei in den Formeln **(XIV)** und **(XV) R⁵**, **R⁷**, **R⁸** und **m** wie oben für **(X)** und **(XI)** definiert sind, insbesondere:
• **R⁵** und **R⁸**, die gleich oder verschieden sein können, für ein Wasserstoffatom oder eine (C₁-C₄)Alkoxygruppe wie Methoxy stehen, vorzugsweise **R⁵** und **R⁸** für ein Wasserstoffatom stehen;
• **R**⁷ für eine (C₁-C₄)Alkoxygruppe oder NR¹⁰R¹¹ steht, wobei R¹⁰ und R¹¹, die gleich oder verschieden sein können, für a) ein Wasserstoffatom oder b) eine (C₁-C₈)Alkylgruppe, die gegebenenfalls durch eine oder mehrere Gruppen, die aus i) Hydroxyl, ii) R-Z-C(X)-Y-, wobei X, Y und Z für ein Sauerstoff- oder Schwefelatom oder N(R') stehen oder alternativ dazu X und/oder Z für eine Bindung stehen, R und R', die gleich oder verschieden sein können, für ein Wasserstoffatom oder eine (C₁-C₆)Alkylgruppe stehen, vorzugsweise X für ein Sauerstoffatom steht, iii) Sulfonsäure SO₃H, iv) Sulfonat SO₃⁻, Q⁺, v) Carboxylat C(O)O⁻ ausgewählt sind, substituiert ist, stehen, wobei Q⁺ für ein kationisches Gegenion wie ein Alkalimetall oder Erdalkalimetall steht; insbesondere **R**⁷ für eine Gruppe NR¹⁰R¹¹ steht, wobei R¹⁰ und R¹¹, die gleich oder verschieden sein können, für a) ein Wasserstoffatom oder b) eine (C₁-C₆) Alkylgruppe, die gegebenenfalls durch eine oder mehrere Gruppen, die aus i) Hydroxyl, ii) Carboxyl, iii) Carboxylat, iv) Sulfonsäure und v) Sulfonat, ausgewählt sind, substituiert ist, stehen, spezieller aus gleichen oder verschiedenen Gruppen, die für a) ein Wasserstoffatom oder b) eine (C₁-C₆) Alkylgruppe, die gegebenenfalls durch eine oder mehrere Gruppen, die aus i) Hydroxyl, ii) Carboxyl und iii) Carboxylat ausgewählt sind, substituiert ist, ausgewählt sind;
• **m** für eine ganze Zahl zwischen 1 und 18 inklusive, insbesondere eine ganze Zahl zwischen 1 und 6 inklusive, bevorzugt eine ganze Zahl zwischen 1 und 4, weiter bevorzugt eine ganze Zahl zwischen 1 und 2, steht; und
• **M'** für ein anionisches Gegenion steht, das sich von einem Salz einer organischen oder mineralischen Säure oder einer organischen oder mineralischen Base ableitet und die elektrische Neutralität des Farbstoffs gewährleistet;
wobei es sich versteht, dass dann, wenn das Molekül eine Alkoxidgruppe umfasst, M' und Q⁺ fehlen können, um die elektrische Neutralität des Moleküls zu gewährleisten.

20. Verfahren nach einem der vorhergehenden Ansprüche, wobei:
• der oder die kationischen Poly(methin)-Fluoreszenzfarbstoffe (b) der Formel (X) entsprechen, wobei:
| **R¹** | **R₂** | **R³** | **R⁴** | **R⁵** | **R⁶** | **R**⁷ | **R⁸** | **R⁹** | m |
|---|---|---|---|---|---|---|---|---|---|
| H | H | H | H | H | H | H | H | H | 1 |
| H | H | H | H | H | t-Bu | OH | t-Bu | H | 5 |
| H | H | H | H | H | t-Bu | OH | t-Bu | H | 5 |
| H | H | H | H | H | H | NH₂ | H | H | 1 |
| H | H | H | H | H | H | NH₂ | H | OCH₃ | 1 |
| H | H | H | H | H | H | OH | Br | H | 5 |
| H | H | H | H | H | OCH₃ | OH | OCH₃ | H | 5 |
| H | H | H | H | Cl | H | OH | H | Cl | 5 |
| H | H | H | H | H | H | OH | H | H | 10 |
| H | H | H | H | H | OCH₃ | OH | OCH₃ | H | 10 |
| H | H | H | H | H | t-Bu | OH | t-Bu | H | 10 |
| H | H | H | H | H | H | N(CH₂CH₃)₂ | H | H | 1 |
| H | H | H | H | H | H | N(CH₂CH₃)₂ | H | H | 1 |
| H | H | H | H | H | H | N(CH₂CH₃)₂ | H | H | 1 |
| H | H | H | H | H | t-Bu | OH | t-Bu | H | 10 |
| H | H | H | H | H | H | N(CH₂CH₃)CH₂CH₂OH | H | H | 1 |
| H | H | H | H | H | H | N(CH₂CH₃)₂ | H | H | 1 |
| H | H | H | H | H | H | N(CH₂CH₃)₂ | H | H | 1 |
| H | H | H | H | H | H | N(CH₂CH₃)₂ | H | H | 1 |
| H | H | H | H | H | H | N(CH₂CH₃)₂ | H | H | 1 |
| H | H | H | H | H | H | N(CH₂CH₃)₂ | H | H | 1 |
| H | H | H | H | H | H | N(CH₂CH₃)₂ | H | H | 1 |
| H | H | H | H | H | H | N(CH₂CH₃)₂ | H | H | 1 |
| H | H | H | H | H | H | N(CH₂CH₃)₂ | H | H | 1 |
| H | H | H | H | H | H | N(CH₂CH₃)₂ | H | H | 1 |
| H | H | H | H | H | H | n-C₆H₁₃ | H | H | 1 |
| H | H | H | H | H | H | N(CH₂CH₂OH)₂ | H | H | 2 |
| H | H | H | H | H | H | N(n-Bu)₂ | H | H | 2 |
| H | H | H | H | H | OCH₃ | OH | H | H | 10 |
| H | H | H | H | H | H | OC₂H₅OH | H | H | 1 |
| H | H | H | H | H | H | OH | H | H | 1 |
| H | H | Benzo | | H | H | H | H | H | 1 |
| H | H | Benzo | | H | H | N(CH₂CH₂OH)₂ | H | H | 1 |
| H | H | Benzo | | H | H | N(CH₂CH₂OH)₂ | H | H | 1 |
sowie den organischen oder mineralischen Säure- oder Basensalzen davon, den optischen Isomeren, geometrischen Isomeren und Tautomeren davon und den Solvaten davon wie Hydraten;
der oder die kationischen Poly(methin)-Fluoreszenz-farbstoffe (b) der Formel **(XI)** entsprechen, wobei:
| **R¹** | **R²** | **R³** | R⁴ | **R⁵** | **R⁶** | **R**⁷ | **R⁸** | **R⁹** | **m** |
|---|---|---|---|---|---|---|---|---|---|
| CH₃ | H | H | H | OH | H | OCH₃ | H | H | 2 |
| CH₃ | H | H | H | H | H | OCH₃ | OCH₂-Ph | H | 2 |
| CH₃ | H | H | H | H | H | H | H | OCH₃ | 2 |
| CH₃ | H | H | H | F | H | H | H | H | 2 |
| CH₃ | H | H | H | H | H | H | OPh | H | 2 |
| CH₃ | H | H | H | H | H | N(CH₂CH₂OAC)₂ | H | H | 2 |
| CH₃ | H | H | H | OCH₃ | H | OCH₃ | OCH₃ | H | 2 |
| CH₃ | H | H | H | H | H | H | CH₃ | H | 2 |
| CH₃ | H | H | H | H | H | OH | H | H | 2 |
| CH₃ | H | H | H | H | OCH₃ | OCH₃ | OCH₃ | H | 2 |
| CH₃ | H | H | H | H | OCH₃ | OH | OH | H | 2 |
| CH₃ | H | H | H | H | H | OCH₃ | OCH₃ | OCH₃ | 2 |
| CH₃ | H | H | H | H | H | H | OCH₃ | OH | 2 |
| CH₃ | H | H | H | H | H | N(n-Butyl)₂ | H | H | 2 |
| CH₃ | H | H | H | H | OCH₃ | OCH₃ | H | H | 2 |
| CH₃ | H | H | H | H | H | H | H | H | 2 |
| CH₃ | H | H | H | H | H | i-Propyl | H | H | 2 |
| H | H | H | H | H | H | OH | H | H | 6 |
| H | H | H | H | H | OCH₃ | OCH₃ | OCH₃ | H | 6 |
| H | H | H | H | OH | H | OCH₃ | H | H | 2 |
| H | H | H | H | OCH₃ | H | H | OCH₃ | OCH₃ | 2 |
| H | H | H | H | H | H | H | H | Br | 2 |
| H | H | H | H | H | H | OH | H | H | 2 |
| H | H | H | H | OCH₃ | OCH₃ | OCH₃ | H | H | 6 |
| H | H | H | H | OH | OCH₃ | H | H | H | 6 |
| H | H | H | H | H | OCH₃ | OCH₃ | H | H | 6 |
| H | H | H | H | H | OCH₃ | OCH₃ | H | H | 2 |
| H | H | H | H | H | H | C(O)-OH | H | H | 6 |
| H | H | H | H | H | H | C(O)-OH | H | H | 2 |
| H | H | H | H | H | H | i-Propyl | H | H | 2 |
| H | H | H | H | H | H | N(CH₃)CH₂CH₂CN | H | H | 2 |
| H | H | H | H | H | H | OCH₃ | OCH₂Ph | H | 2 |
| H | H | H | H | H | H | H | OPh | H | 2 |
| H | H | H | H | H | H | N(CH₂CH₂C(O)CH₃)₂ | H | H | 2 |
| H | H | H | H | OH | H | OCH₃ | H | H | 6 |
| H | H | H | H | H | OCH₃ | OCH₃ | OCH₃ | H | 2 |
| H | H | H | H | OCH₃ | H | OCH₃ | OCH₃ | H | 6 |
| H | H | H | H | H | H | H | CH₃ | H | 2 |
| H | H | H | H | H | H | N(CH₃)CH₂CH₂OH | H | H | 2 |
sowie den organischen oder mineralischen Säure- oder Basensalzen davon, den geometrischen Isomeren und Tautomeren davon und den Solvaten davon wie Hydraten;
• der oder die kationischen Poly(methin)-Fluoreszenzfarbstoffe (b) den Formeln **(X')** und **(XI')** entsprechen: 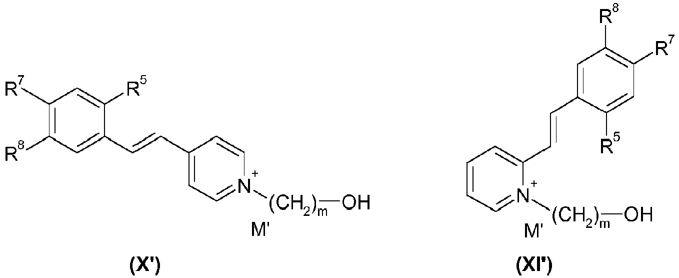 wobei M' wie im vorhergehenden Anspruch definiert ist; und
| **R⁵** | **R**⁷ | **R⁸** | **m** | | **R⁵** | **R**⁷ | **R⁸** | **m** |
|---|---|---|---|---|---|---|---|---|
| H | N(CH₂CH₂OH)₂ | H | 2 | | OCH₃ | OCH₃ | OCH₃ | 2 |
| H | N(CH₂CH₂OH)₂ | H | 3 | | OCH₃ | OCH₃ | OCH₃ | 3 |
| H | N(CH₂CH₂OH)₂ | H | 4 | | OCH₃ | OCH₃ | OCH₃ | 3 |
| H | N(CH₂CH₂OH)₂ | H | 5 | | OCH₃ | OCH₃ | OCH₃ | 4 |
| H | N(CH₂CH₂OH)₂ | H | 6 | | OCH₃ | OCH₃ | OCH₃ | 5 |
| H | N(CH₂CH₂OH)₂ | H | 8 | | OCH₃ | OCH₃ | OCH₃ | 8 |
| H | N(CH₂CH₂OH)₂ | H | 10 | | OCH₃ | OCH₃ | OCH₃ | 10 |
| H | N(CH₂CH₂OH)₂ | H | 12 | | OCH₃ | OCH₃ | OCH₃ | 12 |
| H | N(CH₂CH₂OH)₂ | H | 14 | | OCH₃ | OCH₃ | OCH₃ | 14 |
| H | N(CH₂CH₂OH) ₂ | H | 16 | | OCH₃ | OCH₃ | OCH₃ | 16 |
| H | CH₃CH₂N(CH₂CH₂OH) | H | 2 | | | | | |
| H | CH₃CH₂N(CH₂CH₂OH) | H | 4 | und | | | | |
sowie den organischen oder mineralischen Säure- oder Basensalzen davon, den geometrischen Isomeren und Tautomeren davon und den Solvaten davon wie Hydraten;
der oder die kationischen Poly(methin)-Fluoreszenz-farbstoffe der Formel **(XII)** entsprechen, wobei:
| **R¹** | **R²** | **R³** | **R⁴** | **R⁵** | **R⁶** | **R**⁷ | **R⁸** | **R⁹** | **m** |
|---|---|---|---|---|---|---|---|---|---|
| H | H | H | H | H | H | OCH₃ | OCH₃ | OCH₃ | 2 |
| H | H | H | H | OH | OCH₃ | H | H | H | 2 |
| H | H | H | H | H | H | H | H | H | 2 |
| H | H | H | H | H | OCH₃ | OCH₃ | H | H | 2 |
| H | H | H | H | OH | H | OH | H | H | 6 |
| H | H | H | H | OCH₃ | H | OCH₃ | OCH₃ | H | 6 |
| H | H | H | H | H | H | OH | H | H | 6 |
| H | H | H | H | OCH₃ | H | H | H | F | 2 |
| H | H | H | H | H | H | C(O)-OH | H | H | 2 |
| H | H | H | H | H | H | Isopropyl | H | H | 2 |
| H | H | H | H | H | H | N(CH₂CH₂C(O)CH₃)₂ | H | H | 2 |
| H | H | H | H | OH | H | OCH₃ | H | H | 2 |
| H | H | H | H | H | H | OH | H | H | 2 |
| H | H | H | H | H | OCH₃ | OH | OH | H | 2 |
| H | H | H | H | H | CH₃ | OCH₂Ph | CH₃ | H | 2 |
| H | H | H | H | H | OCH₃ | OCH₃ | OCH₃ | H | 2 |
| H | H | H | H | H | OCH₃ | OCH₃ | OCH₃ | H | 6 |
| H | H | H | H | H | H | N(CH₃)₂ | H | H | 6 |
| H | H | H | H | H | H | OCH₃ | OCH₃ | OCH₃ | 6 |
| H | H | H | H | H | H | Phenyl | H | H | 6 |
| H | H | H | H | H | OCH₃ | OCH₃ | H | H | 6 |
| H | H | H | H | H | H | C(O)-OH | H | H | 6 |
| H | H | H | H | H | H | N(n-Butyl)₂ | H | H | 2 |
| H | H | H | H | H | H | OCH₃ | OCH₃ | H | 3 |
| H | H | H | H | H | H | OCH₃ | OCH₃ | H | 2 |
| H | H | H | H | H | H | OCH₃ | OCH₃ | H | 5 |
| H | H | H | H | H | H | OCH₃ | H | H | 3 |
| H | H | H | H | H | H | N(CH₃)₂ | H | H | 3 |
| H | H | H | H | H | H | H | H | H | 3 |
sowie den organischen oder mineralischen Säure- oder Basensalzen davon, den optischen Isomeren, geometrischen Isomeren und Tautomeren davon und den Solvaten davon wie Hydraten;
• der oder die kationischen Poly(methin)-Fluoreszenzfarbstoffe der Formel **(XIII)** entsprechen, wobei:
| **R¹** | **R²** | **R³** | **R⁴** | **R⁵** | **R⁶** | **R**⁷ | **R⁸** | **R⁹** | **m** |
|---|---|---|---|---|---|---|---|---|---|
| CH₃ | H | H | H | OH | H | OCH₃ | H | H | 2 |
| CH₃ | H | H | H | H | H | OCH₃ | OCH₂-Ph | H | 2 |
| CH₃ | H | H | H | H | H | H | H | OCH₃ | 2 |
| CH₃ | H | H | H | F | H | H | H | H | 2 |
| CH₃ | H | H | H | H | H | H | OPh | H | 2 |
| CH₃ | H | H | H | H | H | N(CH₂CH₂OAC)₂ | H | H | 2 |
| CH₃ | H | H | H | OCH₃ | H | OCH₃ | OCH₃ | H | 2 |
| CH₃ | H | H | H | H | H | H | CH₃ | H | 2 |
| CH₃ | H | H | H | H | H | OH | H | H | 2 |
| CH₃ | H | H | H | H | OCH₃ | OCH₃ | OCH₃ | H | 2 |
| CH₃ | H | H | H | H | OCH₃ | OH | OH | H | 2 |
| CH₃ | H | H | H | H | H | OCH₃ | OCH₃ | OCH₃ | 2 |
| CH₃ | H | H | H | H | H | H | OCH₃ | OH | 2 |
| CH₃ | H | H | H | H | H | N(n-Butyl)₂ | H | H | 2 |
| CH₃ | H | H | H | H | OCH₃ | OCH₃ | H | H | 2 |
| CH₃ | H | H | H | H | H | H | H | H | 2 |
| CH₃ | H | H | H | H | H | i-Propyl | H | H | 2 |
| H | H | H | H | H | H | OH | H | H | 6 |
| H | H | H | H | H | OCH₃ | OCH₃ | OCH₃ | H | 6 |
| H | H | H | H | OH | H | OCH₃ | H | H | 2 |
| H | H | H | H | OCH₃ | H | H | OCH₃ | OCH₃ | 2 |
| H | H | H | H | H | H | H | H | Br | 2 |
| H | H | H | H | H | H | OH | H | H | 2 |
| H | H | H | H | H | H | N(CH₃)₂ | H | H | 6 |
| H | H | H | H | OCH₃ | OCH₃ | OCH₃ | H | H | 6 |
| H | H | H | H | OH | OCH₃ | H | H | H | 6 |
| H | H | H | H | H | OCH₃ | OCH₃ | H | H | 6 |
| H | H | H | H | H | OCH₃ | OCH₃ | H | H | 2 |
| H | H | H | H | H | H | C(O)-OH | H | H | 6 |
| H | H | H | H | H | H | C(O)-OH | H | H | 2 |
| H | H | H | H | H | H | i-Propyl | H | H | 2 |
| H | H | H | H | H | H | N(CH₃)CH₂CH₂CN | H | H | 2 |
| H | H | H | H | H | H | OCH₃ | OCH₂Ph | H | 2 |
| H | H | H | H | H | H | H | OPh | H | 2 |
| H | H | H | H | H | H | N(CH₂CH₂C(O)CH₃)₂ | H | H | 2 |
| H | H | H | H | OH | H | OCH₃ | H | H | 6 |
| H | H | H | H | H | OCH₃ | OCH₃ | OCH₃ | H | 2 |
| H | H | H | H | OCH₃ | H | OCH₃ | OCH₃ | H | 6 |
| H | H | H | H | H | H | H | CH₃ | H | 2 |
| H | H | H | H | H | H | N(CH₃)CH₂CH₂OH | H | H | 2 |
| CH₃ | H | H | H | H | H | N(CH₃)₂ | H | H | 2 |
sowie den organischen oder mineralischen Säure- oder Basensalzen davon, den geometrischen Isomeren und Tautomeren davon und den Solvaten davon wie Hydraten;
• der oder die kationischen Poly(methin)-Fluoreszenzfarbstoffe (b) den nachstehenden Formeln **(XII')** und **(XIII')** entsprechen: 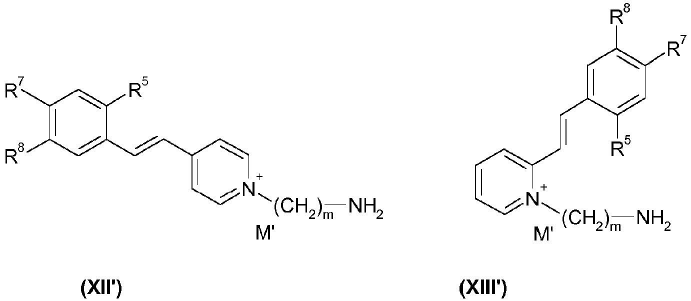 wobei:
| **R⁵** | **R**⁷ | **R⁸** | **m:** | | **R⁵** | **R**⁷ | **R⁸** | **m** |
|---|---|---|---|---|---|---|---|---|
| H | N(CH₂CH₂OH)₂ | H | 2 | | OCH₃ | OCH₃ | OCH₃ | 2 |
| H | N(CH₂CH₂OH)₂ | H | 3 | | OCH₃ | OCH₃ | OCH₃ | 3 |
| H | N(CH₂CH₂OH)₂ | H | 4 | | OCH₃ | OCH₃ | OCH₃ | 3 |
| H | N(CH₂CH₂OH)₂ | H | 5 | | OCH₃ | OCH₃ | OCH₃ | 4 |
| H | N(CH₂CH₂OH)₂ | H | 6 | | OCH₃ | OCH₃ | OCH₃ | 5 |
| H | N(CH₂CH₂OH)₂ | H | 8 | | OCH₃ | OCH₃ | OCH₃ | 8 |
| H | N(CH₂CH₂OH)₂ | H | 10 | | OCH₃ | OCH₃ | OCH₃ | 10 |
| H | N(CH₂CH₂OH)₂ | H | 12 | | OCH₃ | OCH₃ | OCH₃ | 12 |
| H | N(CH₂CH₂OH)₂ | H | 14 | | OCH₃ | OCH₃ | OCH₃ | 14 |
| H | N(CH₂CH₂OH)₂ | H | 16 | und | OCH₃ | OCH₃ | OCH₃ | 16 |
sowie den organischen oder mineralischen Säure- oder Basensalzen davon, den geometrischen Isomeren und Tautomeren davon und den Solvaten davon wie Hydraten;
• der oder die kationischen Poly(methin)-Fluoreszenzfarbstoffe (b) aus den folgenden Verbindungen ausgewählt sind:
| | |
|---|---|
| 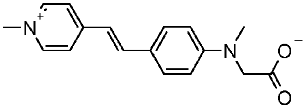 | 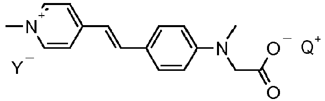 |
| **(d)** | **(d')** |
| 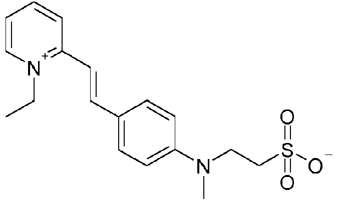 | 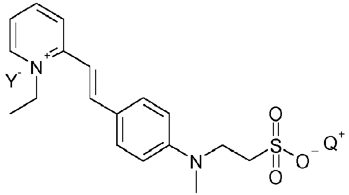 |
| **(e)** | **(e')** |
| 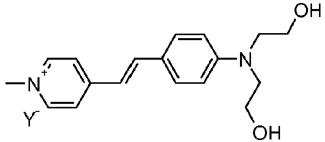 | |
| **(f)** | |
| 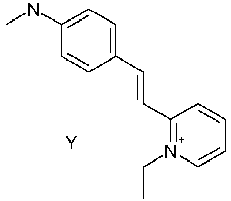 | |
| | |
| **(g)** | |
| 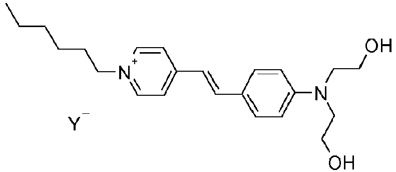 | |
| **(h)** | |
sowie den organischen oder mineralischen Säure- oder Basensalzen davon, den geometrischen Isomeren und Tautomeren davon und den Solvaten davon wie Hydraten;
wobei M' und Q⁺ wie im vorhergehenden Anspruch definiert sind.

21. Verfahren nach einem der vorhergehenden Ansprüche, **dadurch gekennzeichnet, dass** es außerdem das Aufbringen einer kosmetischen Zusammensetzung, die c) ein oder mehrere Reduktionsmittel, die vorzugsweise aus i) den Reduktionsmitteln der nachstehenden Formel (XIV) sowie den Additionssalzen davon und Mischungen davon:
H(X)_{q}(R₁₀)ₜ (XVI)
wobei in Formel (XVI):
- X für P, S oder SO₂ steht,
- q für eine ganze Zahl mit einem Wert von 0 oder 1 steht,
- t für eine ganze Zahl mit einem Wert von 1 oder 2 steht und
- R für einen linearen oder verzweigten, gesättigten oder ungesättigten Ci- bis C₂₀-Alkylrest, der gegebenenfalls durch ein Heteroatom unterbrochen und/oder gegebenenfalls durch einen oder mehrere Reste, die aus Hydroxyl-, Halogen-, Amin-, Carboxyl-, ((C₁-C₃₀)Alkoxy) carbonyl-, Amido-, ((C₁-C₃₀) Alkyl) aminocarbonyl-, ((C₁-C₃₀) Acyl) amino-, Mono- oder Dialkylamino- und Mono- oder Dihydroxylaminoresten ausgewählt sind, substituiert ist, steht;
ii) Thioglykolsäure, iii) Thiomilchsäure, iv) Glycerylmonothioglykolat, v) Cysteamin, vi) N-Acetylcysteamin, vii) N-Propionylcysteamin, viii) Cystein, ix) N-Acetylcystein, x) Thioäpfelsäure, xi) Pantethein, xii) 2,3-Dimercaptobernsteinsäure, xiii) N-(Mercaptoalkyl)-ω-hydroxyalkylamiden, xiv) N-Mono- oder N,N-Dialkylmercapto-4-butyramiden, xv) Aminomercaptoalkylamiden, xvi) N-(Mercaptoalkyl)bernsteinsäuremonoamid-Derivativen, xvii) N-(Mercaptoalkyl)succinimid-Derivativen, xviii) Alkylaminomercaptoalkylamiden, xix) dem azeotropen Gemisch von 2-Hydroxypropylthioglykonat und von (2-Hydroxy-l-methyl)ethylthioglykolat, xx) Mercaptoalkylaminoamiden, xxi) N-Mercaptoalkylalkandiamiden, xxii) Formamidinsulfinsäure-Derivativen, Additionssalzen davon und Mischungen davon ausgewählt ist, auf die Keratinfasern umfasst.

22. Verfahren nach einem der Ansprüche 1 bis 20, bei dem kein Reduktionsmittel verwendet wird.

23. Verfahren nach einem der vorhergehenden Ansprüche, bei dem die Anthrachinon-Verbindung (en) (a) gemäß einem der Ansprüche 1 bis 11 und der bzw. die kationischen (Poly)methin-Fluoreszenzdirekt-farbstoffe (b) gemäß einem der Ansprüche 1 und 12 bis 20 zusammen auf die Keratinfasern aufgebracht werden; vorzugsweise das Verfahren einen Schritt des Aufbringens einer kosmetischen Zusammensetzung, die a) einen oder mehrere Anthrachinon-Farbstoffe der Formeln **(I)** und/oder **(II)** gemäß einem der Ansprüche 1 bis 11 und b) einen oder mehrere kationische(Poly)methin-Fluoreszenzfarbstoffe gemäß einem der Ansprüche 1 und 12 bis 20 umfasst, auf die Keratinfasern umfasst.

24. Verfahren nach einem der Ansprüche 1 bis 22, das mindestens zwei aufeinanderfolgende Schritte umfasst:
- einen Schritt des Aufbringens einer kosmetischen Zusammensetzung, die (b) einen oder mehrere kationische (Poly)methin-Fluoreszenzfarbstoffe gemäß einem der Ansprüche 1 und 12 bis 20 umfasst, auf die Keratinfasern, gefolgt von
- einem Schritt des Aufbringens einer kosmetischen Zusammensetzung die (a) einen oder mehrere Anthrachinon-Farbstoffe der Formel (I) und/oder (II) gemäß einem der Ansprüche 1 bis 11 umfasst, auf die Keratinfasern.

25. Verfahren nach einem der Ansprüche 1 bis 22, das mindestens zwei aufeinanderfolgende Schritte umfasst:
- einen Schritt des Aufbringens einer kosmetischen Zusammensetzung die (a) einen oder mehrere Anthrachinon-Farbstoffe der Formel (I) und/oder (II) gemäß einem der Ansprüche 1 bis 11 umfasst, auf die Keratinfasern, gefolgt von
- einem Schritt des Aufbringens einer kosmetischen Zusammensetzung, die (b) einen oder mehrere kationische (Poly)methin-Fluoreszenzfarbstoffe gemäß einem der Ansprüche 1 und 12 bis 20 umfasst, auf die Keratinfasern.

26. Verfahren nach einem der vorhergehenden Ansprüche, wobei der pH-Wert der kosmetischen Zusammensetzung bzw. der kosmetischen Zusammensetzungen zwischen 6 und 11 inklusive, insbesondere zwischen 7 und 10, spezieller zwischen 7,5 und 9,5, wie zwischen 9 und 9,5, liegt.

27. Kosmetische Zusammensetzung, umfassend (a) einen oder mehrere Anthrachinon-Farbstoffe der Formel (I) und/oder **(II)** gemäß einem der Ansprüche 1 bis 11 und (b) einen oder mehrere kationische (Poly)methin-Fluoreszenzfarbstoffe gemäß einem der Ansprüche 1 und 13 bis 20 gegebenenfalls bei einem wie im vorhergehenden Anspruch definierten pH-Wert.

## Revendications

1. Procédé de coloration des fibres kératiniques, en particulier des fibres kératiniques humaines, telles que les cheveux comprenant l'application sur lesdites fibres kératiniques des ingrédients (a) et (b) suivants :
(a) un ou plusieurs colorants anthraquinones choisis parmi les composés de formules (I) et/ou (II) suivantes, leurs isomères optiques, leurs isomères géométriques, leurs tautomères, leurs solvates et leurs mélanges : 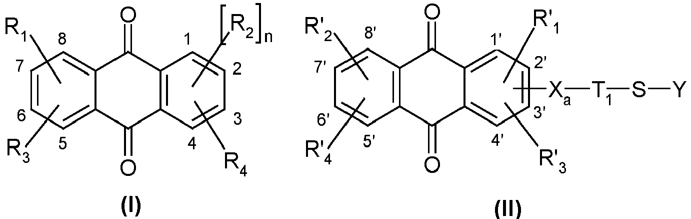 Formules (I) ou (II) dans lesquelles,
▪ Xₐ, identique ou différent, représente une liaison, un hétéroatome ou groupe choisi parmi un atome d'oxygène ou de soufre, N(Rₐ), CO, SO, SO₂, ou leur associations telles que -O-CO-, -CO-O-, -NH-CO-ou -CO-NH-, avec Rₐ représentant un atome d'hydrogène, ou un groupe (C₁-C₆) alkyle éventuellement substitué par un ou plusieurs groupes hydroxyle ; de préférence, Xₐ représentent N(Rₐ) et particulièrement NH ;
▪ Y représente : i) un atome d'hydrogène ; ii) un métal alcalin ; iii) un métal alcalino-terreux ; iv) un groupe ammonium : N⁺R^{a}R^{b}R^{g}R^{d} ou un groupe phosphonium : P⁺R^{a}R^{b}R^{g}R^{d} avec **R_{a,}** R^{b}, R^{g} et R_{d}**,** qui peuvent être identiques ou différents, représentant un atome d'hydrogène ou un groupe (C₁-C₄) alkyle ; ou v) un groupe protecteur de fonction thiol ; ou vi) le groupe (III) suivant : 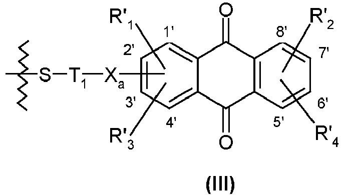
▪ R₁, R₂**,** R₃, R₄, R'₁, R'₂, R'₃ et R'₄, identiques ou différents, représentent un atome ou groupe choisi parmi :
o hydrogène ;
o halogène tel que le brome et le chlore,
o hydroxyle,
o (C₁-C₄) alcoxy,
o hydroxysulfonyle (-SO₃H) ou sulfonate (-SO₃⁻, M⁺), avec M⁺ représentant un contre-ion cationique en particulier un métal alcalin, alcalino terreux, ou ammonium, tel que Na⁺ ou K⁺ ;
o (C₁-C₆)alkyle, éventuellement substitué,
o -NR₅R₆ dans lequel R⁵ et R₆, identiques ou différents, représentent un atome ou radical choisi parmi : i) hydrogène, ii) (C₁-C₄)alkylcarbonyle tel que methylcarbonyl (-COCH₃), iii) arylsulfonyle tel que phénylsulfonyle (-SO₂Ph), iv) Het-ALK-C(O)- avec Het représentant un groupe hétérocycloalkyle éventuellement substitué, en particulier par un ou plusieurs groupes (C₁-C₄) alkyle et ALK représente un groupe (C₁-C₆) alkylène éventuellement substitué par un ou plusieurs groupes hydroxyle ou (di) (hydroxy) (C₁-C₄) alkylamino ; Het-ALK-C(O)-est tel que (pipéridin-1-yl)acétyle, 2-méthyl-2-(pipéridin-1-yl)propanoyle, 2-(morpholin-4-yl)ethyl-β-alaninoyle, v) aryle éventuellement substitué, en particulier phényle éventuellement substitué par au moins un radical choisi parmi a) (C₁-C₆)alkyle, b) hydroxyle, c) hydroxysulfonyle, d) (C₁-C₄) alcoxy, e) carboxyle (-COOH), f) (C₁-C₄) alcoxycarbonyle, g) amino, h) (di) (C₁-C₄) alkylamino, l'un des radicaux alkyle pouvant être substitué par un radical hydroxy ou hydroxysulfonyle -SO₃H, ou -OSO₃H, vi) aryl(C₁-C₄)alkyle éventuellement substitué, en particulier benzyle éventuellement substitué par un groupe (di) (C₁-C₄) alkylamino, viii) (C₁-C₂₀) alkyle éventuellement substitué, éventuellement interrompu par un ou plusieurs hétéroatomes et/ou par un ou plusieurs groupes comprenant au moins un hétéroatome, de préférence choisis parmi l'oxygène, l'azote ou le soufre, CO, SO, SO₂ ou leurs combinaisons ; lorsque ledit radical alkyle est substitué, il est substitué par un ou plusieurs atomes ou groupes choisis parmi : a) des halogènes, de préférence un ou plusieurs atomes de chlore, b) hydroxyle, c) (C₁-C₆)alkylcarbonylamino, en particulier acylamino, d) hétérocycloalkyle de 5 ou 6 chaînons tels que tétrahydro-2H-pyran-4-amine, morpholino, pipéridino, ou pipérazino, e) (di) (C₁-C₄) alkylamino, f) hydroxysulfonyl(C₁-C₄)alkylamino, ou hydroxysulfonyloxy(C₁-C₄)alkylamino, g) (di) (hydroxy) (C₁-C₄) alkylamino, h) hétéroaryle de 5 ou 6 chaînons tel qu'imidazole, et i) formylamino (-NHCOH) ;
o un groupe de formule (a) suivante : -N(R₇)-X₁-W₁ (a)
formule (a) dans laquelle :
R⁷ représente un hydrogène ou un radical (C₁-C₄) alkyle,
X₁ représente un radical divalent choisi parmi (C₁-C₂₀)alkylène éventuellement interrompu par un ou plusieurs hétéroatomes ou groupes choisis parmi l'oxygène, l'azote ou le soufre, CO, SO, SO₂, arylène tel que phénylène, ou leurs combinaisons ; de préférence X₁ représente :
• (C₁-C₁₀) alkylène,
• - (C₁-C₁₀) alkylcarbonyl-,
• -carbonyl (C₁-C₁₀) alkyl-,
• - (C₁-C₁₀) alkylaminocarbonyl (C₁-C₁₀) alkyl-,
• - (C₁-C₁₀) alkylcarbonylamino (C₁-C₁₀) alkyl-,
• -phényl (C₁-C₁₀) alkyl-,
• - (C₁-C₁₀) alkylphényl-, ou
• phénylène,
W₁ représente un radical cationique choisi parmi : 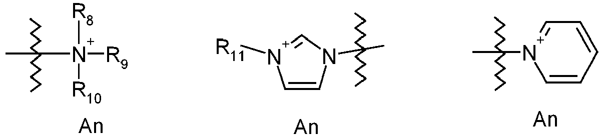
avec R₈, R₉, R₁₀, R₁₁ qui peuvent être identiques ou différents, représentent un groupe (C₁-C₆)alkyle, un radical benzyle, un radical (C₁-C₆)alkylsulfonate ; les radicaux R₈ et R₉ peuvent former, avec l'atome d'azote auquel ils sont liés, un hétérocycle de 5 à 7 chaînons, saturé ou insaturé, éventuellement substitué, comprenant éventuellement un autre hétéroatome non-azote de préférence un atome d'oxygène ;
▪ n est un entier dans la plage de 1 à 3, de préférence n est égal à 1 ou 2,
T₁ représente une chaîne hydrocarbonée divalente, linéaire ou ramifiée, comprenant de 1 à 20 atomes de carbone, éventuellement interrompue par un ou plusieurs hétéroatomes ou groupes, ou leurs combinaisons, choisis parmi oxygène, soufre, N(R_{b}), C(O), -N⁺(R₈) (R₉) -An, hétéroaryle éventuellement cationique et éventuellement substitué, tel qu'imidazolium, An avec R₈ et Rg, qui peuvent être identiques ou différents, représentent un radical (C₁-C₆)alkyle ; R_{b} représentant un atome d'hydrogène ou un groupe (hydroxy) (C₁-C₄) alkyle, de préférence ladite chaîne hydrocarbonée est interrompue par un ou plusieurs groupes choisis parmi N(R_{b}), C(O), et leur combinaison, tels que -C(O)-N(R_{b})- ou -N(R_{b})-C(O)- et -N⁺(R₈) (R₉) -An, An est un contre-ion anionique, organique ou inorganique, qui assure l'électroneutralité des colorants de formules (I) et (II) ;
▪ 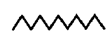 étant la partie de la liaison qui est reliée au reste de la molécule ; et
(b) un ou plusieurs colorants fluorescents (poly)méthines cationiques ;
étant entendu que :
- le ou les colorants anthraquinones de formules (I) comprennent au moins un radical R₁, R₃ ou R₄, autre qu'un atome d'hydrogène et le ou les colorants anthraquinones de formules (II) comprennent au moins un radical R'₁, R'₂, R'₃ ou R'₄ différent d'un atome d'hydrogène ;
- (a) le ou les colorants anthraquinones de formules (I) ou (II) et (b) le ou les colorants fluorescents sont appliqués sur lesdites fibres kératiniques conjointement ou séquentiellement ; et
- lorsque les composés de formule (I) ou (II) sont cationiques et comprennent un groupe sulfonate, alors M⁺ et An peuvent être absents pour assurer l'électroneutralité de ladite molécule.

2. Procédé selon la revendication précédente, dans lequel le ou les colorants anthraquinones de formules (I) et/ou (II) sont des colorants qui absorbent la lumière dans la gamme bleu-violet, de préférence la gamme bleue.

3. Procédé selon l'une des revendications précédentes, dans lequel le ou les colorants anthraquinones de formules (I) et/ou (II) sont des colorants de formule (I) et n est égal à 0 ou 1, de préférence, les substituants R₁, R₂**,** R₃ et R₄, sont aux positions 1, 4, 5 et 8.

4. Procédé selon l'une quelconque des revendications précédentes, dans lequel le ou les colorants anthraquinones de formules (I) et/ou (II) sont des colorants de formule (I) avec R₁ et R₃ représentant un atome ou groupe choisi parmi i) hydroxyle, ii) arylamino ou aryl (C₁-C₆)alkylamino avec le groupe aryle représentant un groupe aryle éventuellement substitué, en particulier un groupe phényle éventuellement substitué par un ou plusieurs groupes choisis parmi (C₁-C₄)alkyle, carboxyle, (C₁-C₄) alcoxycarbonyle, (di) (hydroxy) (C₁-C₄) alkylamino, tri (C₁-C₄) alkylammonium, (C₁-C₄) alcoxy, (di)hydroxysulfonyloxy(C₁-C₄) alkylamino, hydroxysulfonyle, iii) (di) (hydroxy) (C₁-C₆) alkylamino, iv) (di) (C₁-C₄) alkylamino (C₁-C₆) alkylamino, v) (C₁-C₄) alkylcarbonylamino (C₁-C₆) alkylamino, vi) arylsulfonylamino avec le groupe aryle pouvant être éventuellement substitué, aryle représentant de préférence un groupe phényle, vii) (di)halogène(C₁-C₄) alkylamino, viii) (di) (hydroxy) (C₁-C₄) alcoxy (C₁-C₄) alkylamino, ix) (di) tri (C₁-C₄) alkylammonium (C₁-C₆) alkylamino, x) hétérocycloalkyl(C₁-C₆) alkylamino, hétérocycloalkyl(C₁-C₄)alkylcarbonylamino, hétérocycloalkyl(C₁-C₄)alkylamino(C₁-C₄)alkylcarbonylamin o ou hétérocycloalkyl(C₁-C₄) alkylcarbonylamino (C₁-C₆) alkylamin o, ledit hétérocycloalkyle pouvant être éventuellement cationique et/ou substitué en particulier par un ou plusieurs groupes (C₁-C₄)alkyle ou benzyle, xi) halogéno (C₁-C₄) alkylcarbonylamino (C₁-C₆) alkylamino, xii) hydroxysulfonyle, xiii) halogéno tel que chlore, xiv) hétéroaryl(C₁-C₆)alkylamino ledit hétéroaryle pouvant être éventuellement cationique et/ou substitué en particulier par un ou plusieurs groupes (C₁-C₄) alkyle, xv) hétéroaryl(C₁-C₄) alkylcarbonylamino (C₁-C₆) alkylamino, ledit hétéroaryle pouvant être éventuellement cationique et/ou substitué en particulier par un ou plusieurs groupes (C₁-C₄) alkyle, xvi) R'R"N- avec R' et R", qui peuvent être identiques ou différents, représentant un groupe (halogéno) (C₁-C₄) alkylcarbonylamino (C₁-C₆) alkyle, un groupe (halogéno) (C₁-C₄) alkylaminocarbonyl (C₁-C₆) alkyle, un groupe (halogéno) (C₁-C₄) alkylcarbonyle, un groupe carboxy(C₁-C₆)alkyle, le groupe alkyle pouvant être substitué par un ou plusieurs groupes amino ou hydroxyle, un groupe polyhydroxy(C₁-C₆)alkyle, xvii) (C₁-C₆) alkylaminocarbonylamino (C₁-C₆) alkylamino, xviii) formylamino (C₁-C₆) alkylamino, xix) (hydroxy) (C₁-C₆) alkylamino (C₁-C₆) alkylamino, xix) hydroxysulfonyl (C₁-C₆) alkylamino (C₁-C₆) alkylamino, xx) sulfonato (C₁-C₆) alkyl (di (C₁-C₄) alkyl) ammonium (C₁-C₆) alkyl amino, xxi) hydroxysulfonyl (C₁-C₆) alcoxy (C₁-C₆) alkylamino, xxi) hétéroaryl (C₁-C₄)alkylcarbonylamino, ledit hétéroaryle pouvant être éventuellement cationique et/ou substitué en particulier par un ou plusieurs groupes (C₁-C₄) alkyle, xxii) hétérocycloalkyl(C₁-C₄)alkylcarbonylamino ledit hétérocycloalkyle pouvant être éventuellement cationique et/ou substitué en particulier par un ou plusieurs groupes (C₁-C₄) alkyle ou benzyle, xxiii) (di) (C₁-C₄) alkylamino (C₁-C₆) alkylamino, le groupe (C₁-C₆)alkyle pouvant être substitué par un ou plusieurs groupes hydroxyle, xxiv) hétérocycloalkylamino (C₁-C₆)alkylamino ou hétérocycloalkylamino, ledit hétérocycloalkyle pouvant être éventuellement cationique et/ou substitué en particulier par un ou plusieurs groupes (C₁-C₄)alkyle ou benzyle, xxv) hétéroarylalkylamino (C₁-C₆) alkylamino ou hétéroarylalkylamino, ledit hétéroaryle pouvant être éventuellement cationique et/ou substitué, en particulier par un ou plusieurs groupes (C₁-C₄) alkyle, xxvi) tri (C₁-C₆) alkylammonium(C₁-C₆) alkylamino, xxvii) (C₁-C₄) alcoxycarbonyl (C₁-C₆) alkyl (di (C₁-C₄) alkyl) ammonium (C₁-C₆) alkylamino, xxviii) carboxylato (C₁-C₆) alkyl (di (C₁-C₄) alkyl) ammonium (C₁-C₆) alk ylamino, xxix) carboxy (C₁-C₆) alkylamino (C₁-C₆) alkylamino, xxx) aryl (C₁-C₄) alkyl (di (C₁-C₄) alkyl) ammonium (C₁-C₆) alkylamino, xxxi) sulfonique SO₃H ou sulfonate SO₃⁻, M⁺ avec M⁺ représentant un contre-ion cationique, xxxii) (C₁-C₆) alkyle, xxxiii) hydroxysulfonyl (C₁-C₄)amino, xxxiv) phénylsulfonylamino.

5. Procédé selon l'une quelconque des revendications précédentes, dans lequel le ou les colorants anthraquinones de formules (I) et/ou (II) sont des colorants de formule (I) et R₂ et R₄**,** identiques ou différents, représentant un atome d'hydrogène ou un groupe tel que défini précédemment pour R₁ et R₃ i) à xxxii) dans la revendication précédente :
- hydroxyle,
- (di) (C₁-C₄) alkylamino,
- (C₁-C₄) alcoxy, hétérocycloalkyl (C₁-C₆) alkylamino ledit hétérocycloalkyle pouvant être éventuellement cationique et/ou substitué en particulier par un ou plusieurs groupes (C₁-C₄)alkyle ou benzyle,
- arylamino ou aryl(C₁-C₆)alkylamino avec le groupe aryle représentant un groupe aryle éventuellement substitué, en particulier un groupe phényle éventuellement substitué par un ou plusieurs groupes choisis parmi (C₁-C₄) alkyle, (di) (hydroxy) (C₁-C₄) alkylamino, (C₁-C₄) alcoxy et tri (C₁-C₄) alkylammonium,
- aryl (C₁-C₄) alkyl (di (C₁-C₄) alkyl) ammonium (C₁-C₆) alkylam ino,
- (di) (C₁-C₄) (alkyl) amino (C₁-C₆) alkylamino, et
- hydroxy (C₁-C₄) alkylamino (C₁-C₆) alkylamino.

6. Procédé selon l'une quelconque des revendications précédentes, dans lequel le ou les colorants anthraquinones de formules (I) et/ou (II) sont des colorants de formule (I) et R₂ et R₄ représentent un atome d'hydrogène.

7. Procédé selon l'une quelconque des revendications 1 à 3, dans lequel le ou les colorants anthraquinones de formules (I) et/ou (II) sont des colorants de formule (I) avec R₂ et R₃ représentant un atome d'hydrogène, et R₁ et R₄ sont de préférence aux positions 8 et 4 respectivement, et R₁ et R₄, sont tels que définis dans la revendication 1, et en particulier représentent un atome ou groupe tel que défini précédemment pour R₁ et R³ i) à xxxii) dans la revendication 4, de préférence R₁ et R₄ sont choisis parmi :
- halogène tel que chlore,
- (di) (C₁-C₄) (alkyl)amino,
- (C₁-C₄) alkylcarbonylamino,
- hétérocycloalkyl(C₁-C₆)alkylamino, hétérocycloalkyl(C₁-C₄)alkylcarbonylamino ou hétérocycloalkyl(C₁-C₄)alkylamino(C₁-C₄)alkylcarbonyla mino, ledit hétérocycloalkyle pouvant être éventuellement cationique et/ou substitué en particulier par un ou plusieurs groupes (C₁-C₄)alkyle ou benzyle,
- tri (C₁-C₄) alkylammonium(C₁-C₆) alkylamino,
- arylamino ou aryl (C₁-C₆) alkylamino avec le groupe aryle représentant un groupe aryle éventuellement substitué, en particulier un groupe phényle éventuellement substitué par un ou plusieurs groupes choisis parmi (C₁-C₄) alkyle, (di) (hydroxy) (C₁-C₄) alkylamino, (C₁-C₄) alcoxy et tri (C₁-C₄) alkylammonium,
- (di) (C₁-C₄) alkylamino (C₁-C₆) alkylamino, et
- (C₁-C₄) alkylcarbonylamino (C₁-C₆) alkylamino.

8. Procédé selon l'une quelconque des revendications 1 à 3, dans lequel le ou les colorants anthraquinones de formules (I) et/ou (II) sont des colorants de formule (I) avec R₃ et R₄ représentant un atome d'hydrogène, et R₁ et R₂**,** sont de préférence aux positions 8 et 1 respectivement, et R₁ et R₂**,** sont tels que définis dans la revendication 1, en particulier représentent un atome ou groupe tel que défini précédemment pour R₁ et R₃ i) à xxxii) dans la revendication 4, de préférence R₁ et R₂ sont choisis parmi :
- (di) (hydroxy) (C₁-C₄) alkylamino,
- (di) (hydroxy) (C₁-C₄) alkylamino (C₁-C₆) alkylamino,
- tri (C₁-C₆) alkylammonium (C₁-C₆) alkylammonium ;
- tri (C₁-C₆) alkylammonium (C₁-C₆) alkylamino
- hétérocycloalkyl(C₁-C₆)alkylamino, ledit heterocycloalkyle pouvant être éventuellement cationique et/ou substitué en particulier par un ou plusieurs groupes (C₁-C₄)alkyle ou benzyle et
- arylamino ou aryl (C₁-C₆) alkylamino avec le groupe aryle représentant un groupe aryle éventuellement substitué, en particulier un groupe phényle éventuellement substitué par un ou plusieurs groupes choisis parmi (C₁-C₄) alkyle, (di) (hydroxy) (C₁-C₄) alkylamino, (C₁-C₄) alcoxy, tri (C₁-C₄) alkylammonium.

9. Procédé selon l'une quelconque des revendications 1 à 3, dans lequel le ou les colorants anthraquinones de formules (I) et/ou (II) sont des colorants de formule (I) avec R₁ et R₃ représentant un atome d'hydrogène, et n est égal à 2, et R₂ et R₄ sont de préférence aux positions 1, 2 et 4 respectivement, et R₂ et R₄, sont tels que définis dans la revendication 1, et représentent en particulier un atome ou groupe tel que défini précédemment pour R₁ et R₃ i) à xxxii) dans la revendication 4, de préférence R₂ et R₄ sont choisis parmi :
- (C₁-C₆) alkyle,
- (di) (hydroxy) (C₁-C₄) alkylamino,
- SO₃H, ou SO₃⁻M⁺,
- (di) (hydroxy) (C₁-C₄) alkylamino (C₁-C₆) alkylamino,
- tri (C₁-C₄) alkylammonium(C₁-C₆) alkylamino, et
- hétérocycloalkyl(C₁-C₆)alkylamino, ledit hétérocycloalkyle pouvant être éventuellement cationique et/ou substitué en particulier par un ou plusieurs groupes (C₁-C₄)alkyle ou benzyle, et
- arylamino ou aryl (C₁-C₆) alkylamino avec le groupe aryle représentant un groupe aryle éventuellement substitué, en particulier un groupe phényle éventuellement substitué par un ou plusieurs groupes choisis parmi (C₁-C₄) alkyle, (di) (hydroxy) (C₁-C₄) alkylamino, (C₁-C₄) alcoxy, tri (C₁-C₄) alkylammonium.

10. Procédé selon l'une quelconque des revendications 1 à 3, dans lequel le ou les colorants anthraquinones de formules (I) et/ou (II) sont des colorants de formule (II') : 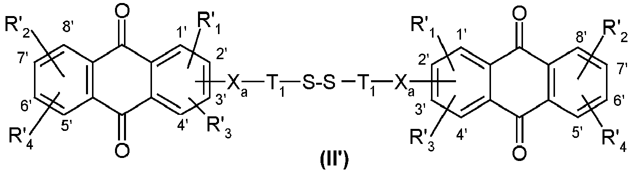 avec R'₁, R'₂, R'₃ et R'₄, T₁ et Xₐ tels que définis dans la revendication 1, Xₐ représente de préférence un N(Rₐ) et particulièrement NH, plus particulièrement R'₂ et R'₄ représentent un atome d'hydrogène et R'₁ et R'₃ sont en particulier tels que définis dans la revendication 4 pour R₁ et R₃ i) à xxxii), de préférence R'₃ et R'₄, qui peuvent être identiques ou différents, représentent un groupe choisi parmi (C₁-C₆) alkyle et (di) (hydroxy) (C₁-C₄) alkylamino ; de préférence T₁ représente une chaîne hydrocarbonée divalente, linéaire, saturée comprenant de 1 à 20 atomes de carbone, de préférence entre 2 et 10 atomes de carbone, éventuellement interrompue par un ou plusieurs groupes, choisis parmi N(R_{b}), C(O), -N⁺(R₈) (R₉) -An, hétéroaryle cationique tel qu'imidazolium, An ou leur combinaisons, avec R₈ et Rg, qui peuvent être identiques ou différents, représentant un radical (C₁-C₆)alkyle ; R_{b} représentant un atome d'hydrogène ou un groupe (C₁-C₄) alkyle, de préférence ladite chaîne hydrocarbonée est interrompue par un ou plusieurs groupes choisis parmi -N⁺(R₈) (R₉) -An, N(R_{b}), C(O), et leur combinanison tels que -C(O)- N(R_{b})-ou -N(R_{b})-C(O)-, N⁺(R₈) (R₉) -An ; plus préférentiellement T₁ représente un groupe divalent-(CH₂)ₙ-Tₐ- (CH₂)ₘ-T_{b}- (CH₂)ₚ- avec Tₐ et T_{b}, qui peuvent être identiques ou différents, représentant une liaison, ou un groupe choisi parmi N(R_{b}), C(O), -N⁺(R₈) (R₉) -An, hétéroaryle cationique tel qu'imidazolium, An ou leur combinaisons, avec R₈ et Rg, qui peuvent être identiques ou différents, représentant un radical (C₁-C₆)alkyle ; R_{b} représentant un atome d'hydrogène ou un groupe (C₁-C₄) alkyle, de préférence -N⁺(R₈) (Rg)-An, -C(O)-N(R_{b})-ou -N(R_{b})-C(O)-, n, m et p, qui peuvent être identiques ou différents, représentant un entier compris entre 1 et 10 inclus, avec la somme n+m+p comprise entre 1 et 20 inclus, de préférence entre 2 et 10 ; préférentiellement les groupes R'₁ et R'₃ sont aux positions 2' et 4', et R'₂ et R'₄ sont aux positions 5' et 8'.

11. Procédé selon la revendication précédente, dans lequel le ou les colorants anthraquinones de formules (I) et/ou (II) sont des colorants de formule (II") : 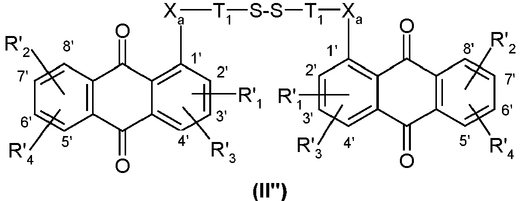 formule (II") dans laquelle R'₁, R'₂, R'₃ et R'₄, T₁ et Xₐ sont tels que définis dans la revendication précédente pour (II').

12. Procédé selon l'une quelconque des revendications précédentes dans lequel (a) le ou les colorants anthraquinones de formules (I) et/ou (II) sont choisis parmi les composés suivants, leurs isomères optiques, leurs isomères géométriques, leurs tautomères, leurs solvates et leurs mélanges : 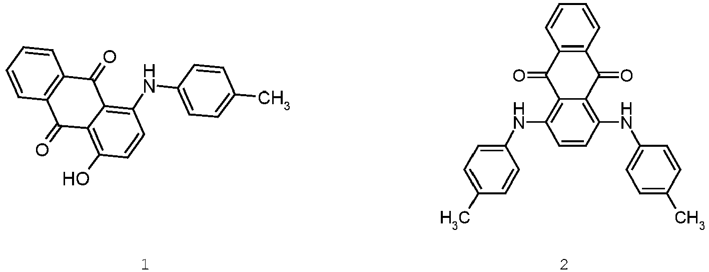 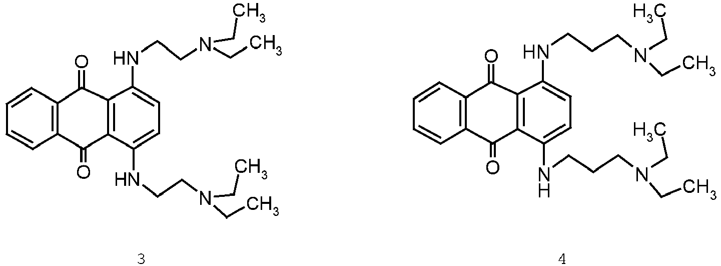 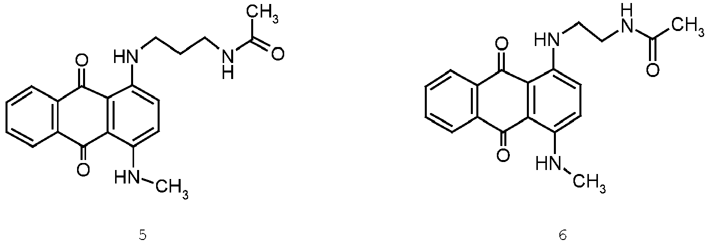 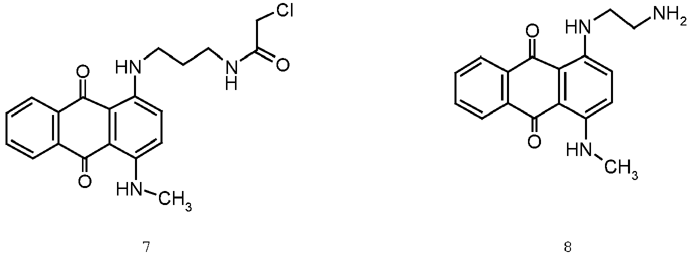 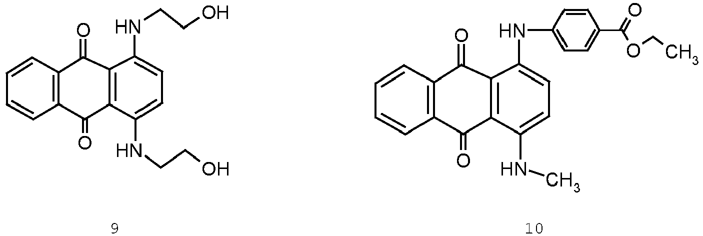 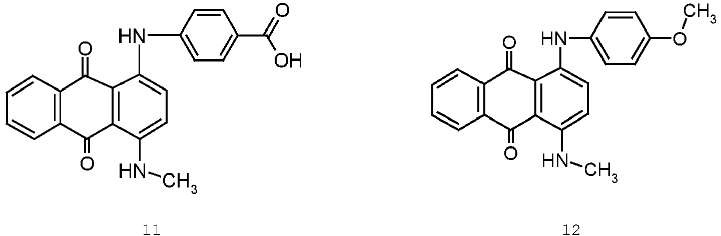 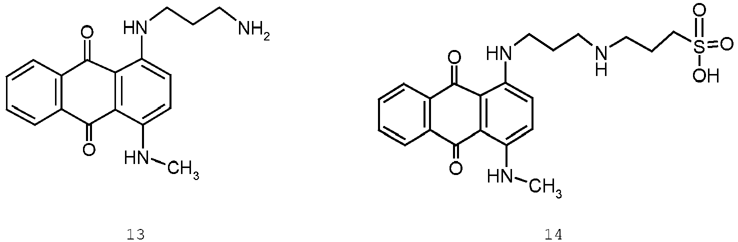 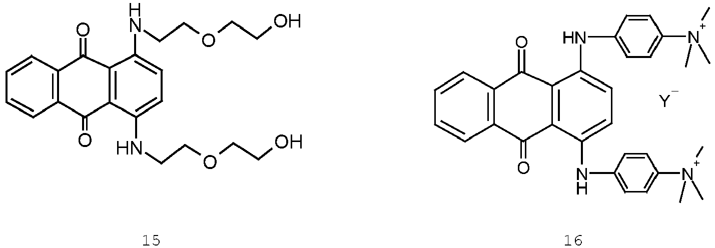 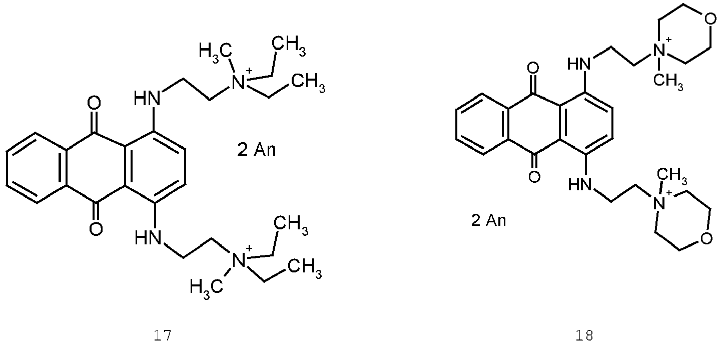 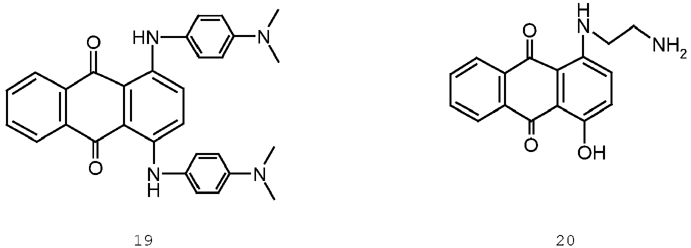 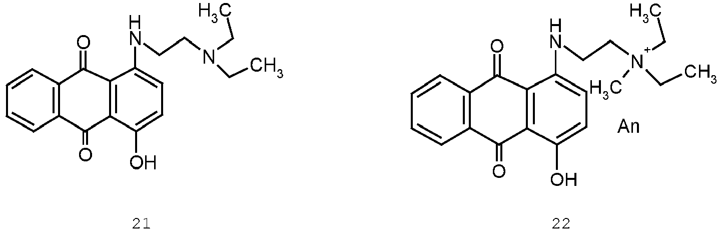 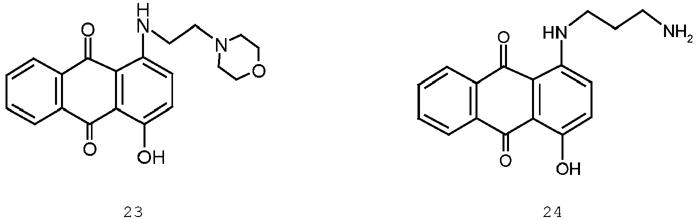 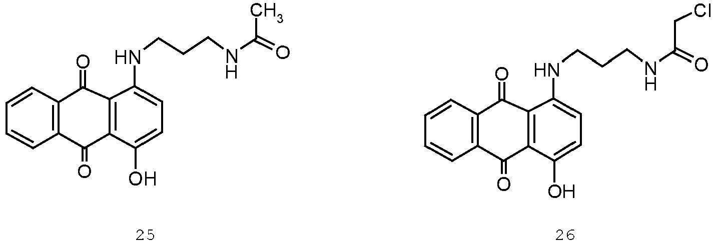 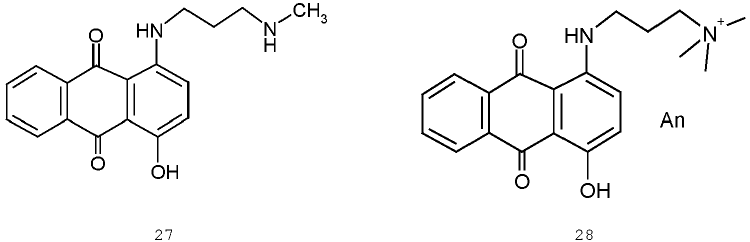 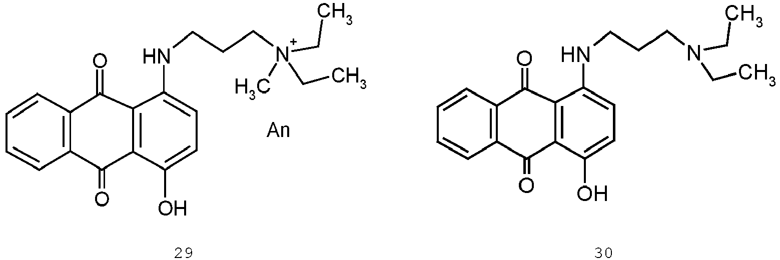 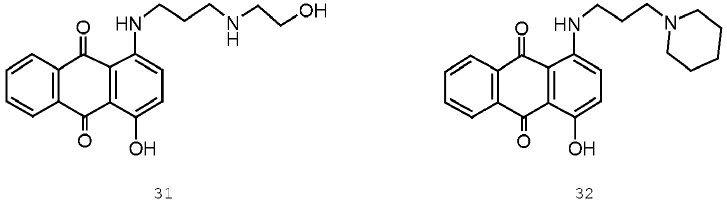 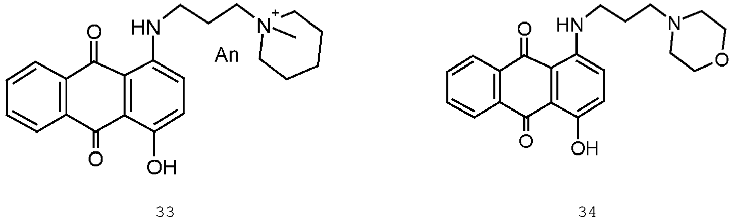 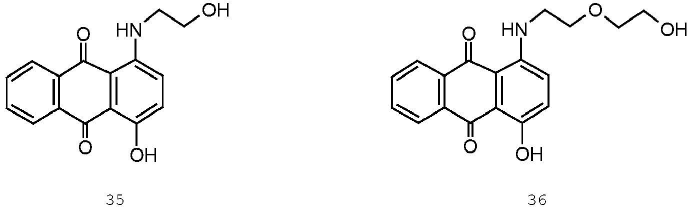 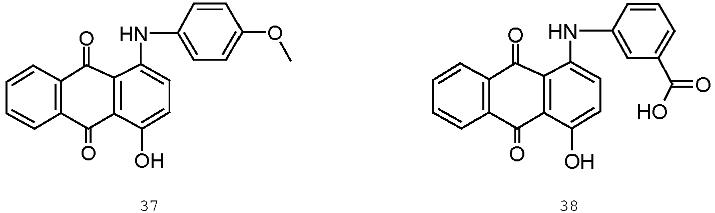 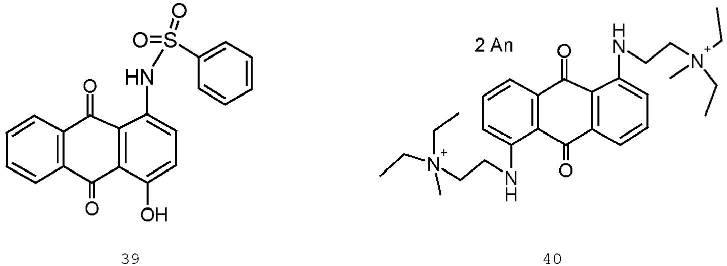 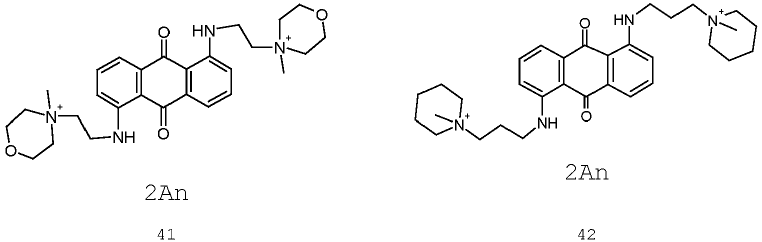 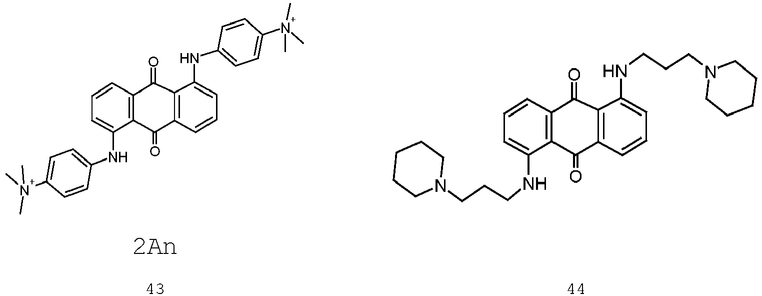 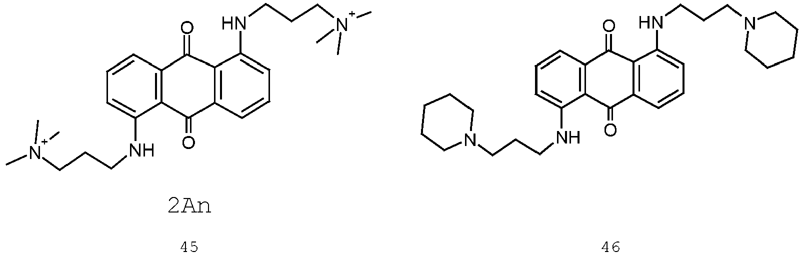 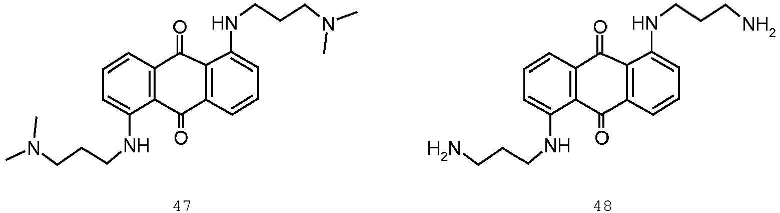 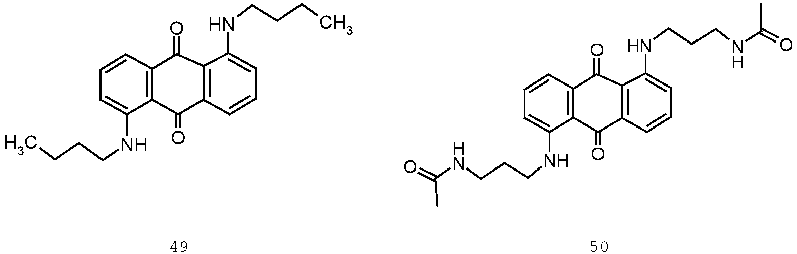 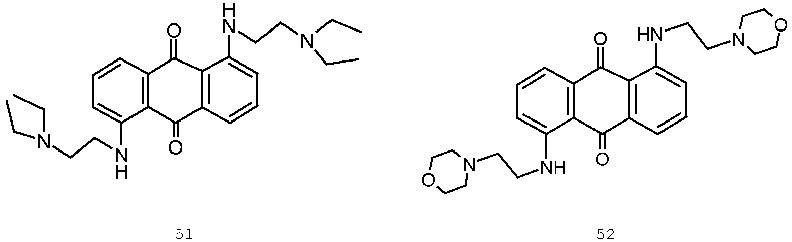 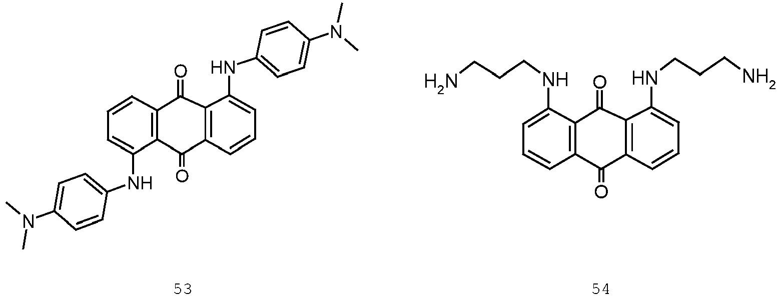 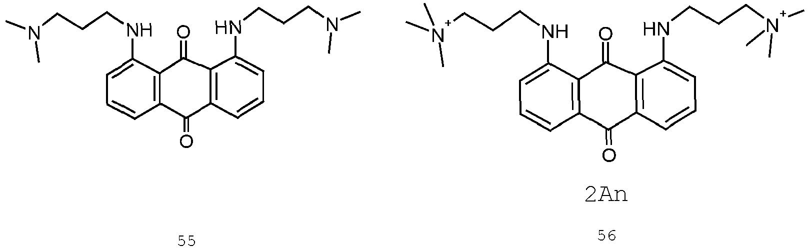 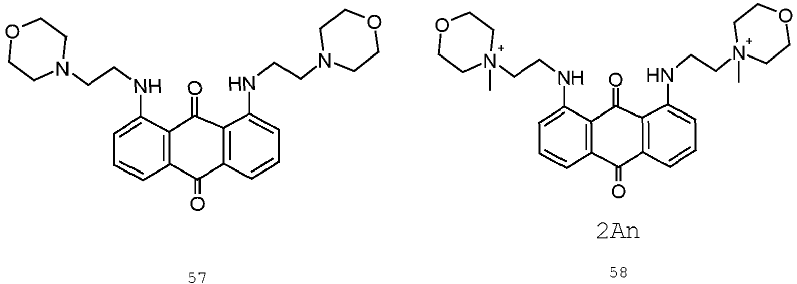 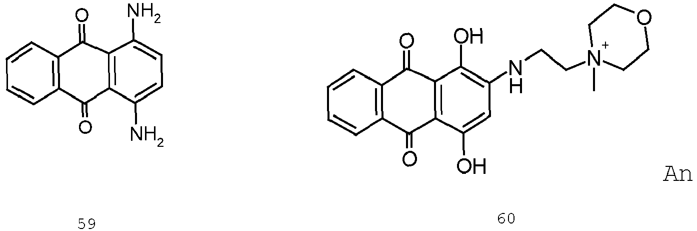 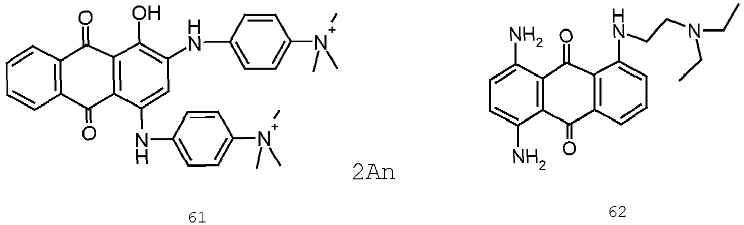 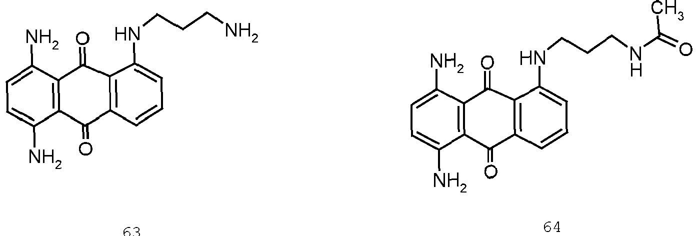 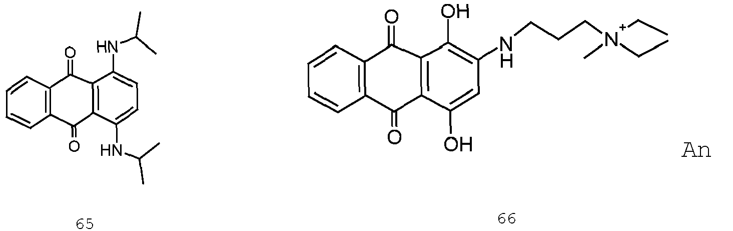 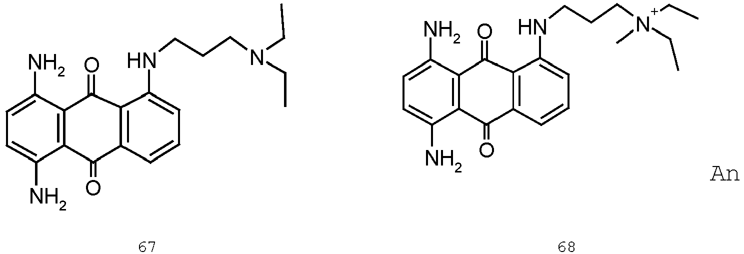 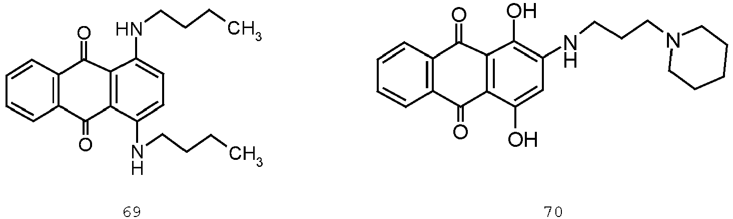 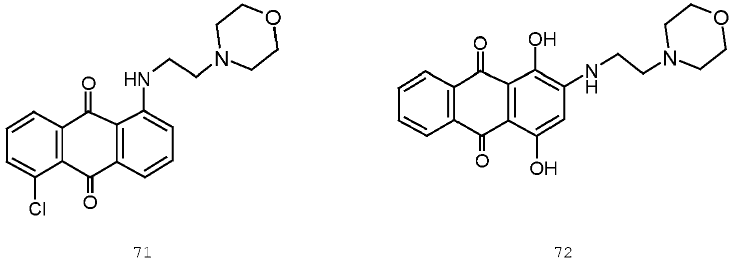 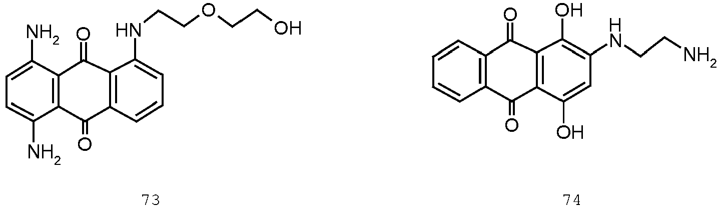 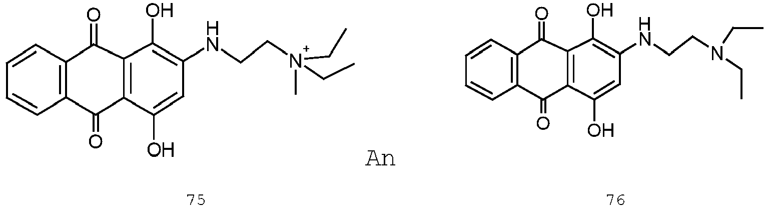 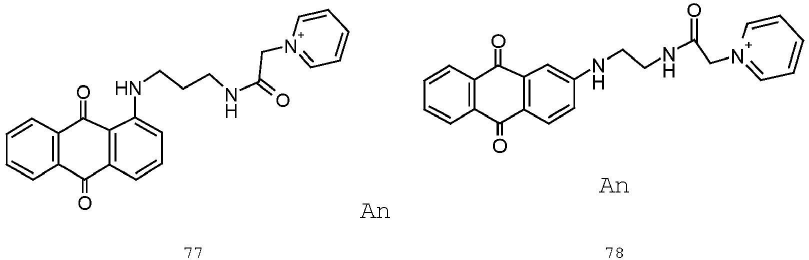 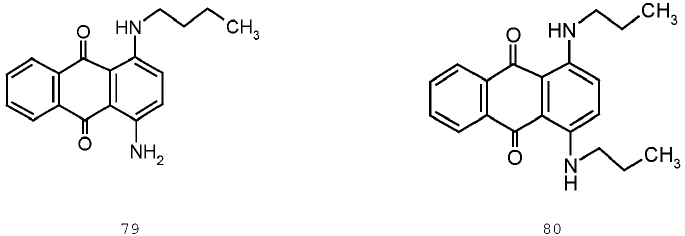 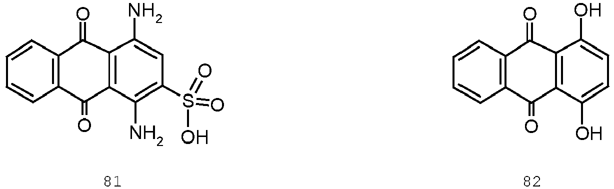 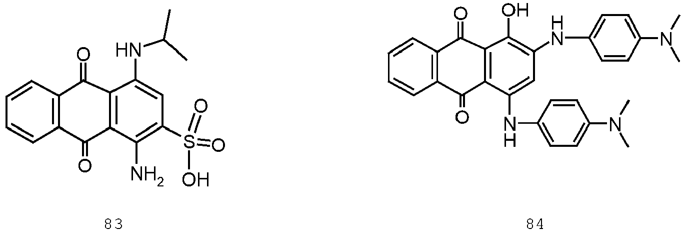 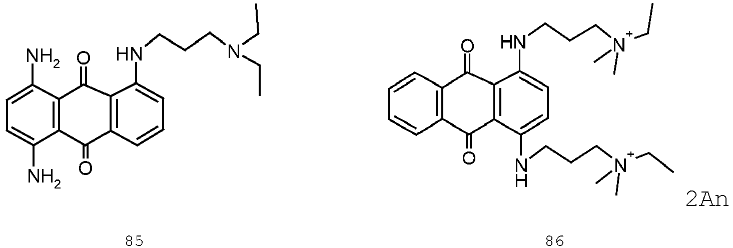 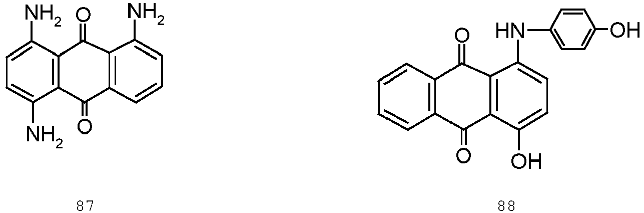 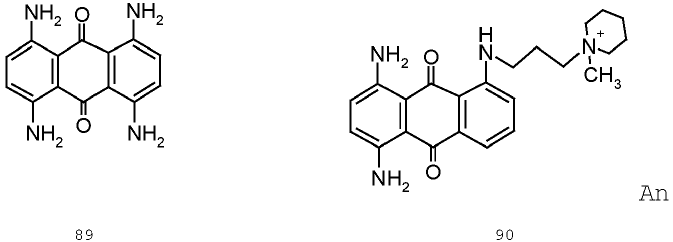 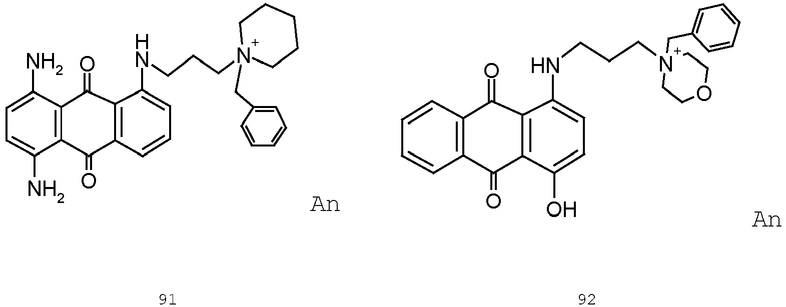 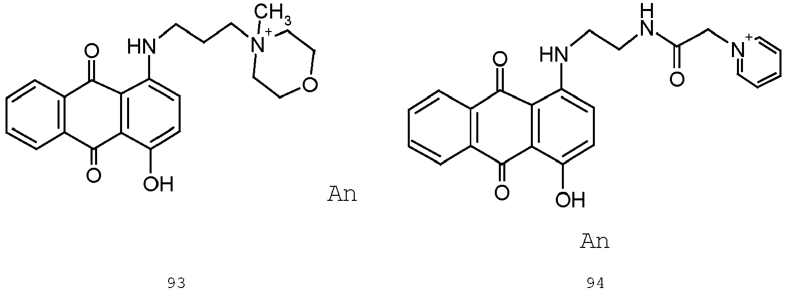 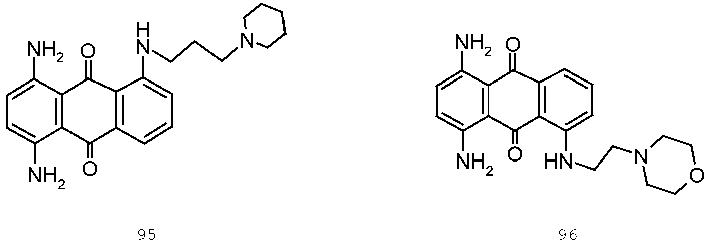 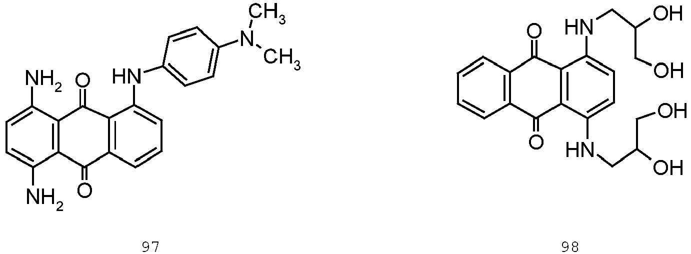 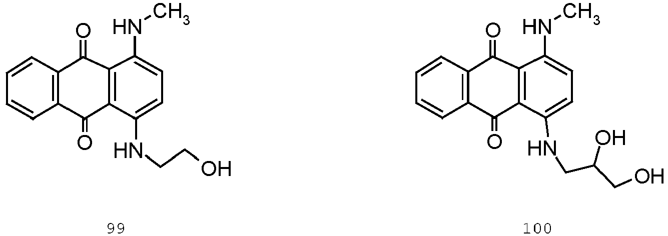 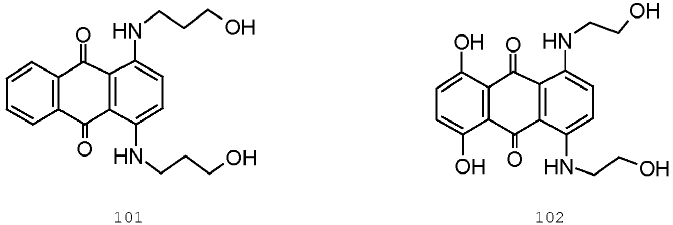 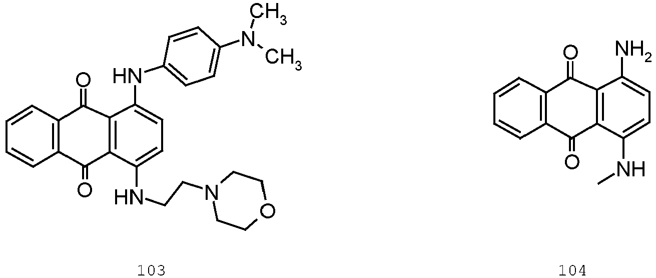 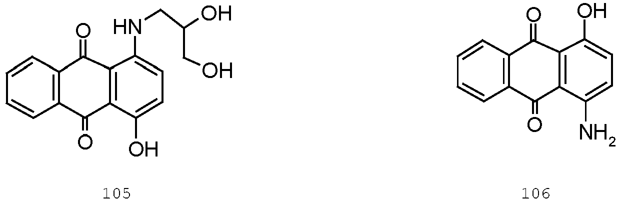 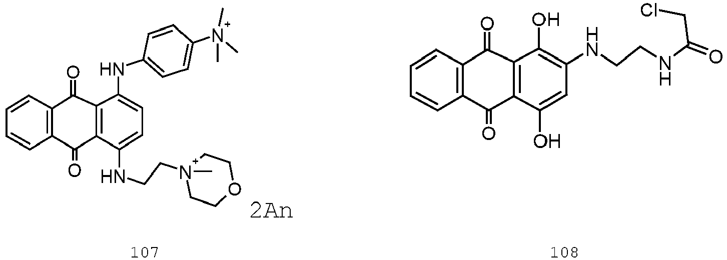 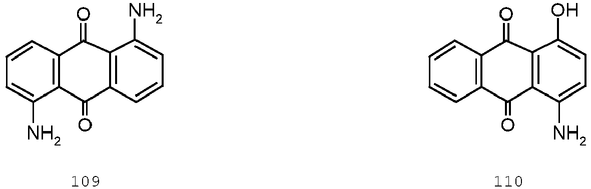 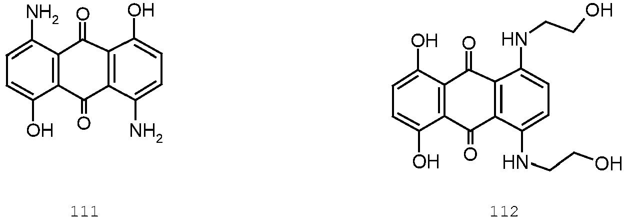 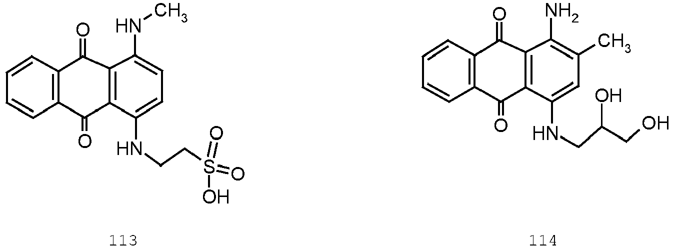 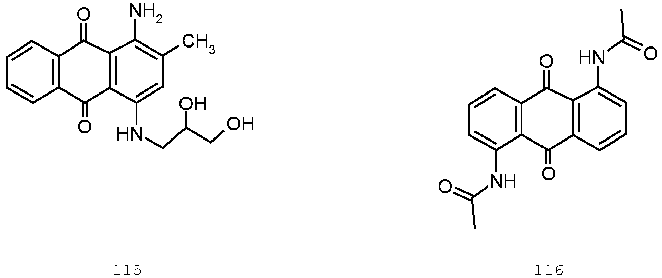 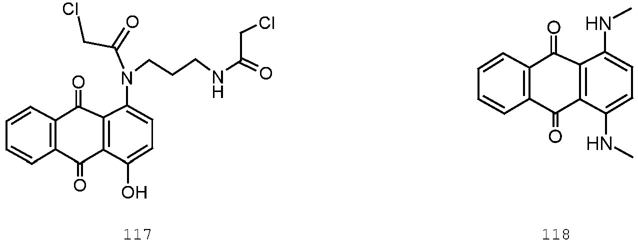 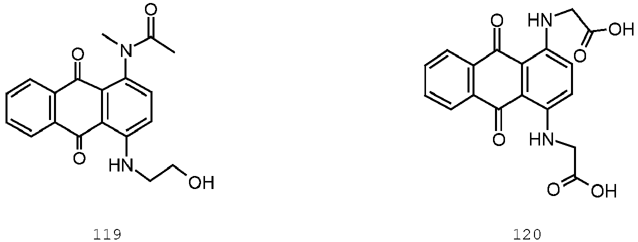 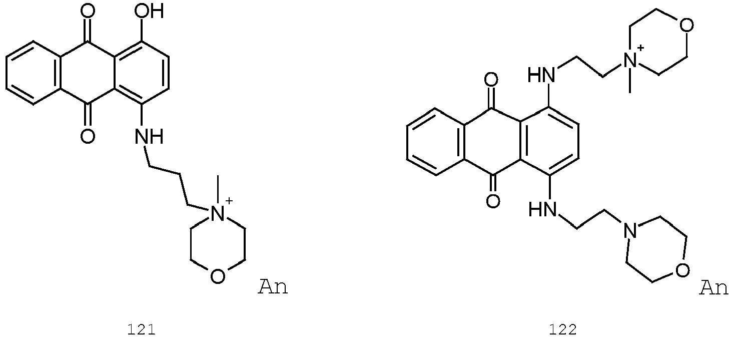 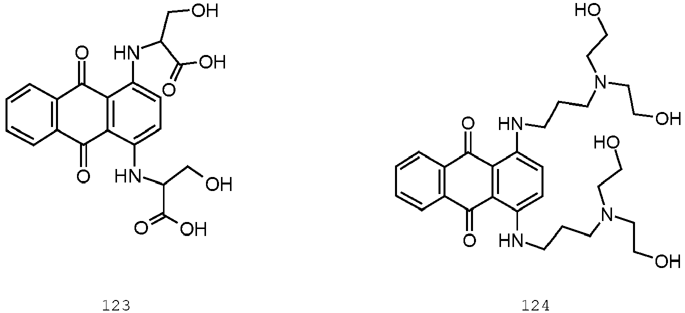 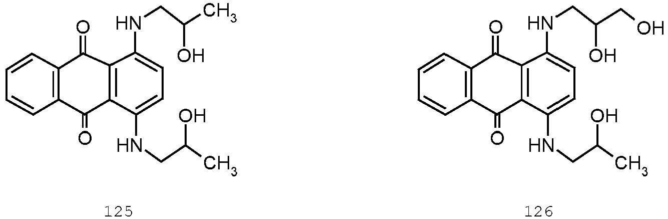 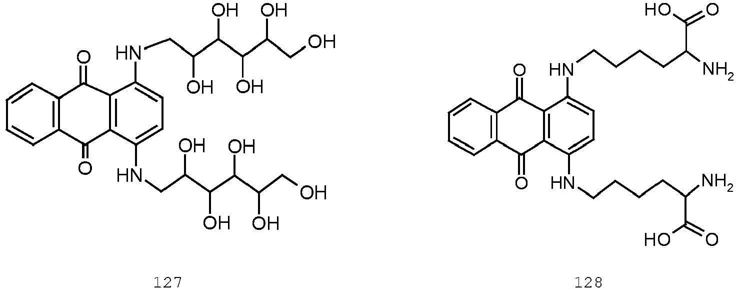 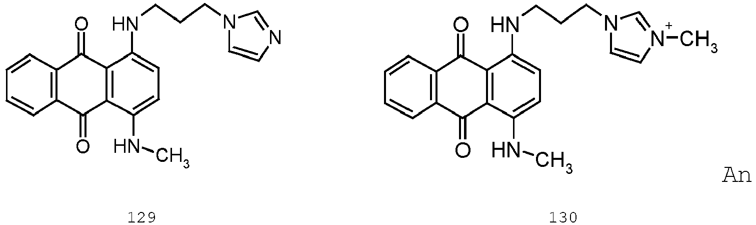 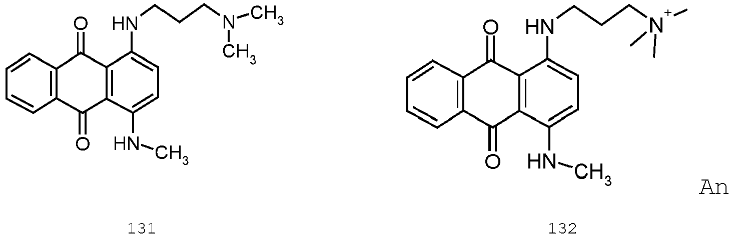 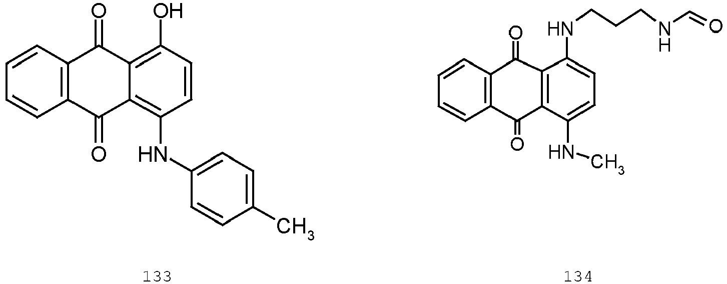 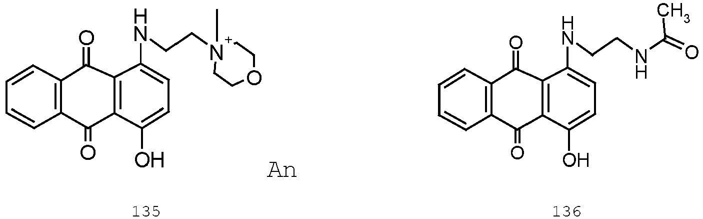 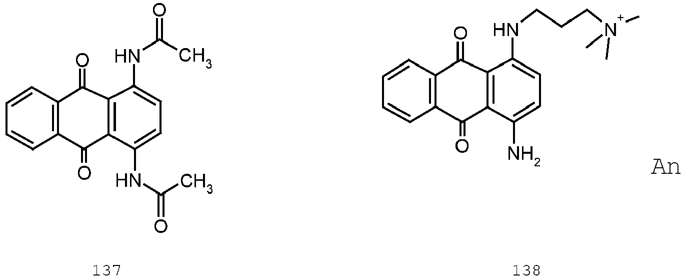 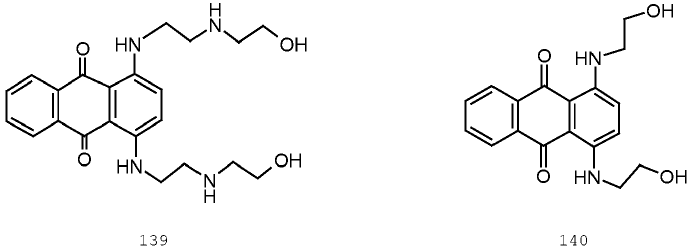 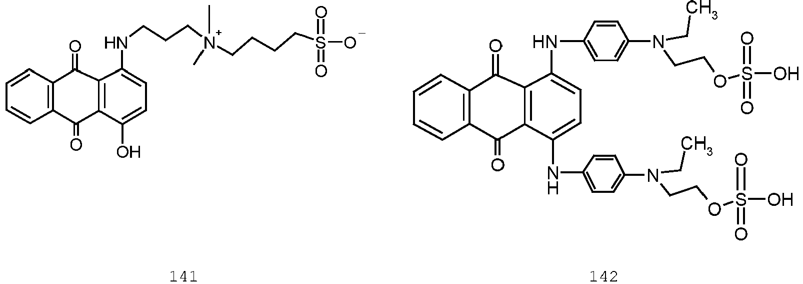 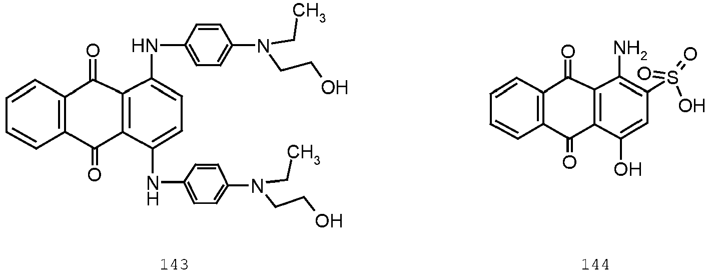 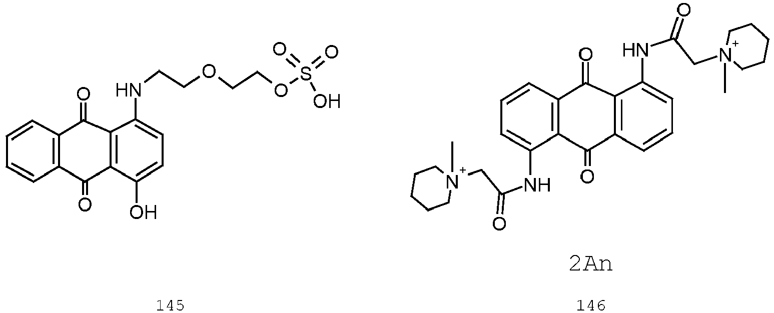 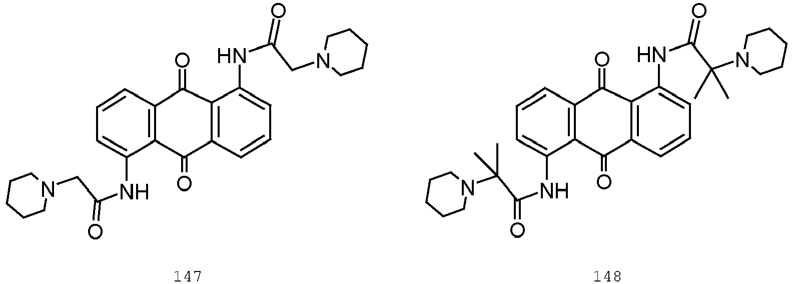 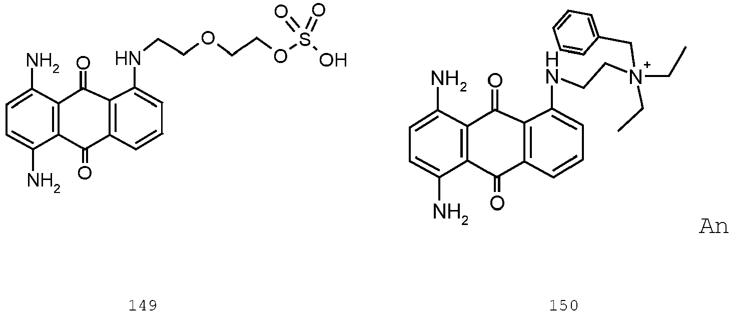 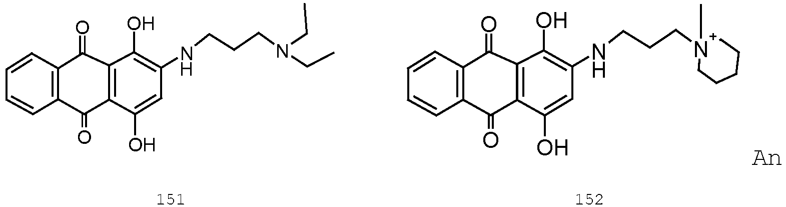 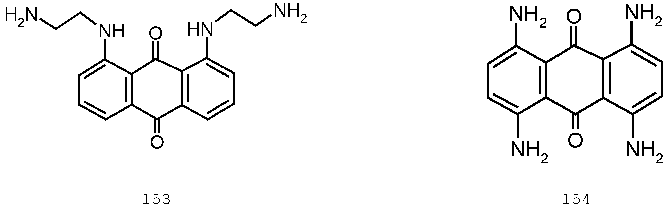 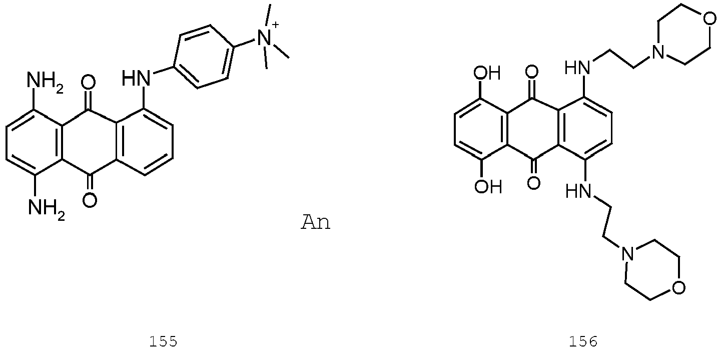 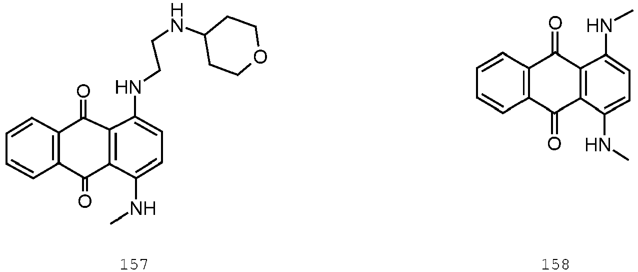 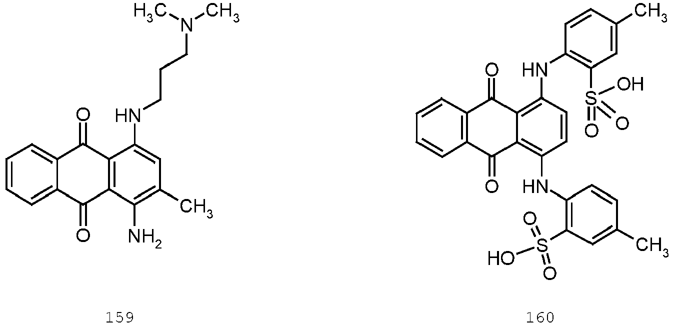 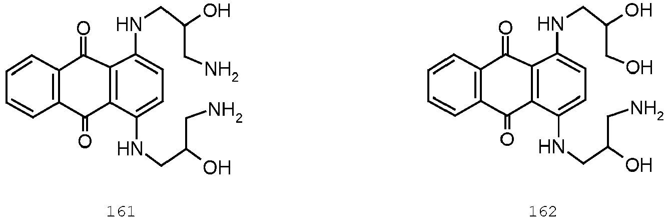 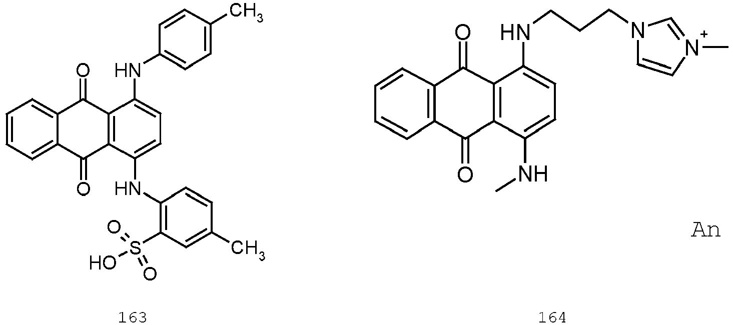 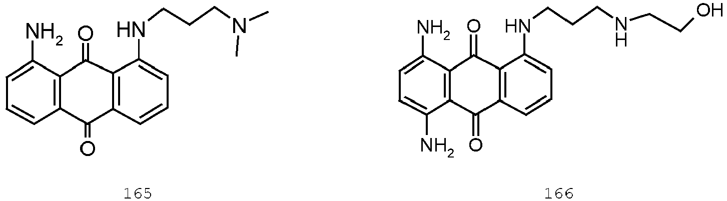 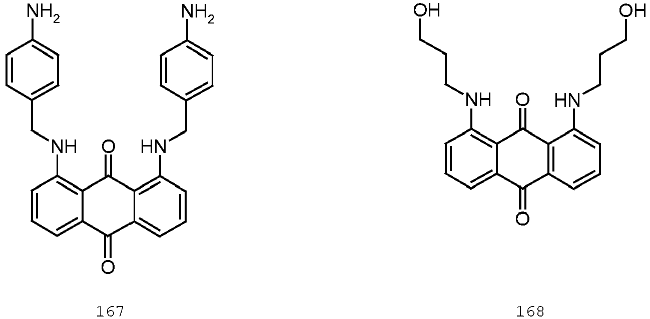 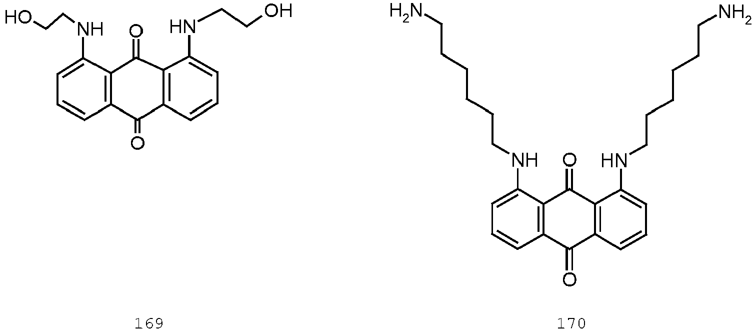 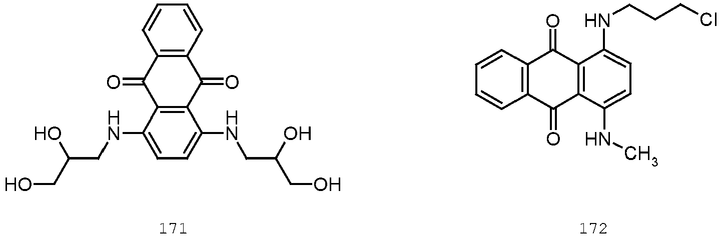 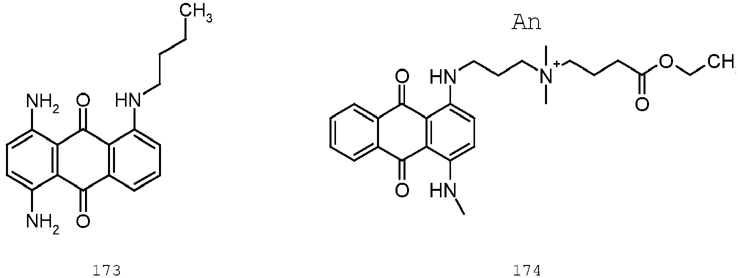 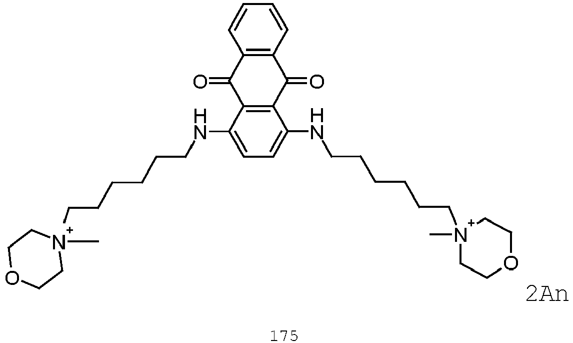 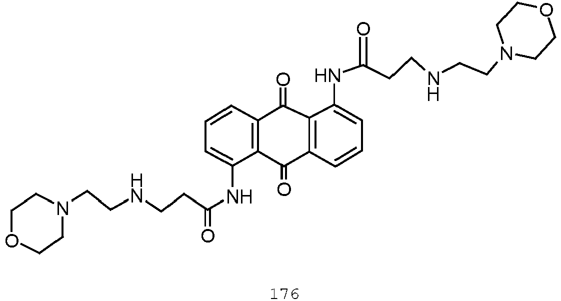 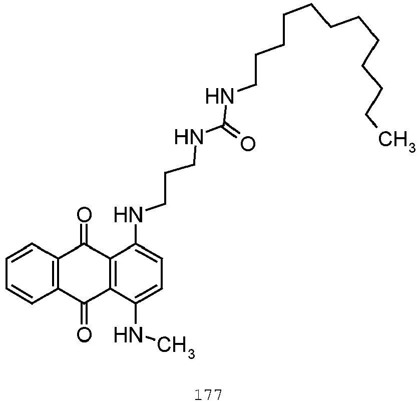 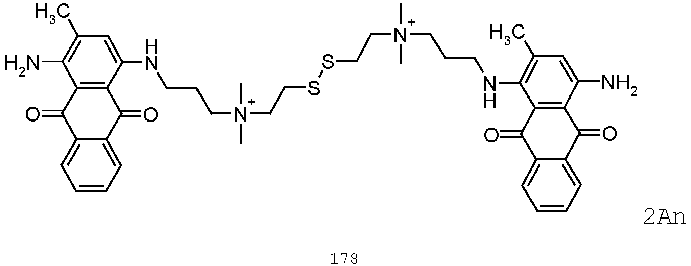 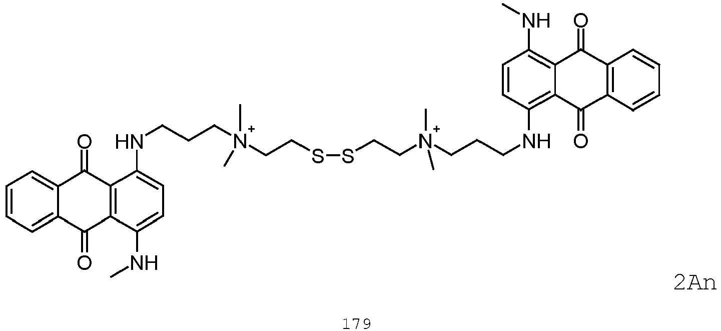 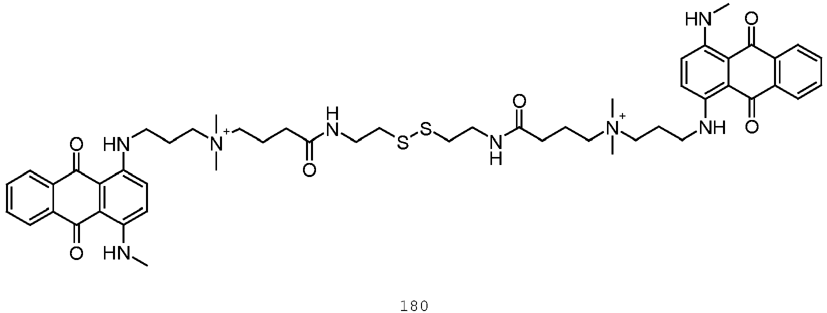 avec An, qui peuvent être identiques ou différents, représentant un contre-ion anionique tel que défini précédemment, de préférence choisi parmi mésylate, tosylate et halogénure tel que Cl⁻ et I⁻.

13. Procédé selon l'une quelconque des revendications précédentes, dans lequel le ou les colorants fluorescents (poly)méthines cationiques sont des colorants directs choisis parmi des colorants cyanines et des colorants styryles/hémicyanines, et des colorants naphtalimides, et leurs mélanges ; plus particulièrement, les colorants fluorescents cationiques sont directs et sont choisis parmi les colorants cationiques styryles ou hémicyanines.

14. Procédé selon l'une quelconque des revendications précédentes, dans lequel le ou les colorants fluorescents (poly)méthines cationiques sont des colorants qui absorbent la lumière dans la gamme jaune, orange et rouge, de préférence dans la plage de longueur d'onde d'absorption λ_{abs} entre 400 nm et 500 nm inclus.

15. Procédé selon l'une quelconque des revendications précédentes, dans lequel le ou les colorants fluorescents (poly)méthines cationiques (b) sont des colorants qui portent au moins un chromophore cationique choisi parmi les formules (III),(IV), (IIIa) et (IVa) ci-dessous :
W⁺-[C (R^{c}) =C(R^{d})]_{m'}-Ar'-(*) Q⁻ (III)
Ar-[C(R^{d}) =C (R^{c}) ]_{m'}-W'⁺- (*) Q⁻ (IV),
formules (III) et (IV) dans lesquelles :
• W⁺ représente un groupe hétéroaryle cationique, comprenant en particulier un ammonium quaternaire éventuellement substitué par un ou plusieurs groupes (C₁-C₈) alkyle éventuellement substitués en particulier avec un ou plusieurs groupes hydroxyle ;
• W'⁺ représente un radical hétéroaryle divalent tel que défini pour W⁺ ;
• Ar représente un groupe aryle tel que phényle ou naphtyle, éventuellement substitué de préférence par i) un ou plusieurs atomes d'halogène tels que le chlore ou le fluor ; ii) un ou plusieurs groupes (C₁-C₈) alkyle, de préférence des groupes (C₁-C₄) alkyle tels que méthyle ; iii) un ou plusieurs groupes hydroxyle ; iv) un ou plusieurs groupes (C₁-C₈)alcoxy tels que méthoxy ; v) un ou plusieurs groupes hydroxy(C₁-C₈)alkyle tels qu'hydroxyéthyle, vi) un ou plusieurs groupes amino ou (di)(C₁-C₈)alkylamino, de préférence avec la partie (C₁-C₄) alkyle éventuellement substituée par un ou plusieurs groupes hydroxyle, tels que (di)hydroxyéthylamino, vii) avec un ou plusieurs groupes acylamino ; viii) un ou plusieurs groupes hétérocycloalkyle, tels que pipérazinyle, pipéridyle ou hétéroaryle de 5 ou 6 chaînons tels que pyrrolidinyle, pyridyle et imidazolinyle ;
• Ar' est un radical aryle divalent tel que défini pour Ar ;
• m' représente un entier compris entre 1 et 4 inclus, en particulier m est 1 ou 2 ; de préférence 1 ;
• R^{c} et R^{d}**,** qui peuvent être identiques ou différents, représentent un atome d'hydrogène ou éventuellement un groupe (C₁-C₈) alkyle, de préférence un groupe (C₁-C₄) alkyle, ou en variante, R^{c} est contigu à W ou W' et/ou R^{d} est contigu à Ar ou Ar' et forment, avec les atomes qui les portent, un (hétéro)cycloalkyle ; en particulier, Rc est contigu à W⁺ ou W'⁺ et forme un (hétéro)cycloalkyle tel que cyclohexyle ;
• Q⁻ est un contre-ion anionique organique ou minéral ;
• (*) représente la partie du chromophore fluorescent qui est liée au reste du colorant ;
de préférence, W⁺ ou W'⁺ est un radical imidazolium, pyridinium, benzimidazolium, pyrazolium, benzothiazolium ou quinoléinium éventuellement substitué avec un ou plusieurs radicaux (C₁-C₄) alkyle identiques ou différents ; de façon particulièrement préférée, le ou les chromophores fluorescents sont choisis parmi ceux avec m' = 1, Ar représentant un groupe phényle substitué en para par rapport au groupe styryle -C(R^{d}) =C (R^{c}) - par un groupe (di) (hydroxy) (C₁-C₆) alkylamino tel que dihydroxy(C₁-C₄)alkylamino, et W'⁺ représentant un groupe imidazolium ou pyridinium, de préférence ortho- ou para-pyridinium ; 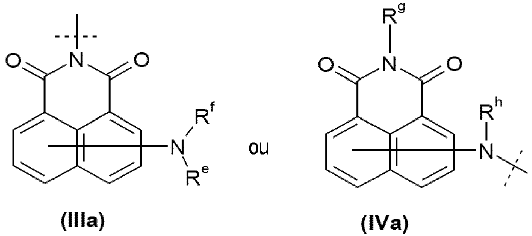 où 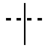 représente la liaison avec le colorant formules (IIIa) et (IVa) dans lesquelles R^{e}, R^{f}, R^{g} et R^{h}, qui peuvent être identiques ou différents, représentent un atome d'hydrogène ou un groupe (C₁-C₆)alkyle qui est éventuellement substitué, de préférence avec un groupe di (C₁-C₆) alkylamino ou tri (C₁-C₆) alkylammonium tel que triméthylammonium.

16. Procédé selon l'une quelconque des revendications précédentes dans lequel le ou les colorants fluorescents (poly)méthines cationiques sont choisis parmi les colorants de formules (V), (VI) et (VII) : 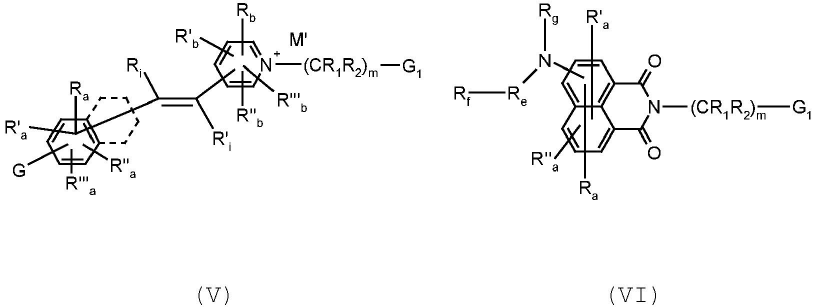 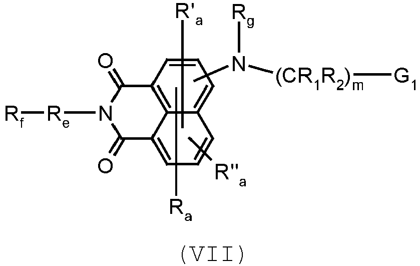 ainsi que leurs sels d'acide ou de base, organique ou minéral, leurs isomères optiques, isomères géométriques et tautomères et leurs solvates tels que des hydrates ; formules (V), (VI) et (VII) dans lesquelles :
• R₁ et R₂, qui peuvent être identiques ou différents, représentent un atome d'hydrogène ou un groupe (C₁-C₆) alkyle ; préférentiellement un atome d'hydrogène ;
• G₁ représente un atome d'hydrogène ou un groupe choisi parmi NH₂ ou OH ; de préférence hydrogène ;
• Rₐ, R'ₐ, R"ₐ, R'''ₐ, Rb, R'_{b}, R"_{b} et R'''_{b}, qui peuvent être identiques ou différents, représentent a) un atome d'hydrogène, b) un atome d'halogène, un groupe parmi : c) amino, d) (C₁-C₄)alkylamino, e) (C₁-C₄) dialkylamino, f) cyano, g) carboxyle -C(O)OH ou carboxylate -C(O)O⁻, Q⁺, h) hydroxy -OH ou alcoolate -O⁻Q⁺, i) (poly)halogéno (C₁-C₆) alkyle tel que trifluorométhyle, j) acylamino, k) (C₁-C₆)alcoxy, 1) (C₁-C₆) alkylthio, m) (poly) hydroxy (C₂-C₄) alcoxy, n) (C₁-C₆) alkylcarbonyloxy, o) (C₁-C₆) alcoxycarbonyle, p) (C₁-C₆) alkylcarbonylamino, q) acylamino, r) carbamoyle , s) (C₁-C₆) alkylsulfonylamino, t) aminosulfonyle, u) -SO₃H ou sulfonate -SO₃⁻ , Q⁺ ou v) (C₁-C₆) alkyle éventuellement substitué par un groupe choisi parmi (C₁-C₆) alcoxy, hydroxyle, cyano, carboxyle, amino, (di) (C₁-C₄) alkylamino, ou, en variante, les deux radicaux alkyles portés par l'atome d'azote du groupe amino forment un hétérocycle de 5 à 7 chaînons comprenant éventuellement un autre hétéroatome d'azote ou non-azote ; en particulier Rₐ, R'ₐ, R"ₐ, R'''ₐ, Rb, R'_{b}, R"_{b} et R"'_{b}, représentent un atome d'hydrogène ou d'halogène ou un groupe (C₁-C₄)alkyle, de préférence un atome d'hydrogène ;
• ou, en variante, deux groupes Rₐ et R'ₐ ; R_{b}, et R'_{b} ; portés par deux atomes de carbone adjacents, forment conjointement un cycle benzo ou indéno, un groupe hétérocycloalkyle fusionné ou hétéroaryle fusionné ; le cycle benzo, indéno, hétérocycloalkyle ou hétéroaryle étant éventuellement substitué par un atome d'halogène, un groupe amino, (C₁-C₄) alkylamino, (C₁-C₄) dialkylamino, nitro, cyano, carboxyle, hydroxyle ou trifluorométhyle, un radical acylamino, (C₁-C₄) alcoxy (poly) hydroxy (C₁-C₄) alcoxy, (C₁-C₄) alkylcarbonyloxy (C₁-C₄) alcoxycarbonyle ou (C₁-C₄) alkylcarbonylamino, un radical acylamino, carbamoyle ou alcoxyalkylsulfonylamino, un radical aminosulfonyle, ou un radical (C₁-C₆) alkyle éventuellement substitué par : un groupe choisi parmi (C₁-C₆) alcoxy, hydroxyle, cyano, carboxyle, amino, (C₁-C₄) alkylamino et (C₁-C₄) dialkylamino, ou, en variante, les deux radicaux alkyles portés par l'atome d'azote du groupe amino forment un hétérocycle de 5 à 7 chaînons comprenant éventuellement un autre hétéroatome d'azote ou non-azote ; préférentiellement, Rₐ et R'ₐ forment conjointement un groupe benzo ;
• ou, en variante, deux groupes Rᵢ et Rₐ ; et/ou un groupe R'ᵢ et R'ₐ forment conjointement un (hétéro)cycloalkyle fusionné, préférentiellement cycloalkyle tel que cyclohexyle ;
• R_{g} représente un atome d'hydrogène, un groupe (hétéro) aryl (C₁-C₄) alkyle ou un groupe (C₁-C₆) alkyle qui est éventuellement substitué ; préférentiellement, R_{b} représente un atome d'hydrogène ou un groupe (C₁-C₃) alkyle ou benzyle ;
• Rₑ représente une liaison covalente, une chaîne hydrocarbonée (C₁-C₈) alkylène ou (C₂-C₈) alcénylène, linéaire ou ramifiée, éventuellement substituée, de préférence Rₑ représente un (C₁-C₆) alkylène non substitué ;
• R_{f} représente un atome d'hydrogène, un groupe (C₁-C₄) alcoxy, un groupe amino R₃R₄N-, ammonium quaternaire M', R₃R₄R₅N⁺- dans lequel R₃, R₄ et R₅, qui peuvent être identiques ou différents, représentent un atome d'hydrogène ou un groupe (C₁-C₄) alkyle ou R₃R₄N- représente un groupe hétéroaryle éventuellement substitué, préférentiellement un groupe imidazolyle éventuellement substitué ou, en variante, M', R₃R₄R₅N⁺- représente un groupe hétéroaryle cationique éventuellement substitué, préférentiellement un groupe imidazolinium éventuellement substitué par un groupe (C₁-C₃) alkyle ;
• G représente i) un groupe -NR_{c}R_{d}, ii) -OR avec R représentant a) un atome d'hydrogène, b) un groupe (C₁-C₆) alkyle éventuellement substitué, préférentiellement non substitué c) un groupe (hétéro)aryle éventuellement substitué, d) un groupe (hétéro)aryl(C₁-C₆) alkyle éventuellement substitué tel que benzyle, e) un groupe (hétéro)cycloalkyle éventuellement substitué, f) un groupe (hétéro) cycloalkyl (C₁-C₆) alkyle éventuellement substitué ; selon un mode de réalsation particulier, G représente un groupe -NR_{c}R_{d}, selon un autre mode de réalisation particulier, G représente un groupe (C₁-C₆) alcoxy ;
ou, en variante, lorsque G représente -NR_{c}R_{d}, deux groupes R_{c} et R'ₐ et/ou R_{d} et Rₐ forment conjointement un hétéroaryle ou hétérocycle saturé, éventuellement substitué par un ou plusieurs groupes (C₁-C₆) alkyle, préférentiellement un hétérocycle de 5 à 7 chaînons contenant un ou deux hétéroatomes choisis parmi l'azote et l'oxygène ; plus préférerntiellement et comprenant entre 5 et 7 chaînons ; plus préférentiellement, l'hétérocycle est choisi parmi les groupes morpholinyle, pypérazinyle, pypéridinyle et pyrrolidinyle ;
• R_{c} et R_{d}, qui peuvent être identiques ou différents, représentent un atome d'hydrogène ou un groupe parmi : a) (hétéro)aryle éventuellement substitué tel que phényle, b) (hétéro) aryl (C₁-C₄) alkyle éventuellement substitué, c) (hétéro)cycloalkyle éventuellement substitué, d) (hétéro)cycloalkyl(C₁-C₄) alkyle éventuellement substitué, f) (C₂-C₅) alkyle ou g) (C₁-C₈) alkyle qui est éventuellement substitué, de préférence éventuellement substitué par un groupe hydroxyle, carboxyle, carboxylate, sulfate ou sulfonate ;
ou, en variante, deux radicaux R_{c} et R_{d} adjacents, portés par le même atome d'azote forment ensemble un groupe hétérocyclique éventuellement substitué ou hétéroaryle éventuellement substitué ;
• Rᵢ, et R'ᵢ, identiques ou différents, représentent un atome d'hydrogène ou un groupe (C₁-C₄)alkyle ;
• 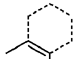 représente un groupe (hétéro)aryle fusionné avec le cycle phényle ; ou, en variante, est absent du phényle ; préférentiellement lorsque le cycle est présent le cycle est un benzo ;
• m représente un entier compris entre 1 et 18 inclus, en particulier un entier compris entre 1 et 14 inclus ; préférentiellement, un entier compris entre 2 et 10 inclus ; plus préférentiellement un entier compris entre 3 et 8 ; plus particulièrement un entier compris entre 4 et 6 ;
• M' représente un contre-ion anionique, dérivé d'un sel d'un acide organique ou minéral ou d'une base organique ou minérale qui assure l'électroneutralité de la molécule ;
• Q⁺ représente un contre-ion cationique, dérivé d'un sel d'un acide organique ou minéral, ou d'une base organique ou minérale qui assure l'électroneutralité de la molécule telle qu'un métal alcalin, alcalino-terreux ou ammonium ;
étant entendu que, lorsque la molécule comprend un groupe carboxylate, sulfonate alcoolate ou alcoolate alors M' et Q⁺ peuvent être absents pour assurer l'électroneutralité de ladite molécule.

17. Procédé selon l'une quelconque des revendications précédentes dans lequel le ou les colorants fluorescents (poly)méthines cationiques (b) sont choisis parmi les colorants styryles de formule (VIII) suivants : 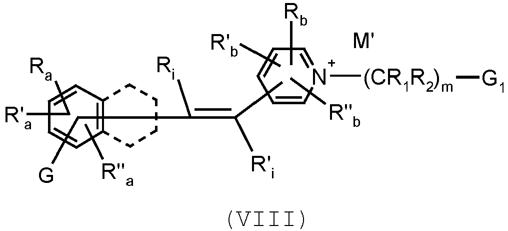 ainsi que leurs sels d'acide ou de base, organique ou minéral, leurs isomères optiques, siomères géométriques et tautomères et leurs solvates tels que des hydrates, formule (VIII) dans laquelle G, G₁, Rₐ, R'ₐ, R"ₐ, R_{b}, R'_{b}, R"_{b}, Rᵢ, R'ᵢ, R₁, R₂ et m sont tels que définis dans la revendication précédente ;
en particulier :
• R₁ et R₂, qui peuvent être identiques ou différents, représentent un atome d'hydrogène ;
• Rᵢ et Rᵢ', qui peuvent être identiques ou différents, représentent un atome d'hydrogène ou un groupe (C₁-C₄) alkyle, de préférence hydrogène ;
• Rₐ, R'ₐ et R"ₐ, identiques ou différents, représentent un atome d'hydrogène, un atome d'halogène tel que fluor, ou un groupe -OH, -O⁻Q⁺, (C₁-C₆)alcoxy, nitro ou cyano, avec Q⁺ tel que défini dans la revendication précédente ;
• R_{b}, R'_{b}, et R"_{b}, qui peuvent être identiques ou différents, représentent un atome d'hydrogène ou un groupe (C₁-C₆)alkyle ;
• ou, en variante, deux radicaux contigus R_{b} et R'_{b} forment, conjointement avec les atomes de carbone qui les portent, un groupe benzo qui est condensé ou fusionné avec le groupe pyridinium, ledit groupe benzo pouvant être substitué, de préférence, ledit groupe benzo n'est pas substitué ;
• G représente un groupe -NR_{c}R_{d}, ou (C₁-C₆)alcoxy, qui est éventuellement substitué, préférentiellement non substitué ; selon un mode de réalsation particulier, G représente un groupe -NR_{c}R_{d}, selon un autre mode de réalisation particulier, G représente un groupe (C₁-C₆)alcoxy ;
• G₁ représente un atome d'hydrogène, ou un groupe OH ou NH₂, de préférence hydrogène ;
• Rᵢ, et R'ᵢ, qui peuvent être identiques ou différents, représentent un atome d'hydrogène, ou un groupe (C₁-C₄) alkyle ;
• 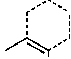 représente un groupe aryle ou hétéroaryle fusionné au cycle phényle ; ou, en variante, est absent du cycle phényle ; préférentiellement, lorsque le cycle est présent, le cycle est un benzo ;
• m, représente un entier compris entre 1 et 18 inclus ; en particulier un entier compris entre 2 et 16 inclus ; préférentiellement, un entier compris entre 3 et 10 ; plus préférentiellement, un entier compris entre 4 et 6 ;
• R_{c} et R_{d}, qui peuvent être identiques ou différents, représentent un atome d'hydrogène, un groupe (C₂-C₄) alkyle, ou un groupe (C₁-C₈) alkyle substitué, de préférence (C₂-C₄) alkyle substitué en particulier par un ou plusieurs groupes choisis parmi i) cyano, ii) (C₁-C₃) alcoxy, iii) hydroxyle et iv) (C₁-C₃)alkylcarbonyle, de préférence par un ou plusieurs groupes hydroxyle ; et
• M' représente un contre-ion anionique, dérivé d'un sel d'un acide organique ou minéral, ou d'une base organique ou minérale qui assure l'électroneutralité de la molécule ;
étant entendu que, lorsque la molécule comprend un groupe alcoolate alors M' et Q⁺ peuvent être absents en assurant l'électroneutralité de ladite molécule.

18. Procédé selon l'une quelconque des revendications précédentes, dans lequel le ou les colorants fluorescents (poly)méthines cationiques (b) sont choisis parmi les colorants des composés de formules (X), (XI), (XII) et (XIII) ci-dessous : 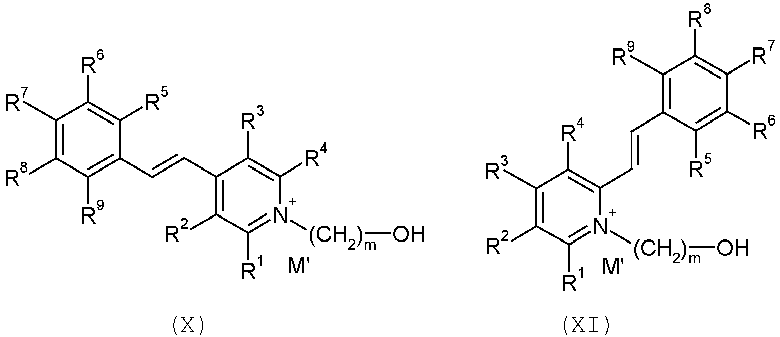 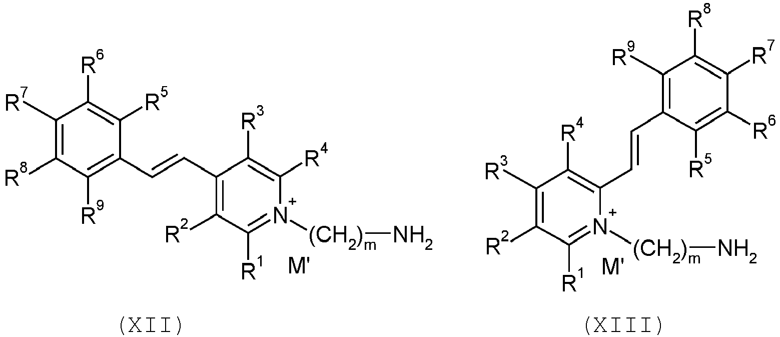 ainsi que leurs sels d'acide organique ou minéral, leurs isomères optiques, isomères géométriques et tautomères et leurs solvates tels que des hydrates,
formules (X),(XI), (XII) ou (XIII) dans lesquelles :
• R₁, R₂, R₃ et R₄, qui peuvent être identiques ou différents, représentent un atome d'hydrogène ou un groupe (C₁-C₆) alkyle ; de préférence R₂ et R₃ représentent un atome d'hydrogène et R₁ et R₄, qui peuvent être identiques ou différents, représentent un atome d'hydrogène ou un groupe (C₁-C₄) alkyle ;
• R₅, R₆, R₇, R₈ et R₉, identiques ou différents, représentent i) un atome d'hydrogène, ou ii) un atome d'halogène tel que Cl, Br ou F, iii) un groupe OR dans lequel R représente un atome d'hydrogène ou Q⁺ tel que décrit précédemment, ou un groupe (C₁-C₃) alkyle, iv) aryle tel que benzène, v) un groupe aryl(C₁-C₃) alkyle tel que benzyle, vi) cyano, vii) nitro, viii) (C₁-C₃) alkylthio, ix) amino NR¹⁰R¹¹ avec R¹⁰ et R¹¹, qui peuvent être identiques ou différents, représentant a) un atome d'hydrogène, b) un groupe (C₂-C₄) alkyle, ou c) un groupe (C₁-C₈) alkyle substitué, de préférence (C₂-C₄) alkyle éventuellement substitué par un ou plusieurs groupes choisis parmi :
- cyano,
- (C₁-C₃) alcoxy,
- hydroxyle, et
- (C₁-C₃) alkylcarbonyle ;
en particulier, R₁₀ et Ru, qui peuvent être identiques ou différents, représentent un atome d'hydrogène, ou un groupe (C₁-C₆) alkyle substitué par un ou plusieurs groupes hydroxyle, cyano ou (C₁-C₃)alkylcarbonyle tel qu'hydroxyéthyle ;
• m représente un entier compris entre 1 et 18 inclus ; en particulier un entier compris entre 2 et 16 inclus ; préférentiellement un entier compris entre 3 et 10 ; plus préférentiellement un entier compris entre 4 et 6 ;
• M' représente un contre-ion anionique tel que défini dans la revendication précédente ;
étant entendu que, lorsque la molécule comprend un groupe alcoolate, alors M' et Q⁺ peuvent être absents pour assurer l'électroneutralité de ladite molécule.

19. Procédé selon l'une quelconque des revendications 1 à 18 dans lequel le ou les colorants fluorescents (poly)méthines cationiques de l'invention (b) sont choisis parmi ceux de formules (XIV) et (XV) suivantes : 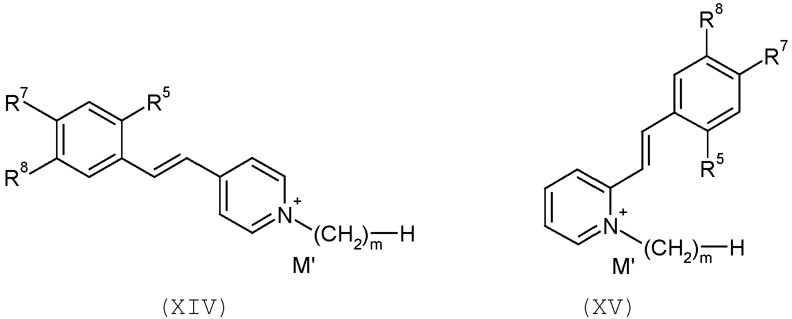 formules (XIV) et (XV) dans lesquelles R₅, R₇, R₈ et m sont tels que définis précédemment pour (X) et (XI), en particulier :
• R₅ et R₈, qui peuvent être identiques ou différents, représentent un atome d'hydrogène ou un groupe (C₁-C₄)alcoxy tel que méthoxy, de préférence, R₅ et R⁸ représentent un atome d'hydrogène ;
• R₇ représente un groupe (C₁-C₄) alcoxy ou NR¹⁰R¹¹, avec R¹⁰ et R¹¹, qui peuvent être identiques ou différents, représentant a) un atome d'hydrogène, ou b) un groupe (C₁-C₈) alkyle éventuellement substitué par un ou plusieurs groupes choisis parmi i) hydroxyle, ii) R-Z-C(X)-Y- avec X, Y et Z représentant un atome d'oxygène ou de soufre ou N(R'), ou, en variante, X et/ou Z représente (nt) une liaison, R et R', qui peuvent être identiques ou différents, représentent un atome d'hydrogène ou un groupe (C₁-C₆) alkyle, de préférence, X représente un atome d'oxygène, iii) sulfonique SO₃H, iv) sulfonate SO₃⁻, Q⁺, v) carboxylate C(O)O⁻, Q⁺ avec Q⁺ représentant un contre-ion cationique tel qu'un métal alcalin ou un métal alcalino-terreux ; en particulier, R₇ représente un groupe NR¹⁰R¹¹ avec R¹⁰ et R¹¹, qui peuvent être identiques ou différents, représentant a) un atome d'hydrogène, ou b) un groupe (C₁-C₆) alkyle éventuellement substitué par un ou plusieurs groupes choisis parmi i) hydroxyle, ii) carboxyle, iii) carboxylate, iv) sulfonique, et v) sulfonate, plus particulièrement choisis parmi des groupes identiques ou différents représentant a) un atome d'hydrogène, ou b) un groupe (C₁-C₆) alkyle éventuellement substitué par un ou plusieurs groupes choisis parmi i) hydroxyle, ii) carboxyle, et iii) carboxylate ;
• m représente un entier compris entre 1 et 18 inclus ; en particulier un entier compris entre 1 et 6 inclus ; préférentiellement un entier compris entre 1 et 4 ; plus préférentiellement un entier compris entre 1 et 2 ; et
• M' représente un contre-ion anionique, dérivé d'un sel d'acide organique ou minéral, ou d'une base organique ou minérale qui assure l'électroneutralité de la molécule ;
• étant entendu que, lorsque la molécule comprend un groupe alcoolate, alors M' et Q⁺ peuvent être absents pour assurer l'électroneutralité de ladite molécule.

20. Procédé selon l'une quelconque des revendications précédentes dans lequel :
• le ou les colorants fluorescents (poly)méthines cationiques (b) sont de formule (X) avec :
| R₁ | R₂ | R₃ | R₄ | R₈ | R₆ | R₇ | R₈ | R₉ | m |
|---|---|---|---|---|---|---|---|---|---|
| H | H | H | H | H | H | H | H | H | 1 |
| H | H | H | H | H | t-Bu | OH | t-Bu | H | 5 |
| H | H | H | H | H | t-Bu | OH | t-Bu | H | 5 |
| H | H | H | H | H | H | NH₂ | H | H | 1 |
| H | H | H | H | H | H | NH₂ | H | OCH₃ | 1 |
| H | H | H | H | H | H | OH | Br | H | 5 |
| H | H | H | H | H | OCH₃ | OH | OCH₃ | H | 5 |
| H | H | H | H | Cl | H | OH | H | Cl | 5 |
| H | H | H | H | H | H | OH | H | H | 10 |
| H | H | H | H | H | OCH₃ | OH | OCH₃ | H | 10 |
| H | H | H | H | H | t-Bu | OH | t-Bu | H | 10 |
| H | H | H | H | H | H | N(CH₂CH₃)₂ | H | H | 1 |
| H | H | H | H | H | H | N(CH₂CH₃)₂ | H | H | 1 |
| H | H | H | H | H | H | N(CH₂CH₃)₂ | H | H | 1 |
| H | H | H | H | H | t-Bu | OH | t-Bu | H | 10 |
| H | H | H | H | H | H | N(CH₂CH₃)CH₂CH₂OH | H | H | 1 |
| H | H | H | H | H | H | N(CH₂CH₃)₂ | H | H | 1 |
| H | H | H | H | H | H | N(CH₂CH₃)₂ | H | H | 1 |
| H | H | H | H | H | H | N(CH₂CH₃)₂ | H | H | 1 |
| H | H | H | H | H | H | N(CH₂CH₃)₂ | H | H | 1 |
| H | H | H | H | H | H | N(CH₂CH₃)₂ | H | H | 1 |
| H | H | H | H | H | H | N(CH₂CH₃)₂ | H | H | 1 |
| H | H | H | H | H | H | N(CH₂CH₃)₂ | H | H | 1 |
| H | H | H | H | H | H | N(CH₂CH₃)₂ | H | H | 1 |
| H | H | H | H | H | H | N(CH₂CH₃)₂ | H | H | 1 |
| H | H | H | H | H | H | n-C₆H₁₃ | H | H | 1 |
| H | H | H | H | H | H | N(CH₂CH₂OH)₂ | H | H | 2 |
| H | H | H | H | H | H | N(n-Bu)₂ | H | H | 2 |
| H | H | H | H | H | OCH₃ | OH | H | H | 10 |
| H | H | H | H | H | H | OC₂H₅OH | H | H | 1 |
| H | H | H | H | H | H | OH | H | H | 1 |
| H | H | benzo | | H | H | H | H | H | 1 |
| H | H | benzo | | H | H | N(CH₂CH₂OH)₂ | H | H | 1 |
| H | H | benzo | | H | H | N(CH₂CH₂OH)₂ | H | H | 1 |
ainsi que leurs sels d'acide ou de base, organique ou minéral, leurs isomères optiques, isomères géométriques et tautomères et leurs solvates tels que des hydrates ;
• le ou les colorants fluorescents (poly)méthines cationiques (b) de formule (XI) avec :
| R₁ | R₂ | R₃ | R₄ | R₅ | R₆ | R₇ | R₈ | R₉ | m |
|---|---|---|---|---|---|---|---|---|---|
| CH₃ | H | H | H | OH | H | OCH₃ | H | H | 2 |
| CH₃ | H | H | H | H | H | OCH₃ | OCH₂-Ph | H | 2 |
| CH₃ | H | H | H | H | H | H | H | OCH₃ | 2 |
| CH₃ | H | H | H | F | H | H | H | H | 2 |
| CH₃ | H | H | H | H | H | H | OPh | H | 2 |
| CH₃ | H | H | H | H | H | N(CH₂CH₂OAc)₂ | H | H | 2 |
| CH₃ | H | H | H | OCH₃ | H | OCH₃ | OCH₃ | H | 2 |
| CH₃ | H | H | H | H | H | H | CH₃ | H | 2 |
| CH₃ | H | H | H | H | H | OH | H | H | 2 |
| CH₃ | H | H | H | H | OCH₃ | OCH₃ | OCH₃ | H | 2 |
| CH₃ | H | H | H | H | OCH₃ | OH | OH | H | 2 |
| CH₃ | H | H | H | H | H | OCH₃ | OCH₃ | OCH₃ | 2 |
| CH₃ | H | H | H | H | H | H | OCH₃ | OH | 2 |
| CH₃ | H | H | H | H | H | N(n-butyle)₂ | H | H | 2 |
| CH₃ | H | H | H | H | OCH₃ | OCH₃ | H | H | 2 |
| CH₃ | H | H | H | H | H | H | H | H | 2 |
| CH₃ | H | H | H | H | H | i-propyle | H | H | 2 |
| H | H | H | H | H | H | OH | H | H | 6 |
| H | H | H | H | H | OCH₃ | OCH₃ | OCH₃ | H | 6 |
| H | H | H | H | OH | H | OCH₃ | H | H | 2 |
| H | H | H | H | OCH₃ | H | H | OCH₃ | OCH₃ | 2 |
| H | H | H | H | H | H | H | H | Br | 2 |
| H | H | H | H | H | H | OH | H | H | 2 |
| H | H | H | H | OCH₃ | OCH₃ | OCH₃ | H | H | 6 |
| H | H | H | H | OH | OCH₃ | H | H | H | 6 |
| H | H | H | H | H | OCH₃ | OCH₃ | H | H | 6 |
| H | H | H | H | H | OCH₃ | OCH₃ | H | H | 2 |
| H | H | H | H | H | H | C(O)-OH | H | H | 6 |
| H | H | H | H | H | H | C(O)-OH | H | H | 2 |
| H | H | H | H | H | H | i-propyle | H | H | 2 |
| H | H | H | H | H | H | N(CH₃)CH₂CH₂CN | H | H | 2 |
| H | H | H | H | H | H | OCH₃ | OCH₂Ph | H | 2 |
| H | H | H | H | H | H | H | OPh | H | 2 |
| H | H | H | H | H | H | N(CH₂CH₂C(O)CH₃)₂ | H | H | 2 |
| H | H | H | H | OH | H | OCH₃ | H | H | 6 |
| H | H | H | H | H | OCH₃ | OCH₃ | OCH₃ | H | 2 |
| H | H | H | H | OCH₃ | H | OCH₃ | OCH₃ | H | 6 |
| H | H | H | H | H | H | H | CH₃ | H | 2 |
| H | H | H | H | H | H | N(CH₃)CH₂CH₂OH | H | H | 2 |
ainsi que leurs sels d'acide ou de base, organique ou minéral, leurs isomères géométriques et tautomères et leurs solvates tels que des hydrates ;
• le ou les colorants fluorescents (poly)méthines cationiques (b) de formules (X') et (XI') : 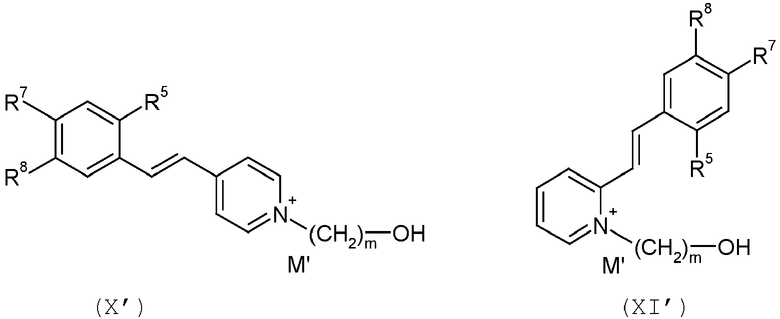 avec M' tel que défini dans la revendication précédente et :
| **R⁵** | **R**⁷ | **R⁸** | **m** | | **R⁵** | **R**⁷ | **R⁸** | **m** |
|---|---|---|---|---|---|---|---|---|
| H | N(CH₂CH₂OH)₂ | H | 2 | | OCH₃ | OCH₃ | OCH₃ | 2 |
| H | N(CH₂CH₂OH)₂ | H | 3 | | OCH₃ | OCH₃ | OCH₃ | 3 |
| H | N(CH₂CH₂OH)₂ | H | 4 | | OCH₃ | OCH₃ | OCH₃ | 3 |
| H | N(CH₂CH₂OH)₂ | H | 5 | | OCH₃ | OCH₃ | OCH₃ | 4 |
| H | N(CH₂CH₂OH)₂ | H | 6 | | OCH₃ | OCH₃ | OCH₃ | 5 |
| H | N(CH₂CH₂OH)₂ | H | 8 | | OCH₃ | OCH₃ | OCH₃ | 8 |
| H | N(CH₂CH₂OH)₂ | H | 10 | | OCH₃ | OCH₃ | OCH₃ | 10 |
| H | N(CH₂CH₂OH)₂ | H | 12 | | OCH₃ | OCH₃ | OCH₃ | 12 |
| H | N(CH₂CH₂OH)₂ | H | 14 | | OCH₃ | OCH₃ | OCH₃ | 14 |
| H | N(CH₂CH₂OH) ₂ | H | 16 | | OCH₃ | OCH₃ | OCH₃ | 16 |
| H | CH₃CH₂N(CH₂CH₂OH) | H | 2 | | | | | |
| H | CH₃CH₂N(CH₂CH₂OH) | H | 4 | et | | | | |
ainsi que leurs sels d'acide ou de base, organique ou minéral, leurs isomères géométriques et tautomères et leurs solvates tels que des hydrates ;
• le ou les colorants fluorescents (poly)méthines cationiques de formule (XII) avec :
| R₁ | R₂ | R₃ | R₄ | R₅ | R₆ | R₇ | R₈ | R₉ | m |
|---|---|---|---|---|---|---|---|---|---|
| H | H | H | H | H | H | OCH₃ | OCH₃ | OCH₃ | 2 |
| H | H | H | H | OH | OCH₃ | H | H | H | 2 |
| H | H | H | H | H | H | H | H | H | 2 |
| H | H | H | H | H | OCH₃ | OCH₃ | H | H | 2 |
| H | H | H | H | OH | H | OH | H | H | 6 |
| H | H | H | H | OCH₃ | H | OCH₃ | OCH₃ | H | 6 |
| H | H | H | H | H | H | OH | H | H | 6 |
| H | H | H | H | OCH₃ | H | H | H | F | 2 |
| H | H | H | H | H | H | C(O)-OH | H | H | 2 |
| H | H | H | H | H | H | Isopropyle | H | H | 2 |
| H | H | H | H | H | H | N(CH₂CH₂C(O)CH₃)₂ | H | H | 2 |
| H | H | H | H | OH | H | OCH₃ | H | H | 2 |
| H | H | H | H | H | H | OH | H | H | 2 |
| H | H | H | H | H | OCH₃ | OH | OH | H | 2 |
| H | H | H | H | H | CH₃ | OCH₂Ph | CH₃ | H | 2 |
| H | H | H | H | H | OCH₃ | OCH₃ | OCH₃ | H | 2 |
| H | H | H | H | H | OCH₃ | OCH₃ | OCH₃ | H | 6 |
| H | H | H | H | H | H | N(CH₃)₂ | H | H | 6 |
| H | H | H | H | H | H | OCH₃ | OCH₃ | OCH₃ | 6 |
| H | H | H | H | H | H | Phényle | H | H | 6 |
| H | H | H | H | H | OCH₃ | OCH₃ | H | H | 6 |
| H | H | H | H | H | H | C(O)-OH | H | H | 6 |
| H | H | H | H | H | H | N(n-Butyle)₂ | H | H | 2 |
| H | H | H | H | H | H | OCH₃ | OCH₃ | H | 3 |
| H | H | H | H | H | H | OCH₃ | OCH₃ | H | 2 |
| H | H | H | H | H | H | OCH₃ | OCH₃ | H | 5 |
| H | H | H | H | H | H | OCH₃ | H | H | 3 |
| H | H | H | H | H | H | N(CH₃)₂ | H | H | 3 |
| H | H | H | H | H | H | H | H | H | 3 |
ainsi que leurs sels d'acide ou de base, organique ou minéral, leurs isomères optiques, isomères géométriques et tautomères et leurs solvates tels que des hydrates ;
• le ou les colorants fluorescents (poly)méthines cationiques de formule (XIII) avec :
| R₁ | R₂ | R₃ | R₄ | R₅ | R₆ | R₇ | Rs | R₉ | m |
|---|---|---|---|---|---|---|---|---|---|
| CH₃ | H | H | H | OH | H | OCH₃ | H | H | 2 |
| CH₃ | H | H | H | H | H | OCH₃ | OCH₂-Ph | H | 2 |
| CH₃ | H | H | H | H | H | H | H | OCH₃ | 2 |
| CH₃ | H | H | H | F | H | H | H | H | 2 |
| CH₃ | H | H | H | H | H | H | OPh | H | 2 |
| CH₃ | H | H | H | H | H | N(CH₂CH₂OAC)₂ | H | H | 2 |
| CH₃ | H | H | H | OCH₃ | H | OCH₃ | OCH₃ | H | 2 |
| CH₃ | H | H | H | H | H | H | CH₃ | H | 2 |
| CH₃ | H | H | H | H | H | OH | H | H | 2 |
| CH₃ | H | H | H | H | OCH₃ | OCH₃ | OCH₃ | H | 2 |
| CH₃ | H | H | H | H | OCH₃ | OH | OH | H | 2 |
| CH₃ | H | H | H | H | H | OCH₃ | OCH₃ | OCH₃ | 2 |
| CH₃ | H | H | H | H | H | H | OCH₃ | OH | 2 |
| CH₃ | H | H | H | H | H | N(n-butyle)₂ | H | H | 2 |
| CH₃ | H | H | H | H | OCH₃ | OCH₃ | H | H | 2 |
| CH₃ | H | H | H | H | H | H | H | H | 2 |
| CH₃ | H | H | H | H | H | i-propyle | H | H | 2 |
| H | H | H | H | H | H | OH | H | H | 6 |
| H | H | H | H | H | OCH₃ | OCH₃ | OCH₃ | H | 6 |
| H | H | H | H | OH | H | OCH₃ | H | H | 2 |
| H | H | H | H | OCH₃ | H | H | OCH₃ | OCH₃ | 2 |
| H | H | H | H | H | H | H | H | Br | 2 |
| H | H | H | H | H | H | OH | H | H | 2 |
| H | H | H | H | H | H | N(CH₃)₂ | H | H | 6 |
| H | H | H | H | OCH₃ | OCH₃ | OCH₃ | H | H | 6 |
| H | H | H | H | OH | OCH₃ | H | H | H | 6 |
| H | H | H | H | H | OCH₃ | OCH₃ | H | H | 6 |
| H | H | H | H | H | OCH₃ | OCH₃ | H | H | 2 |
| H | H | H | H | H | H | C(O)-OH | H | H | 6 |
| H | H | H | H | H | H | C(O)-OH | H | H | 2 |
| H | H | H | H | H | H | i-propyle | H | H | 2 |
| H | H | H | H | H | H | N(CH₃)CH₂CH₂CN | H | H | 2 |
| H | H | H | H | H | H | OCH₃ | OCH₂Ph | H | 2 |
| H | H | H | H | H | H | H | OPh | H | 2 |
| H | H | H | H | H | H | N(CH₂CH₂C(O)CH₃)₂ | H | H | 2 |
| H | H | H | H | OH | H | OCH₃ | H | H | 6 |
| H | H | H | H | H | OCH₃ | OCH₃ | OCH₃ | H | 2 |
| H | H | H | H | OCH₃ | H | OCH₃ | OCH₃ | H | 6 |
| H | H | H | H | H | H | H | CH₃ | H | 2 |
| H | H | H | H | H | H | N(CH₃)CH₂CH₂OH | H | H | 2 |
| CH₃ | H | H | H | H | H | N(CH₃)₂ | H | H | 2 |
ainsi que leurs sels d'acide ou de base, organique ou minéral, leurs isomères géométriques et tautomères et leurs solvates tels que des hydrates ;
• le ou les colorants fluorescents (poly)méthines cationiques (b) de formules (XII') et (XIII') suivantes : 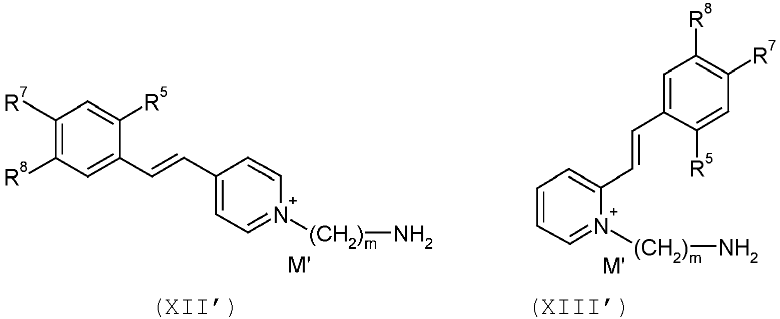 avec :
| **R⁵** | **R**⁷ | **R⁸** | **m:** | | **R⁵** | **R**⁷ | **R⁸** | **m** |
|---|---|---|---|---|---|---|---|---|
| H | N(CH₂CH₂OH)₂ | H | 2 | | OCH₃ | OCH₃ | OCH₃ | 2 |
| H | N(CH₂CH₂OH)₂ | H | 3 | | OCH₃ | OCH₃ | OCH₃ | 3 |
| H | N(CH₂CH₂OH)₂ | H | 4 | | OCH₃ | OCH₃ | OCH₃ | 3 |
| H | N(CH₂CH₂OH)₂ | H | 5 | | OCH₃ | OCH₃ | OCH₃ | 4 |
| H | N(CH₂CH₂OH)₂ | H | 6 | | OCH₃ | OCH₃ | OCH₃ | 5 |
| H | N(CH₂CH₂OH)₂ | H | 8 | | OCH₃ | OCH₃ | OCH₃ | 8 |
| H | N(CH₂CH₂OH)₂ | H | 10 | | OCH₃ | OCH₃ | OCH₃ | 10 |
| H | N(CH₂CH₂OH)₂ | H | 12 | | OCH₃ | OCH₃ | OCH₃ | 12 |
| H | N(CH₂CH₂OH)₂ | H | 14 | | OCH₃ | OCH₃ | OCH₃ | 14 |
| H | N(CH₂CH₂OH)₂ | H | 16 | et | OCH₃ | OCH₃ | OCH₃ | 16 |
ainsi que leurs sels d'acide ou de base, organique ou minéral, leurs isomères géométriques et tautomères et leurs solvates tels que des hydrates ;
• le ou les colorants fluorescents (poly)méthines cationiques (b) choisis parmi les composés suivants :
| | |
|---|---|
| 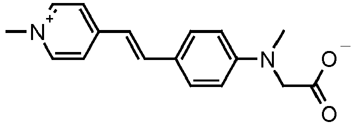 | 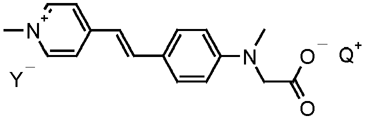 |
| (d) | (d') |
| 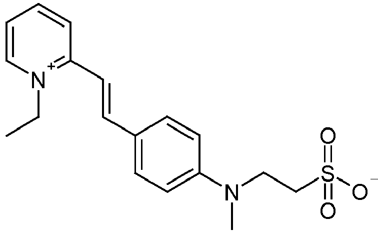 | 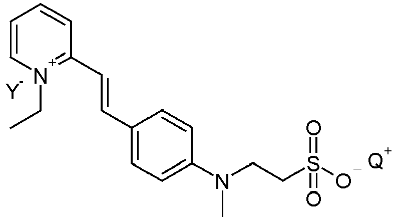 |
| (e) | (e') |
| 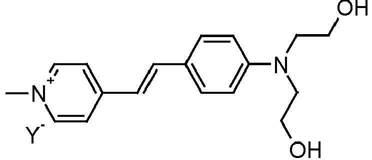 | |
| (f) | |
| 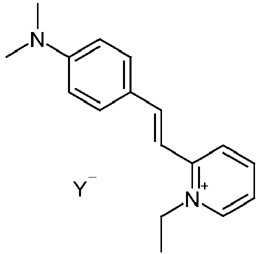 | |
| (g) | |
| 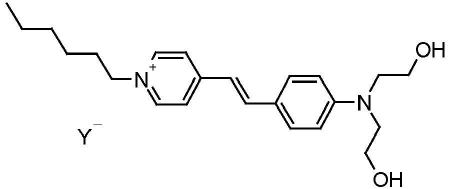 | |
| (h) | |
ainsi que leurs sels d'acide ou de base, organique ou minéral, leurs isomères géométriques et tautomères et leurs solvates tels que des hydrates ;
avec Y et Q⁺ tels que définis dans la revendication précédente.

21. Procédé selon l'une quelconque des revendications précédentes, **caractérisé en ce qu'**il comprend en outre l'application sur lesdites fibres kératiniques d'une composition cosmétique comprenant c) un ou plusieurs agents réducteurs, et de préférence choisis parmi i) les agents réducteurs de formule (XVI) ci-dessous, ainsi que leurs sels d'addition et leurs mélanges !
H(X)_{q}(R₁₀)ₜ (XVI)
formule (XVI) dans laquelle :
- X représente P, S ou SO₂,
- q représente un entier égal à 0 ou 1,
- t représente un entier égal à 1 ou 2, et
- R représente un radical alkyle en C₁ à C₂₀, linéaire ou ramifié, saturé ou insaturé, éventuellement interrompu par un hétéroatome et/ou éventuellement substitué par un ou plusieurs radicaux choisis parmi les radicaux hydroxyle, halogéno, amine, carboxyle, ((C₁-C₃₀)alcoxy) carbonyle, amido, ((C₁-C₃₀) alkyl) aminocarbonyle, ((C₁-C₃₀) acyl) amino, mono- ou dialkylamino et mono- ou dihydroxylamino ; ii) l'acide thioglycolique, iii) l'acide thiolactique, iv) le monothioglycolate de glycéryle, v) la cystéamine, vi) la N-acétylcystéamine, vii) la N-propionylcystéamine, viii) la cystéine, ix) la N-acétylcystéine, x) l'acide thiomalique, xi) la panthétéine, xii) l'acide 2,3-dimercaptosuccinique, xiii) les N-(mercaptoalkyl)-ω-hydroxyalkylamides, xiv) les N-mono ou N,N-dialkylmercapto-4-butyramides, xv) les aminomercaptoalkylamides, xvi) les dérivés d'acide N-(mercaptoalkyl)-succinamiques, xvii) les dérivés des acides N-(mercaptoalkyl)succinimides, xviii) les alkylamino-mercaptoalkylamides, xix) le mélange azéotrope de thioglyconate de 2-hydroxypropyle et de thioglycolate de (2-hydroxy-1-méthyl)éthyle, xx) les mercaptoalkylaminoamides, xxi) les N-mercaptoalkylalcanediamides, xxii) les dérivés d'acide formamidinesulfinique, leurs sels d'addition et leurs mélanges.

22. Procédé selon l'une quelconque des revendications 1 à 20, qui ne met en œuvre aucun agent réducteur.

23. Procédé selon l'une quelconque des revendications précédentes, dans lequel le ou les composés anthraquinones (a), tels que définis dans l'une quelconque des revendications 1 à 11 et le ou les colorants directs fluorescents (poly)méthines cationiques (b) tels que définis dans l'une quelconque des revendications 1 et 12 à 20, sont appliqués conjointement sur les fibres kératiniques ; de préférence, le procédé comprend un étape d'application sur les fibres kératiniques d'une composition cosmétique qui comprend a) un ou plusieurs colorants anthraquinones de formules (I) et/ou (II), tels que définis dans l'une quelconque des revendications 1 à 11 et (b) un ou plusieurs colorants fluorescents (poly)méthines cationiques, tels que définis dans l'une quelconque des revendications 1 et 12 à 20.

24. Procédé selon l'une quelconque des revendications 1 à 22, qui comprend au moins deux étapes successives :
- une étape d'application sur les fibres kératiniques d'une composition cosmétique comprenant (b) un ou plusieurs colorants fluorescents (poly)méthines cationiques, tels que définis dans l'une quelconque des revendications 1 et 12 à 20, suivie de
- une étape d'application sur lesdites fibres kératiniques d'une composition cosmétique comprenant (a) un ou plusieurs colorants anthraquinones de formules (I) et/ou (II), tels que définis dans l'une quelconque des revendications 1 à 11.

25. Procédé selon l'une quelconque des revendications 1 à 22, qui comprend au moins deux étapes successives :
- une étape d'application sur lesdites fibres d'une composition cosmétique comprenant (a) un ou plusieurs colorants anthraquinones de formules (I) et/ou (II), tels que définis dans l'une quelconque des revendications 1 à 11, suivie de
- une étape d'application sur les fibres kératiniques d'une composition cosmétique comprenant (b) un ou plusieurs colorants fluorescents (poly)méthines cationiques, tels que définis dans l'une quelconque des revendications 1 et 12 à 20.

26. Procédé selon l'une quelconque des revendications précédentes, dans lequel le pH de la ou des compositions cosmétiques est compris entre 6 et 11 inclus, en particulier entre 7 et 10, plus particulièrement entre 7,5 et 9,5, par exemple entre 9 et 9,5.

27. Composition cosmétique comprenant (a) un ou plusieurs colorants anthraquinones de formules (I) et/ou (II), tels que définis dans l'une quelconque des revendications 1 à 11, et (b) un ou plusieurs colorants fluorescents (poly)méthines cationiques, tels que définis dans l'une quelconque des revendications 1 et 13 à 20, éventuellement à un pH tel que défini dans la revendication précédente.
